(19) Europäisches Patentamt
European Patent Office
Office européen des brevets

(11) Publication number: **0 030 142**
**B1**

(12) **EUROPEAN PATENT SPECIFICATION**

(45) Date of publication of patent specification: **05.12.84**

(21) Application number: **80304287.8**

(22) Date of filing: **28.11.80**

(51) Int. Cl.³: **A 01 N 47/36,** C 07 D 409/12, C 07 D 407/12, C 07 D 307/08, C 07 D 491/04 // C07D333/38 ,(C07D491/04, 307/00, 239/00)

(54) **Herbicidal ureas, preparation, compositions and use thereof.**

(30) Priority: **30.11.79 US 98723**
**22.10.80 US 196267**

(43) Date of publication of application:
**10.06.81 Bulletin 81/23**

(45) Publication of the grant of the patent:
**05.12.84 Bulletin 84/49**

(84) Designated Contracting States:
**AT BE CH DE FR IT LI LU NL SE**

(56) References cited:
**EP-A-0 001 485**
**EP-A-0 001 514**
**EP-A-0 007 687**
**EP-A-0 015 683**
**US-A-4 127 405**
**US-A-4 169 719**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Levitt, George**
**3218 Romilly Road**
**Wilmington Delaware 19810 (US)**

(74) Representative: **Hildyard, Edward Martin et al**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 030 142 B1**

Courier Press, Leamington Spa, England.

**Description**

Background of the Invention

This invention relates to ureas and isoureas and in particular their use as agricultural chemicals and particularly as herbicides.

U.S. Patent 4,127,405 teaches compounds which are useful for controlling weeds in wheat having the formula

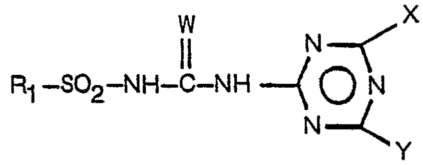

wherein

$R_1$ is 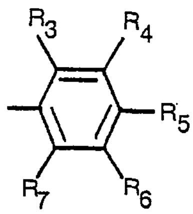 , 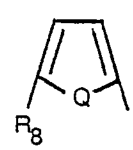 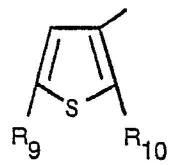 or 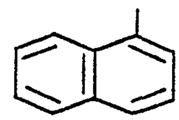 ;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur; n is 0, 1 or 2;

X is hydrogen, chlorine, bromine, methyl, ethyl, alkoxy of 1—3 carbon atoms, trifluoromethyl, $CH_2S$— or $CH_3OCH_2$—; and

Y is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must he hydrogen;

(b) when $R_4$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

French Patent No. 1,468,747 discloses the following *para*-substituted phenylsulfonamides, useful as antidiabetic agents:

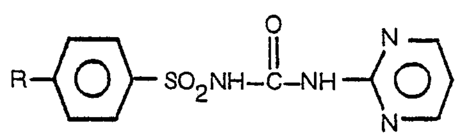

wherein

R = H, halogen, $CF_3$ or alkyl.

Logemann et al. Chem. Ab., *53,* 18052 g (1959), disclose a number of sulfonamides, including uracil derivatives and those having the formula:

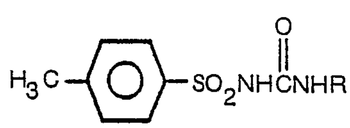

wherein

R is butyl, phenyl or 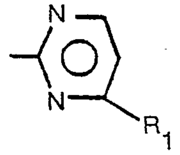

and $R_1$ is hydrogen or methyl. When tested for hypoglycemic effect in rats (oral doses of 25 mg/100 g),

the compounds in which R is butyl and phenyl were most potent. The others were of low potency or inactive.

Wojciechowski, J. Acta. Polon. Pharm. *19,* p. 121—5 (1962) [Chem Ab., *59* 1633 e] describes the synthesis of N-[(2,6-dimethoxypyrimidin-4-yl)aminocarbonyl]-4-methylbenzenesulfonamide:

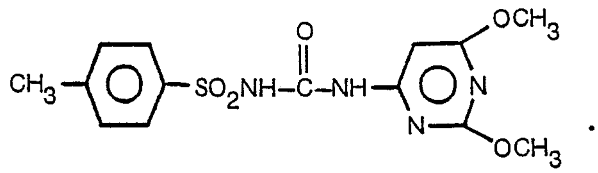

Based upon similarity to a known compound, the author predicted hypoglycemic activity for the foregoing compound.

Netherlands Patent 121,788, published September 15, 1966, teaches the preparation of compounds of Formula (i), and their use as general or selective herbicides,

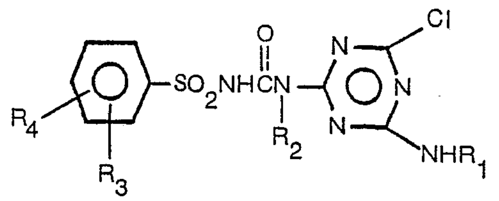

( i )

wherein

$R_1$ and $R_2$ may independently be alkyl of 1—4 carbon atoms; and

$R_3$ and $R_4$ may independently be hydrogen, chlorine or alkyl of 1—4 carbon atoms.

Compounds of Formula (ii), and their use as antidiabetic agents, are reported in *J. Drug. Res. 6,* 123 (1974),

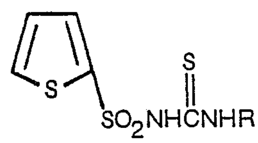

( ii )

wherein

R is pyridyl.

In our EP—A—1514, EP—A—1485, US—A—4,169,719 and US—A—4,127,405 we have disclosed herbicidal sulfonylureas containing *inter alia* thienyl or furyl moieties which are unsubstituted or, in some cases, substituted by methyl or halogen. These Specifications do not suggest the particular substituents envisaged by the present invention, which substituents are thought to contribute substantially to the herbicidal activity of our compounds.

The presence of undesired vegetation causes substantial damage to useful crops, especially agricultural products that satisfy man's basic food needs, such as soybeans, barley, wheat, and the like. The current population explosion and concomitant world food shortage demand improvements in the efficiency of producing these crops. Prevention or minimizing the loss of a portion of such valuable crops by killing, or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or retard weeds without causing significant damage to useful crops.

## Summary of the Invention

This invention relates to novel compounds of Formulas I, II and III and their agriculturally suitable salts, suitable agricultural compositions containing them, and methods of using them as selective, as well as general herbicides having both pre-emergence and post-emergence activity and for regulating plant growth. Some of the compounds are especially useful for controlling weeds in crops such as soybeans:

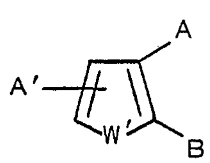

I

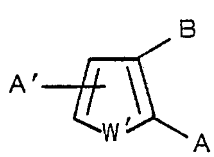

II

or

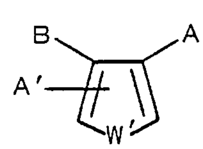

III

wherein

W' is O or S;

A' is H, Cl, Br, $C_1$—$C_4$ alkyl, $OCH_3$, $NO_2$ or $CF_3$;

A is 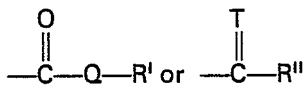

where Q is O, S or 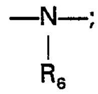

T is O or 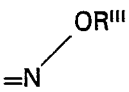

where

R''' is H, $C_1$—$C_4$ alkyl or $C_3$—$C_4$ alkenyl when Q is O or S then R' is $C_1$—$C_6$ alkyl; $C_3$—$C_6$ alkenyl; $C_3$—$C_6$ alkynyl; $C_2$—$C_6$ alkyl substituted with 1—3 Cl, F or Br, or one of CN or $OCH_3$; $C_3$—$C_6$ alkenyl substituted with 1—3 Cl; $C_3$—$C_6$ alkynyl substituted with Cl; $C_5$—$C_6$ cycloalkyl; cyclohexenyl; cyclohexyl substituted with 1—3 $CH_3$; $C_4$—$C_7$ cycloalkylalkyl or

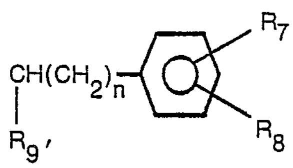

where $R_7$ and $R_8$ are independently H, Cl, $CH_3$ or $OCH_3$;

n is 0 or 1; and

$R_{9'}$ is H or $CH_3$; when Q is O then R' can be $CH_2CH_2OR_{15}$; $CH_2CH_2CH_2OR_{15}$; 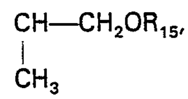

where $R_{15}$ is $C_2H_5$, $CH(CH_3)_2$, phenyl, $CH_2CH_2Cl$, $CH_2CCl_3$; $-(CH_2CH_2O)-_{n'}R_{16}$, 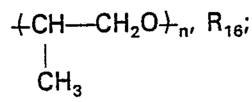

where $R_{16}$ is $CH_3$, $C_2H_5$, $CH(CH_3)_2$, phenyl, $CH_2CH_2Cl$, $CH_2CCl_3$, and n' is 2 or 3;

$CH_2CN$; 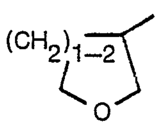 ; 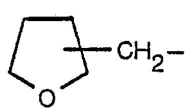 ; 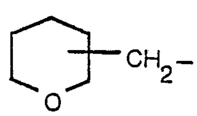 ; 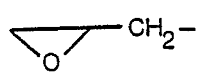 ; $CH_2OR'_6$ ;

where $R'_6$ is $C_1$—$C_4$ alkyl; provided R' has a total of ≤13 carbon atoms; when Q is 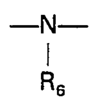 then

R' is H; $C_1$—$C_6$ alkyl; —$CH_2CH_2OR_{10}$; —$CH_2CH_2CH_2OR_{10}$; where $R_{10}$ is $CH_3$, $CH_3CH_2$, $CH(CH_3)_2$, or phenyl; $C_3$—$C_6$ alkenyl; $C_3$—$C_6$ cycloalkyl; $C_5$—$C_6$ cycloalkenyl; $C_6$ cycloalkyl substituted with any one of 1—2 $OCH_3$, 1—3 $CH_3$, —$CH_2CH_3$ or $CF_3$; $C_4$—$C_7$ cycloalkylalkyl; —$CH_2CN$; —$CH_2CH_2CN$;

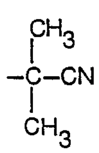 ; $OCH_3$ ; $OCH_2CH_3$ ; 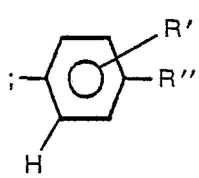 or 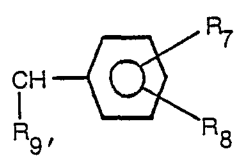 ;

where

R' is H, $C_1$—$C_4$ alkyl, $OCH_3$, F, Cl, Br, CN, $NO_2$ or $CF_3$;

R'' is H, $CH_3$, Cl, F or Br;

$R_7$, $R_8$ and $R_{9'}$ are as previously defined;

$R_6$ is N, $C_1$—$C_3$ alkyl; $CH_2CN$; $CH_2CH_2$—CN or —$CH_2$—$CH{=}CH_2$ and $R_6$ and $R^I$ may be taken together to form —$(CH_2)_4$—, —$(CH_2)_5$— or —$CH_2CH_2O$—$CH_2CH_2$—; with the proviso that when $R_6$ is $CH_2CH_2CN$ or $CH_2CN$, then $R^I$ is $CH_2CH_2CN$ or $CH_2CN$; and $R^I$ and $R_6$ have a total carbon atom count of $\leq 13$; and when $R^I$ is $OCH_3$ or $OCH_2CH_3$ then $R_6$ is $CH_3$ or H;

when A is 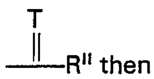 then

$R^{II}$ is H, $C_1$—$C_6$ alkyl; $C_3$—$C_6$ alkenyl; phenyl; benzyl; benzyl or phenyl substituted with 1—2 Cl, 1—2 $OCH_3$, 1—2 $CH_3$; $C_5$—$C_6$ cycloalkyl; $C_4$—$C_7$ cycloalkylalkyl with the proviso that when T is $=N$—$OR^{III}$, then $R^{II}$ must be $C_1$—$C_6$ alkyl or $C_3$—$C_6$ alkenyl; B is

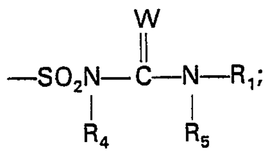

where $R_4$ is H or $CH_3$; W is O or S; $R_5$ is H, $CH_3$ or $CH_3O$; with the proviso that either $R_4$ or $R_5$ must be H;

$R_1$ is 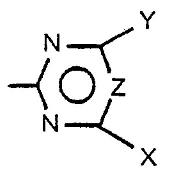 , 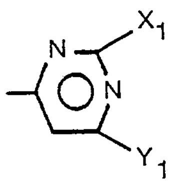 , 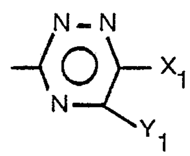 , 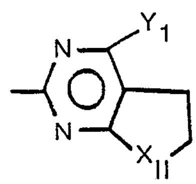 or 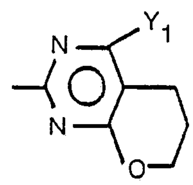 ;

where Z is N, CH or C—F;

X = H, Cl, —$CH_3$, —$OCH_3$ or —$OCH_2CH_3$;

Y = H; Cl; $C_1$—$C_4$ alkyl; $C_1$—$C_4$ alkyl substituted with —$OCH_3$, —$OC_2H_5$, —CN, —$CO_2CH_3$, —$CO_2C_2H_5$,

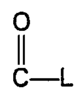

or 1—3 atoms of F, Cl, Br; $C_3$—$C_4$ alkenyl; —O—$(CH_2)_{n'}$O—($C_1$—$C_3$ alkyl) where n′ is 2 or 3;

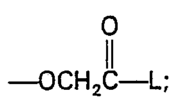 ; 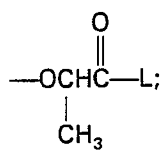 ; 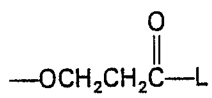

where L is OH, —$NH_2$, 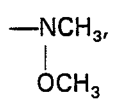

—NH($C_1$—$C_4$ alkyl), —N($C_1$—$C_4$ alkyl)$_2$ or $C_1$—$C_6$ alkoxy; SCN; —$N_3$; $NR_{11}R_{12}$ where $R_{11}$ is H or $CH_3$ and $R_{12}$ is H, —$OCH_3$, $C_1$—$C_4$ alkyl, $C_3$—$C_6$ cycloalkyl, $C_3$—$C_4$ alkenyl, $C_2$—$C_3$ alkyl substituted with $OCH_3$ or $OC_2H_5$, $C_1$—$C_2$ alkyl substituted with —CN, $CO_2H$, $CO_2CH_3$ or $CO_2C_2H_5$, and $R_{11}$ and $R_{12}$ can be taken together to form —$CH_2CH_2CH_2CH_2$— or $CH_2CH_2OCH_2CH_2$—; —O—$R_9$ where $R_9$ is $C_1$—$C_4$ alkyl, $C_2$—$C_4$ alkyl substituted with 1—3 atoms of F, Cl or Br, $C_1$—$C_2$ alkyl substituted with cyano, $C_3$—$C_4$ alkenyl, —$CH_2C{\equiv}CR_{13}$,

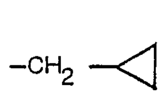 , 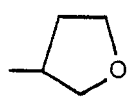 or 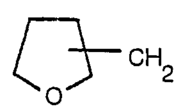 :

$R_{13}$ is H, $CH_3$ or $CH_2Cl$; $SR_{14}$; where $R_{14}$ is $C_1$—$C_4$ alkyl, alkyl, propargyl or $C_1$—$C_2$ alkyl substituted with CN; with the proviso that when X and Y are both H, then $R^I$ and $R^{II}$ are less than 5 carbons;

$X_1$ = H, Cl, $OCH_3$, $OCH_2CH_3$ or $CH_3$;

$Y_1$ = H, $OCH_3$ or $CH_3$; and

$X_{II}$ = O or $CH_2$ and further provided that when A contains greater than 5 carbon atoms, then Y must contain ≤4 carbon atoms, and their agriculturally suitable salts.

Preferred Compounds

Preferred for reasons of higher activity and/or lower cost and/or greater ease of synthesis are compounds:

1) of the *Generic* scope in which

B is 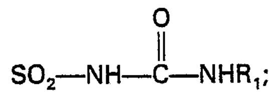;

More Preferred and in increasing order for reasons of higher activity and/or even lower cost and/or even greater ease of synthesis are compounds:

2) Preferred 1) in which W' is sulfur;

2a) More Preferred 2) in which T is oxygen;

3) More Preferred 2 or 2a) in which

$R_1$ is 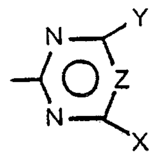, 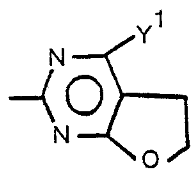 or 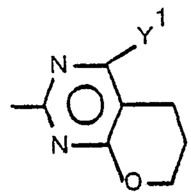

4) More Preferred 3) where Q is O or S and $R^I$ is $C_1$—$C_4$ alkyl; $C_3$—$C_4$ alkenyl; $C_3$—$C_4$ alkynyl; $C_2$—$C_3$ alkyl substituted with CN, $OCH_3$ or 1—3 F, Cl or Br; $C_3$—$C_4$ alkenyl substituted with 1—3 Cl; $C_3$—$C_4$ alkynyl substituted with Cl;

5) More Preferred 3) in which Q is oxygen and $R^I$ is $CH_2CH_2OR_{15}$; $CH_2CH_2CH_2OR_{15}$;

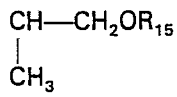

where $R_{15}$ is $CH_2CH_3$;

$CH_2CN$; $CH_2OR_6'$ wherein $R_6'$ is $CH_3$ or $CH_3CH_2$; 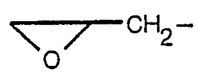 ;

6) More Preferred 3) in which Q is 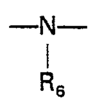

$R^I$ is H, $C_1$—$C_4$ alkyl, $CH_2CH_2OR_{10}$, $CH_2CH_2CH_2OR_{10}$ where $R_{10}$ is $CH_3$ or $CH_3CH_2$; $C_3$—$C_4$ alkenyl; $CH_2CN$; $CH_2CH_2CN$; $OCH_3$ or $OCH_2CH_3$; $R^6$ is H, $C_1$—$C_2$ alkyl, $CH_2CN$ or $CH_2CH_2CN$ and $R_6$ and $R^I$ can be taken together to form $-(CH_2)_4-$

7. More Preferred 3) in which $R^{II}$ is H or $C_1$—$C_3$ alkyl;

8) More Preferred 4), 5), 6) and 7) in which Z is CH or N;

X is $CH_3$ or $CH_3O$; and

Y is $C_1$—$C_2$ alkyl; $C_1$—$C_2$ alkyl substituted with $OCH_3$; $OCH_2CH_3$, CN or 1—3 atoms of F, Cl or Br;

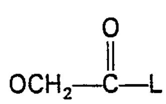 or 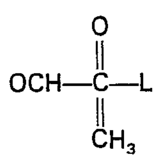

where L is $NH_2$, OH, 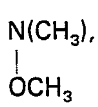

$N(CH_3)_2$, $NHCH_3$, $C_1$—$C_2$ alkoxy; SCN; $N_3$; $NR_{11}R_{12}$ where $R_{11}$ is H or $CH_3$; $R_{12}$ is H, $CH_3$, $CH_3CH_2$, $OCH_3$; $OR_9$ where $R_9$ is $CH_3$, $CH_3CH_2$; $CH_2CH{=}CH_2$ or $CH_2C{\equiv}CH$; $R_9$ is also $C_2$ alkyl substituted with 1—3 F, Cl or Br; $CH_3S$;

9) More Preferred 3) in which A' is H, Cl or Br;

10) More Preferred 9) in which Q is O or S and $R^I$ is $C_1$—$C_4$ alkyl, $CH_2CH{=}CH_2$ or $CH_2CH_2Cl$;

11) More Preferred 9) in which Q is O and $R^I$ is $CH_2CH_2OCH_3$, CH—$OCH_3$,
|
$CH_3$

$CH_2OCH_3$ or $CH_2OCH_2CH_3$;

12) More Preferred 9) in which Q is —N—
|
$R_6'$

and $R^I$ is H, $C_1$—$C_3$ alkyl, $OCH_3$ or $OCH_2CH_3$ and $R_6$ is H or $C_1$—$C_2$ alkyl;

13) More Preferred 9) in which $R^{II}$ is H or $CH_3$;

14) More Preferred 10), 11), 12) and 13) in which A' is H; Y is $CH_3$, $OCH_3$, $OCH_2CH_3$, $OCH_2CF_3$, $OCH_2CH{=}CH_2$ or $OCH_2C{\equiv}CH$;

15) More Preferred 8) in which A is 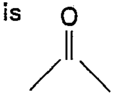

Q—$R^I$ and Q is oxygen or sulfur and $R^I$ is $CH_3$ or $CH_2CH_3$; Q is —N—
|
$R_6$

and $R^I$ is H, $CH_3$ or $OCH_3$ and $R_6$ is $CH_3$;

$R_1$ is 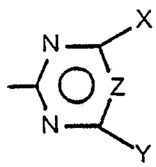

and Y is $CH_3$ or $OCH_3$;

16) More Preferred 15) of Formula I;

17) More Preferred 15) of Formula II;

18) More Preferred 15) of Formula III.

Equally More Preferred in increasing order and for reasons of higher activity and/or even lower cost and/or even greater ease of synthesis are:

19) Compounds of Preferred 1 in which W' is oxygen-

20) Compounds of More Preferred 19) in which T is oxygen;

21) More Preferred 19 or 20) in which

$R_1$ is 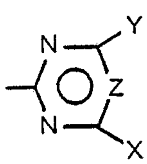 , 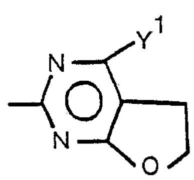 or 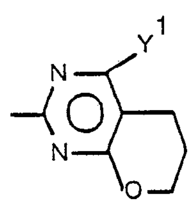

22) More Preferred 21) where Q and O or S and $R_1$ is $C_1$—$C_4$ alkyl; $C_3$—$C_4$ alkenyl; $C_3$—$C_4$ alkynyl; $C_2$—$C_3$ alkyl substituted with CN, $OCH_3$ or 1—3 F, Cl or Br; $C_3$—$C_4$ alkenyl substituted with 1—3 Cl; $C_3$—$C_4$ alkynyl substituted with Cl;

23) More Preferred 21) in which Q is oxygen and $R^I$ is $CH_2CH_2OR_{15}$; $CH_2CH_2CH_2OR_{15}$;

$CHOR_{15}$
|
$CH_3$

where $R_{15}$ is $CH_2CH_3$; $CH_2CN$; $CH_2OR_6'$ where $R_6'$ is $CH_3$ or $CH_3CH_2$; 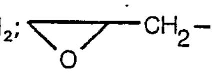 ;

24) More Preferred 21) in which Q is —N—
|
$R_6$

and $R^1$ is H, $C_1$—$C_4$ alkyl, $CH_2CH_2OR_{10}$, $CH_2CH_2CH_2OR_{10}$ where $R_{10}$ is $CH_3$ or $CH_3CH_2$; $C_3C_4$ alkenyl; $CH_2CN$;

$CH_2CH_2CN$; $OCH_3$ or $OCH_2CH_3$; $R^6$ is H, $C_1$—$C_2$ alkyl, $CH_2CN$ or $CH_2CH_2CN$ and $R_6$ and $R^I$ can be taken together to form $-(CH_2)_4-$.

25) More Preferred 21) in which $R^{II}$ is H or $C_1$—$C_3$ alkyl;

26) More Preferred 22), 23), 24) and 25) in which Z is CH or N;

X is $CH_3$ or $CH_3O$; and

Y is $C_1$—$C_2$ alkyl; $C_1$—$C_2$ alkyl substituted with $OCH_3$; $OCH_2CH_3$, CN or 1—3 atoms of F, Cl or Br;

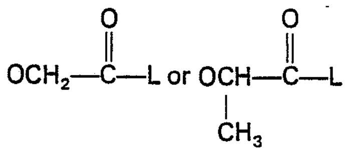

where L is $NH_2$, OH, 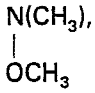

$N(CH_3)_2$, $NHCH_3$, $C_1$—$C_2$ alkoxy; SCN; $N_3$; $NR_{11}R_{12}$ where $R_{11}$ is H or $CH_3$; $R_{12}$ is H, $CH_3$, $CH_3CH_2$, $OCH_3$; $OR_9$ where $R_9$ is $CH_3$, $CH_3CH_2$, $CH_2CH{=}CH_2$ or $CH_2C{\equiv}CH$; $R_9$ is also $C_2$ alkyl substituted with 1—3 F, Cl or Br; $CH_3S$;

27) Preferred 21) in which A′ is H, Cl or Br;

28) More Preferred 27) in which Q is O or S and $R^I$ is $C_1$—$C_4$ alkyl, $CH_2CH{=}CH_2$ or $CH_2CH_2Cl$;

29) More Preferred 27) in which Q is O and $R^I$ is $CH_2CH_2OCH_3$, 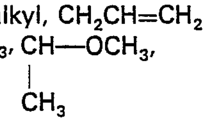

$CH_2OCH_3$ or $CH_2OCH_2CH_3$;

30) More Preferred 27) in which Q is 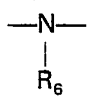

and $R^I$ is H, $C_1$—$C_3$ alkyl, $OCH_3$ or $OCH_2CH_3$ and $R_6$ is H or $C_1$—$C_2$ alkyl;

31) More Preferred 27) in which $R^{II}$ is H or $CH_3$;

32) More Preferred 28), 29), 30) and 31) in which A′ is H; Y is $CH_3$, $OCH_3$, $OCH_2CH_3$, $OCH_2CF_3$, $OCH_2CH{=}CH_2$ or $OCH_2C{\equiv}CH$;

33) More Preferred 27) in which A is 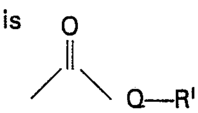

and Q is oxygen or sulfur and $R^I$ is $CH_3$ or $CH_2CH_3$; Q is 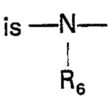

and $R^I$ is H, $CH_3$ or $OCH_3$ and $R_6$ is $CH_3$;

$R_1$ is 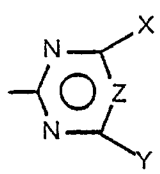

and Y is $CH_3$ or $OCH_3$;

34) More Preferred 33) of Formula I;

35) More Preferred 33) of Formula II;

36) More Preferred 33) of Formula III.

Specifically Preferred for reasons of highest activity and/or lowest cost and/or greatest ease of synthesis are:

methyl 3-[[(4,6-dimethoxypyrimidin-2-yl)aminocarbonyl]aminosulfonyl]-2-thiophenecarboxylate

methyl 3-[[(4,6-dimethylpyrimidin-2-yl)aminocarbonyl]aminosulfonyl]-2-thiophenecarboxylate

methyl 3-[[(4-methoxy-6-methylpyrimidin-2-yl)aminocarbonyl]aminosulfonyl]-2-thiophenecarboxylate

methyl 3-[[(4,6-dimethoxy-1,3,5-triazin-2-yl)aminocarbonyl]aminosulfonyl]-2-thiophenecarboxylate

methyl 3-[[(4,6-dimethyl-1,3,5-triazin-2-yl)aminocarbonyl]aminosulfonyl]-2-thiophenecarboxylate

methyl 3-[[(4-methoxy-6-methyl-1,3,5-triazin-2-yl)aminocarbonyl]aminosulfonyl]-2-thiophenecarboxylate

methyl 3-[[(4,6-dimethoxypyrimidin-2-yl)aminocarbonyl]aminosulfonyl]-2-furancarboxylate

methyl 3-[[(4,6-dimethylpyrimidin-2-yl)aminocarbonyl]aminosulfonyl]-2-furancarboxylate

methyl 3-[[(4-methoxy-6-methylpyrimidin-2-yl)aminocarbonyl]aminosulfonyl]-2-furancarboxylate

methyl 3-[[(4,6-dimethoxy-1,3,5-triazin-2-yl)aminocarbonyl]aminosulfonyl]-2-furancarboxylate

methyl 3-[[(4,6-dimethyl-1,3,5-triazin-2-yl)aminocarbonyl]aminosulfonyl]-2-furancarboxylate

methyl 3-[[(4-methoxy-6-methyl-1,3,5-triazin-2-yl)aminocarbonyl]aminosulfonyl]-2-furancarboxylate

methyl 2-[[(4,6-dimethoxypyrimidin-2-yl)aminocarbonyl]aminosulfonyl]-3-thiophenecarboxylate

methyl 2-[[(4,6-dimethylpyrimidin-2-yl)aminocarbonyl]aminosulfonyl]-3-thiophenecarboxylate

methyl 2-[[(4-methoxy-6-methylpyrimidin-2-yl)aminocarbonyl]aminosulfonyl]-3-thiophenecarboxylate

methyl 2-[[(4,6-dimethoxy-1,3,5-triazin-2-yl)aminocarbonyl]aminosulfonyl]-3-thiophenecarboxylate

methyl 2-[[(4,6-dimethyl-1,3,5-triazin-2-yl)aminocarbonyl]aminosulfonyl]-3-thiophenecarboxylate

methyl 2-[[(4-methoxy-6-methyl-1,3,5-triazin-2-yl)aminocarbonyl]aminosulfonyl]-3-thiophenecarboxylate

methyl 2-[[(4,6-dimethoxypyrimidin-2-yl)aminocarbonyl]aminosulfonyl]-3-furancarboxylate

methyl 2-[[(4,6-dimethylpyrimidin-2-yl)aminocarbonyl]aminosulfonyl]-3-furancarboxylate

methyl 2-[[4-methoxy-6-methylpyrimidin-2-yl)aminocarbonyl]aminosulfonyl]-3-furancarboxylate

methyl 2-[[(4,6-dimethoxy-1,3,5-triazin-2-yl)aminocarbonyl]aminosulfonyl]-3-furancarboxylate

methyl 2-[[(4,6-dimethyl-1,3,5-triazin-2-yl)aminocarbonyl]aminosulfonyl]-3-furancarboxylate

methyl 2-[[(4-methoxy-6-methyl-1,3,5-triazin-2-yl)aminocarbonyl]aminosulfonyl]-3-furancarboxylate

methyl 4-[[(4,6-dimethoxypyrimidin-2-yl)aminocarbonyl]aminosulfonyl]-3-thiophenecarboxylate

methyl 4-[[(4,6-dimethylpyrimidin-2-yl)aminocarbonyl]aminosulfonyl]-3-thiophenecarboxylate

methyl 4-[[(4-methoxy-6-methylpyrimidin-2-yl)aminocarbonyl]aminosulfonyl]-3-thiophenecarboxylate

methyl 4-[[(4,6-dimethoxy-1,3,5-triazin-2-yl)aminocarbonyl]aminosulfonyl]-3-thiophenecarboxylate

methyl 4-[[(4,6-dimethyl-1,3,5-triazin-2-yl)aminocarbonyl]aminosulfonyl]-3-thiophenecarboxylate

methyl 4-[[(4-methoxy-6-methyl-1,3,5-triazin-2-yl)aminocarbonyl]aminosulfonyl]-3-thiophenecarboxylate

methyl 4-[[(4,6-dimethoxypyrimidin-2-yl)aminocarbonyl]aminosulfonyl]-3-furancarboxylate

methyl 4-[[(4,6-dimethylpyrimidin-2-yl)aminocarbonyl]aminosulfonyl]-3-furancarboxylate

methyl 4-[[(4-methoxy-6-methylpyrimidin-2-yl)aminocarbonyl]aminosulfonyl]-3-furancarboxylate

methyl 4-[[(4,6-dimethoxy-1,3,5-triazin-2-yl)aminocarbonyl]aminosulfonyl]-3-furancarboxylate

methyl 4-[[(4,6-dimethyl-1,3,5-triazin-2-yl)aminocarbonyl]aminosulfonyl]-3-furancarboxylate

methyl 4-[[(4-methoxy-6-methyl-1,3,5-triazin-2-yl)aminocarbonyl]aminosulfonyl]-3-furancarboxylate

N-[(4-methoxy-6-methylpyrimidin-2-yl)aminocarbonyl]-2-(1-pyrrolidinylcarbonyl)-3-thiophenesulfonamide

1-methylethyl 3-[[(4,6-dimethoxypyrimidin-2-yl)aminocarbonyl]aminosulfonyl]-2-thiophenecarboxylate

2-propenyl 3-[[(4-methoxy-6-methylpyrimidin-2-yl)aminocarbonyl]aminosulfonyl]-2-thiophene carboxylate

1-methylethyl 3-[[(4-methoxy-6-methylpyrimidin-2-yl)aminocarbonyl]aminosulfonyl]-2-thiophenecarboxylate.

Intermediates

Compounds of Formulas I', II' and III' are used for the preparation of compounds of Formulas I, II and III

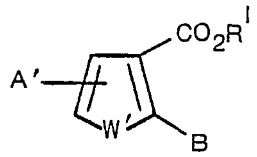

I'

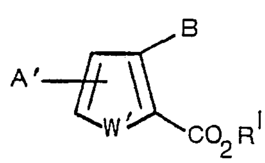

II'

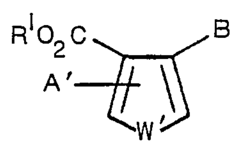

III'

wherein

R' is H or M;

M is a cation of an alkali metal or of a tertiary amine of up to 12 carbon atoms;

A', W', B, are as previously defined.

Preferred and in increasing order for reasons of lower cost and/or greater ease of synthesis and/or higher activity of derived compounds are those intermediate

1) Compounds of the Generic scope in which W' is sulfur, B is $SO_2NHCONHR_1$, A' is H, Cl or Br and

$R_1$ is 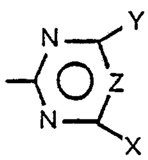 , 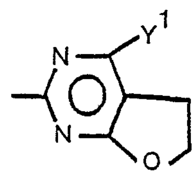 or 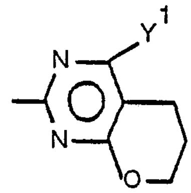

2) Compounds of Preferred 1) in which X is $CH_3$ or $OCH_3$, Y is $CH_3$, $OCH_3$, $OCH_2CH_3$, $OCH_2CF_3$, $OCH_2CH{=}CH_2$ or $OCH_2{-}C{\equiv}C{-}H$; Z is CH or N;

3) Compounds of Preferred 2) in which Y is $CH_3$ or $OCH_3$;

$R_1$ is 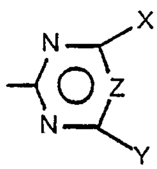 ;

A' is H;

4) Compounds of Preferred 3) of Formula I';

5) Compounds of Preferred 4) of Formula II';

6) Compounds of Preferred 5) of Formula III';

Equally Preferred in increasing order, for reasons of lower cost and/or greater ease of synthesis and/or higher activity of derived compound are those intermediate:

7) Compounds of the Generic scope in which W' is oxygen; B is $SO_2NHCONHR_1$, A' is H, Cl or Br, and

$R_1$ is 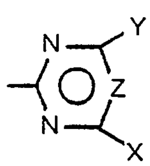 , 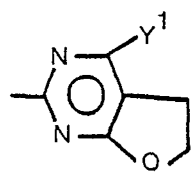 or 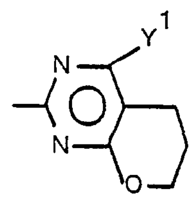 .

8) Compounds of Preferred 7) in which X is $CH_3$ or $OCH_3$; Y is $CH_3$, $OCH_3$, $OCH_2CH_3$, $OCH_2CF_3$, $OCH_2CH{=}CH_2$ or $OCH_2C{\equiv}C{-}H$; and Z is CH or N;

9) Compounds of Preferred 8) in which Y is $CH_3$ or $OCH_3$;

$R_1$ is 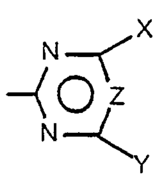 ;

A' is H;

10) Compounds of Preferred 9) of Formula IV;

11) Compounds of Preferred 10) of Formula V;

12) Compounds of Preferred 11) of Formula VI;

Specifically Preferred for reasons of lowest cost and/or greatest ease of synthesis and/or highest activity of desired compounds are:

3-[[(4,6-dimethoxypyrimidin-2-yl)aminocarbonyl]aminosulfonyl]-2-thiophenecarboxylic acid

3-[[(4,6-dimethylpyrimidin-2-yl)aminocarbonyl]aminosulfonyl]-2-thiophenecarboxylic acid

3-[[(4-methoxy-6-methylpyrimidin-2-yl)aminocarbonyl]aminosulfonyl]-2-thiophenecarboxylic acid

3-[[(4,6-dimethoxy-1,3,5-triazin-2-yl)aminocarbonyl]aminosulfonyl]-2-thiophenecarboxylic acid

3-[[(4,6-dimethyl-1,3,5-triazin-2-yl)aminocarbonyl]aminosulfonyl]-2-thiophenecarboxylic acid

3-[[(4-methoxy-6-methyl-1,3,5-triazin-2-yl)aminocarbonyl]aminosulfonyl]-2-thiophenecarboxylic acid
3-[[(4,6-dimethoxypyrimidin-2-yl)aminocarbonyl]aminosulfonyl]-2-furancarboxylic acid
3-[[(4,6-dimethylpyrimidin-2-yl)aminocarbonyl]aminosulfonyl]-2-furancarboxylic acid
3-[[(4-methoxy-6-methylpyrimidin-2-yl)aminocarbonyl]aminosulfonyl]-2-furancarboxylic acid
3-[[(4,6-dimethoxy-1,3,5-triazin-2-yl)aminocarbonyl]aminosulfonyl]-2-furancarboxylic acid
3-[[(4,6-dimethyl-1,3,5-triazin-2-yl)aminocarbonyl]aminosulfonyl]-2-furancarboxylic acid
3-[[(4-methoxy-6-methyl-1,3,5-triazin-2-yl)aminocarbonyl]aminosulfonyl]-2-furancarboxylic acid
2-[[(4,6-dimethoxypyrimidin-2-yl)aminocarbonyl]aminosulfonyl]-3-thiophenecarboxylic acid
2-[[(4,6-dimethylpyrimidin-2-yl)aminocarbonyl]aminosulfonyl]-3-thiophenecarboxylic acid
2-[[(4-methoxy-6-methylpyrimidin-2-yl)aminocarbonyl]aminosulfonyl]-3-thiophenecarboxylic acid
2-[[(4,6-dimethoxy-1,3,5-triazin-2-yl)aminocarbonyl]aminosulfonyl]-3-thiophenecarboxylic acid
2-[[(4,6-dimethyl-1,3,5-triazin-2-yl)aminocarbonyl]aminosulfonyl]-3-thiophenecarboxylic acid
2-[[(4-methoxy-6-methyl-1,3,5-triazin-2-yl)aminocarbonyl]aminosulfonyl]-3-thiophenecarboxylic acid
2-[[(4,6-dimethoxypyrimidin-2-yl)aminocarbonyl]aminosulfonyl]-3-furancarboxylic acid
2-[[(4,6-dimethylpyrimidin-2-yl)aminocarbonyl]aminosulfonyl]-3-furancarboxylic acid
2-[[(4-methoxy-6-methylpyrimidin-2-yl)aminocarbonyl]aminosulfonyl]-3-furancarboxylic acid
2-[[(4,6-dimethoxy-1,3,5-triazin-2-yl)aminocarbonyl]aminosulfonyl]-3-furancarboxylic acid
2-[[(4,6-dimethyl-1,3,5-triazin-2-yl)aminocarbonyl]aminosulfonyl]-3-furancarboxylic acid
2-[[(4-methoxy-6-methyl-1,3,5-triazin-2-yl)aminocarbonyl]aminosulfonyl]-3-furancarboxylic acid
4-[[(4,6-dimethoxypyrimidin-2-yl)aminocarbonyl]aminosulfonyl]-3-thiophenecarboxylic acid
4-[[(4,6-dimethylpyrimidin-2-yl)aminocarbonyl]aminosulfonyl]-3-thiophenecarboxylic acid
4-[[(4-methoxy-6-methylpyrimidin-2-yl)aminocarbonyl]aminosulfonyl]-3-thiophenecarboxylic acid (B5854)
4-[[(4,6-dimethoxy-1,3,5-triazin-2-yl)aminocarbonyl]aminosulfonyl]-3-thiophenecarboxylic acid
4-[[(4,6-dimethyl-1,3,5-triazin-2-yl)aminocarbonyl]aminosulfonyl]-3-thiophenecarboxylic acid
4-[[(4-methoxy-6-methyl-1,3,5-triazin-2-yl)aminocarbonyl]aminosulfonyl]-3-thiophenecarboxylic acid
4-[[(4,6-dimethoxypyrimidin-2-yl)aminocarbonyl]aminosulfonyl]-3-furancarboxylic acid
4-[[(4,6-dimethylpyrimidin-2-yl)aminocarbonyl]aminosulfonyl]-3-furancarboxylic acid
4-[[(4-methoxy-6-methylpyrimidin-2-yl)aminocarbonyl]aminosulfonyl]-3-furancarboxylic acid
4-[[(4,6-dimethoxy-1,3,5-triazin-2-yl)aminocarbonyl]aminosulfonyl]-3-furancarboxylic acid
4-[[(4,6-dimethyl-1,3,5-triazin-2-yl)aminocarbonyl]aminosulfonyl]-3-furancarboxylic acid
4-[[(4-methoxy-6-methyl-1,3,5-triazin-2-yl)aminocarbonyl]aminosulfonyl]-3-furancarboxylic acid

The following compounds of Formulas I'', II'' and III'' can also be used for the preparation of compounds of Formulas I, II and III

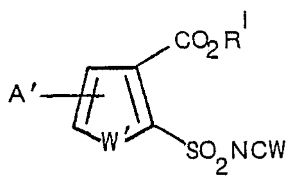
I''

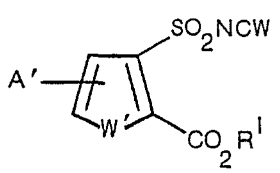
II''

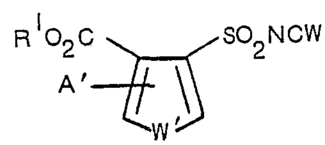
III''

wherein

W is oxygen or sulfur;

W' is oxygen or sulfur;

A' is H, Cl, Br, $C_1$—$C_4$ alkyl, $OCH_3$, $NO_2$ or $CF_3$;

$R^I$ is $C_1$—$C_6$ alkyl; $C_3$—$C_6$ alkenyl; $C_3$—$C_6$ alkynyl; $C_2$—$C_6$ alkyl substituted with Cl, CN or $OCH_3$; $C_3$—$C_6$ alkenyl substituted with 1—3 Cl; $C_3$—$C_6$ alkynyl substituted with Cl; $C_5$—$C_6$ cycloalkyl; cyclohexenyl; cyclohexyl substituted with 1—3 $CH_3$; $C_4$—$C_7$ cycloalkylalkyl or

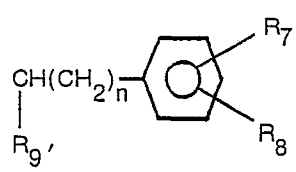

where $R_7$ and $R_8$ are independently H, Cl, $CH_3$ or $OCH_3$;

n is 0 or 1; and

$R_{9'}$ is H or $CH_3$; $CH_2CH_2OR_{15}$, $CH_2CH_2CH_2OR_{15}$, $\underset{\displaystyle CH_3}{\underset{|}{CH}}-CH_2OR_{15}$

where $R_{15}$ is $C_2H_5$, $CH(CH_3)_2$, phenyl, $CH_2CH_2Cl$, $CH_2CCl_3$; $-(CH_2CH_2O)_{n'}R_{16}$, $(\underset{\displaystyle CH_3}{\underset{|}{CH}}-CH_2O)_{n'}R_{16}$

where $R_{16}$ is $CH_3$, $C_2H_5$, $CH(CH_3)_2$, phenyl, $CH_2CH_2Cl$, $CH_2CCl_3$, and n′ is 2 or 3;

$CH_2CN$; 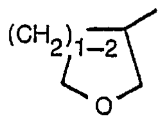 ; 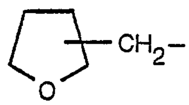 ; 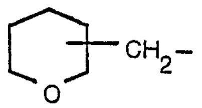 ; 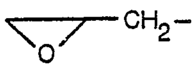 ; $CH_2OR'_6$ ;

where $R'_6$ is $C_1$—$C_4$ alkyl; provided R' has a total of $\leq 13$ carbon atoms.

Preferred in increasing order for reasons of lower cost and/or greater ease of synthesis and/or higher activity of desired compounds are those intermediate:

1) Compounds of the Generic scope in which W′ is sulfur; W is oxygen; A′ is H, Cl or Br; and R' is $C_1$—$C_4$ alkyl; $CH_2CH{=}CH_2$; or $CH_2CH_2Cl$; $CH_2CH_2OCH_3$; or $\underset{\displaystyle CH_3}{\underset{|}{CH}}-OCH_3$

2) Compounds of Preferred 1) in which R' is $CH_3$ or $CH_2CH_3$; A' is H;

3) Compounds of Preferred 2) of Formula VII;

4) Compounds of Preferred 2) of Formula VIII;

5) Compounds of Preferred 2) of Formula IX.

Equally Preferred in increasing order for reasons of lower cost and/or greater ease of synthesis and/or higher activity of derived compounds are those intermediate:

6) Compounds of the generic scope in which W′ is oxygen; W is oxygen; A′ is H, Cl or Br;

7) Compounds of Preferred 6) in which R' is $CH_3$ or $CH_2CH_3$; $A^1$ is H;

8) Compounds of Preferred 7) of Formula I″.

9) Compounds of Preferred 7) of Formula II″.

10) Compounds of Preferred 7) of Formula III″.

Specifically Preferred for reasons of lowest cost and/or greatest ease of synthesis and/or highest activity of desired compounds are:

methyl 3-(isocyanatosulfonyl)-2-thiophenecarboxylate
methyl 2-(isocyanatosulfonyl)-3-thiophenecarboxylate
methyl 4-(isocyanatosulfonyl)-3-thiophenecarboxylate
methyl 3-(isocyanatosulfonyl)-2-furancarboxylate
methyl 2-(isocyanatosulfonyl)-3-furancarboxylate
methyl 4-(isocyanatosulfonyl)-3-furancarboxylate

Synthesis

Many of the compounds of Formulas I—III are prepared as shown in Equation 3 by the reaction of an appropriately substituted alkoxycarbonylthiophene or -furan sulfonylisocyanate or sulfonylisothiocyanate with an appropriate aminopyrimidine or aminotriazine. These compounds of Formulas I—III can be converted to other compounds of Formulas I—III as will be shown in subsequent equations.

The novel sulfonylisocyanates are important intermediates for the preparation of the compounds of this invention. Their synthesis is described in Equations 1 and 2.

EQUATION 1

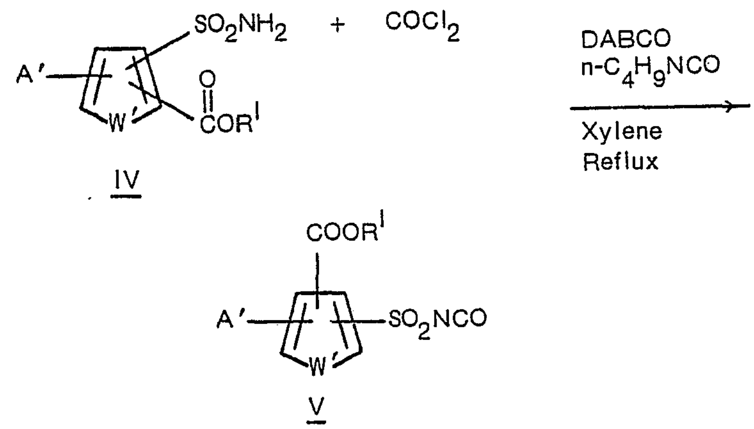


A mixture of the appropriate sulfonamide, e.g. an 2-alkoxycarbonyl-3-thiophene sulfonamide IV such as the methyl ester, which is known in the art, an alkyl isocyanate such as butyl isocyanate and a catalytic amount of 1,4-diaza[2.2.2]bicyclooctane (DABCO) in xylene or other inert solvent of sufficiently high boiling point (e.g. > 135°) is heated to approximately 130—150°C. Phosgene is added to the mixture until an excess of phosgene is present as indicated by a drop in the boiling point. After the mixture is cooled and filtered to remove a small amount of insoluble by-products, the solvent and alkyl isocyanate are distilled off *in-vacuo* leaving a residue which is the crude sulfonyl isocyanate V. In Equation 1.

A′, W′ and $CO_2R^I$ are as defined previously for structures I″, II″ and III″.

The novel sulfonylisothiocyanate intermediates of formula $V^a$, prepared according to Equations 2 and 2′, are useful for the preparation of compounds of Formulas I—III where W = S.

EQUATION 2

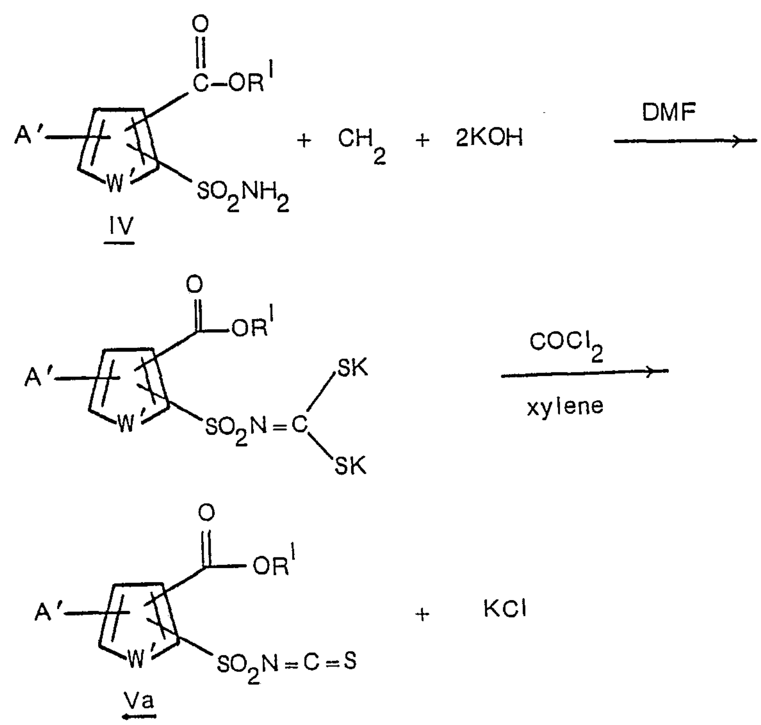

The alkoxycarbonyl substituted sulfonamide is dissolved in dimethylformamide (DMF) with an equivalent amount of carbon disulfide and two equivalents of potassium hydroxide are added portionwise at room temperature. The mixture is stirred for 1—8 hours and diluted with ethylacetate, ethyl ether or similar aprotic solvent to cause the dipotassium salt of the dithiocarbamic acid to precipitate. The salt is isolated, dried and suspended in an inert solvent such as xylene, benzene, carbon tetrachloride or methylene chloride. Phosgene is added to the stirred suspension at below room temperature and the mixture stirred for 1—3 hours. In place of phosgene, a chloroformic ester (e.g. methyl chloroformate), phosphorous pentachloride, sulfuryl chloride or thionyl chloride can be used.

The sulfonylisothiocyanate which is formed is usually soluble in the solvent and is isolated by filtering off the insoluble potassium chloride and concentrating the filtrate. These isothiocyanates tend to be unstable and dimerize readily, (Equation 2') however, the dimers can be used

EQUATION 2'

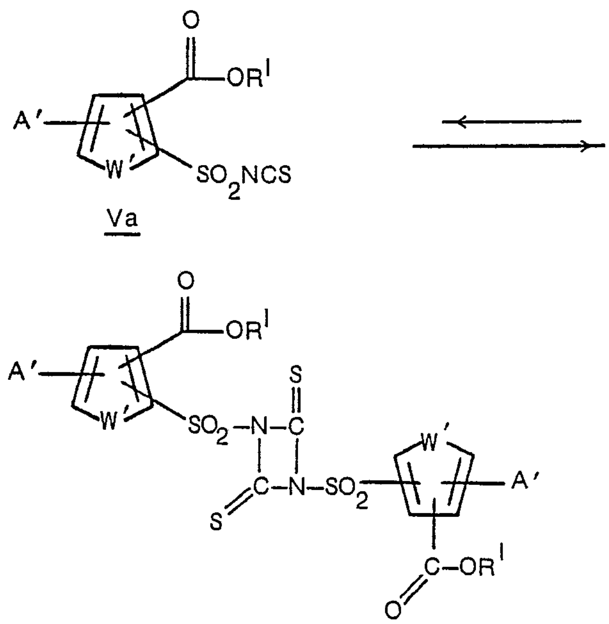

Va

in the same manner as the parent isothiocyanates for the purposes of this invention.

The synthetic method chosen for the preparation of compounds of Formulas I—III depends largely on the substituents QR' and $R_4$. As shown in Equation 3, compounds of Formulas I—III wherein R' or A' are as defined for Equation 1, are conveniently prepared by reacting an appropriately substituted carbonyl thiophene or furan sulfonyl isocyanate or isothiocyanate of Formula V with an appropriately substituted aminopyrimidine or aminotriazine of Formula VI:

EQUATION 3

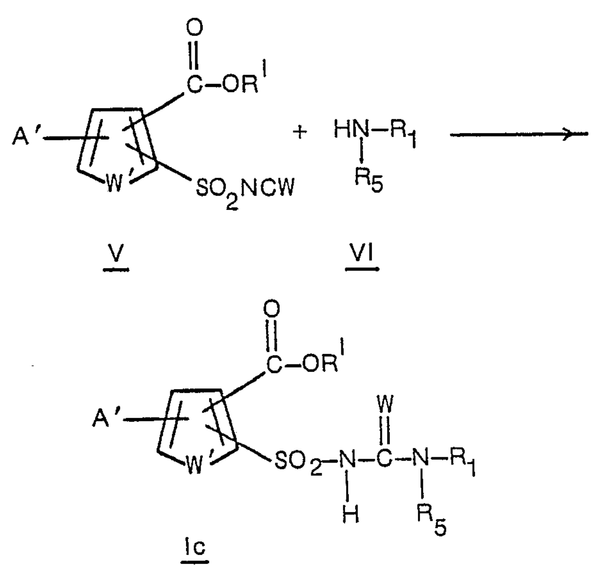

V VI

Ic

The reaction of Equation 3 is best carried out in inert aprotic organic solvents such as methylene chloride, tetrahydrofuran or acetonitrile, at ambient pressure and temperature. The mode of addition is not critical; however, it is often convenient to add the sulfonyl isocyanate or isothiocyanate to a stirred suspension of amine VI. Since such isocyanates and isothiocyanates are liquids, low melting solids or are readily soluble in solvents such as those listed above, their addition can be easily controlled.

The reaction is generally exothermic. In some cases, the desired product is soluble in the warm reaction medium and on cooling crystallizes in pure form. Other products which are soluble in the reaction medium are isolated by evaporation of the solvent, trituration of the solid residue with solvents such as 1-chlorobutane or ethyl ether, and filtration.

As shown in Equation 4, compounds of Formulas I—III, wherein W is S, A and A′ are as previously defined and $R_5$ is H, are alternatively prepared by the reaction of an appropriately substituted thiophene or furan sulfonamide with the appropriate triazine or pyrimidine isothiocyanate of Formula VIa.

EQUATION 4

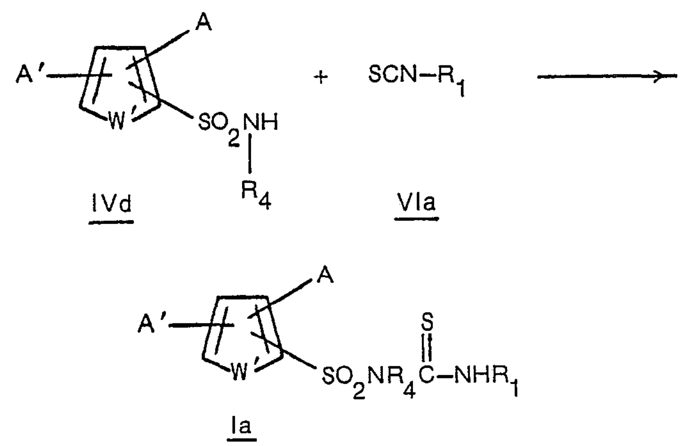

IVd VIa

Ia

The reaction of Equation 4 is best carried out by dissolving or suspending the sulfonamide and isothiocyanate in a polar solvent such as acetone, acetonitrile, ethyl acetate or methylethylketone, adding an equivalent of a base such as potassium carbonate and stirring the mixture at a temperature from ambient up to the reflux temperature from one to twenty-four hours. In some cases, the product precipitates from the reaction mixture and can be removed by filtration. The product is stirred in dilute mineral acid, filtered and washed with cold water. If the product does not precipitate from the reaction mixture, it can be isolated by evaporation of the solvent, trituration of the residue with dilute mineral acid and filtering off the insoluble product.

The heterocycle isothiocyanates which are used in the procedure of Equation 4 are prepared, for example, according to the method of Japan Patent Application Pub: Kokai 51—143686, June 5, 1976, or that of W. Abraham and G. Barnikow, Tetrahedron 29, 691—7 (1973).

As shown in Equation 5, compounds of Formulas I—III, wherein A, A′, $R_1$, W′, and $R_5$ are as defined previously, and W is O, can be prepared by methylation of salts VII wherein M is an alkali metal cation such as sodium (derived from compounds of Formulas I—III wherein $R_4$ is hydrogen):

EQUATION 5

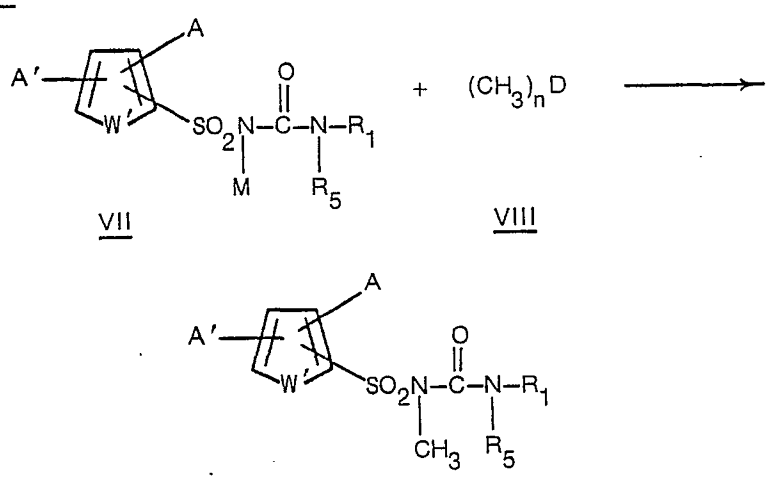

VII VIII

D being an incipient anion and n being an integer corresponding to the valence of D.

The reaction of Equation 5 is best carried out in aprotic organic solvents such as tetrahydrofuran, dimethylformamide, or dimethylacetamide, at ambient pressure and temperature. Methylating agents VIII such as dimethyl sulfate or methyl iodide, can be employed. The desired product can be isolated by pouring the reaction mixture into water and filtering off the precipitated solid.

As shown in Equation 6, compounds of Formulas I—III wherein A, A', $R_1$, W', and $R_5$ are as defined for Equation 5, and W is O or S, can also be prepared by the reaction of an appropriately substituted sulfonyl-N-methylcarbamyl chloride or sulfonyl-N-methylthiocarbamyl chloride of Formula IX with an appropriate aminopyrimidine or aminotriazine of Formula V:

EQUATION 6

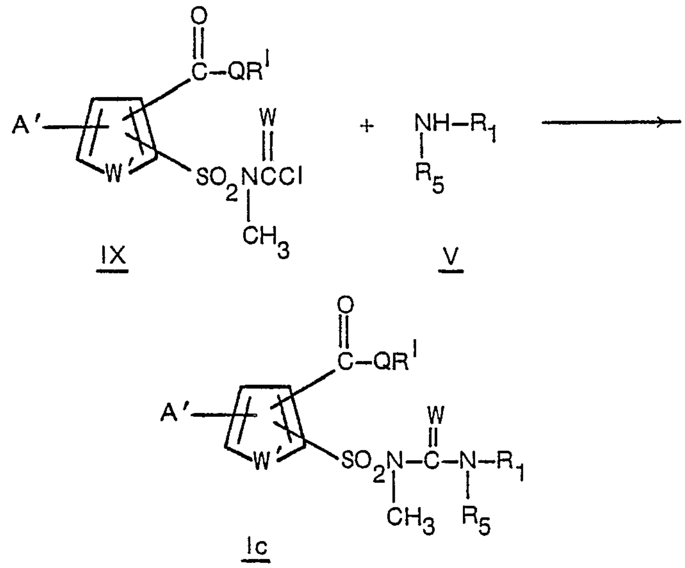

The preparation of ureas and thioureas, like those of Formula Ic, from amines and carbamyl chlorides and thiocarbamyl chlorides is well known to the art. The reaction can best be carried out by adding equivalent amounts of chloride IX and amine V to an inert organic solvent, such as tetrahydrofuran, xylene, or methylene chloride, in the presence of an acid acceptor, such as triethylamine, pyridine, or sodium carbonate employing temperatures from 20°—130°. Soluble products can be isolated by filtering off the precipitated salts and concentration of the filtrate. Insoluble products can be filtered off and washed free of salts with water.

The chlorides of Formula IX can be prepared by phosgenation or thiophosgenation of N-methylsulfonamide salts. The sulfonamide salt is added to an excess of phosgene or thiophosgene in an inert organic solvent, such as tetrahydrofuran, toluene, or xylene, whereupon, after removal of the excess phosgene, the chloride IX can be isolated or reacted *in situ* with the amine VI.

The esters of Formulas I—III hydrolyze to the parent acid as shown in Equation 7. Alkali metal base catalyzed hydrolysis in aqueous methanol produces the alkali metal carboxylate from which the carboxylic acid is obtained by treatment with mineral acids such as HCl:

EQUATION 7

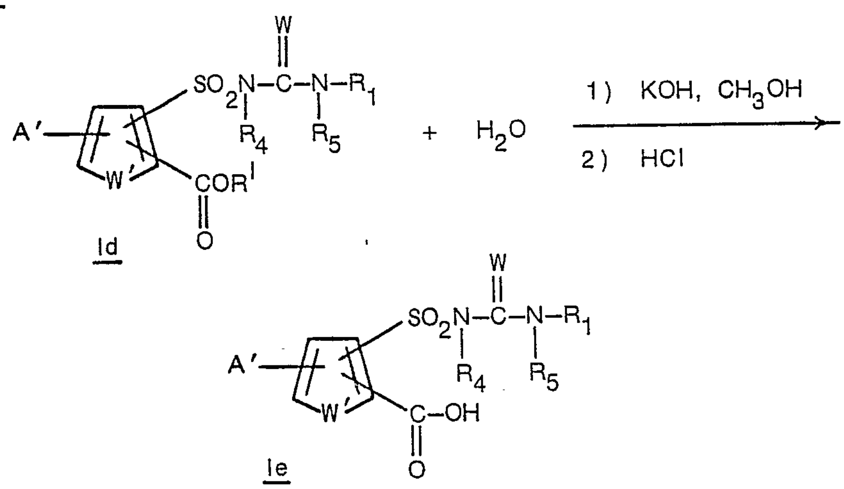

The reaction of Equation 7 is best carried out in a solution containing the compound being hydrolyzed, 2 to 10 parts of methanol, 10—50 parts of water and 2—10 equivalents of a base such as sodium or potassium hydroxide maintaining the temperature at 30—90°C for 3—24 hours. The reaction yields the soluble alkali metal salt of the carboxylic acid, which is suitable for the purposes of this invention. Conversion of these salts to the acid form is easily carried out by addition to the reaction medium of strong mineral acids, such as hydrochloric or sulfuric acid, causing the desired carboxylic acids to precipitate from solution.

The acids of Formula Ie prepared as in Equation 7 wherein W is O can be converted to compounds of this invention where $R^I$ is a higher alkyl or substituted hydrocarbyl group, as already disclosed herein, by the reaction of salts of the parent acid with $R^I$-halogen as shown in Equation 8.

EQUATION 8

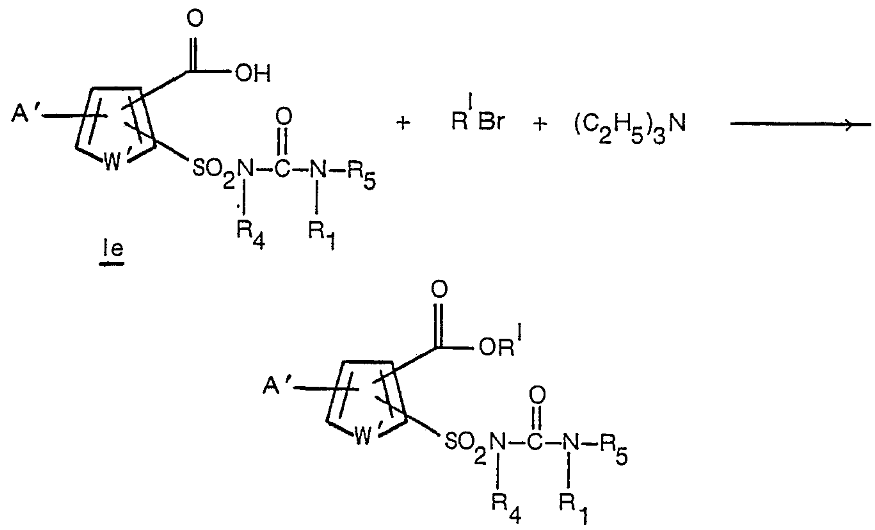


Ie

The reaction of Equation 8 is of use where the intermediate compound $R^I$-halogen contains a readily replaceable halogen as is the case for substituted or unsubstituted allylic or benzylic halides, $\alpha$-halonitriles, or $\alpha$-halocarbonyl compounds.

The procedure of Equation 8 is best carried out in inert polar solvents such as tetrahydrofuran, acetonitrile or acetone by combining the appropriately substituted carboxylic acid and base such as triethylamine or 1,4-diaza[2,2,2]bicyclooctane adding the appropriate halide and heating the mixture to reflux with stirring for 1 to 16 hours. The reaction mixture can be evaporated to dryness and the residue triturated with water, filtered and washed with water to separate the desired product from the water soluble salt.

The procedure of Equation 8 can also be used for the synthesis of compounds wherein $R^I$-halogen of Equation 8 is of a less reactive species than described above. In these cases, the silver salt of the carboxylic acid is used rather than the amine salt. The silver salt which is precipitated by adding silver nitrate to an aqueous solution of the sodium salt of the acid of Formula Ie is combined with the appropriate $R^I$-halide using the same solvents and conditions as shown above for the amine salt.

When Q is $NR_6$, the compounds can be prepared from the esters of this invention where $R^I$ is

$C_1$—$C_4$ (preferably $C_1$) by the reaction of the esters with dialkylaluminum-N-alkylamide derivatives according to Equation 9; $R^I$, A′, W′, $R_1$, $R_4$, $R_5$ and $R_6$ being as previously defined.

EQUATION 9

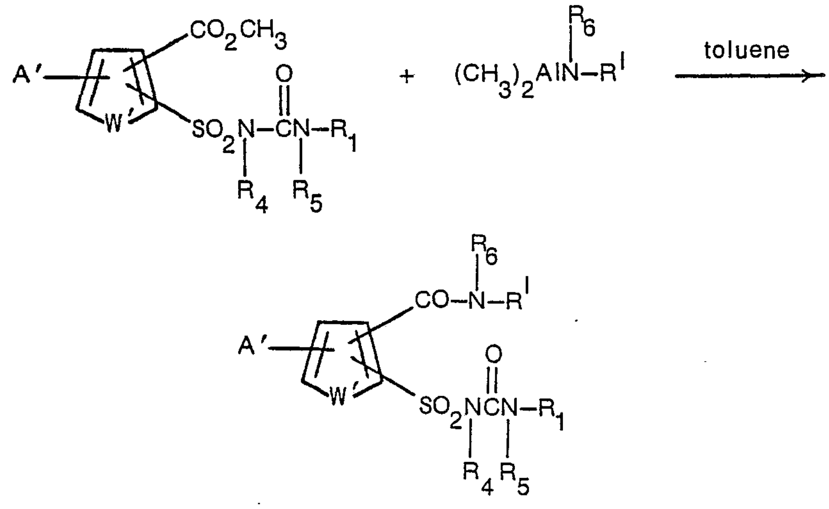


The intermediate alkylaminoaluminum compounds prepared according to A. Basha, M. Lipton and S. W. Weinreb, *Tetrahedron Letters* 4171 (1977), are co-mingled with a suspension of the esters in toluene or similar inert solvent and the mixture is refluxed for one to six hours. The product can be isolated by evaporation of the solvent, addition of methylene chloride and aqueous hydrochloric acid to decompose the residual reaction mass and extracting the desired product into methylene chloride. Evaporation of the methylene chloride yields the desired product in sufficiently pure form for the purpose of this invention.

Compounds of Formula X, wherein Q is $R^I$
|
$NR_6$,

A′, W′ and $R_4$ are as previously defined in the general formula, which are useful as intermediates in Equation 4, are prepared as shown in Equation 10.

EQUATION 10

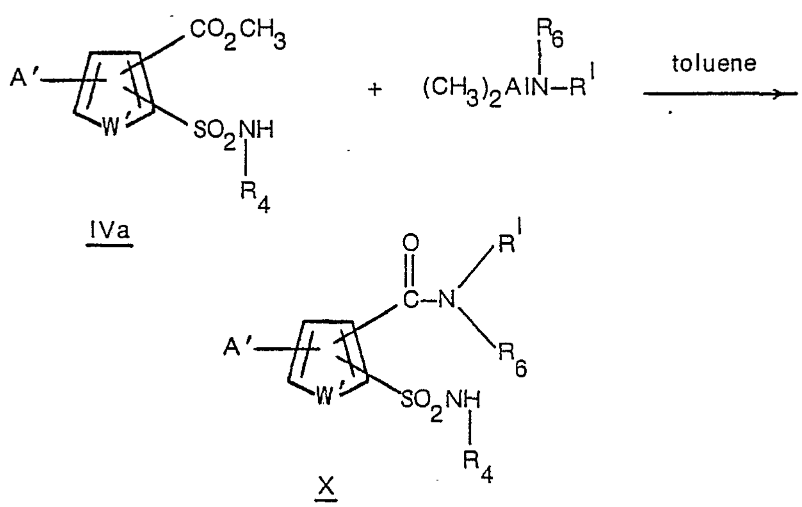


IVa

X

The conditions described for Equation 8 are suitable for the conversion of the esters of Formula IVa to the carboxamides as shown in Equation 10.

The products of Equation 10 are especially useful for the preparation of compounds of Formulas I—III wherein Y has an ester substituent $CO_2(C_1$—$C_6)$, by the route described in Equation 4.

When Q is S, these compounds can be prepared from the esters of this invention wherein $QR^I$ is

O($C_1$—$C_4$ alkyl) (preferably $C_1$) by the reaction of the esters with the appropriate dialkylaluminum alkylthiolate according to Equation 11.

EQUATION 11

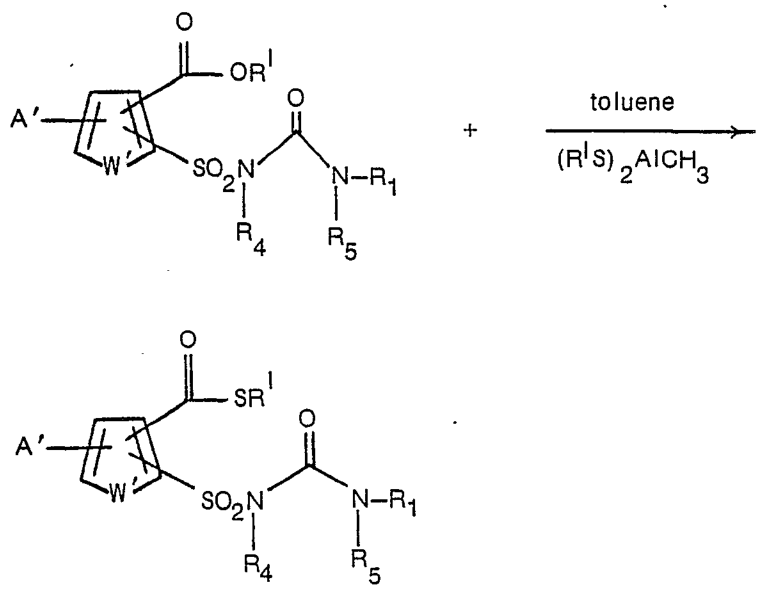


The intermediate aluminum thiolates can be prepared according to R. P. Hatch and S. W. Weinreb, *Journal of Organic Chemistry,* Vol. 42, 3960 (1977). The reaction of the thiolate with the ester of this invention is best carried out in a neutral solvent such as toluene or xylene at reflux for one to three hours. Best results are obtained when the aluminum thiolate compound is present in excess of the stoichiometric amount required.

Sulfonamides of Formula IVc are also converted from carboxylic acid esters to the thiolesters as shown in Equation 12 according to the method of R. P. Hatch and S. W. Weinreb as described for Equation 11 wherein R', A', W' and $R_4$ are as previously defined.

EQUATION 12

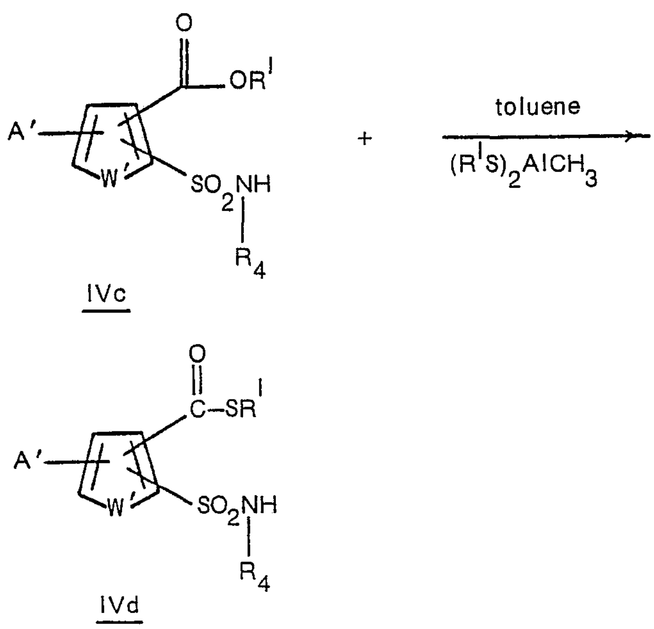


IVc

IVd

The conditions described for Equation 11 are suitable for the conversion of the sulfonamides of Formula IVc as shown in Equation 12.

The products obtained by the procedure of Equation 12 are especially useful for the preparation of

compounds of formulas I—III where Y has a substituent ($CO_2C_1$—$C_6$) by the route described for Equation 4 and Q = S.

An alternate route to prepare compounds where $R^I$ is bonded to Q (Q=O) at a secondary carbon involves the reaction of the appropriate dialkylaluminum alcoholate and an ester of this invention wherein $R^I$ is a lower primary alkyl group, preferably methyl, according to Equation 13.

EQUATION 13

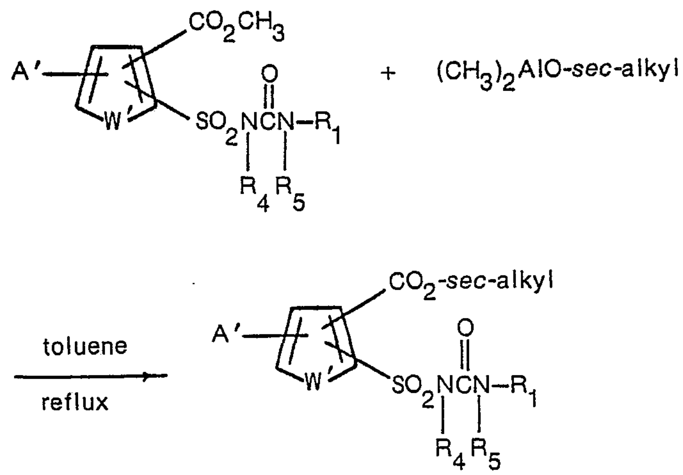


The reaction is carried out in a neutral solvent such as toluene with a boiling point sufficiently high to bring about the desired reaction during reflux. The dialkylaluminum alcoholate being present in greater than an equivalent amount to the ester for best yields. After refluxing for 1—15 hours, the reaction mixture is decomposed with dilute hydrochloric acid and the product extracted into methylene chloride. Evaporation of the methylene chloride yields the desired compound sufficiently pure for the purposes of this invention. The product can be triturated with a solvent, e.g. 1-chlorobutane to remove impurities.

Ketones wherein A is $\overset{O}{\overset{\|}{C}}—R^{II}$ and A′, W′, W, $R_1$, $R_4$ and $R_5$ are as defined by the scope of this invention, are prepared according to Equation 14, from the carboxylic acids of Formula Ie whose preparation is described in Equation 7.

EQUATION 14

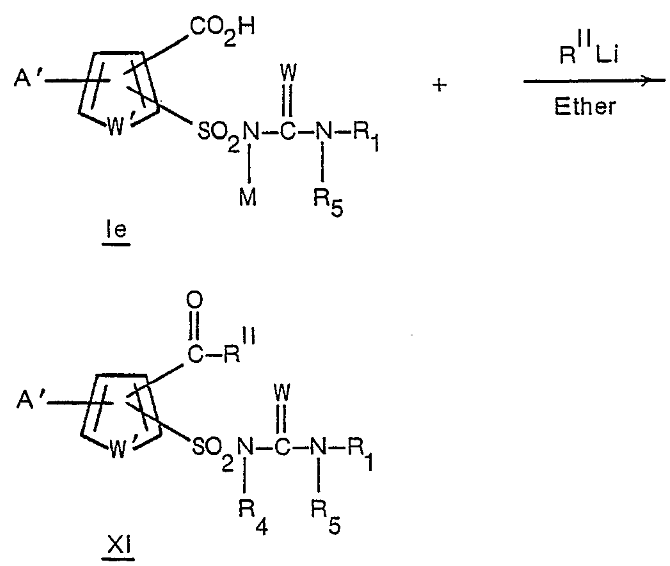


Ie

XI

The reaction of an organolithium compound with a carboxylic acid to yield a ketone as in Equation 14 is described in the work of H. Gilman and P. R. Van Ess, JACS, *55,* 1258 (1933); H. Gilman, W. Langham and F. W. Moore, ibid., *62* (1940)); C. Tegner, Chem. Scand., *6,* 782 (1952); J. F. Arens and

D. A. Van Dorp, Rec. Trav., *65* 338 (1946); *66,* 759 (1947); C. H. Depuy, G. M. Dappen, K. L. Eilers and R. A. Klein, J. Org., *29,* 2813 (1964).

An excess of the organolithium compound in a suitable solvent such as diethyl ether, hexane, pentane or benzene is added to a solution or slurry of XII in a similar solvent at temperatures between −100 and 0°C. The mixture is allowed to warm to room temperature and stir for 30 minutes. Aqueous acid is then added and the ketosulfonamide extracted into a suitable solvent to free it from salts followed by evaporation of the solvent.

The synthesis of a wide variety of organolithium compounds by many different procedures is known in the art. A summary of methods with bibliography is contained in *Organo-Metallic Compounds,* G. E. Coates, John Wiley and Sons, 1960, p. 3—21.

Oximes of the ketones of Formula XI, for example, can be prepared from the appropriate hydroxylamine derivative, wherein $R^{III}$ is as previously defined, and the ketone of Formula XI according to Equation 15.

EQUATION 15

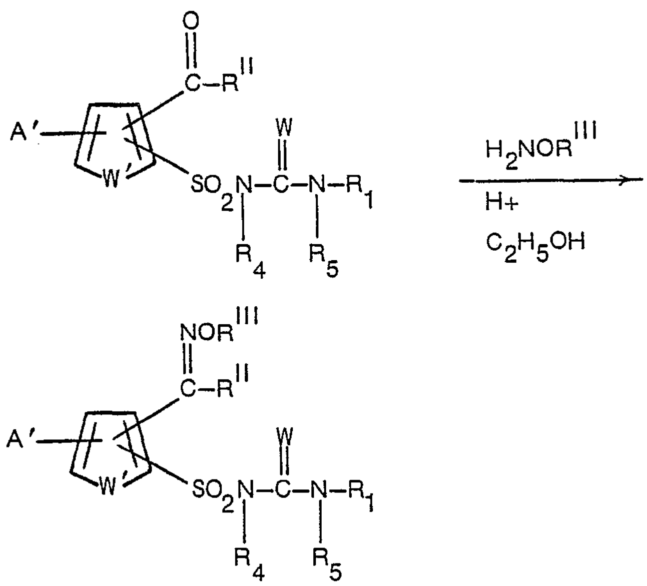


A procedure such as that described in *Preparative Organic Chemistry* by G. Hilgetag and A. Martini, Ed., John Wiley and Sons, p. 513 is suitable for the preparation of the oximes of this invention.

Compounds of Formulas I—III wherein Y of group $R_1$ contains 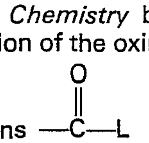 and L is OH can be prepared according to the procedure of Equation 16 wherein A′, W′, X, $R_4$, W and $R_5$ are as defined previously; and Q′ is $C_1$—$C_4$ alkyl, $OCH_2$, $OCH_2CH_2$, 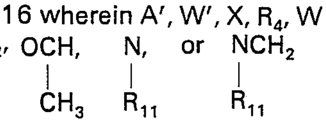 where $R_{11}$ is as previously defined.

EQUATION 16

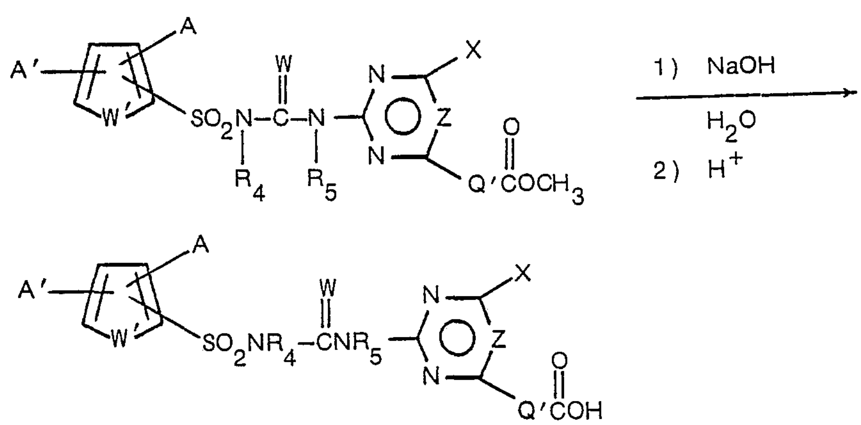

The reaction of Equation 16 is best carried out by suspending the compound being hydrolyzed in 10 to 100 parts of water with enough of a base such as sodium hydroxide or potassium hydroxide to obtain a pH 10 to 14, ideally a pH of 12, heating until a clear solution is obtained and then adjusting the pH to 1—3, preferably 3. The product is thus caused to precipitate in some instances and can be removed by filtration or it can be extracted into a polar organic solvent such as methylene chloride and isolated by evaporation of the solvent.

Thiophene derivatives with sulfamoyl and alkoxycarbonyl substituents on adjacent carbon atoms are prepared by the methods taught by O. Hromatka and D. Binder, U.S. Patent 4,028,373 and P. A. Rossy et al, U.S. Patent 4,143,050. The analogous furan derivatives are prepared similarly or as taught in Belgian Patent 871,772.

Compounds of structure XXIX wherein A is an aldehyde group and A′ does not equal —$NO_2$ are prepared by the procedure of Equation 17.

EQUATION 17

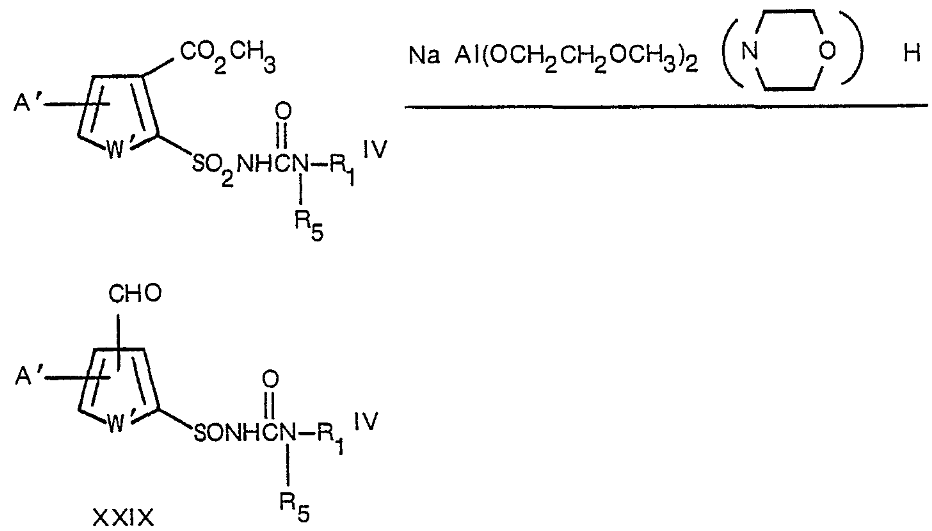


XXIX

Following the procedure os R. Kanazawa and T. Tokoroyama, *Synthesis,* 526 (1976), a solution of sodium bis-(2-methoxyethoxy)aluminum hydride in THF is reacted with one equivalent of morpholine. To this solution at −40°C is added a methyl ester of Formula XXVIII and the solution is allowed to warm to 25°C. The product is isolated by addition of aqueous acid and extraction into ether or methylene chloride. Evaporation of the solvent and crystallization or column chromatography on silica gel affords the aldehyde XXX.

Aldehydes of Formula XXX may also be prepared from the esters by treatment with diisobutylaluminum hydride according to the procedures of E. Winterfeldt, *Synthesis, 617* (1975).

Compounds of Formulas I, II and III may also be prepared by the reaction of the appropriately substituted thiophene or furan sulfonamides with the appropriate heterocyclic isocyanate using the methods described in our U.S. Patent Applications Serial Nos. 098,722, and 098,722 filed November 20, 1979 corresponding to European Patent Application of even date herewith).

According to this process a sulfonamide of formula

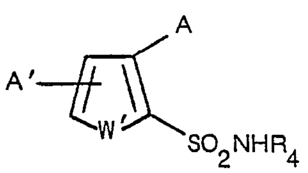

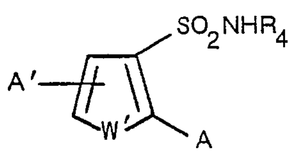

or

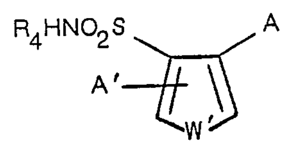

is reacted with an isocyanate of formula:

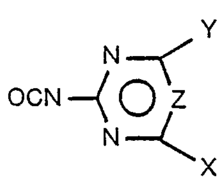

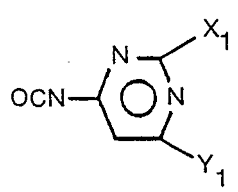

or

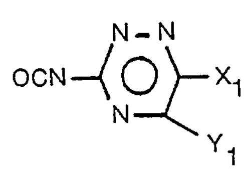

The reaction is best performed in an inert organic solvent e.g. acetonitrile, THF, toluene acetone or butanone, optionally in the presence of a catalytic amount of base and preferably at a temperature in the range 25 to 110°C.

The synthesis of heterocyclic amines has been reviewed in "The Chemistry of Heterocyclic Compounds" a series published by Interscience Publ., New York and London. 2-Aminopyrimidines are described by D. J. Brown in The Pyrimidines, Vol. XVI of this series. The 2-amino-1,3,5-triazines are reviewed by K. R. Huffman and in The Triazines of this same series. The synthesis of triazines are also described by F. C. Schaefer, U.S. Patent No. 3,154,547 and by K. R. Huffman and F. C. Schaeffer, J. Org. Chem. *28,* 1816—1821 (1963).

The preparation of the aminoheterocycles described by Formula XXX varies according to the definition of $Y_1$ and $X_{II}$.

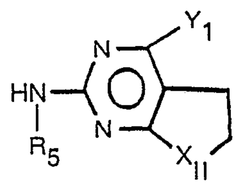

XXX

Braker, Sheehan, Spitzmiller and Lott, *J. Am. Chem. Soc. 69,* 3072 (1947) describe the preparation of 6,7-dihydro-4-methoxy-5H-cyclopentapyrimidin-2-amine by the following sequence of reactions.

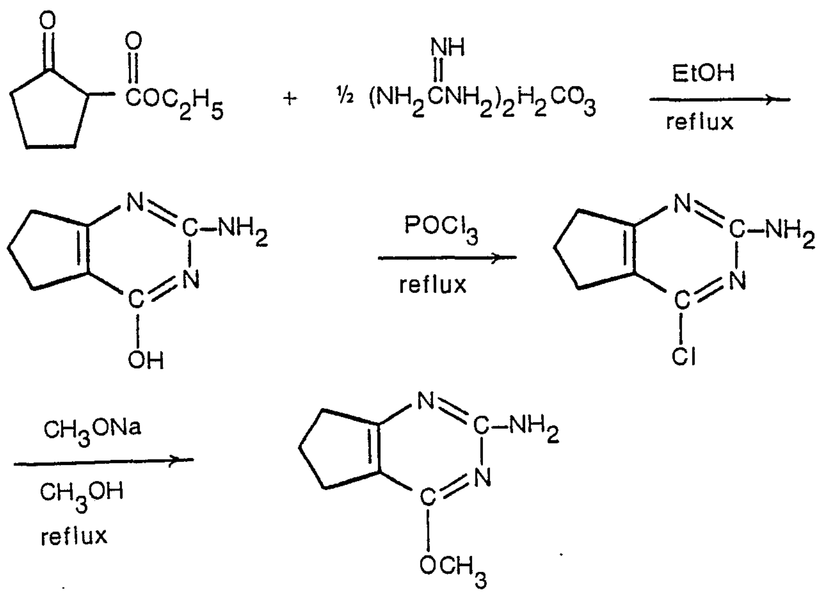


6,7-dihydro-4-methoxy-5H-cyclopentapyrimidin-2-amine

Similarly, 6,7-dihydro-4-methyl-5H-cyclopentapyrimidin-2-amine can be prepared by the condensation of 2-acetylcyclopentanone with guanidine carbonate, but preferably under acidic conditions, removing the water formed.

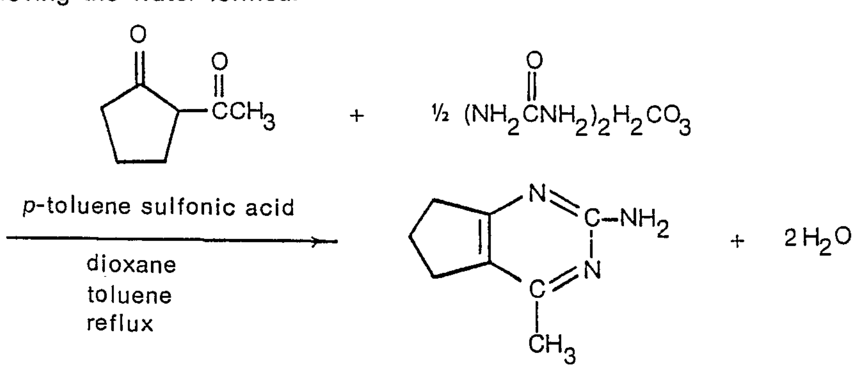


6,7-dihydro-4-methyl-5H-cyclopentapyrimidin-2-amine

Shrage and Hitchings, *J. Org. Chem. 16,* 1153 (1951) describe the preparation of 5,6-dihydro-4-methylfuro[2,3-d]pyrimidin-2-amine by the following sequence of reactions

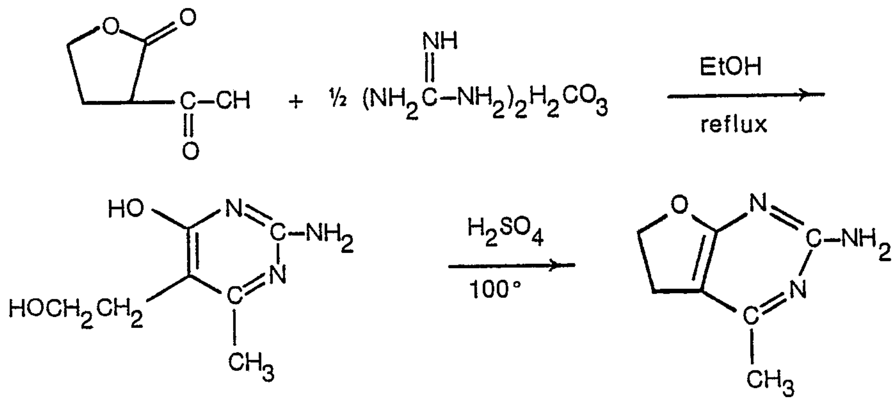


5,6-Dihydro-4-methoxyfuro[2,3-d]pyrimidin-2-amine can be prepared by the method of Braker et al., *J. Am. Chem. Soc. 69,* 3072 (1947), using 5,6-dihydro-4-hydroxyfuro[2,3-d]pyrimidin-2-amine [Svab, Budesinski and Vavrina, *Collection Czech. Chem. Commun. 32,* 1582 (1967)].

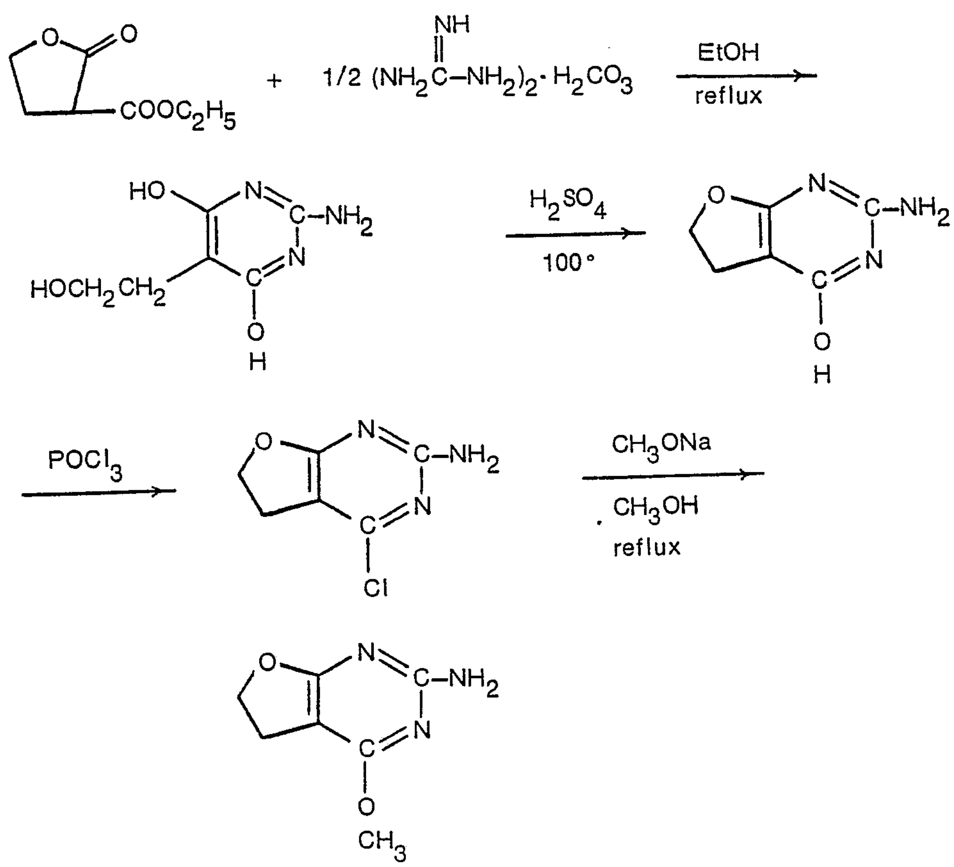

Caldwell, Kornfield and Donnell, *J. Am. Chem. Soc. 63,* 2188 (1941), describe the preparation of 6,7-dihydro-5H-cyclopentapyrimidin-2-amine by the following sequence of reactions.

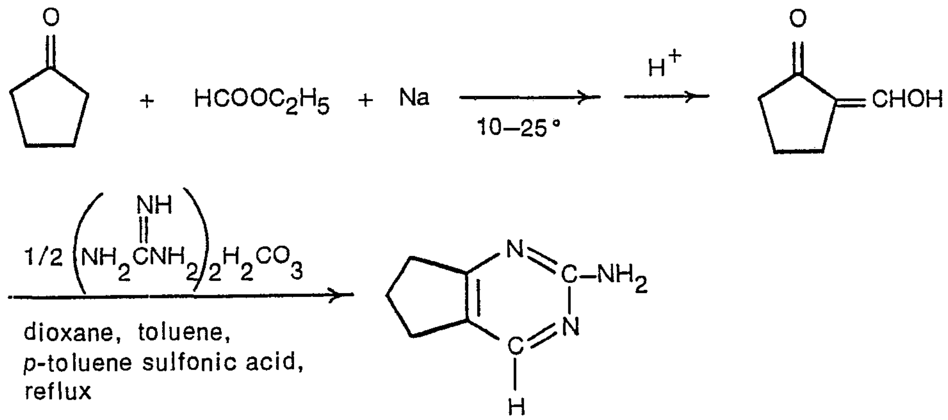


Fissekis, Myles and Brown, *J. Org. Chem. 29,* 2670 (1964), describe the preparation of 2-amino-4-hydroxy-5-(2-hydroxyethyl)pyrimidine which can be converted to 5,6-dihydrofuro[2,3-d]pyrimidin-2-amine by dehydration.

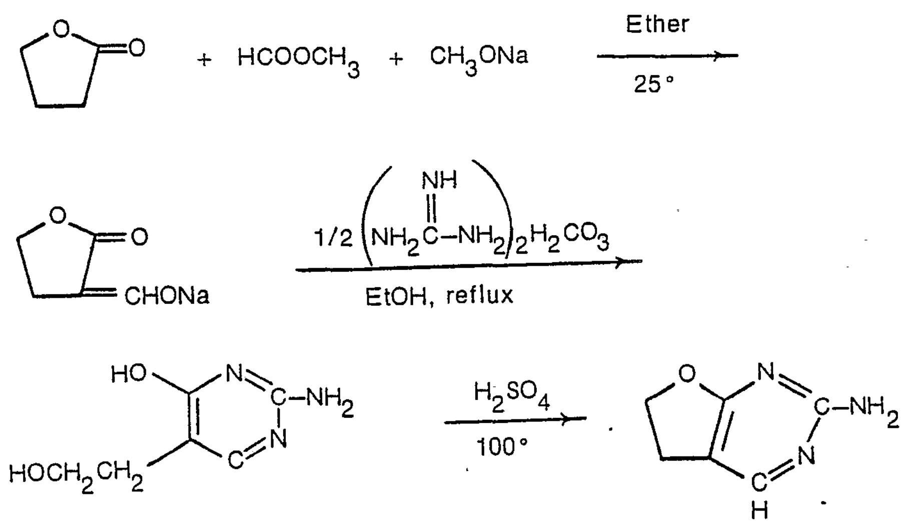


Agriculturally suitable salts of compounds of Formulas I—III are also useful herbicides and can be prepared by a number of ways known to the art. For example, metal salts can be made by treating compounds of Formulas I—III with a solution of alkali or alkaline earth metal salt having a sufficiently basic anion (e.g., hydroxide, alkoxide, carbonate or hydride). Quaternary amine salts can be made by similar techniques.

Salts of compounds of Formulas I—III can also be prepared by exchange of one cation to another. Cationic exchange can be effected by direct treatment of an aqueous solution of a salt of a compound of Formulas I—III (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formulas I—III (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water soluble, e.g., a potassium, sodium or calcium salt.

Acid addition salts, useful in this invention, can be obtained by reacting a compound of Formulas I—III with a suitable acid, e.g., *p*-toluenesulfonic acid, trichloroacetic acid or the like.

The compounds of this invention and their preparation are further illustrated by the following examples wherein temperatures are given in degrees centigrade.

The desired product is underscored at the top of each example.

Example 1

2-Methoxycarbonyl-3-thiophenesulfonyl isocyanate

A mixture containing 22.1 g of methyl-3-sulfamoylthiophene-2-carboxylate, 9.9 g of *n*-butyl isocyanate, 0.3 g of 1,4-diaza[2,2,2]bicyclooctane and 150 ml of dry xylene was placed in a 4 neck round bottom flask equipped with a gas inlet tube, mechanical stirrer, thermometer and dry ice cooled reflux condenser. This mixture was heated to 135°C and phosgene was passed into the flask so that after several minutes, the reflux temperature dropped to 120°C. The phosgene addition was halted until the temperature rose to 130 and then additional phosgene was added to cause the temperature to drop again to 120°. The phosgene addition cycle was repeated until the reflux temperature of the reaction mixture remained at 120° with no further phosgene addition.

Cooling the reaction mixture caused a small amount of a precipitate to form which was removed by filtration and the filtrate was concentrated *in vacuo* to yield an oil which showed a strong absorption peak in the infrared region at 2200 $cm^{-1}$ consistent for the desired sulfonyl isocyanate. This highly reactive intermediate was used without further purification.

Example 2

Methyl 3-[[(4,6-dimethylpyrimidin-2-yl)aminocarbonyl]aminosulfonyl]-2-thiophenecarboxylate

To 1.23 g of 2-amino-4,6-dimethyl pyrimidine in 30 ml of anhydrous acetonitrile was added with stirring 2.7 g of 2-methoxycarbonyl-3-thiophenesulfonylisocyanate. The mixture was heated to the boiling point, whereupon all of the insoluble material dissolved and the mixture was allowed to cool. After stirring for two hours the mixture was filtered to remove the desired product which had precipitated as a white solid. After washing with anhydrous ethyl ether the product melted at 191—193° with decomposition and showed absorption peaks by nuclear magnetic resonance at 3.8 ppm for the methoxy group, 2.44 ppm for the two methyl groups on the pyrimidine ring, a peak at 7.0 consistent for the hydrogen in the pyrimidine ring and a peak at 7.42 for the hydrogens on the thiophene ring. The infrared absorption spectrum showed absorption peaks at 1720 and 1700 $cm^{-1}$ consistent for the two carbonyl groups present in the desired product.

Example 3

Methyl 3 [[(4,6-dimethoxypyrimidin-2-yl)aminocarbonyl]aminosulfonyl]-2-thiophenecarboxylate

To 1.5 g of 2-amino-4,6-dimethoxypyrimidine in 30 ml of anhydrous acetonitrile was added 2.7 g of 2-methoxycarbonyl-3-thiophenesulfonyl isocyanate with stirring at ambient temperature. All of the solid reactant dissolved and after twenty minutes of stirring a precipitate started to form. After two hours the mixture was filtered and the solid which was washed with anhydrous ethyl ether, melted at 191—193°. The solid showed peaks by nuclear magnetic resonance spectroscopy at 4.0 ppm and 3.8 ppm for the methoxy groups, 6.0 ppm for the H of pyrimidine and 7.6 ppm for the hydrogens on thiophene. The infrared absorption spectrum showed absorption peaks at 1730 and 1700 $cm^{-1}$ consistent for the two carbonyl peaks in the desired product.

Example 4

Methyl 3-[[(4-methoxy-6-methylpyrimidin-2-yl)aminocarbonyl]aminosulfonyl]-2-thiophenecarboxylate

To 1.4 g of 2-amino-4-methoxy-6-methylpyrimidine in 30 ml of anhydrous acetonitrile was added at ambient temperature, with stirring 3.6 g of 2-methoxycarbonyl-3-thiophenesulfonylisocyanate. The mixture was heated to the boiling point and then allowed to cool to ambient temperature. After stirring for sixteen hours the precipitate present in the mixture was filtered off and washed with anhydrous ethyl ether. The product thus obtained which melted at 165—173° showed absorption peaks by infrared spectroscopy at 1720 and 1700 $cm^{-1}$, consistent for the carbonyl groups in the desired product.

Example 5

Methyl 3-[[(4,6-dimethyl-1,3,5-triazin-2-yl)aminocarbonyl]aminosulfonyl]-2-thiophenecarboxylate

To 1.23 g of 2-amino-4,6-dimethyl-1,3,5-triazine in 30 ml of anhydrous methylene chloride was added with stirring 2.7 g of 2-methoxycarbonyl-3-thiophenesulfonylisocyanate. The mixture was heated to the boiling point and allowed to cool and stir at ambient temperature for sixteen hours. The solid thus obtained was removed by filtration to yield 2.3 g of the crude desired product melting at 158—178°. The product showed peaks at 1720 and 1710 $cm^{-1}$, by infrared absorption spectroscopy, consistent for the desired product.

Example 6

Methyl 3-[[(5,6 dimethyl-1,2,4-triazinyl-2-yl)aminocarbonyl]aminosulfonyl]-2-thiophenecarboxylate

To 1.2 g of 3-amino-5,6-dimethyl-1,2,4-triazine in 30 ml of anhydrous methylene chloride was added with stirring 2.7 g of 2-methoxycarbonyl-3-thiophenesulfonylisocyanate. After stirring for 16 hours at ambient temperature the solution was filtered to remove some insoluble material and the filtrate evaporated to dryness. The residue thus obtained was triturated with ethyl ether and the insoluble product filtered to yield 2.9 g of the desired compound melting at 129° with decomposition.

Infrared analysis of this product showed absorption peaks at 1730 and 1700 $cm^{-1}$ as expected for the desired product.

Example 7

3-[[(4,6-Dimethylpyrimidin-2-yl)aminocarbonyl]aminosulfonyl]-2-thiophene carboxylic acid

A solution of 1.0 g of methyl 3-[[(4,6-dimethylpyrimidin-2-yl)aminocarbonyl]aminosulfonyl]-2-thiophenecarboxylate in 10 ml of ethanol and 1 ml of 50% sodium hydroxide in water was stirred overnight at room temperature. To this was added ice-water and aqueous hydrochloric acid until acidic, and the precipitate was filtered and washed first with acetone and then with methylene chloride. The product .8 g, which melted at 127°.

Example 8

1-[3-[[(4-methoxy-6-methylpyrimidin-2-yl)aminocarbonyl]aminosulfonyl]thiene-2-yl carbonyl] pyrrolidone

To 2.5 ml of a 2M-solution of trimethylaluminum in toluene was added 20 ml of methylene chloride and 400 $\mu$l of pyrrolidine. To this solution was added .66 g of methyl 3-[[(4-methoxy-6-methylpyrimidin-2-yl)amino carbonyl]aminosulfonyl]-2-thiophenecarboxylate. The resulting solution was stirred under nitrogen overnight at room temperature, quenched with 5N aqueous hydrochloric acid, and extracted with ethyl acetate. The residue obtained from evaporation of solvent was washed with ether to afford .6 g of product, mp 184—5°, which showed absorption peaks at 1.8—2.2 ppm and 3.2—3.8 ppm for the pyrrolidine ring and no methyl ester peak, and all other signals indicative of the desired product.

TABLE I—a

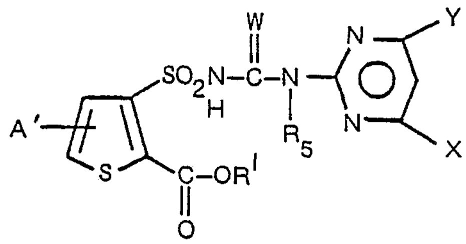

| A' | R' | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |

TABLE I—a (cont'd.)

| A' | $R^I$ | W | $R_5$ | X | Y |
|---|---|---|---|---|---|
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_1Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |
| H | $CH_3$ | O | H | $CH_3$ | $O(CH_2)_3O(CH_2)_2CH_3$ |
| H | $CH_3$ | O | H | $CH_3$ | 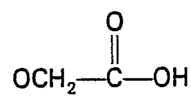 |
| H | $CH_3$ | O | H | $CH_3$ | 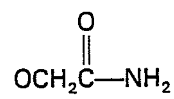 |
| H | $CH_3$ | O | H | $CH_3$ | 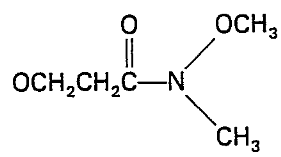 |
| H | $CH_3$ | O | H | $CH_3$ | 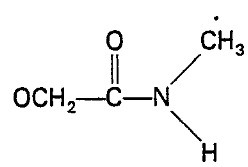 |
| H | $CH_3$ | O | H | $CH_3$ | 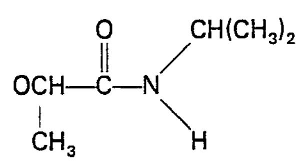 |
| H | $CH_3$ | O | H | $CH_3$ | 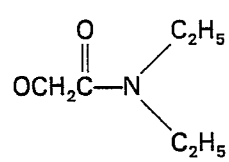 |
| H | $CH_3$ | O | H | $CH_3$ | 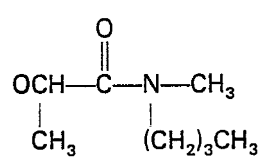 |

TABLE I—a (cont'd.)

| A′ | R' | W | $R_5$ | X | Y |
|---|---|---|---|---|---|
| H | $CH_3$ | O | H | $CH_3$ | 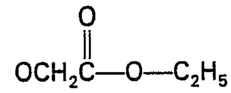 |
| H | $CH_3$ | O | H | $CH_3$ | 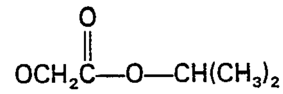 |
| H | $CH_3$ | O | H | $CH_3$ | 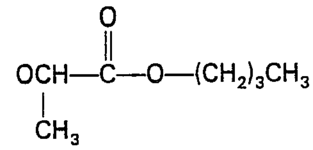 |
| H | $CH_3$ | O | H | $CH_3$ | 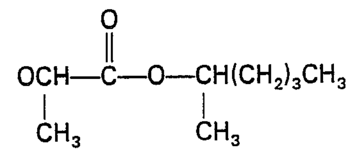 |
| H | $CH_3$ | O | H | $CH_3$ | SCN |
| H | $CH_3$ | O | H | $CH_3$ | $N_3$ |
| H | $CH_3$ | O | H | $CH_3$ | $NH_3$ |
| H | $CH_3$ | O | H | $CH_3$ | $NHCH_3$ |
| H | $CH_3$ | O | H | $CH_3$ | 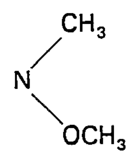 |
| H | $CH_3$ | O | H | $CH_3$ | 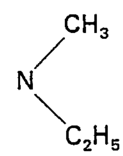 |
| H | $CH_3$ | O | H | $CH_3$ | 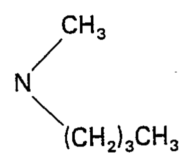 |
| H | $CH_3$ | O | H | $CH_3$ | 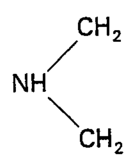 |
| H | $CH_3$ | O | H | $CH_3$ | 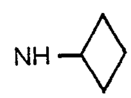 |

TABLE I—a (cont'd.)

| A' | $R^I$ | W | $R_5$ | X | Y |
|---|---|---|---|---|---|
| H | $CH_3$ | O | H | $CH_3$ | NH — 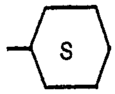 |
| H | $CH_3$ | O | H | $CH_3$ | $NH—CH_2CH{=}CH_2$ |
| H | $CH_3$ | O | H | $CH_3$ | $NH—CH_2CH{=}CHCH_2$ |
| H | $CH_3$ | O | H | $CH_3$ | $CH_3$<br>\|<br>$N—(CH_2)_2OCH_3$ |
| H | $CH_3$ | O | H | $CH_3$ | $CH_3$<br>\|<br>$N—CH—CH_2OCH_3$<br>\|<br>$CH_3$ |
| H | $CH_3$ | O | H | $CH_3$ | $NH—(CH_2)_2OC_2H_5$ |
| H | $CH_3$ | O | H | $CH_3$ | $CH_3$<br>\|<br>$N—CH_2CN$ |
| H | $CH_3$ | O | H | $CH_3$ | $NH—CH—CN$<br>\|<br>$CH_3$ |
| H | $CH_3$ | O | H | $CH_3$ | $CH_3$<br>\|<br>$N—CH—CO_2H$<br>\|<br>$CH_3$ |
| H | $CH_3$ | O | H | $CH_3$ | $NH—CH—CO_2CH_3$<br>\|<br>$CH_3$ |
| H | $CH_3$ | O | H | $CH_3$ | $CH_3$<br>\|<br>$N—CH_2CO_2C_2H_5$ |
| H | $CH_3$ | O | H | $CH_3$ | 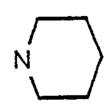 |
| H | $CH_3$ | O | H | $CH_3$ | 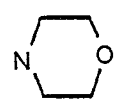 |
| H | $CH_3$ | O | H | $CH_3$ | $O—C_2H_5$ |
| H | $CH_3$ | O | H | $CH_3$ | O-*n*-$C_3H$— |
| H | $CH_3$ | O | H | $CH_3$ | $O—CH(CH_3)_2$ |

TABLE I—a (cont'd.)

| A' | R' | W | $R_5$ | X | Y |
|---|---|---|---|---|---|
| H | $CH_3$ | O | H | $CH_3$ | O—CH—$C_2H_5$<br>\|<br>$CH_3$ |
| H | $CH_3$ | O | H | $CH_3$ | O—$CH_2CF_3$ |
| H | $CH_3$ | O | H | $CH_3$ | O—$CH_2CH_2Cl$ |
| H | $CH_3$ | O | H | $CH_3$ | O—$CH_2CH_2Br$ |
| H | $CH_3$ | O | H | $CH_3$ | O—$CH_2CCl_3$ |
| H | $CH_3$ | O | H | $CH_3$ | O—$CH_2CN$ |
| H | $CH_3$ | O | H | $CH_3$ | O—CH—CN<br>\|<br>$CH_3$ |
| H | $CH_3$ | O | H | $CH_3$ | O—$CH_2$—CH=CH=$CH_3$ |
| H | $CH_3$ | O | H | $CH_3$ | O—$CH_2$—C≡CH |
| H | $CH_3$ | O | H | $CH_3$ | O—$CH_2$—C≡C—$CH_2Cl$ |
| H | $CH_3$ | O | H | $CH_3$ | 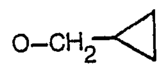 |
| H | $CH_3$ | O | H | $CH_3$ | 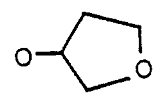 |
| H | $CH_3$ | O | H | $CH_3$ | S—$CH_3$ |
| H | $CH_3$ | O | H | $CH_3$ | S—$CH(CH_3)_2$ |
| H | $CH_3$ | O | H | $CH_3$ | S-$n$-$C_4H_9$ |
| H | $CH_3$ | O | H | $CH_3$ | S—$CH_2CH$—$CH_2$ |
| 5-$CH_3$ | $CH_3$ | O | H | $CH_3$ | $OCH_3$ |
| 5-$CH_3$ | $CH_3$ | O | H | $CH_3$ | $OCH_3$ |
| 5-Cl | $CH_3$ | O | H | $CH_3$ | $OCH_3$ |
| 5-Cl | $CH_3$ | O | H | $CH_3$ | $OCH_3$ |
| 5-Cl | $CH_3$ | O | H | $CH_3$ | $OCH_3$ |
| 5-Br | $CH_3$ | O | H | $CH_3$ | $OCH_3$ |
| 5-Br | $CH_3$ | O | H | $CH_3$ | $OCH_3$ |
| 5-Br | $CH_3$ | O | H | $CH_3$ | $OCH_3$ |
| 5-$C_2H_5$ | $CH_3$ | O | H | $CH_3$ | $OCH_3$ |
| 5-n-$C_4H_9$ | $CH_3$ | O | H | $CH_3$ | $OCH_3$ |
| H | $(CH_2)_5CN$ | O | H | $CH_3$ | $OCH_3$ |

TABLE I—a (cont'd.)

| A′ | R′ | W | $R_5$ | X | Y |
|---|---|---|---|---|---|
| H | $CH_2CH{=}CHCH_2Cl$ | O | H | $CH_3$ | $OCH_3$ |
| H | $CH_2CH{=}CH(CH_2)_2Cl$ | O | H | $CH_3$ | $OCH_3$ |
| H | $CH_2C{\equiv}CCH_2Cl$ | O | H | $CH_3$ | $OCH_3$ |
| H | $CH_2C{\equiv}C(CH_2)_3Cl$ | O | H | $CH_3$ | $OCH_3$ |
| H | 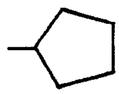 | O | H | $CH_3$ | $OCH_3$ |
| H | 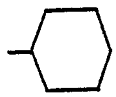 | O | H | $CH_3$ | $OCH_3$ |
| H | 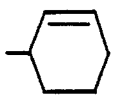 | O | H | $CH_3$ | $OCH_3$ |
| H | 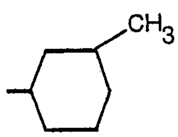 | O | H | $CH_3$ | $OCH_3$ |
| H | 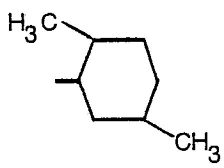 | O | H | $CH_3$ | $OCH_3$ |
| H | 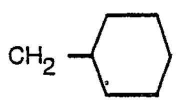 | O | H | $CH_3$ | $OCH_3$ |
| H | 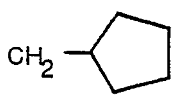 | O | H | $CH_3$ | $OCH_3$ |
| H | 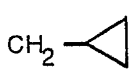 | O | H | $CH_3$ | $OCH_3$ |
| H | 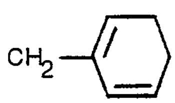 | O | H | $CH_3$ | $OCH_3$ |
| H | 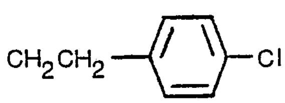 | O | H | $CH_3$ | $OCH_3$ |
| H | 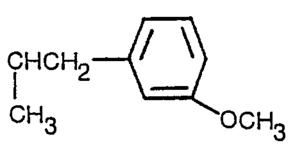 | O | H | $CH_3$ | $OCH_3$ |

TABLE I—a (cont'd.)

| A′ | R^I | W | $R_5$ | X | Y |
|---|---|---|---|---|---|
| H | $CH_3$ | O | H | $OCH_3$ | $S—CH_2—C≡CH$ |
| H | $CH_3$ | O | H | $OCH_3$ | S—CH—CN<br>\|<br>$CH_3$ |
| H | $CH_3$ | O | H | $OCH_3$ | $S—CH_2CN$ |
| H | $CH_3$ | S | H | $OCH_3$ | $CH_3$ |
| H | $CH_3$ | S | H | $CH_3$ | $CH_3$ |
| H | $CH_3$ | S | H | $OCH_3$ | $CH_3$ |
| H | $CH_3$ | S | H | $OCH_3$ | $CH_3$ |
| H | $CH_3$ | S | $CH_3$ | $OCH_3$ | $OCH_3$ |
| H | $CH_3$ | S | $CH_3$ | $CH_3$ | $OCH_3$ |
| H | $CH_3$ | S | H | $OCH_3$ | $OC_2H_5$ |
| H | $CH_3$ | O | $CH_3$ | $OCH_3$ | $OCH_3$ |
| H | $CH_3$ | O | $CH_3$ | $CH_3$ | $OCH_3$ |
| H | $CH_3$ | O | $CH_3$ | $CH_3$ | $CH_3$ |
| H | $CH_3$ | O | $CH_3$ | $CH_3$ | $OCH_2CO_2CH_3$ |
| 4-Cl | $CH_3$ | O | H | $CH_3$ | $OCH_3$ |
| 4-Cl | $CH(CH_3)_2$ | O | H | $CH_3$ | $OCH_3$ |
| 4-Cl | $CH_2CH{=}CH_2$ | O | H | $CH_3$ | $OCH_3$ |
| 4-Br | $C_2H_5$ | O | H | $CH_3$ | $OCH_3$ |
| 4-$CH_3$ | $CH_2CH_2Cl$ | O | H | $CH_3$ | $OCH_3$ |
| 4-$C_2H_5$ | $CH(CH_3)_2$ | O | H | $CH_3$ | $OCH_3$ |
| 4-*n*-$C_4H_9$ | $CH_2CH{=}CH_2$ | O | H | $CH_3$ | $OCH_3$ |
| 4-*i*-$C_3$H— | $CH_3$ | O | H | $CH_3$ | $OCH_3$ |
| 5-Cl | $C_2H_5$ | O | H | $CH_3$ | $OCH_3$ |
| 5-Br | $CH_3$ | O | H | $CH_3$ | $OCH_3$ |
| 5-$CH_3$ | $CH(CH_3)_2$ | O | H | $CH_3$ | $OCH_3$ |
| H | $C_2H_5$ | O | H | $CH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |
| H | $(CH_2)_3CH_3$ | O | H | $OCH_3$ | $OCH_3$ |
| H | $(CH_2)_4CH_3$ | O | H | $OCH_3$ | $OCH_3$ |

TABLE I—a (cont'd.)

| A' | R' | W | $R_5$ | X | Y |
|---|---|---|---|---|---|
| H | $CH(CH_2)_2CH_3$<br>$CH_3$ | O | H | $OCH_3$ | $OCH_3$ |
| H | $(CH_2)_5CH_3$ | O | H | $OCH_3$ | $OCH_3$ |
| H | $CH_2CH{=}CHCH_3$ | O | H | $OCH_3$ | $OCH_3$ |
| H | $CH_2CH{=}CHC_2H_5$ | O | H | $OCH_3$ | $OCH_3$ |
| H | $CH_2CH{=}CHCH(CH_3)_2$ | O | H | $OCH_3$ | $OCH_3$ |
| H | $(CH_2)_3Cl$ | O | H | $OCH_3$ | $OCH_3$ |
| H | $(CH_2)_5Cl$ | O | H | $OCH_3$ | $OCH_3$ |
| H | $(CH_2)_6OCH_3$ | O | H | $OCH_3$ | $OCH_3$ |
| H | $CH_2CN$ | O | H | $OCH_3$ | $OCH_3$ |
| 4-$OCH_3$ | $CH_3$ | O | H | $CH_3$ | $CH_3$ |
| 5-$OCH_3$ | $CH_3$ | O | H | $CH_3$ | $OCH_3$ |
| 4-$NO_2$ | $CH_3$ | O | H | $CH_3$ | $OCH_3$ |
| 5-$NO_2$ | $CH_3$ | O | H | $CH_3$ | $OCH_3$ |
| 4-$CF_3$ | $CH_3$ | O | H | $CH_3$ | $OCH_3$ |
| 5-$CF_3$ | $CH_3$ | O | H | $CH_3$ | $OCH_3$ |
| H | $CH_2CH_2CH_2Cl$ | O | H | $CH_3$ | $OCH_3$ |
| H | $CH_2CCl_3$ | O | H | $CH_3$ | $OCH_3$ |
| H | $(CH_2)_6Cl$ | O | H | $CH_3$ | $OCH_3$ |
| H | $(CH_2)_4Cl$ | O | H | $CH_3$ | $OCH_3$ |
| H | $CH_2CBr_3$ | O | H | $CH_3$ | $OCH_3$ |
| H | $(CH_2)_4Br$ | O | H | $CH_3$ | $OCH_3$ |
| H | $(CH_2)_6Br$ | O | H | $CH_3$ | $OCH_3$ |
| H | $(CH_2)_6F$ | O | H | $CH_3$ | $OCH_3$ |
| H | $(CH_2)_4F$ | O | H | $CH_3$ | $OCH_3$ |
| H | $(CH_2)_3F$ | O | H | $CH_3$ | $OCH_3$ |
| H | $CH_2CF_3$ | O | H | $CH_3$ | $OCH_3$ |
| H | $CH_2CN$ | O | H | $CH_3$ | $OCH_3$ |
| H | 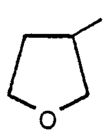 | O | H | $CH_3$ | $OCH_3$ |

TABLE I—a (cont'd.)

| A′ | R$^I$ | W | $R_5$ | X | Y |
|---|---|---|---|---|---|
| H | 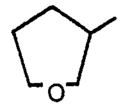 | O | H | $CH_3$ | $OCH_3$ |
| H | 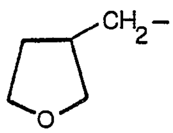 | O | H | $CH_3$ | $OCH_3$ |
| H | 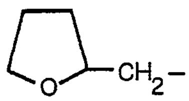 | O | H | $CH_3$ | $OCH_3$ |
| H | 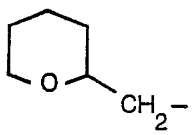 | O | H | $CH_3$ | $OCH_3$ |
| H | 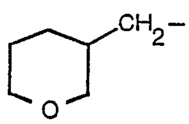 | O | H | $CH_3$ | $OCH_3$ |
| H | 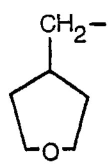 | O | H | $CH_3$ | $OCH_3$ |
| H | 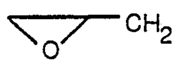 | O | H | $CH_3$ | $OCH_3$ |
| H | $—CH_2OCH_3$ | O | H | $CH_3$ | $OCH_3$ |
| H | $—CH_2OC_2H_5$ | O | H | $CH_3$ | $OCH_3$ |
| H | $—CH_2OCH(CH_3)_2$ | O | H | $CH_3$ | $OCH_3$ |
| H | $—CH_2O\text{-}n\text{-}C_4H_9$ | O | H | $CH_3$ | $OCH_3$ |
| H | H | O | H | $CH_3$ | $OCH_3$ |
| H | H | O | H | $CH_3$ | $OCH_3$ |
| H | H | O | H | $OCH_3$ | $OCH_3$ |

TABLE I—b

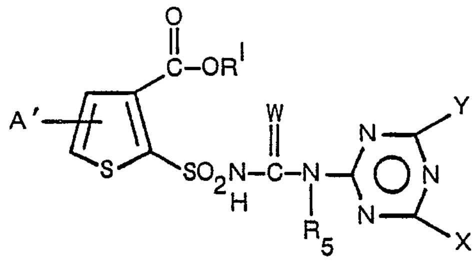

| A′ | R' | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—c

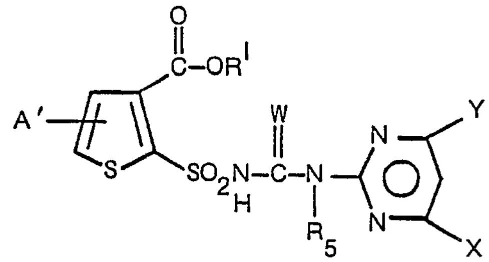

| A′ | $R^I$ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—d

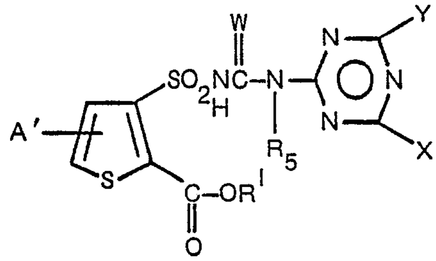

| A′ | R^I | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—e

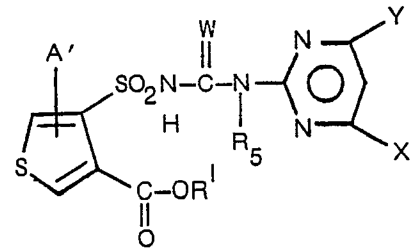

| A′ | R^I | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—f

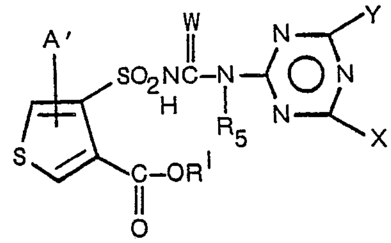

| A′ | $R^I$ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)-CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH=CH_2$ |
| H | $CH_3$ | O | H | $OCH_3$ | $CH_2CH=CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—g

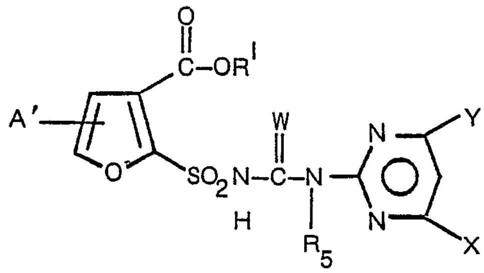

| A′ | R[I] | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)-CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—h

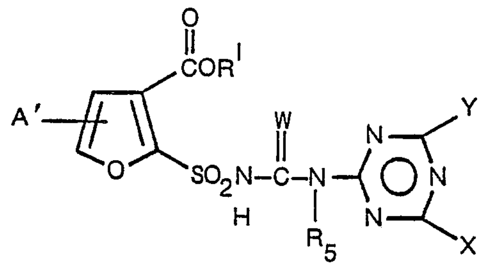

| A′ | $R^I$ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_5$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)—CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—i

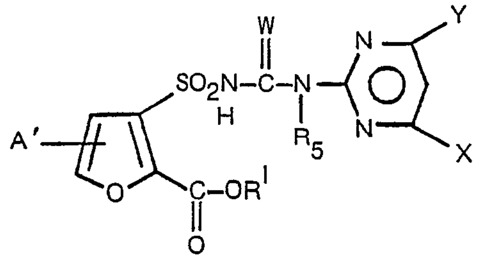

| A′ | R′ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)-CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—j

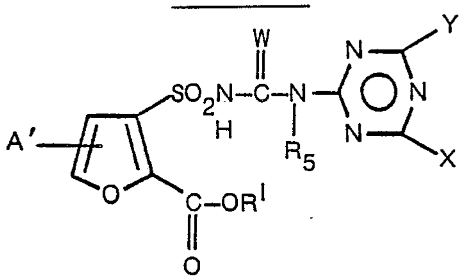

| A′ | R′ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)$—CN |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—k

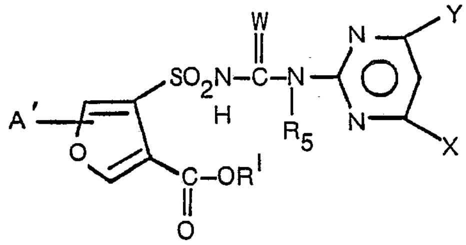

| A' | $R^I$ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)—CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH{=}CH_2$ |
| H | $CH_3$ | O | H | $OCH_3$ | $CH_2CH{=}CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE I—I

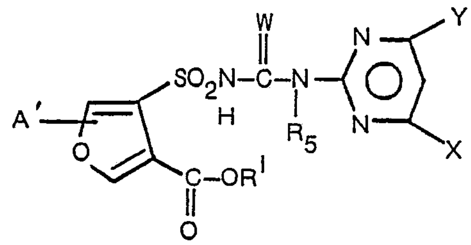

| A′ | $R^I$ | W | $R_5$ | X | Y |
|---|---|---|---|---|---|
| H | $CH_3$ | O | H | $CH_3$ | Cl |
| H | $CH_3$ | O | H | H | H |
| H | $CH_3$ | O | H | Cl | Cl |
| H | $CH_3$ | O | H | $OCH_2CH_3$ | $CH_2CH_3$ |
| H | $CH_3$ | O | H | $CH_3$ | $CH(CH_3)_2$ |
| H | $CH_3$ | O | H | $CH_3$ | $CH_2CH_3$ |
| H | $CH_3$ | O | H | $CH_3$ | $CH_2OCH_3$ |
| H | $CH_3$ | O | H | $CH_3$ | $CH_3CH_2OCH_3$ |
| H | $CH_3$ | O | H | $CH_3$ | $(CH_2)_4OCH_3$ |
| H | $CH_3$ | O | H | $CH_3$ | $(CH_2)_2OC_2H_5$ |
| H | $CH_3$ | O | H | $OCH_3$ | $CH_2CN$ |
| H | $CH_3$ | O | H | $OCH_3$ | $CH(CH_3)-CN$ |
| H | $CH_3$ | O | H | $OCH_3$ | $(CH_2)_3CO_2CH_3$ |
| H | $CH_3$ | O | H | $OCH_3$ | $CH_2CO_2C_2H_5$ |
| H | $CH_3$ | O | H | $OCH_3$ | $CH_2CF_3$ |
| H | $CH_3$ | O | H | $OCH_3$ | $CH_2CH_2Cl$ |
| H | $CH_3$ | O | H | $OCH_3$ | $CF_3$ |
| H | $CH_3$ | O | H | $OCH_3$ | $CH_2CCl_3$ |
| H | $CH_3$ | O | H | $OCH_3$ | $(CH_2)_4Cl$ |
| H | $CH_3$ | O | H | $OCH_3$ | $(CH_2)_3Br$ |
| H | $CH_3$ | O | H | $OCH_3$ | $CH_2CH=CH_2$ |
| H | $CH_3$ | O | H | $OCH_3$ | $CH_2CH=CHCH_2$ |
| H | $CH_3$ | O | H | $CH_3$ | $O(CH_2)_2OCH_3$ |
| H | $CH_3$ | O | H | $CH_3$ | $O(CH_2)_3OC_2H_5$ |
| H | $CH_3$ | O | H | $CH_3$ | $O(CH_2)_2OCH(CH_3)_2$ |

TABLE II—a

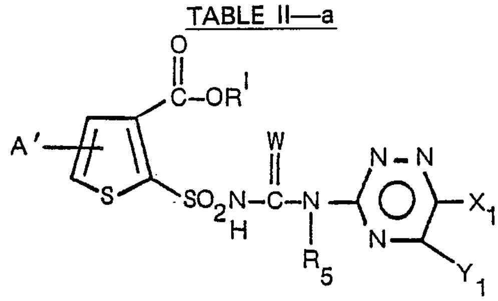

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—b

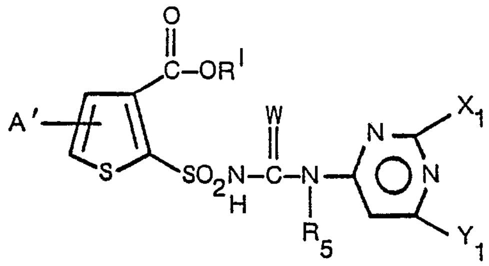

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—c

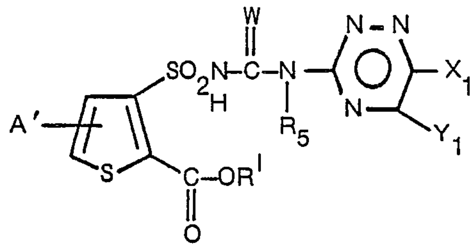

| A′ | R$^{I}$ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—d

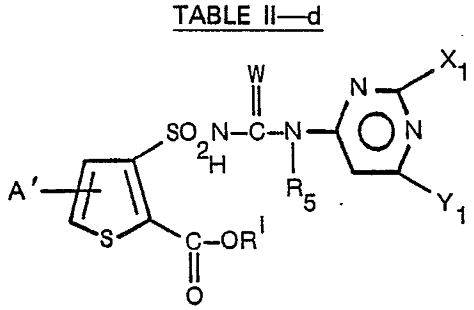

| A′ | $R^I$ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—e

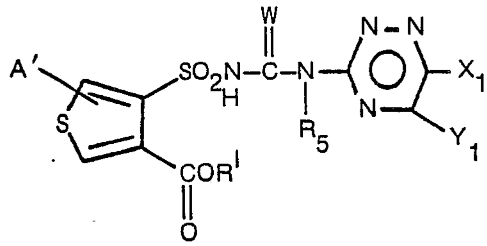

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—f

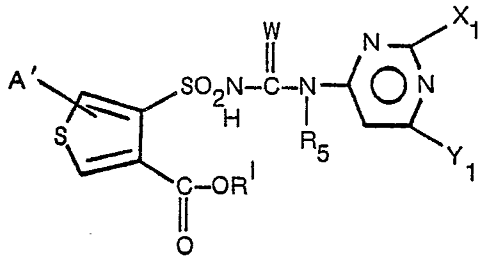

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—g

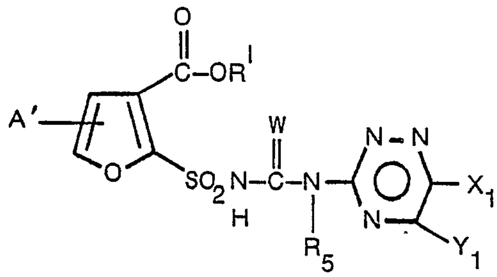

| A′ | $R^I$ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—h

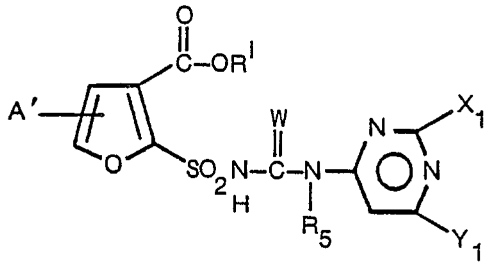

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—i

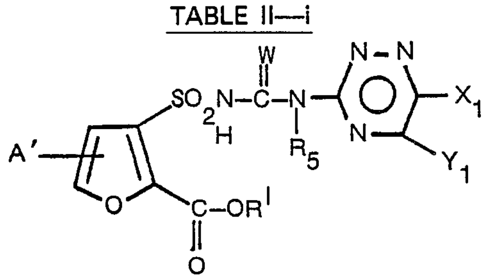

| A' | R' | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—j

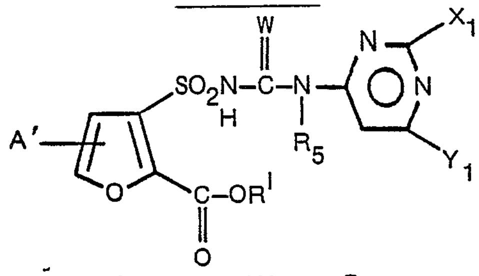

| A′ | R′ | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—k

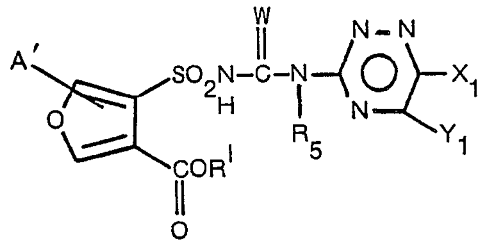

| A′ | R' | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE II—I

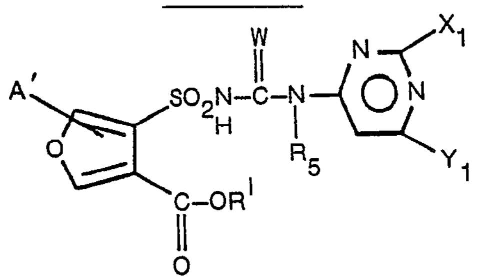

| A' | R' | W | $R_5$ | $X_1$ | $Y_1$ |
|---|---|---|---|---|---|
| H | $CH_3$ | O | H | H | H |
| H | $CH_3$ | O | H | H | $OCH_3$ |
| H | $CH_3$ | O | H | H | $CH_3$ |
| H | $CH_3$ | O | H | Cl | H |
| H | $CH_3$ | O | H | Cl | $OCH_3$ |
| H | $CH_3$ | O | H | Cl | $CH_3$ |
| H | $CH_3$ | O | H | $OCH_3$ | H |
| H | $CH_3$ | O | H | $OCH_3$ | $OCH_3$ |
| H | $CH_3$ | O | H | $OCH_3$ | $CH_3$ |
| H | $CH_3$ | O | H | $OC_2H_5$ | H |
| H | $CH_3$ | O | H | $OC_2H_5$ | $OCH_3$ |
| H | $CH_3$ | O | H | $OC_2H_5$ | $CH_3$ |
| H | $CH_3$ | O | H | $CH_3$ | H |
| H | $CH_3$ | O | H | $CH_3$ | $OCH_3$ |
| H | $CH_3$ | O | H | $CH_3$ | $CH_3$ |
| H | $CH_3$ | O | H | $OCH_3$ | $OCH_3$ |
| 5-Cl | $CH_3$ | O | H | $OCH_3$ | $OCH_3$ |
| 5-$CH_3$ | $CH_3$ | O | H | $OCH_3$ | $OCH_3$ |
| 5-Br | $CH_3$ | O | H | $OCH_3$ | $OCH_3$ |
| 5-$C_2H_5$ | $CH_3$ | O | H | $OCH_3$ | $OCH_3$ |
| H | $CH(CH_3)_2$ | O | CH | $OCH_3$ | $OCH_3$ |
| H | $CH_2CH{=}CH_2$ | O | CH | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $OCH_3$ |
| H | $CH_3$ | O | $CH_3$ | $OCH_3$ | $CH_3$ |
| H | $CH_3$ | S | H | $CH_3$ | $CH_3$ |
| H | $CH_3$ | S | H | $OCH_3$ | $OCH_3$ |
| H | $CH_3$ | S | H | H | H |
| H | $CH(CH_3)_2$ | S | H | $CH_3$ | $CH_3$ |

TABLE III—a

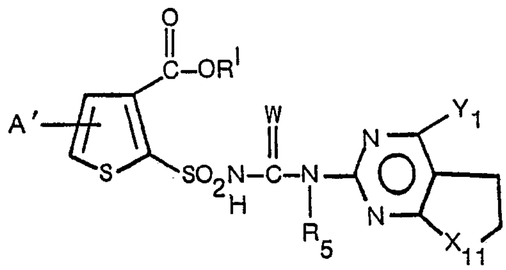

| A′ | R' | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE III—b

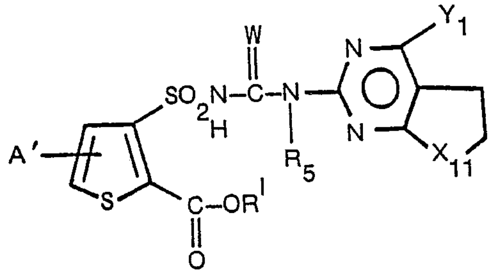

| A′ | $R^I$ | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE III—c

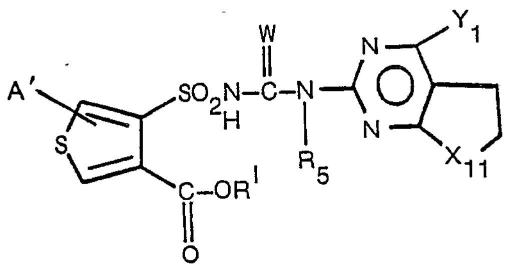

| A' | R' | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE III—d

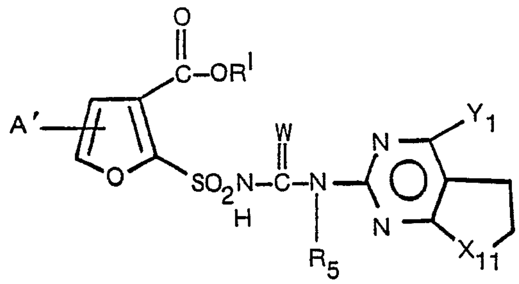

| A′ | $R^I$ | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE III—e

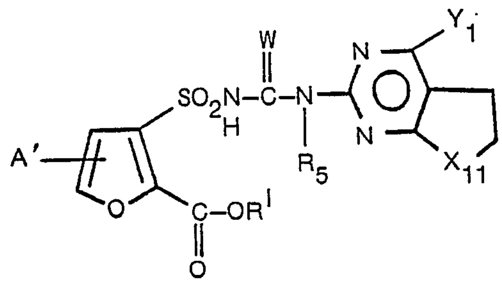

| A′ | $R^I$ | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE III—f

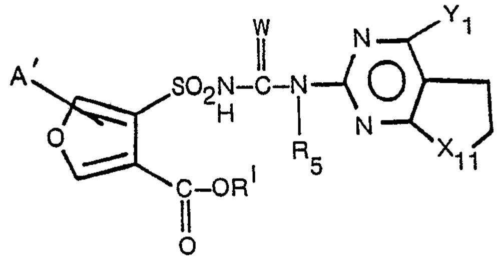

| A′ | R' | W | $R_5$ | $X_{11}$ | $Y_1$ |
|---|---|---|---|---|---|
| H | $CH_3$ | O | H | $CH_2$ | H |
| H | $CH_3$ | O | H | $CH_2$ | $OCH_3$ |
| H | $CH_3$ | O | H | $CH_2$ | $CH_3$ |
| H | $CH_3$ | O | H | O | H |
| H | $CH_3$ | O | H | O | $OCH_3$ |
| H | $CH_3$ | O | H | O | $CH_3$ |
| 5-$CH_3$ | $CH_3$ | O | H | O | $CH_3$ |
| 5-$CH_3$ | $CH_3$ | O | H | O | $CH_3$ |
| 5-Cl | $CH_3$ | O | H | O | $CH_3$ |
| 5-Cl | $CH_3$ | O | H | O | $CH_3$ |
| H | $CH_3$ | S | H | $CH_2$ | $OCH_3$ |
| H | $CH_3$ | S | H | O | $CH_3$ |
| H | $CH_3$ | S | H | O | $CH_3$ |
| H | $CH(CH_3)_2$ | S | H | O | $CH_3$ |
| H | $CH(CH_3)_2$ | O | H | O | $CH_3$ |
| H | $CH_3$ | O | $CH_3$ | O | $CH_3$ |
| H | $CH(CH_3)_2$ | O | $CH_3$ | O | $CH_3$ |

TABLE IV—a

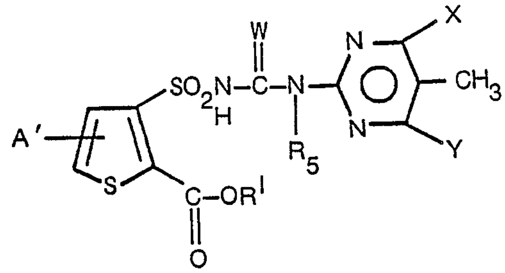

| A′ | R′ | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE IV—b

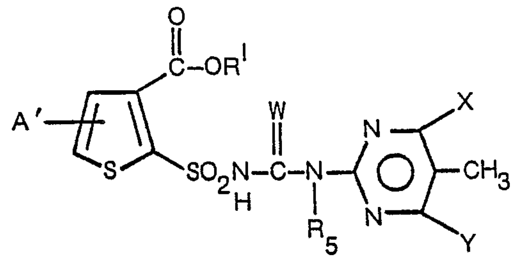

| A′ | $R^I$ | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE IV—c

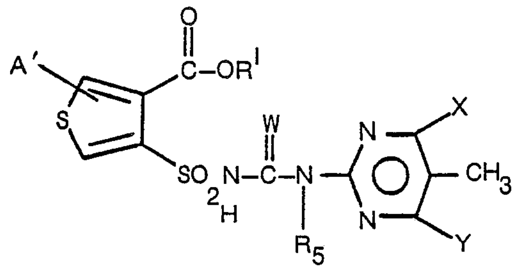

| A′ | $R^I$ | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE IV—d

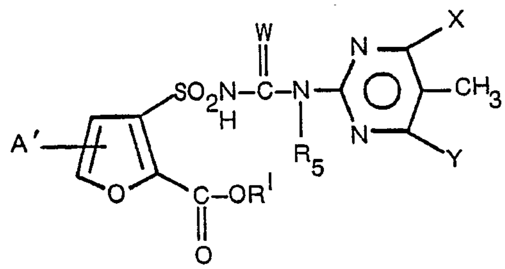

| A′ | R$^{I}$ | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE IV—e

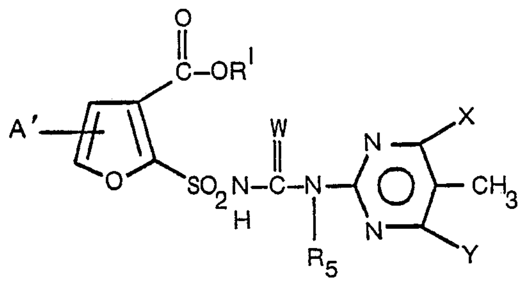

| A′ | $R^I$ | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE IV—f

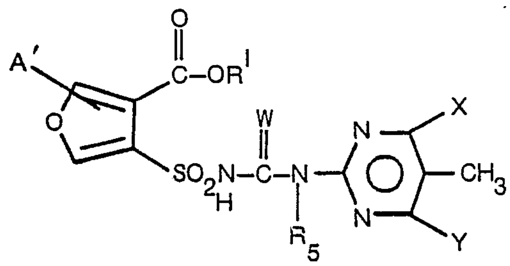

| A' | R' | W | $R_5$ | X | Y |
|---|---|---|---|---|---|
| H | $CH_3$ | O | H | $CH_3$ | $CH_3$ |
| H | $CH_3$ | O | H | $OCH_3$ | $CH_3$ |
| H | $CH_3$ | O | H | $OCH_3$ | $OCH_3$ |
| H | $CH_3$ | O | H | $CH_3$ | $CH_2OCH_3$ |
| H | $CH_3$ | O | H | $OC_2H_5$ | $C_2H_5$ |
| H | $CH_3$ | S | H | $OCH_3$ | $CH_3$ |
| H | $CH_3$ | S | H | $CH_3$ | $CH_3$ |
| H | $CH_3$ | O | $CH_3$ | $CH_3$ | $OCH_3$ |
| H | $CH_3$ | O | $CH_3$ | $OCH_3$ | $OCH_3$ |
| 5-Cl | $CH_3$ | O | H | $OCH_3$ | $OCH_3$ |
| 5-$CH_3$ | $CH_3$ | O | H | $CH_3$ | $CH_3$ |
| 5-$CH_3$ | $CH_3$ | O | H | $OCH_3$ | $OCH_3$ |
| H | $C_2H_5$ | O | H | $OCH_3$ | $OCH_3$ |
| H | $CH(CH_3)_2$ | O | H | $OCH_3$ | $OCH_3$ |
| H | $CH_2CH{=}CH_2$ | O | H | $OCH_3$ | $OCH_3$ |

TABLE V

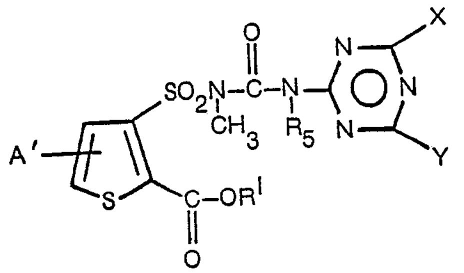

| A′ | R′ | $R_5$ | X | Y | Z |
|---|---|---|---|---|---|
| H | $CH_3$ | H | $CH_3$ | $CH_3$ | CH |
| H | $CH_3$ | H | $CH_3$ | $OCH_3$ | CH |
| H | $CH_3$ | H | $CH_3$ | $OC_2H_5$ | CH |
| H | $CH_3$ | H | $OCH_3$ | $OCH_3$ | CH |
| H | $CH(CH_3)_2$ | H | $OCH_3$ | $CH_3$ | N |
| H | $CH_2CH_2Cl$ | H | $OCH_3$ | $OCH_3$ | N |
| H | $CH_3$ | H | $CH_3$ | $CH_3$ | N |
| H | $CH_3$ | H | $CH_3$ | $CH_3$ | $CCH_3$ |
| H | $CH_3$ | H | $CH_3$ | $OCH_3$ | $CCH_3$ |

TABLE VI

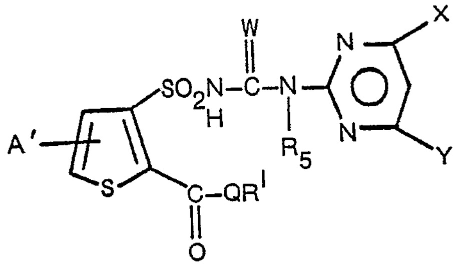

| A′ | QR′ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH(CH_3)—C_2H_5$ | O | H | $CH_3$ | $OCH_3$ |
| H | $SCH(CH_3)—CH(CH_3)_2$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 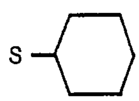 | O | H | $CH_3$ | $OCH_3$ |
| H | 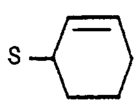 | O | H | $CH_3$ | $OCH_3$ |
| H | 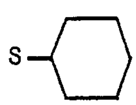 | O | H | $CH_3$ | $OCH_3$ |
| H | 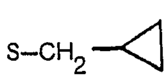 | O | H | $CH_3$ | $OCH_3$ |
| H | 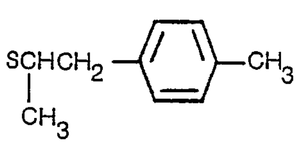 | O | H | $CH_3$ | $OCH_3$ |

TABLE VI (cont'd)

| A' | QR' | W | $R_5$ | X | Y |
|---|---|---|---|---|---|
| H | $NH_2$ | O | H | $CH_3$ | $OCH_3$ |
| H | 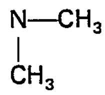 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 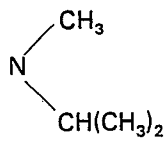 | O | H | $CH_3$ | $OCH_3$ |
| H | $N(CH_2CH{=}CH_2)_2$ | O | H | $CH_3$ | $OCH_3$ |
| 5-Cl | 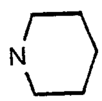 | O | H | $CH_3$ | $OCH_3$ |
| 5-Cl | 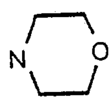 | O | H | $CH_3$ | $OCH_3$ |
| 5-Br | $NHCH_3$ | O | H | $CH_3$ | $CH_3$ |
| 5-$CH_3$ | $NHC_2H_5$ | O | H | $CH_3$ | $CH_3$ |
| 5-$CH_3$ | $NHCH(CH_3)_2$ | O | H | $CH_3$ | $CH_3$ |
| H | 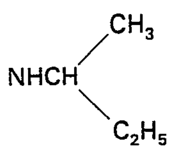 | O | H | $CH_3$ | $CH_3$ |
| H | $NH(CH_2)_5CH_3$ | O | H | $CH_3$ | $CH_3$ |
| H | $NH(CH_2)OCH_3$ | O | H | $CH_3$ | $CH_3$ |
| H | $NH(CH_2)_3OC_2H_5$ | O | H | $CH_3$ | $CH_3$ |
| H | $NH(CH_2)_2OCH(CH_3)_2$ | O | H | $CH_3$ | $CH_3$ |
| H | 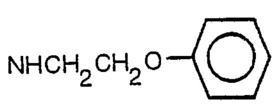 | O | H | $CH_3$ | $CH_3$ |
| H | $NHCH_2CH{=}CH_2$ | O | $CH_3$ | $CH_3$ | $CH_3$ |
| H | $NHCH_2CH{=}CHC_2H_5$ | O | H | $CH_3$ | $CH_3$ |
| H | 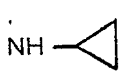 | O | H | $CH_3$ | $CH_3$ |

TABLE VI (cont'd)

| A' | QR' | W | $R_5$ | X | Y |
|---|---|---|---|---|---|
| H | 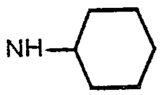 | O | $CH_3$ | $CH_3$ | $CH_3$ |
| H | 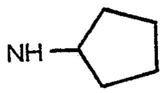 | O | H | $CH_3$ | $CH_3$ |
| H | 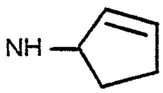 | O | H | $CH_3$ | $CH_3$ |
| H | 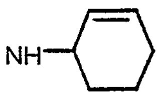 | O | H | $CH_3$ | $CH_3$ |
| H | 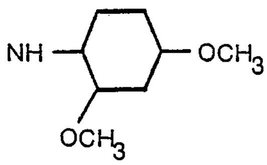 | O | H | $CH_3$ | $CH_3$ |
| H | 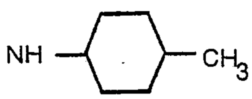 | O | H | $CH_3$ | $CH_3$ |
| H | 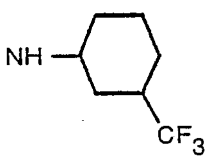 | O | H | $CH_3$ | $CH_3$ |
| H | 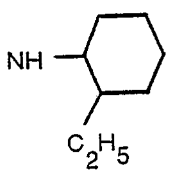 | O | H | $CH_3$ | $CH_3$ |
| H | $CH_3$<br>$N—CH_2CN$ | O | H | $CH_3$ | $CH_3$ |
| H | $CH_3$<br>$N—C(CH_3)_2CN$ | O | H | $CH_3$ | $CH_3$ |
| H | $CH_3$<br>$N—OCH_3$ | O | H | $CH_3$ | $CH_3$ |
| H | 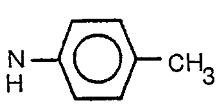 | O | H | $CH_3$ | $OCH_3$ |

TABLE VI (cont'd)

| A' | QR' | W | $R_5$ | X | Y |
|---|---|---|---|---|---|
| H | 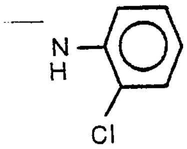 | O | H | $CH_3$ | $OCH_3$ |
| H | 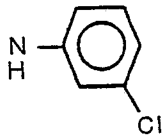 | O | H | $CH_3$ | $OCH_3$ |
| H | 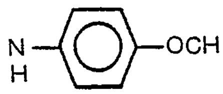 | O | H | $CH_3$ | $OCH_3$ |
| H | 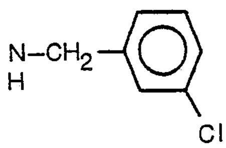 | O | H | $CH_3$ | $OCH_3$ |
| H | 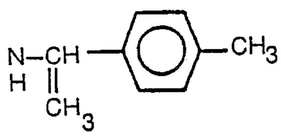 | O | H | $CH_3$ | $OCH_3$ |
| H | 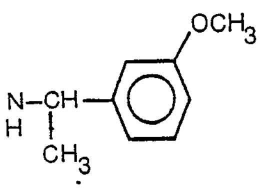 | O | H | $CH_3$ | $OCH_3$ |
| H | 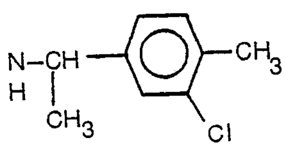 | O | H | $CH_3$ | $OCH_3$ |

TABLE VI-a

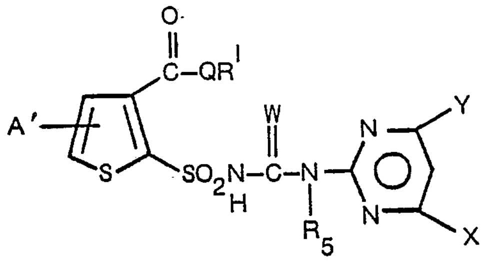

| A' | QR' | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$ (with $CH_3$ on CH) | O | H | $CH_3$ | $OCH_3$ |
| H | $SCH—CH(CH_3)_2$ (with $CH_3$ on CH) | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 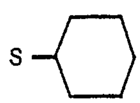 | O | H | $CH_3$ | $OCH_3$ |
| H | 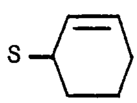 | O | H | $CH_3$ | $OCH_3$ |
| H | 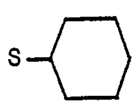 | O | H | $CH_3$ | $OCH_3$ |
| H | 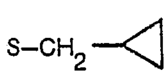 | O | H | $CH_3$ | $OCH_3$ |
| H | 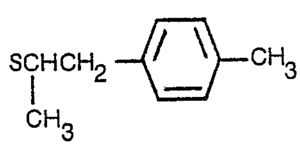 | O | H | $CH_3$ | $OCH_3$ |

TABLE VI-a (cont'd.)

| A' | $QR^I$ | W | $R_5$ | X | Y |
|---|---|---|---|---|---|
| H | $NH_2$ | O | H | $CH_3$ | $OCH_3$ |
| H | $N—CH_3$<br>\|<br>$CH_3$ | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N({}_2H_5)_2$ | O | H | $CH_3$ | $OCH_3$ |
| H | 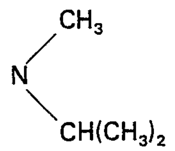 | O | H | $CH_3$ | $OCH_3$ |

TABLE VI-b

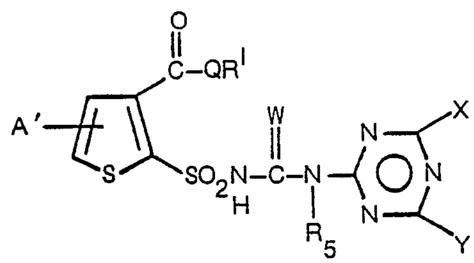

| A' | $QR^I$ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$<br>\|<br>$CH_3$ | O | H | $CH_3$ | $OCH_3$ |
| H | $SCH—CH(CH_3)_2$<br>\|<br>$CH_3$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C≡CC—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| $5-CH_3$ | $SCH_2CH=CHCH_2Cl$ | O | H | $CH_3$ | $OCH_3$ |

TABLE VI-b (cont'd.)

| A' | QR' | W | $R_5$ | X | Y |
|---|---|---|---|---|---|
| $5\text{-}C_2H_5$ | $SCH_2C{\equiv}CCH_2CH_2Cl$ | O | H | $CH_3$ | $OCH_3$ |
| H | 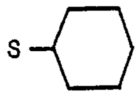 | O | H | $CH_3$ | $OCH_3$ |
| H | 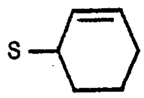 | O | H | $CH_3$ | $OCH_3$ |
| H | 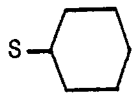 | O | H | $CH_3$ | $OCH_3$ |
| H | 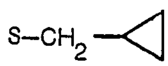 | O | H | $CH_3$ | $OCH_3$ |
| H | 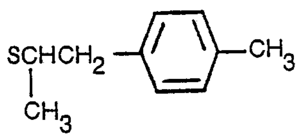 | O | H | $CH_3$ | $OCH_3$ |
| H | $NH_2$ | O | H | $CH_3$ | $OCH_3$ |
| H | 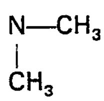 | O | H | $CH_3$ | $OCH_3$ |
| H | $N(CH_2CH)_2$ | O | H | $CH_3$ | $OCH_3$ |
| H | $N(CH_2CH_2CN)$ | O | H | $CH_3$ | $OCH_3$ |
| H | $N(C_2H_5)_2$ | O | H | $CH_3$ | $OCH_3$ |
| H | 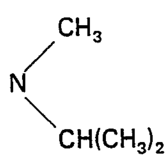 | O | H | $CH_3$ | $OCH_3$ |

TABLE VI-c

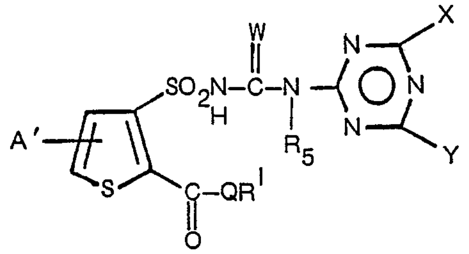

| A′ | QR′ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$ with $CH_3$ on the CH | O | H | $CH_3$ | $OCH_3$ |
| H | $SCH—CH(CH_3)_2$ with $CH_3$ on the CH | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 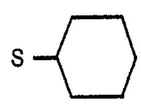 | O | H | $CH_3$ | $OCH_3$ |
| H | 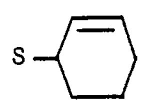 | O | H | $CH_3$ | $OCH_3$ |
| H | 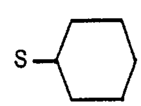 | O | H | $CH_3$ | $OCH_3$ |
| H | 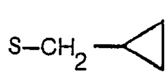 | O | H | $CH_3$ | $OCH_3$ |
| H | 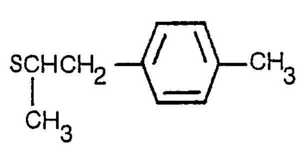 | O | H | $CH_3$ | $OCH_3$ |

TABLE VI-c (cont'd.)

| A' | QR' | W | $R_5$ | X | Y |
|---|---|---|---|---|---|
| H | $NH_2$ | O | H | $CH_3$ | $OCH_3$ |
| H | $N(CH_3)-CH_3$ | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | $N(CH_3)CH(CH_3)_2$ | O | H | $CH_3$ | $OCH_3$ |

TABLE VI-d

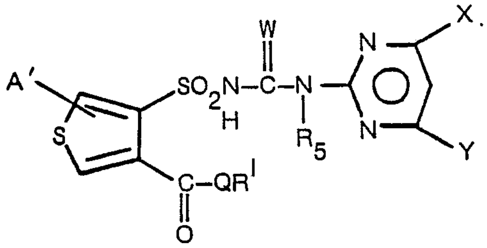

| A' | QR' | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH(CH_3)-C_2H_5$ | O | H | $CH_3$ | $OCH_3$ |
| H | $SCH(CH_3)-CH(CH_3)_2$ | O | H | $CH_3$ | $OCH_3$ |
| H | $SCH_2CH-CH_2$ | O | H | $CH_3$ | $OCH_3$ |
| H | $SCH_2CH-CHC_2H_5$ | O | H | $CH_3$ | $OCH_3$ |
| H | $SCH_2-C\equiv C-C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH=CHCH_2Cl$ | O | H | $CH_3$ | $OCH_3$ |

TABLE VI-d (cont'd.)

| A′ | QR′ | W | $R_5$ | X | Y |
|---|---|---|---|---|---|
| 5-$C_2H_5$ | $SCH_2C{\equiv}CCH_2CH_2Cl$ | O | H | $CH_3$ | $OCH_3$ |
| H | 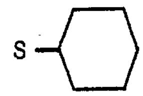 | O | H | $CH_3$ | $OCH_3$ |
| H | 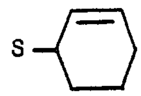 | O | H | $CH_3$ | $OCH_3$ |
| H | 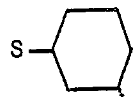 | O | H | $CH_3$ | $OCH_3$ |
| H | 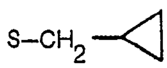 | O | H | $CH_3$ | $OCH_3$ |
| H | 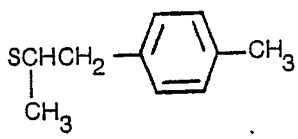 | O | H | $CH_3$ | $OCH_3$ |
| H | $NH_2$ | O | H | $CH_3$ | $OCH_3$ |
| H | 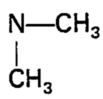 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 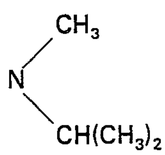 | O | H | $CH_3$ | $OCH_3$ |

TABLE VI-e

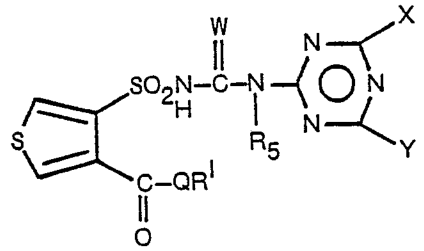

| A' | QR' | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$ with $CH_3$ on CH | O | H | $CH_3$ | $OCH_3$ |
| H | $SCH—CH(CH_3)_2$ with $CH_3$ on CH | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C\equiv C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C\equiv CCH_2CH_2Cl$ | O | H | $CH_3$ | $OCH_3$ |
| H | 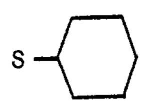 | O | H | $CH_3$ | $OCH_3$ |
| H | 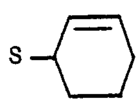 | O | H | $CH_3$ | $OCH_3$ |
| H | 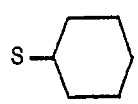 | O | H | $CH_3$ | $OCH_3$ |
| H | 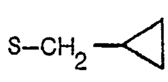 | O | H | $CH_3$ | $OCH_3$ |
| H | 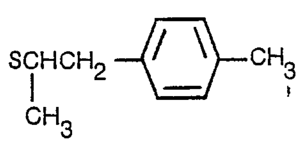 | O | H | $CH_3$ | $OCH_3$ |

TABLE VI-e (cont'd.)

| A' | QR' | W | $R_5$ | X | Y |
|---|---|---|---|---|---|
| H | $NH_2$ | O | H | $CH_3$ | $OCH_3$ |
| H | $N—CH_3$<br>\|<br>$CH_3$ | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 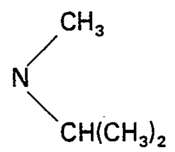 | O | H | $CH_3$ | $OCH_3$ |

TABLE VI-f

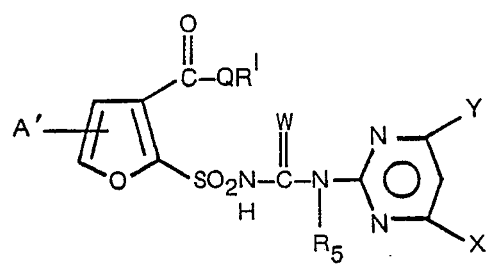

| A' | QR' | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$<br>\|<br>$CH_3$ | O | H | $CH_3$ | $OCH_3$ |
| H | $SCH—CH(CH_3)_2$<br>\|<br>$CH_3$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |

TABLE VI-f (cont'd.)

| A' | QR' | W | $R_5$ | X | Y |
|---|---|---|---|---|---|
| 5-$C_2H_5$ | $SCH_2C{\equiv}CCH_2CH_2Cl$ | O | H | $CH_3$ | $OCH_3$ |
| H | 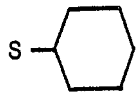 | O | H | $CH_3$ | $OCH_3$ |
| H | 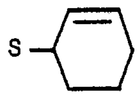 | O | H | $CH_3$ | $OCH_3$ |
| H | 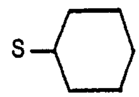 | O | H | $CH_3$ | $OCH_3$ |
| H | 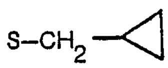 | O | H | $CH_3$ | $OCH_3$ |
| H | 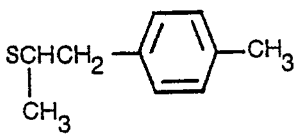 | O | H | $CH_3$ | $OCH_3$ |
| H | $NH_2$ | O | H | $CH_3$ | $OCH_3$ |
| H | 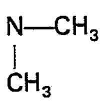 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 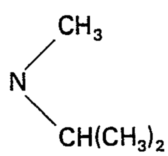 | O | H | $CH_3$ | $OCH_3$ |

TABLE VI-g

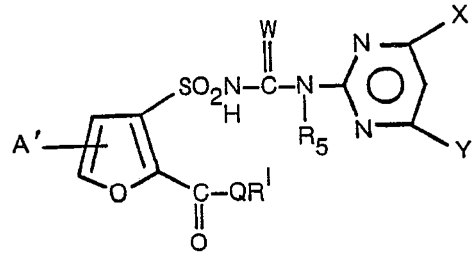

| A′ | QR′ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH(CH_3)—C_2H_5$ | O | H | $CH_3$ | $OCH_3$ |
| H | $SCH(CH_3)—CH(CH_3)_2$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 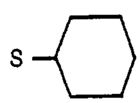 | O | H | $CH_3$ | $OCH_3$ |
| H | 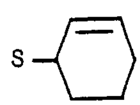 | O | H | $CH_3$ | $OCH_3$ |
| H | 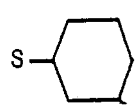 | O | H | $CH_3$ | $OCH_3$ |
| H | 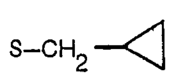 | O | H | $CH_3$ | $OCH_3$ |
| H | 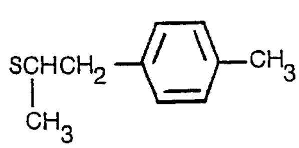 | O | H | $CH_3$ | $OCH_3$ |

TABLE VI-g (cont'd)

| A′ | QR$^I$ | W | $R_5$ | X | Y |
|---|---|---|---|---|---|
| H | $NH_2$ | O | H | $CH_3$ | $OCH_3$ |
| H | $N—CH_3$<br>$CH_3$ | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 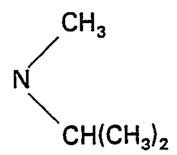 | O | H | $CH_3$ | $OCH_3$ |

TABLE VI-h

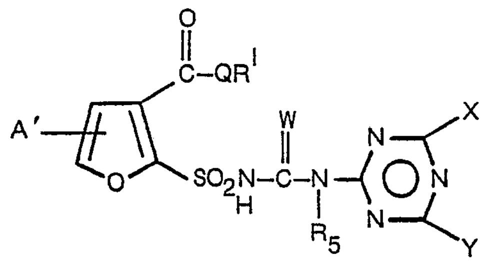

| A′ | QR$^I$ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$<br>$CH_3$ | O | H | $CH_3$ | $OCH_3$ |
| H | $SCH—CH(CH_3)_2$<br>$CH_3$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |

TABLE VI-h (cont'd.)

| A' | QR' | W | $R_5$ | X | Y |
|---|---|---|---|---|---|
| 5-$C_2H_5$ | $SCH_2C{\equiv}CCH_2CH_2Cl$ | O | H | $CH_3$ | $OCH_3$ |
| H | 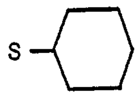 | O | H | $CH_3$ | $OCH_3$ |
| H | 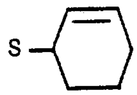 | O | H | $CH_3$ | $OCH_3$ |
| H | 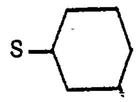 | O | H | $CH_3$ | $OCH_3$ |
| H | 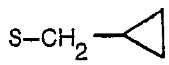 | O | H | $CH_3$ | $OCH_3$ |
| H | 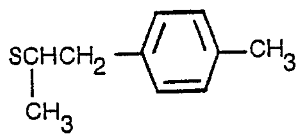 | O | H | $CH_3$ | $OCH_3$ |
| H | $NH_2$ | O | H | $CH_3$ | $OCH_3$ |
| H | 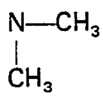 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 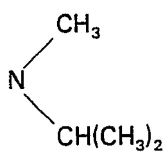 | O | H | $CH_3$ | $OCH_3$ |

TABLE VI-i

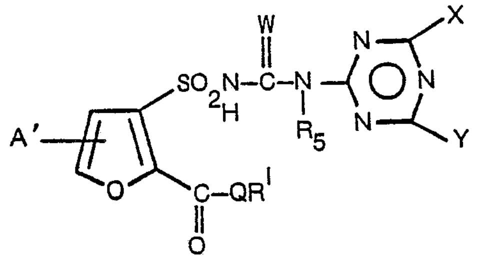

| A′ | QR$^{I}$ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$ (with $CH_3$ on CH) | O | H | $CH_3$ | $OCH_3$ |
| H | $SCH—CH(CH_3)_2$ (with $CH_3$ on CH) | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 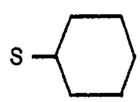 | O | H | $CH_3$ | $OCH_3$ |
| H | 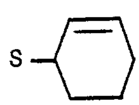 | O | H | $CH_3$ | $OCH_3$ |
| H | 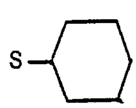 | O | H | $CH_3$ | $OCH_3$ |
| H | 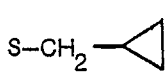 | O | H | $CH_3$ | $OCH_3$ |
| H | 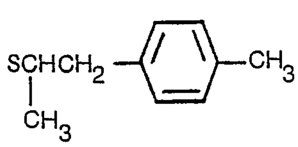 | O | H | $CH_3$ | $OCH_3$ |

TABLE VI-i (cont'd.)

| A' | QR$^I$ | W | $R_5$ | X | Y |
|---|---|---|---|---|---|
| H | $NH_2$ | O | H | $CH_3$ | $OCH_3$ |
| H | N—$CH_3$<br>\|<br>$CH_3$ | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | $CH_3$<br>/<br>N<br>\\<br>$CH(CH_3)_2$ | O | H | $CH_3$ | $OCH_3$ |

TABLE VI-j

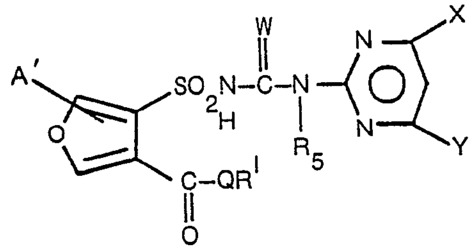

| A' | QR$^I$ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | SCH—$C_2H_5$<br>\|<br>$CH_3$ | O | H | $CH_3$ | $OCH_3$ |
| H | SCH—$CH(CH_3)_2$<br>\|<br>$CH_3$ | O | H | $CH_3$ | $OCH_3$ |
| H | $SCH_2CH$—$CH_2$ | O | H | $CH_3$ | $OCH_3$ |
| H | $SCH_2CH$—$CHC_2H_5$ | O | H | $CH_3$ | $OCH_3$ |
| H | $SCH_2$—C≡C—$C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |

TABLE VI-j (cont'd.)

| A' | QR' | W | $R_5$ | X | Y |
|---|---|---|---|---|---|
| 5-$C_2H_5$ | $SCH_2C{\equiv}CCH_2CH_2Cl$ | O | H | $CH_3$ | $OCH_3$ |
| H | 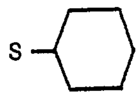 | O | H | $CH_3$ | $OCH_3$ |
| H | 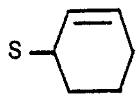 | O | H | $CH_3$ | $OCH_3$ |
| H | 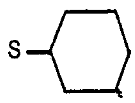 | O | H | $CH_3$ | $OCH_3$ |
| H | 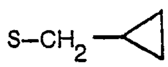 | O | H | $CH_3$ | $OCH_3$ |
| H | 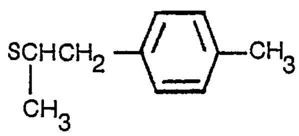 | O | H | $CH_3$ | $OCH_3$ |
| H | $NH_2$ | O | H | $CH_3$ | $OCH_3$ |
| H | 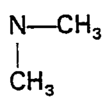 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 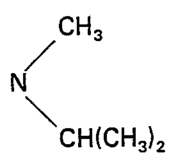 | O | H | $CH_3$ | $OCH_3$ |

TABLE VI-k

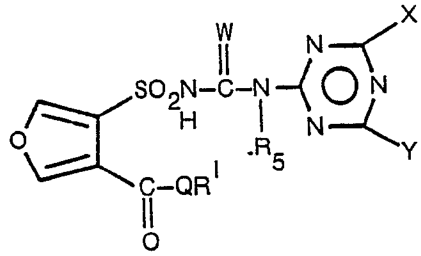

| A' | $QR^I$ | W | $R_5$ | X | Y |
|---|---|---|---|---|---|
| H | $SCH_3$ | O | H | $CH_3$ | $OCH_3$ |
| H | $SCH(CH_3)_2$ | O | H | $CH_3$ | $OCH_3$ |
| H | $SCH—C_2H_5$<br>\|<br>$CH_3$ | O | H | $CH_3$ | $OCH_3$ |
| H | $SCH—CH(CH_3)_2$<br>\|<br>$CH_3$ | O | H | $CH_3$ | $OCH_3$ |
| H | $SCH_2CH—CH_2$ | O | H | $CH_3$ | $OCH_3$ |
| H | $SCH_2CH—CHC_2H_5$ | O | H | $CH_3$ | $OCH_3$ |
| H | $SCH_2—C{\equiv}C—C_2H_5$ | O | H | $CH_3$ | $OCH_3$ |
| H | $S(CH_2)_4Cl$ | O | H | $CH_3$ | $OCH_3$ |
| H | $SCH_2CN$ | O | H | $CH_3$ | $OCH_3$ |
| 5-Cl | $S(CH_2)_2OCH_3$ | O | H | $CH_3$ | $OCH_3$ |
| 5-Cl | $S(CH_2)_4OCH_3$ | O | H | $CH_3$ | $OCH_3$ |
| 5-$CH_3$ | $SCH_2CH{=}CHCH_2Cl$ | O | H | $CH_3$ | $OCH_3$ |
| 5-$C_2H_5$ | $SCH_2C{\equiv}CCH_2CH_2Cl$ | O | H | $CH_3$ | $OCH_3$ |
| H | 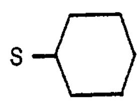 | O | H | $CH_3$ | $OCH_3$ |
| H | 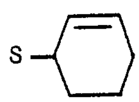 | O | H | $CH_3$ | $OCH_3$ |
| H | 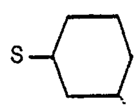 | O | H | $CH_3$ | $OCH_3$ |
| H | 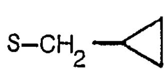 | O | H | $CH_3$ | $OCH_3$ |
| H | 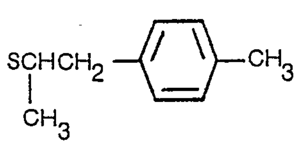 | O | H | $CH_3$ | $OCH_3$ |

TABLE VI-k (cont'd.)

| A' | QR' | W | $R_5$ | X | Y |
|---|---|---|---|---|---|
| H | $NH_2$ | O | H | $CH_3$ | $OCH_3$ |
| H | 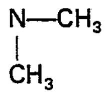 | O | H | $CH_3$ | $OCH_3$ |
| H | $N(CH_2CN)_2$ | O | H | $CH_3$ | $OCH_3$ |
| H | $N(CH_2CH_2CN)_2$ | O | H | $CH_3$ | $OCH_3$ |
| H | $N(C_2H_5)_2$ | O | H | $CH_3$ | $OCH_3$ |
| H | 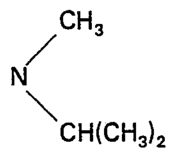 | O | H | $CH_3$ | $OCH_3$ |

TABLE VII-a

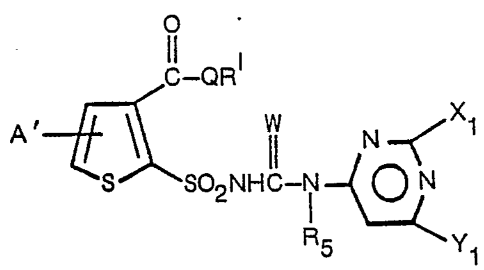

| A' | QR' | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | 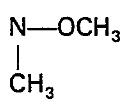 | O | H | $OCH_3$ | $OCH_3$ |
| H | 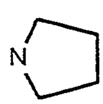 | O | H | $OCH_3$ | $OCH_3$ |

TABLE VII-b

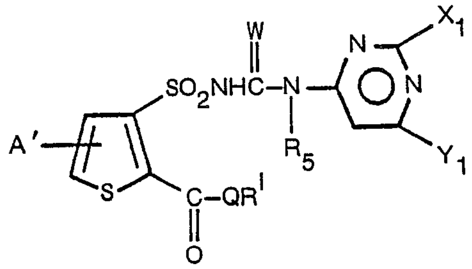

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | N—$OCH_3$<br>$CH_3$ | O | H | $OCH_3$ | $OCH_3$ |
| H | 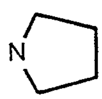 | O | H | $OCH_3$ | $OCH_3$ |

TABLE VII-c

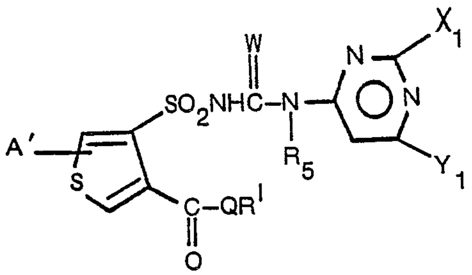

| A' | QR' | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N—OCH_3$<br>$CH_3$ | O | H | $OCH_3$ | $OCH_3$ |
| H | 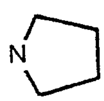 | O | H | $OCH_3$ | $OCH_3$ |

TABLE VII-d

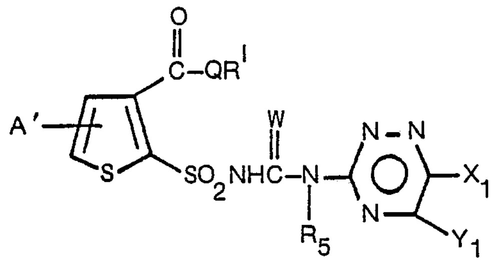

| A′ | QR$^I$ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_2CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | N—$OCH_3$<br>\|<br>$CH_3$ | O | H | $OCH_3$ | $OCH_3$ |
| H | 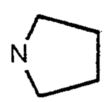 | O | H | $OCH_3$ | $OCH_3$ |

TABLE VII-e

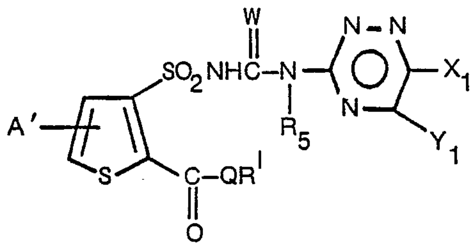

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N(CH_3)-OCH_3$ | O | H | $OCH_3$ | $OCH_3$ |
| H | 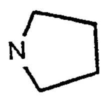 | O | H | $OCH_3$ | $OCH_3$ |

TABLE VII-f

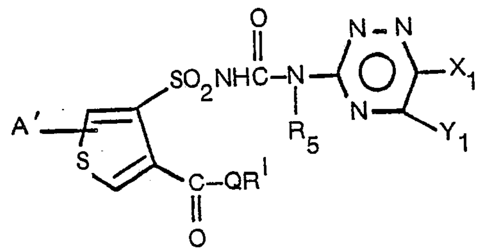

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N—OCH_3$ with $CH_3$ on N | O | H | $OCH_3$ | $OCH_3$ |
| H | 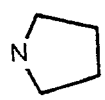 | O | H | $OCH_3$ | $OCH_3$ |

TABLE VII-g

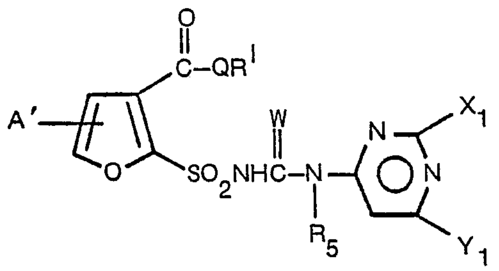

| A′ | $QR^I$ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | N—$OCH_3$<br>\|<br>$CH_3$ | O | H | $OCH_3$ | $OCH_3$ |
| H | 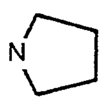 | O | H | $OCH_3$ | $OCH_3$ |

TABLE VII-h

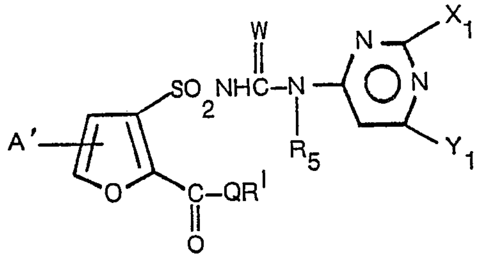

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N(CH_3)-OCH_3$ | O | H | $OCH_3$ | $OCH_3$ |
| H | 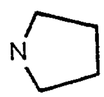 | O | H | $OCH_3$ | $OCH_3$ |

TABLE VII-i

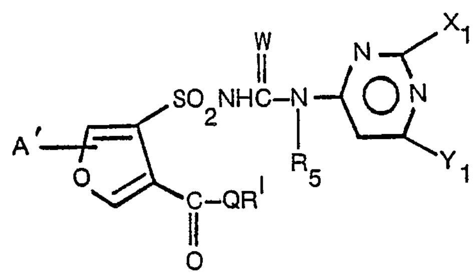

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | N—$OCH_3$<br>\|<br>$CH_3$ | O | H | $OCH_3$ | $OCH_3$ |
| H | 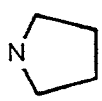 | O | H | $OCH_3$ | $OCH_3$ |

TABLE VII-j

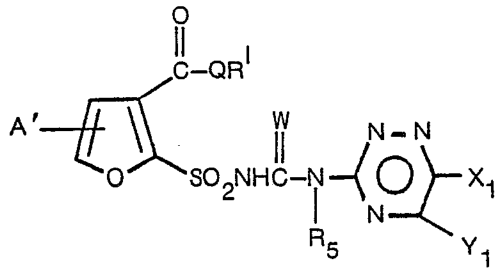

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | 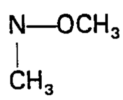 | O | H | $OCH_3$ | $OCH_3$ |
| H | 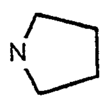 | O | H | $OCH_3$ | $OCH_3$ |

TABLE VII-k

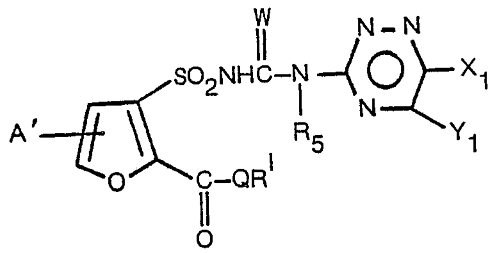

| A′ | $QR^I$ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N(CH_3)-OCH_3$ | O | H | $OCH_3$ | $OCH_3$ |
| H | 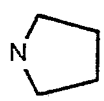 | O | H | $OCH_3$ | $OCH_3$ |

TABLE VII-I

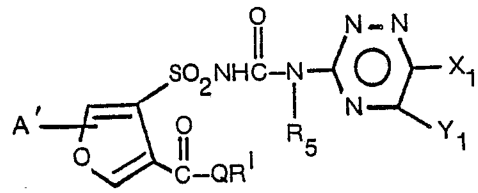

| A′ | QR′ | W | $R_5$ | $X_1$ | $Y_1$ |
|---|---|---|---|---|---|
| H | $SCH(CH_3)_2$ | O | H | $CH_3$ | $CH_3$ |
| H | $SCH_3$ | O | H | $CH_3$ | $CH_3$ |
| H | $SCH_3CH_3$ | O | H | $CH_3$ | $CH_3$ |
| H | $S(CH_2)_3CH_3$ | O | H | $CH_3$ | $CH_3$ |
| H | $NH(CH_2)_3CH_3$ | O | H | $CH_3$ | $CH_3$ |
| H | $NHCH(CH_3)_2$ | O | H | $CH_3$ | $CH_3$ |
| H | $N(CH_3)_2$ | O | H | $OCH_3$ | $OCH_3$ |
| H | $N(C_2H_5)_2$ | O | H | $OCH_3$ | $OCH_3$ |
| H | $N(CH_3)-OCH_3$ | O | H | $OCH_3$ | $OCH_3$ |
| H | 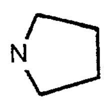 | O | H | $OCH_3$ | $OCH_3$ |

TABLE VIII-a

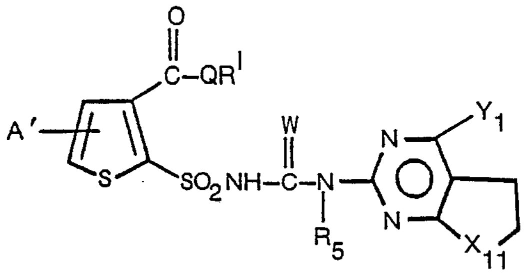

| A′ | QR$^I$ | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE VIII-b

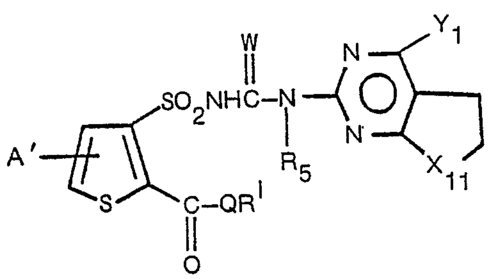

| A′ | QR$^I$ | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE VIII-c

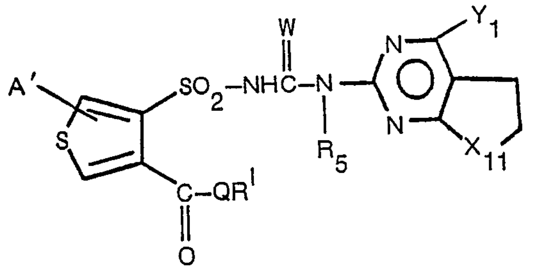

| A′ | $QR^I$ | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE VIII-d

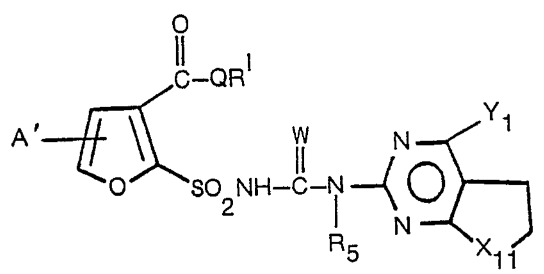

| A′ | $QR^I$ | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE VIII-e

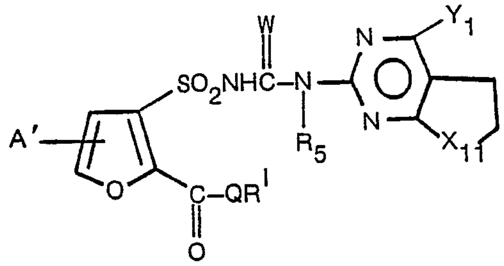

| A′ | QR' | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE VIII-f

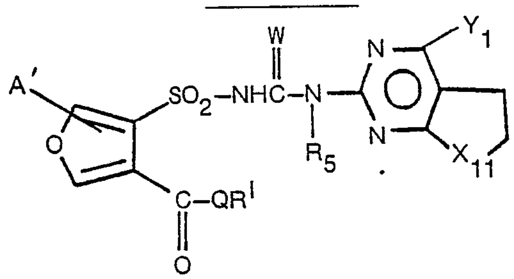

| A′ | QR' | W | $R_5$ | $Y_1$ | $X_{11}$ |
|---|---|---|---|---|---|
| H | $SCH_3$ | O | H | $CH_3$ | $CH_2$ |
| H | $SC_2H_5$ | O | H | $CH_3$ | $CH_2$ |
| H | $SCH(CH_3)_2$ | O | H | $CH_3$ | $CH_2$ |
| H | $SCH_2CH{=}CH_2$ | O | H | $CH_3$ | $CH_2$ |
| H | $NHC_2H_5$ | O | H | $CH_3$ | $CH_2$ |
| H | $NHCH(CH_3)_2$ | O | H | $CH_3$ | O |
| H | $NH(CH_2)_5CH_3$ | O | H | $CH_3$ | O |
| H | $NH(CH_2)_8CH_3$ | O | H | $CH_3$ | O |
| H | $N(CH_3)_2$ | O | H | $CH_3$ | O |

TABLE IX-a

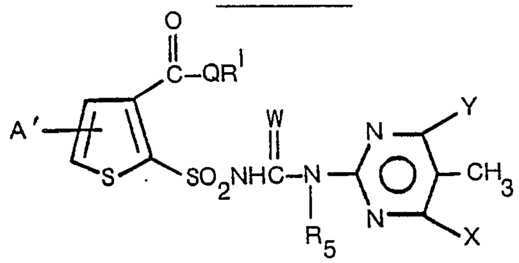

| A′ | QR$^I$ | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)$—$CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE IX-b

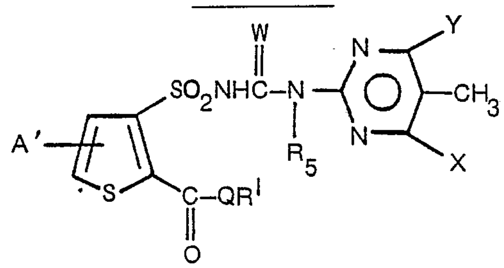

| A′ | QR$^I$ | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)$—$CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE IX-c

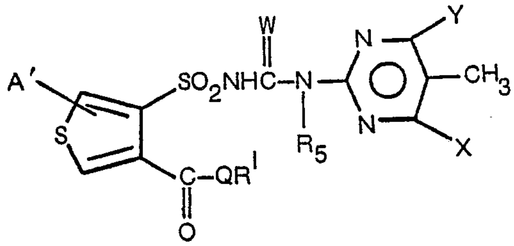

| A' | QR' | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)—CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE IX-d

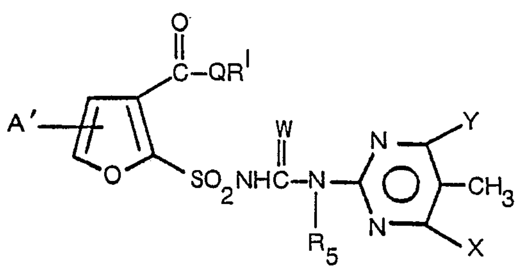

| A' | QR' | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)—CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE IX-e

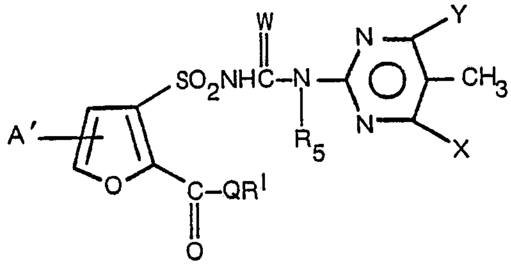

| A′ | QR′ | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)$—$CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE IX-f

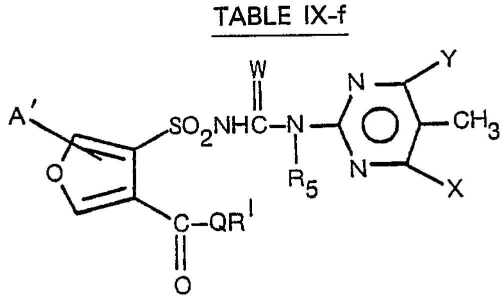

| A′ | QR′ | W | $R_5$ | X | Y |
|---|---|---|---|---|---|
| H | $SCH_3$ | O | H | $CH_3$ | $CH_3$ |
| H | $SC_2H_5$ | O | H | $CH_3$ | $CH_3$ |
| H | $SCH(CH_3)_2$ | O | H | $CH_3$ | $CH_3$ |
| H | $S(CH_2)_3CH_3$ | O | H | $CH_3$ | $CH_3$ |
| H | $SCH_2CH{=}CH_2$ | O | H | $CH_3$ | $CH_3$ |
| H | $S(CH_2)$—$CH_3$ | O | H | $CH_3$ | $CH_3$ |
| H | $NHCH(CH_3)_2$ | O | H | $CH_3$ | $CH_3$ |
| H | $NHC_2H_5$ | O | H | $CH_3$ | $CH_3$ |
| H | $N(CH_3)_2$ | O | H | $CH_3$ | $CH_3$ |
| H | $N(C_2H_5)_2$ | O | H | $CH_3$ | $CH_3$ |

TABLE X-a

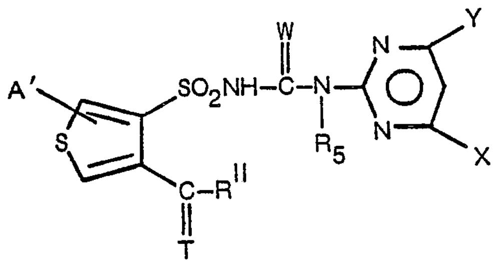

| A′ | 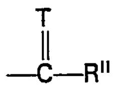 | W | $R_5$ | X | Y |
|---|---|---|---|---|---|
| H | 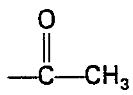 | O | H | $CH_3$ | $CH_3$ |
| H | 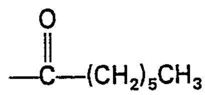 | O | $CH_3$ | $CH_3$ | $CH_3$ |
| H | 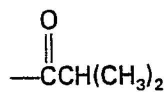 | O | H | $CH_3$ | $CH_3$ |
| H | 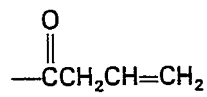 | O | $CH_3$ | $CH_3$ | $CH_3$ |
| H | 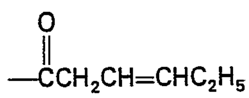 | O | H | $CH_3$ | $CH_3$ |
| H | 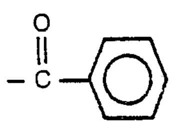 | O | $CH_3$ | $CH_3$ | $CH_3$ |
| H | 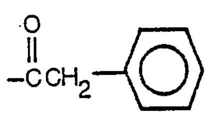 | O | $CH_3$ | $CH_3$ | $CH_3$ |
| H | 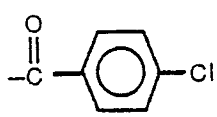 | O | $CH_3$ | $CH_3$ | $CH_3$ |
| H | 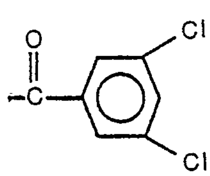 | O | $CH_3$ | $CH_3$ | $CH_3$ |
| H | 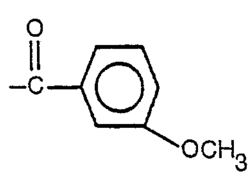 | O | $CH_3$ | $CH_3$ | $CH_3$ |

TABLE X-a (cont'd)

| A' | T<br>‖<br>—C—R'' | W | $R_5$ | X | Y |
|---|---|---|---|---|---|
| H | 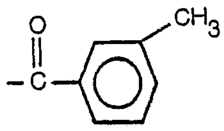 | O | $CH_3$ | $CH_3$ | $CH_3$ |
| H | 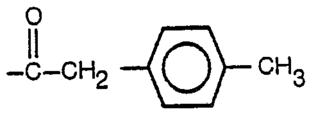 | O | $CH_3$ | $CH_3$ | $CH_3$ |
| H | 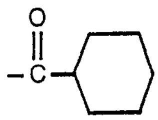 | O | $CH_3$ | $CH_3$ | $CH_3$ |
| H | 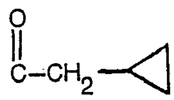 | O | H | $CH_3$ | $CH_3$ |
| H | 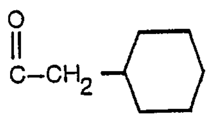 | O | H | $CH_3$ | $CH_3$ |
| H | NOH<br>‖<br>C—$CH_3$ | O | H | $CH_3$ | $CH_3$ |
| H | $NOCH_3$<br>‖<br>C—$C_2H_5$ | O | H | $CH_3$ | $CH_3$ |
| H | $NOCH(CH_3)_2$<br>‖<br>C—$C_2H_5$ | O | H | $CH_3$ | $CH_3$ |
| H | $NOCH_2CH{=}CH_2$<br>‖<br>C—$CH_3$ | | | | |
| H | O<br>‖<br>CH | O | H | $CH_3$ | $CH_3$ |
| H | O<br>‖<br>CH | O | H | $CH_3$ | $CH_3$ |
| H | O<br>‖<br>CH | O | H | $OCH_3$ | $OCH_3$ |

TABLE X-b

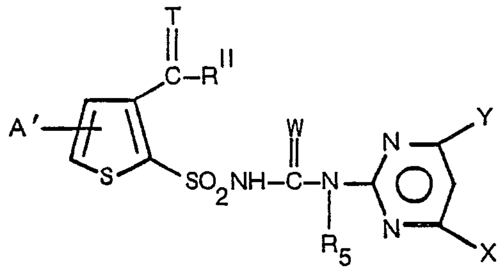

| A′ | 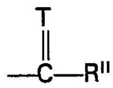 | W | $R_5$ | X | Y |
|---|---|---|---|---|---|
| H | 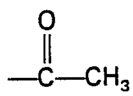 | O | H | $CH_3$ | $CH_3$ |
| H | 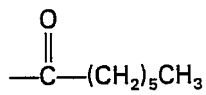 | O | $CH_3$ | $CH_3$ | $CH_3$ |
| H | 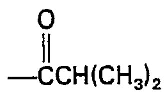 | O | H | $CH_3$ | $CH_3$ |
| H | 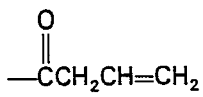 | O | $CH_3$ | $CH_3$ | $CH_3$ |
| H | 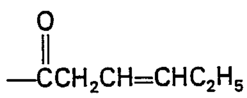 | O | H | $CH_3$ | $CH_3$ |
| H | 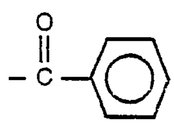 | O | $CH_3$ | $CH_3$ | $CH_3$ |
| H | 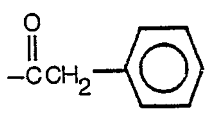 | O | $CH_3$ | $CH_3$ | $CH_3$ |
| H | 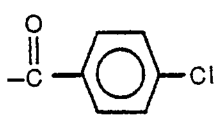 | O | $CH_3$ | $CH_3$ | $CH_3$ |
| H | 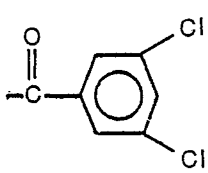 | O | $CH_3$ | $CH_3$ | $CH_3$ |
| H | 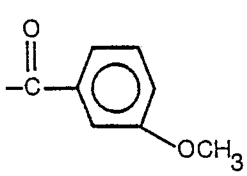 | O | $CH_3$ | $CH_3$ | $CH_3$ |

TABLE X-b (cont'd.)

| A' | 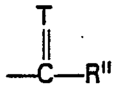 | W | $R_5$ | X | Y |
|---|---|---|---|---|---|
| H | 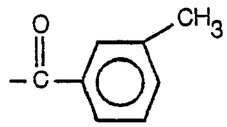 | O | $CH_3$ | $CH_3$ | $CH_3$ |
| H | 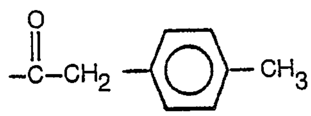 | O | $CH_3$ | $CH_3$ | $CH_3$ |
| H | 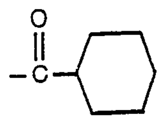 | O | $CH_3$ | $CH_3$ | $CH_3$ |
| H | 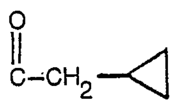 | O | H | $CH_3$ | $CH_3$ |
| H | 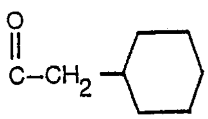 | O | H | $CH_3$ | $CH_3$ |
| H | 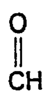 | O | H | $CH_3$ | $OCH_3$ |

TABLE X-c

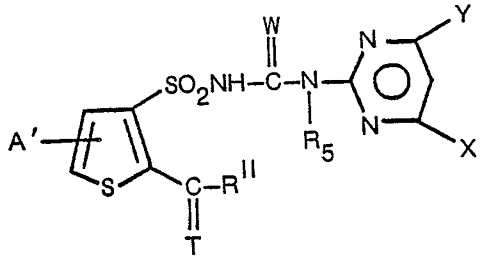

| A′ | 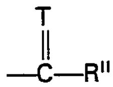 | W | $R_5$ | X | Y |
|---|---|---|---|---|---|
| H | 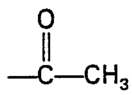 | O | H | $CH_3$ | $CH_3$ |
| H | 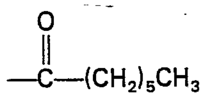 | O | $CH_3$ | $CH_3$ | $CH_3$ |
| H | 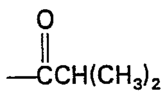 | O | H | $CH_3$ | $CH_3$ |
| H | 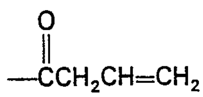 | O | $CH_3$ | $CH_3$ | $CH_3$ |
| H | 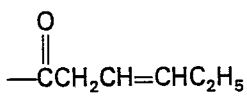 | O | H | $CH_3$ | $CH_3$ |
| H | 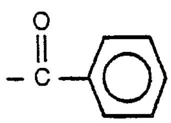 | O | $CH_3$ | $CH_3$ | $CH_3$ |
| H | 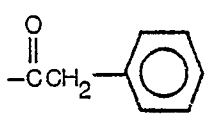 | O | $CH_3$ | $CH_3$ | $CH_3$ |
| H | 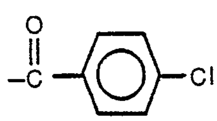 | O | $CH_3$ | $CH_3$ | $CH_3$ |
| H | 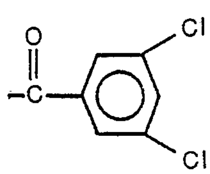 | O | $CH_3$ | $CH_3$ | $CH_3$ |
| H | 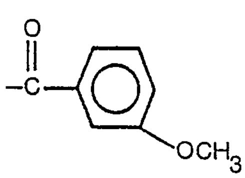 | O | $CH_3$ | $CH_3$ | $CH_3$ |

TABLE X-c (cont'd.)

| A' | 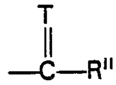 | W | $R_5$ | X | Y |
|---|---|---|---|---|---|
| H | 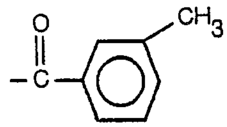 | O | $CH_3$ | $CH_3$ | $CH_3$ |
| H | 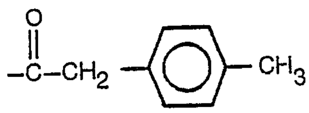 | O | $CH_3$ | $CH_3$ | $CH_3$ |
| H | 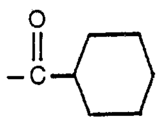 | O | $CH_3$ | $CH_3$ | $CH_3$ |
| H | 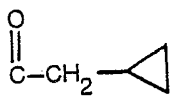 | O | H | $CH_3$ | $CH_3$ |
| H | 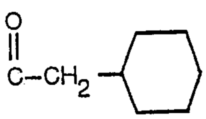 | O | H | $CH_3$ | $CH_3$ |
| H | 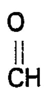 | O | H | $CH_3$ | $OCH_3$ |

TABLE X-d

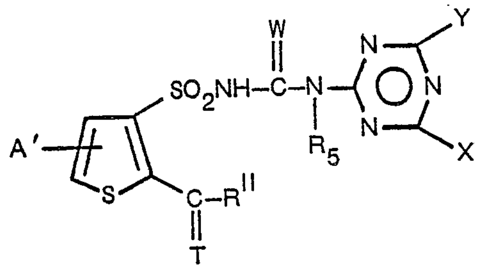

| A′ | 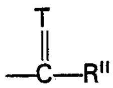 | W | $R_5$ | X | Y |
|---|---|---|---|---|---|
| H | 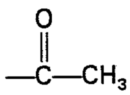 | O | H | $CH_3$ | $CH_3$ |
| H | 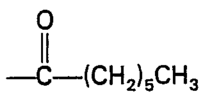 | O | $CH_3$ | $CH_3$ | $CH_3$ |
| H | 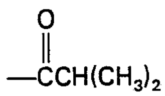 | O | H | $CH_3$ | $CH_3$ |
| H | 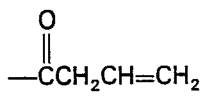 | O | $CH_3$ | $CH_3$ | $CH_3$ |
| H | 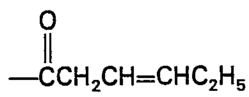 | O | H | $CH_3$ | $CH_3$ |
| H | 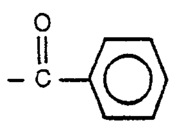 | O | $CH_3$ | $CH_3$ | $CH_3$ |
| H | 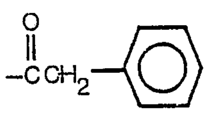 | O | $CH_3$ | $CH_3$ | $CH_3$ |
| H | 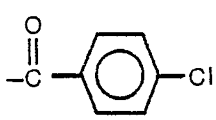 | O | $CH_3$ | $CH_3$ | $CH_3$ |
| H | 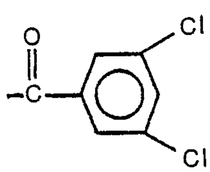 | O | $CH_3$ | $CH_3$ | $CH_3$ |
| H | 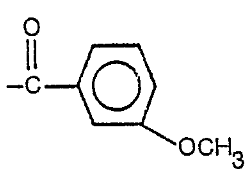 | O | $CH_3$ | $CH_3$ | $CH_3$ |

TABLE X-d (cont'd.)

| A′ | 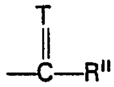 | W | $R_5$ | X | Y |
|---|---|---|---|---|---|
| H | 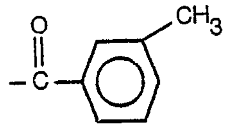 | O | $CH_3$ | $CH_3$ | $CH_3$ |
| H | 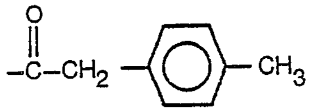 | O | $CH_3$ | $CH_3$ | $CH_3$ |
| H | 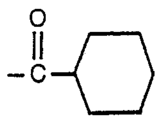 | O | $CH_3$ | $CH_3$ | $CH_3$ |
| H | 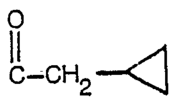 | O | H | $CH_3$ | $CH_3$ |
| H | 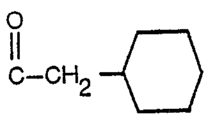 | O | H | $CH_3$ | $CH_3$ |
| H | 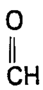 | O | H | $CH_3$ | $OCH_3$ |

TABLE X-e

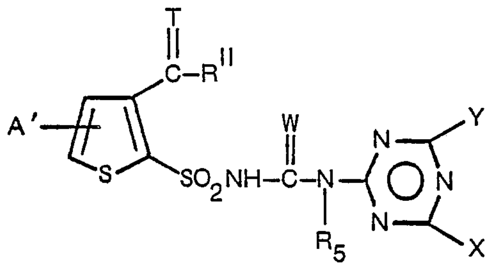

| A′ | 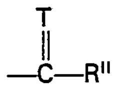 | W | $R_5$ | X | Y |
|---|---|---|---|---|---|
| H | 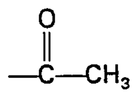 | O | H | $CH_3$ | $CH_3$ |
| H | 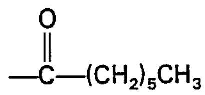 | O | $CH_3$ | $CH_3$ | $CH_3$ |
| H | 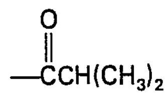 | O | H | $CH_3$ | $CH_3$ |
| H | 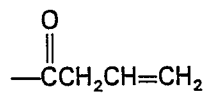 | O | $CH_3$ | $CH_3$ | $CH_3$ |
| H | 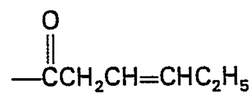 | O | H | $CH_3$ | $CH_3$ |
| H | 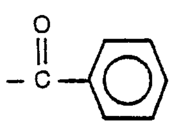 | O | $CH_3$ | $CH_3$ | $CH_3$ |
| H | 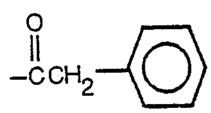 | O | $CH_3$ | $CH_3$ | $CH_3$ |
| H | 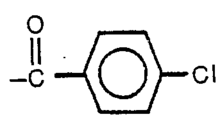 | O | $CH_3$ | $CH_3$ | $CH_3$ |
| H | 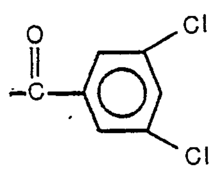 | O | $CH_3$ | $CH_3$ | $CH_3$ |
| H | 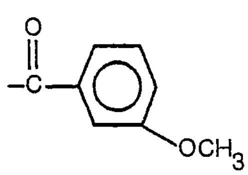 | O | $CH_3$ | $CH_3$ | $CH_3$ |

TABLE X-e (cont'd.)

| A' | 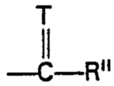 | W | $R_5$ | X | Y |
|---|---|---|---|---|---|
| H | 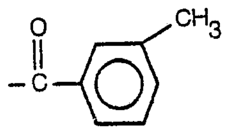 | O | $CH_3$ | $CH_3$ | $CH_3$ |
| H | 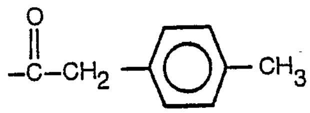 | O | $CH_3$ | $CH_3$ | $CH_3$ |
| H | 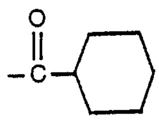 | O | $CH_3$ | $CH_3$ | $CH_3$ |
| H | 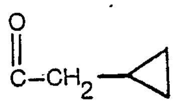 | O | H | $CH_3$ | $CH_3$ |
| H | 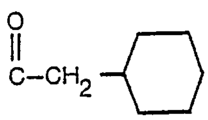 | O | H | $CH_3$ | $CH_3$ |
| H | 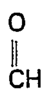 | O | H | $CH_3$ | $OCH_3$ |

TABLE X-f

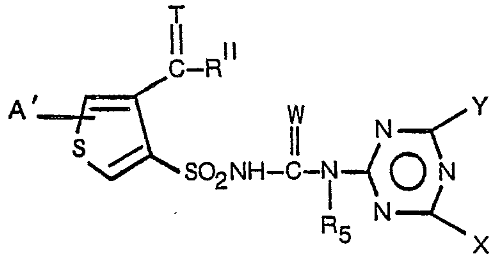

| A′ | $-\overset{T}{\overset{\|}{C}}-R''$ | W | $R_5$ | X | Y |
|---|---|---|---|---|---|
| H | $-\overset{O}{\overset{\|}{C}}-CH_3$ | O | H | $CH_3$ | $CH_3$ |
| H | $-\overset{O}{\overset{\|}{C}}-(CH_2)_5CH_3$ | O | $CH_3$ | $CH_3$ | $CH_3$ |
| H | $-\overset{O}{\overset{\|}{C}}CH(CH_3)_2$ | O | H | $CH_3$ | $CH_3$ |
| H | $-\overset{O}{\overset{\|}{C}}CH_2CH{=}CH_2$ | O | $CH_3$ | $CH_3$ | $CH_3$ |
| H | $-\overset{O}{\overset{\|}{C}}CH_2CH{=}CHC_2H_5$ | O | H | $CH_3$ | $CH_3$ |
| H | 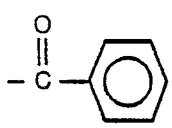 | O | $CH_3$ | $CH_3$ | $CH_3$ |
| H | 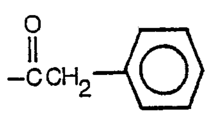 | O | $CH_3$ | $CH_3$ | $CH_3$ |
| H | 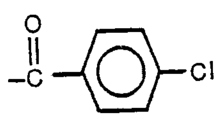 | O | $CH_3$ | $CH_3$ | $CH_3$ |
| H | 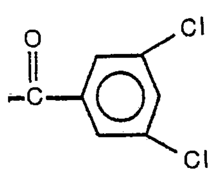 | O | $CH_3$ | $CH_3$ | $CH_3$ |
| H | 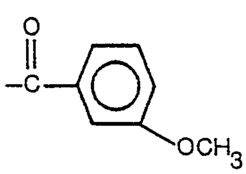 | O | $CH_3$ | $CH_3$ | $CH_3$ |

TABLE X-f (cont'd.)

| A' | 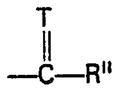 | W | $R_5$ | X | Y |
|---|---|---|---|---|---|
| H | 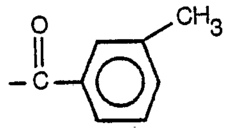 | O | $CH_3$ | $CH_3$ | $CH_3$ |
| H | 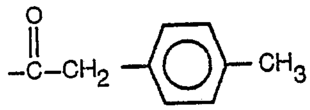 | O | $CH_3$ | $CH_3$ | $CH_3$ |
| H | 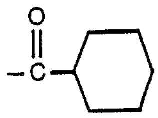 | O | $CH_3$ | $CH_3$ | $CH_3$ |
| H | 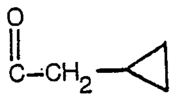 | O | H | $CH_3$ | $CH_3$ |
| H | 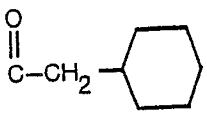 | O | H | $CH_3$ | $CH_3$ |
| H | 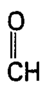 | O | H | $CH_3$ | $OCH_3$ |

TABLE X-g

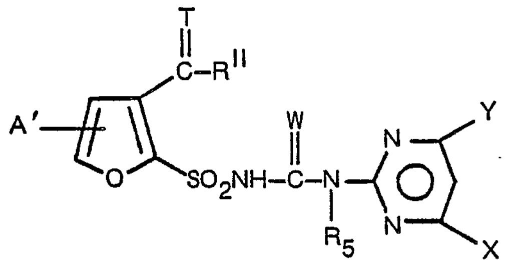

| A′ | 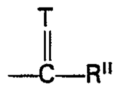 | W | $R_5$ | X | Y |
|---|---|---|---|---|---|
| H | 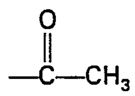 | O | H | $CH_3$ | $CH_3$ |
| H | 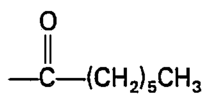 | O | $CH_3$ | $CH_3$ | $CH_3$ |
| H | 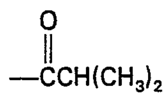 | O | H | $CH_3$ | $CH_3$ |
| H | 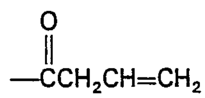 | O | $CH_3$ | $CH_3$ | $CH_3$ |
| H | 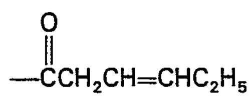 | O | H | $CH_3$ | $CH_3$ |
| H | 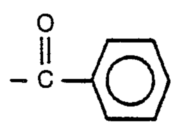 | O | $CH_3$ | $CH_3$ | $CH_3$ |
| H | 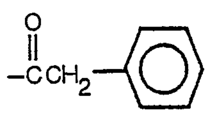 | O | $CH_3$ | $CH_3$ | $CH_3$ |
| H | 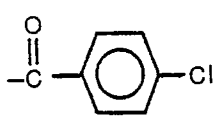 | O | $CH_3$ | $CH_3$ | $CH_3$ |
| H | 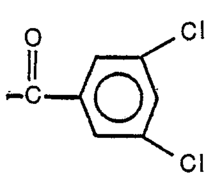 | O | $CH_3$ | $CH_3$ | $CH_3$ |
| H | 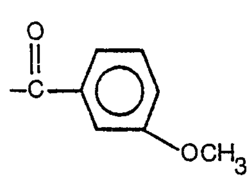 | O | $CH_3$ | $CH_3$ | $CH_3$ |

TABLE X-g (cont'd.)

| A′ | 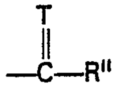 | W | $R_5$ | X | Y |
|---|---|---|---|---|---|
| H | 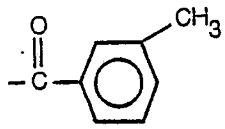 | O | $CH_3$ | $CH_3$ | $CH_3$ |
| H | 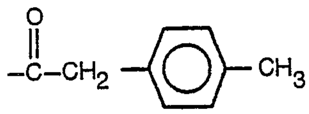 | O | $CH_3$ | $CH_3$ | $CH_3$ |
| H | 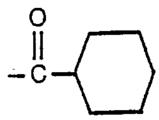 | O | $CH_3$ | $CH_3$ | $CH_3$ |
| H | 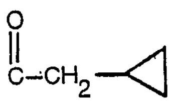 | O | H | $CH_3$ | $CH_3$ |
| H | 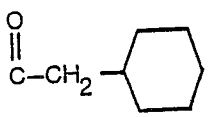 | O | H | $CH_3$ | $CH_3$ |
| H | 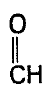 | O | H | $CH_3$ | $OCH_3$ |

TABLE X-h

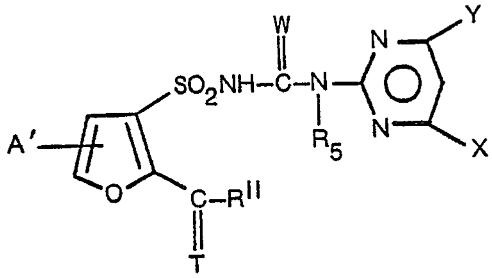

| A′ | $-\overset{T}{\overset{\|}{C}}-R^{II}$ | W | $R_5$ | X | Y |
|---|---|---|---|---|---|
| H | $-\overset{O}{\overset{\|}{C}}-CH_3$ | O | H | $CH_3$ | $CH_3$ |
| H | $-\overset{O}{\overset{\|}{C}}-(CH_2)_5CH_3$ | O | $CH_3$ | $CH_3$ | $CH_3$ |
| H | $-\overset{O}{\overset{\|}{C}}CH(CH_3)_2$ | O | H | $CH_3$ | $CH_3$ |
| H | $-\overset{O}{\overset{\|}{C}}CH_2CH{=}CH_2$ | O | $CH_3$ | $CH_3$ | $CH_3$ |
| H | $-\overset{O}{\overset{\|}{C}}CH_2CH{=}CHC_2H_5$ | O | H | $CH_3$ | $CH_3$ |
| H | 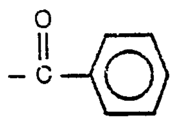 | O | $CH_3$ | $CH_3$ | $CH_3$ |
| H | 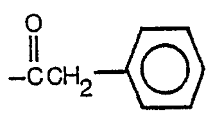 | O | $CH_3$ | $CH_3$ | $CH_3$ |
| H | 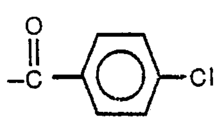 | O | $CH_3$ | $CH_3$ | $CH_3$ |
| H | 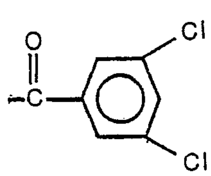 | O | $CH_3$ | $CH_3$ | $CH_3$ |
| H | 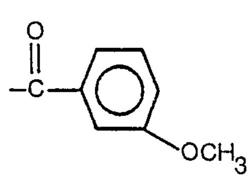 | O | $CH_3$ | $CH_3$ | $CH_3$ |

TABLE X-h (cont'd.)

| A′ | 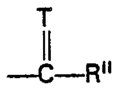 | W | $R_5$ | X | Y |
|---|---|---|---|---|---|
| H | 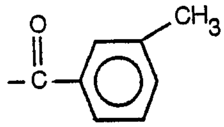 | O | $CH_3$ | $CH_3$ | $CH_3$ |
| H | 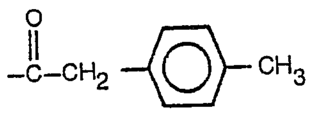 | O | $CH_3$ | $CH_3$ | $CH_3$ |
| H | 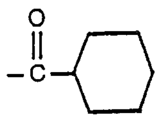 | O | $CH_3$ | $CH_3$ | $CH_3$ |
| H | 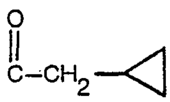 | O | H | $CH_3$ | $CH_3$ |
| H | 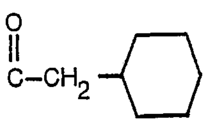 | O | H | $CH_3$ | $CH_3$ |
| H | 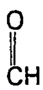 | O | H | $CH_3$ | $OCH_3$ |

TABLE X-i

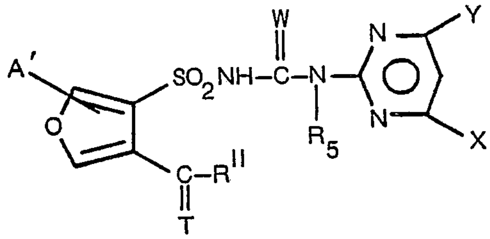

| A′ | 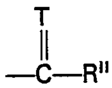 | W | $R_5$ | X | Y |
|---|---|---|---|---|---|
| H | 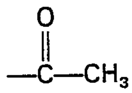 | O | H | $CH_3$ | $CH_3$ |
| H | 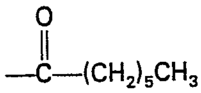 | O | $CH_3$ | $CH_3$ | $CH_3$ |
| H | 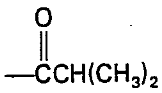 | O | H | $CH_3$ | $CH_3$ |
| H | 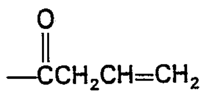 | O | $CH_3$ | $CH_3$ | $CH_3$ |
| H | 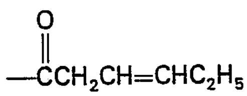 | O | H | $CH_3$ | $CH_3$ |
| H | 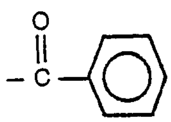 | O | $CH_3$ | $CH_3$ | $CH_3$ |
| H | 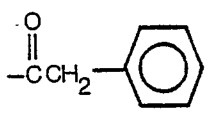 | O | $CH_3$ | $CH_3$ | $CH_3$ |
| H | 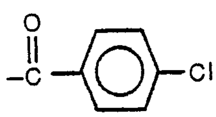 | O | $CH_3$ | $CH_3$ | $CH_3$ |
| H | 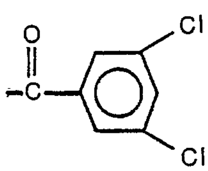 | O | $CH_3$ | $CH_3$ | $CH_3$ |
| H | 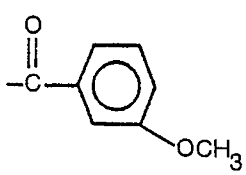 | O | $CH_3$ | $CH_3$ | $CH_3$ |

TABLE X-i (cont'd.)

| A' | 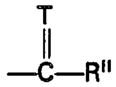 | W | $R_5$ | X | Y |
|---|---|---|---|---|---|
| H | 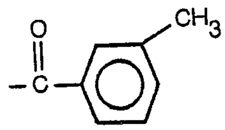 | O | $CH_3$ | $CH_3$ | $CH_3$ |
| H | 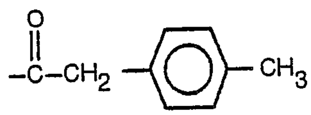 | O | $CH_3$ | $CH_3$ | $CH_3$ |
| H | 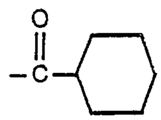 | O | $CH_3$ | $CH_3$ | $CH_3$ |
| H | 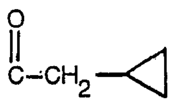 | O | H | $CH_3$ | $CH_3$ |
| H | 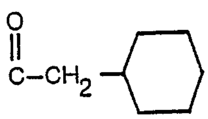 | O | H | $CH_3$ | $CH_3$ |
| H | 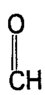 | O | H | $CH_3$ | $OCH_3$ |

TABLE X-j

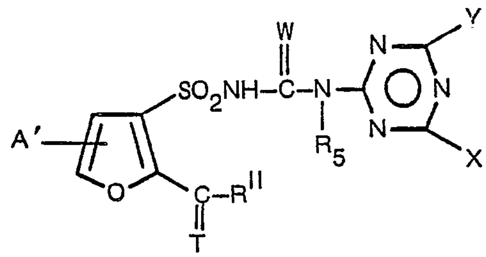

| A′ | 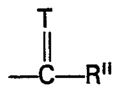 | W | $R_5$ | X | Y |
|---|---|---|---|---|---|
| H | 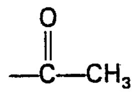 | O | H | $CH_3$ | $CH_3$ |
| H | 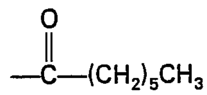 | O | $CH_3$ | $CH_3$ | $CH_3$ |
| H | 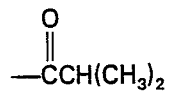 | O | H | $CH_3$ | $CH_3$ |
| H | 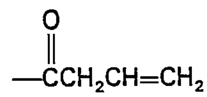 | O | $CH_3$ | $CH_3$ | $CH_3$ |
| H | 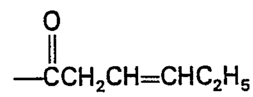 | O | H | $CH_3$ | $CH_3$ |
| H | 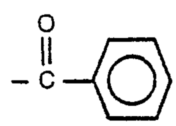 | O | $CH_3$ | $CH_3$ | $CH_3$ |
| H | 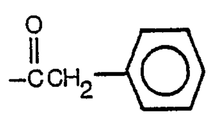 | O | $CH_3$ | $CH_3$ | $CH_3$ |
| H | 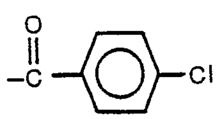 | O | $CH_3$ | $CH_3$ | $CH_3$ |
| H | 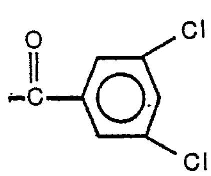 | O | $CH_3$ | $CH_3$ | $CH_3$ |
| H | 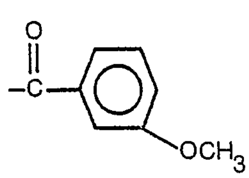 | O | $CH_3$ | $CH_3$ | $CH_3$ |

TABLE X-j (cont'd.)

| A′ | 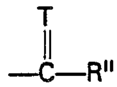 | W | $R_5$ | X | Y |
|---|---|---|---|---|---|
| H | 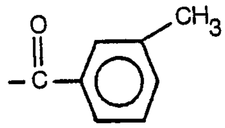 | O | $CH_3$ | $CH_3$ | $CH_3$ |
| H | 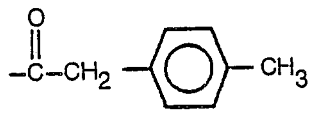 | O | $CH_3$ | $CH_3$ | $CH_3$ |
| H | 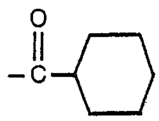 | O | $CH_3$ | $CH_3$ | $CH_3$ |
| H | 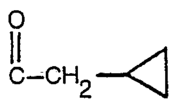 | O | H | $CH_3$ | $CH_3$ |
| H | 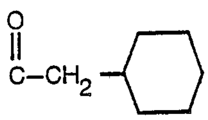 | O | H | $CH_3$ | $CH_3$ |
| H | 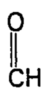 | O | H | $CH_3$ | $OCH_3$ |

TABLE X-k

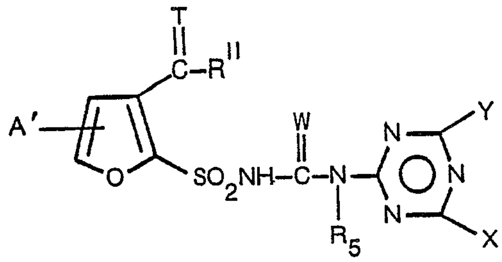

| A′ | $-\overset{T}{\overset{\|}{C}}-R''$ | W | $R_5$ | X | Y |
|---|---|---|---|---|---|
| H | $-\overset{O}{\overset{\|}{C}}-CH_3$ | O | H | $CH_3$ | $CH_3$ |
| H | $-\overset{O}{\overset{\|}{C}}-(CH_2)_5CH_3$ | O | $CH_3$ | $CH_3$ | $CH_3$ |
| H | $-\overset{O}{\overset{\|}{C}}CH(CH_3)_2$ | O | H | $CH_3$ | $CH_3$ |
| H | $-\overset{O}{\overset{\|}{C}}CH_2CH{=}CH_2$ | O | $CH_3$ | $CH_3$ | $CH_3$ |
| H | $-\overset{O}{\overset{\|}{C}}CH_2CH{=}CHC_2H_5$ | O | H | $CH_3$ | $CH_3$ |
| H | 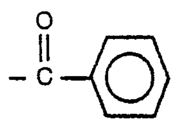 | O | $CH_3$ | $CH_3$ | $CH_3$ |
| H | 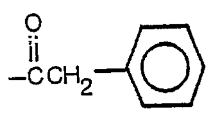 | O | $CH_3$ | $CH_3$ | $CH_3$ |
| H | 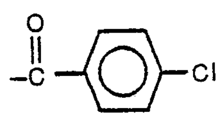 | O | $CH_3$ | $CH_3$ | $CH_3$ |
| H | 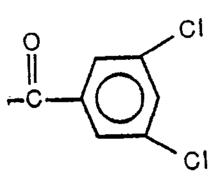 | O | $CH_3$ | $CH_3$ | $CH_3$ |
| H | 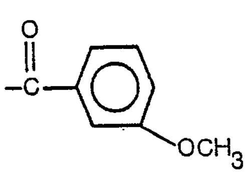 | O | $CH_3$ | $CH_3$ | $CH_3$ |

TABLE X-k (cont'd.)

| A′ | 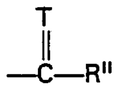 | W | $R_5$ | X | Y |
|---|---|---|---|---|---|
| H | 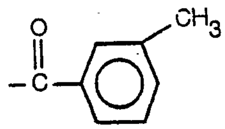 | O | $CH_3$ | $CH_3$ | $CH_3$ |
| H | 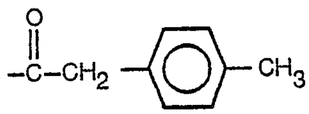 | O | $CH_3$ | $CH_3$ | $CH_3$ |
| H | 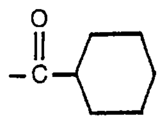 | O | $CH_3$ | $CH_3$ | $CH_3$ |
| H | 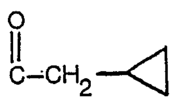 | O | H | $CH_3$ | $CH_3$ |
| H | 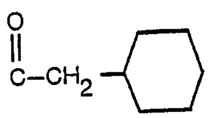 | O | H | $CH_3$ | $CH_3$ |
| H | 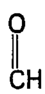 | O | H | $CH_3$ | $OCH_3$. |

TABLE X-I

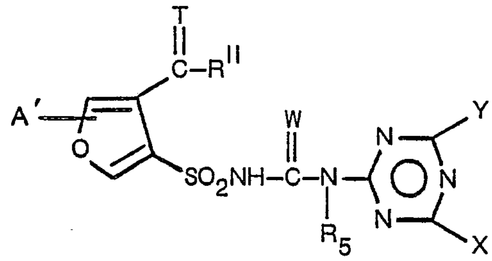

| A′ | 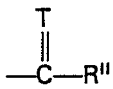 | W | $R_5$ | X | Y |
|---|---|---|---|---|---|
| H | 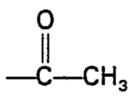 | O | H | $CH_3$ | $CH_3$ |
| H | 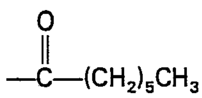 | O | $CH_3$ | $CH_3$ | $CH_3$ |
| H | 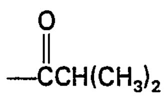 | O | H | $CH_3$ | $CH_3$ |
| H | 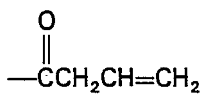 | O | $CH_3$ | $CH_3$ | $CH_3$ |
| H | 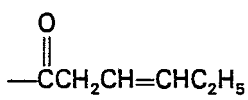 | O | H | $CH_3$ | $CH_3$ |
| H | 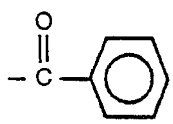 | O | $CH_3$ | $CH_3$ | $CH_3$ |
| H | 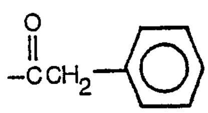 | O | $CH_3$ | $CH_3$ | $CH_3$ |
| H | 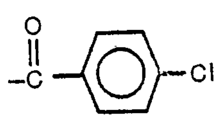 | O | $CH_3$ | $CH_3$ | $CH_3$ |
| H | 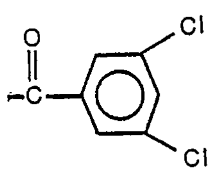 | O | $CH_3$ | $CH_3$ | $CH_3$ |
| H | 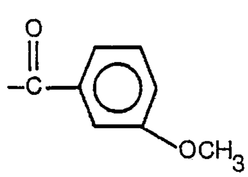 | O | $CH_3$ | $CH_3$ | $CH_3$ |

TABLE X-I (cont'd.)

| A′ | 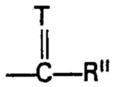 | W | $R_5$ | X | Y |
|---|---|---|---|---|---|
| H | 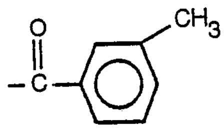 | O | $CH_3$ | $CH_3$ | $CH_3$ |
| H | 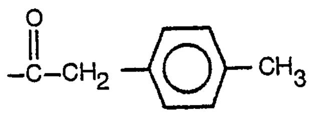 | O | $CH_3$ | $CH_3$ | $CH_3$ |
| H | 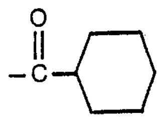 | O | $CH_3$ | $CH_3$ | $CH_3$ |
| H | 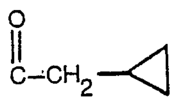 | O | H | $CH_3$ | $CH_3$ |
| H | 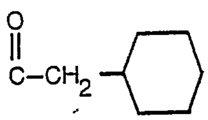 | O | H | $CH_3$ | $CH_3$ |
| H | 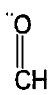 | O | H | $CH_3$ | $OCH_3$ |

TABLE XI

Other compounds within the scope of this invention include:

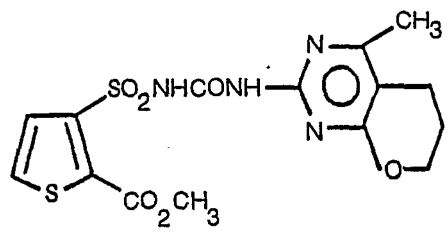

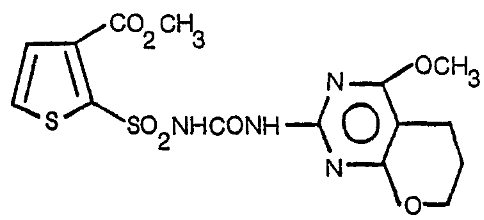

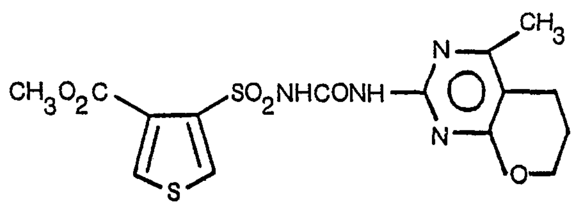

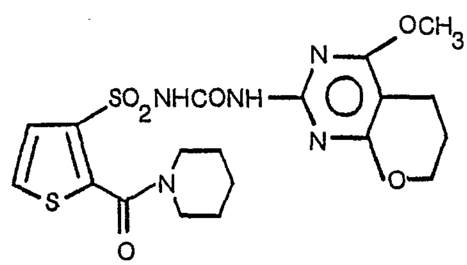

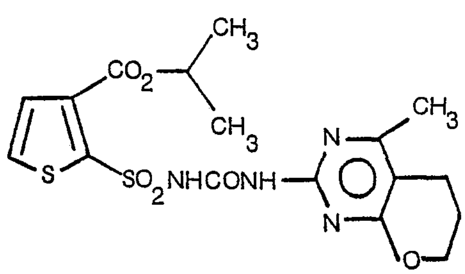

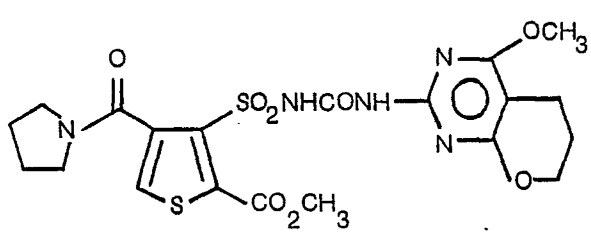

TABLE XI (cont'd)

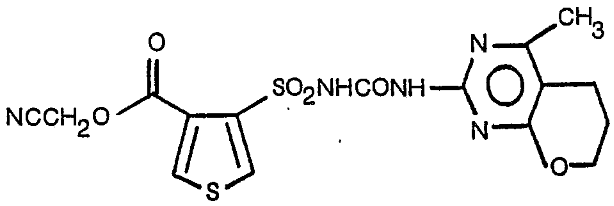

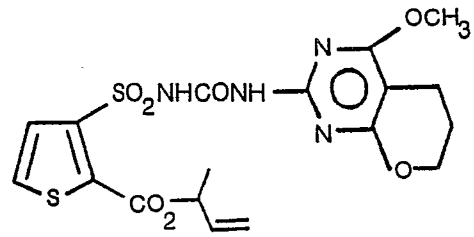

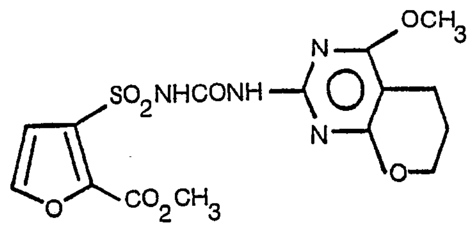

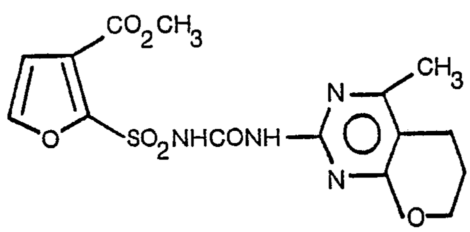

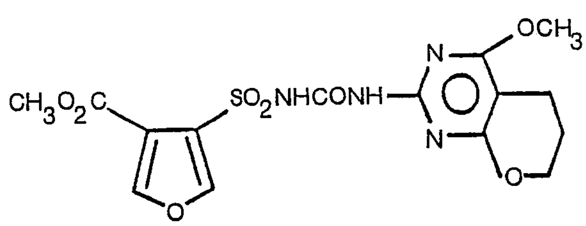

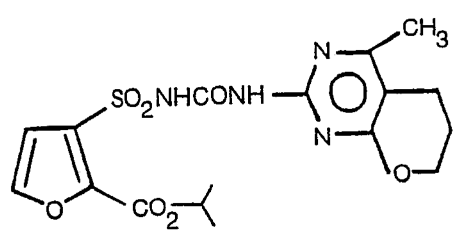

TABLE XI (cont'd)

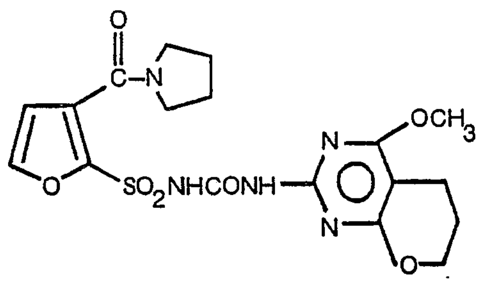

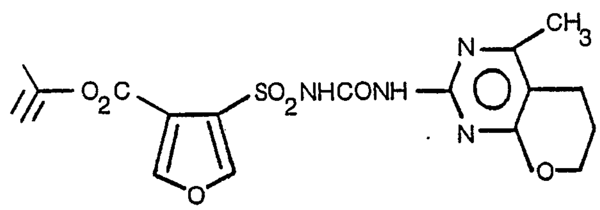

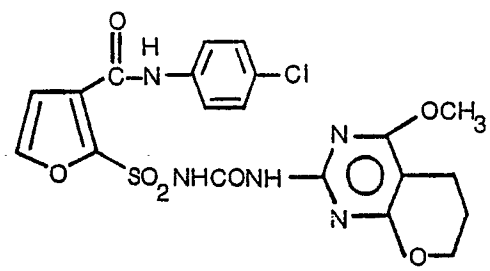

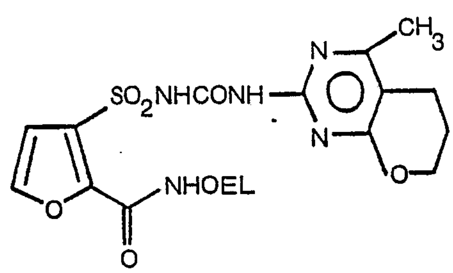

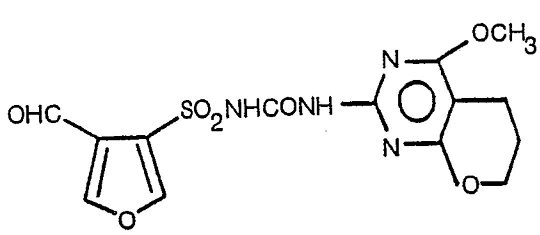

TABLE XI-a

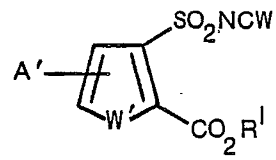

| A′ | W′ | W | $R^I$ |
|---|---|---|---|
| H | S | O | $C_2H_5$ |
| H | S | O | $CH(CH_3)_2$ |
| H | S | O | $CH_2CH_2Cl$ |
| H | S | O | $(CH_2)_6H$ |
| H | S | O | $CH—CH_2CH_3$<br>\|<br>$CH_3$ |
| H | S | O | $CH_2—CH=CH_2$ |
| H | S | O | $CH_2—CH=CH—CH_3$ |
| H | S | O | $CH_2CH=CH—CH_2CH_3$ |
| H | S | O | $CH_2$<br>‖<br>$CH_2—C—CH_3$ |
| H | S | O | $CH_2CH(CH_3)_2$ |
| H | S | O | $CH_2CH(CH_2CH_3)_2$ |
| H | S | O | $CH_2CH_2Cl$ |
| H | S | O | $CH_2CH(CH_2Cl)_2$ |
| H | S | O | $CH_2CCl_3$ |
| H | S | O | $(CH_2)_6Cl$ |
| H | S | O | $(CH_2)_6Br$ |
| H | S | O | $CH_2CH_2Br$ |
| H | S | O | $(CH_2)_6F$ |
| H | S | O | $(CH_2)_4F$ |
| H | S | O | $CH_2CN$ |
| H | S | O | $CH_2CH_2CN$ |
| H | S | O | $CH_2CH_2OCH_3$ |
| H | S | O | $(CH_2)_6OCH_3$ |
| H | S | O | $(CH_2)_3OCH_3$ |
| H | S | O | $CH—CH_2OCH_3$<br>\|<br>$CH_3$ |

TABLE XI-a (cont'd)

| A' | W' | W | R' |
|---|---|---|---|
| H | S | O | $CHCH_2OCH_3$<br>\|<br>$CH_3$ |
| H | S | O | $CH—CH_2OCH(CH_3)_2$<br>\|<br>$CH_3$ |
| H | S | O | $CH—CH_2OCH_2OCl_3$<br>\|<br>$CH_3$ |
| H | S | O | $CH_2CN$ |
| H | S | O | 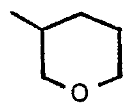 |
| H | S | O | 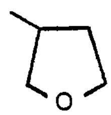 |
| H | S | O | 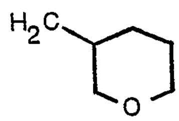 |
| H | S | O | 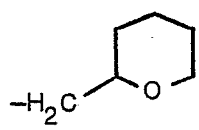 |
| H | S | O | 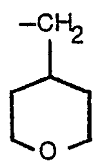 |
| H | S | O | 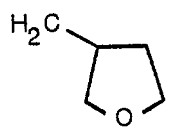 |
| H | S | O | 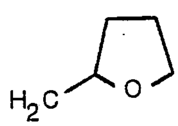 |
| H | S | O | 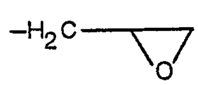 |
| H | S | O | $CH_2OCH_3$ |
| H | S | O | $CH_2OC_2H_5$ |
| H | S | O | $CH_2O(CH_2)_4H$ |

TABLE XI-a (cont'd)

| A′ | W′ | W | R′ |
|---|---|---|---|
| H | S | O | $CH_2CH{=}CHCl$ |
| H | S | O | $CH_2CH{=}CH{-}CH_2Cl$ |
| H | S | O | $CH_2CH{=}CH(CH_2)_3Cl$ |
| H | S | O | $CH_2C{\equiv}C{-}CH_3$ |
| H | S | O | $CH_2C{\equiv}C{-}CH_2Cl$ |
| H | S | O | $CH_2C{\equiv}C{-}CH_2CH_2{-}Cl$ |
| H | S | O | 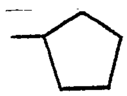 |
| H | S | O | 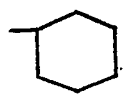 |
| H | S | O | 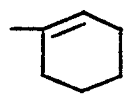 |
| H | S | O | 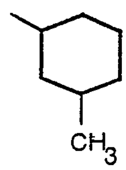 |
| H | S | O | 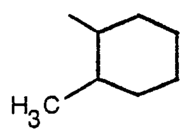 |
| H | S | O | 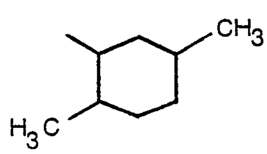 |
| H | S | O | 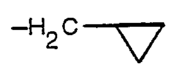 |
| H | S | O | 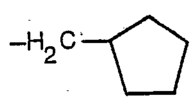 |
| H | S | O | 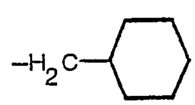 |
| H | S | O | 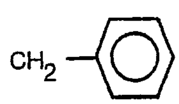 |

TABLE XI-a (cont'd)

| A' | W' | W | R' |
|---|---|---|---|
| H | S | O | 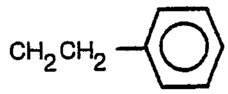 |
| H | S | O | 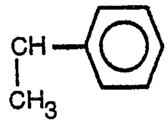 |
| H | S | O | 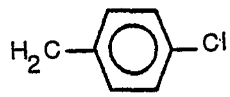 |
| H | S | O | 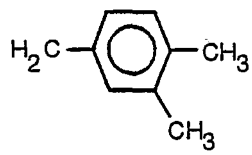 |
| H | S | O | 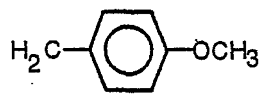 |
| H | S | O | $CH_2CH_2OCH_2CH_3$ |
| H | S | O | $CH_2CH_2OCH(CH_3)_2$ |
| H | S | O | 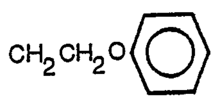 |
| H | S | O | $CH_2CH_2OCH_2CH_2Cl$ |
| H | S | O | $CH_2CH_2OCH_2CCl_3$ |
| H | S | O | $CH\ CH_2OC_2H_5$<br>\|<br>$CH_3$ |
| H | S | O | $CH—CH_2OCH(CH_3)_2$<br>\|<br>$CH_3$ |
| H | S | O | $(CH_2)_3OCH(CH_3)_2$ |
| H | S | O | $(CH_2)_3OCH_2CH_3$ |
| H | S | O | $CH_2CH_2OCH_2CH_2OCH_3$ |
| H | S | O | $CH_2CH_2(OCH_2CH_2)_2OC_2H_5$ |
| 5-$CH_3$ | S | O | $CH_3$ |
| H | O | O | $CH_3$ |
| H | S | S | $CH_3$ |
| 4-$CH_3$ | S | O | $CH_3$ |

TABLE XI-a (cont'd)

| A' | W' | W | R' |
|---|---|---|---|
| 4-Cl | S | O | $CH_3$ |
| 4-Br | S | O | $CH_3$ |
| 5-Br | S | O | $CH_3$ |
| 4-$NO_2$ | S | O | $CH_3$ |
| 4-$C_2H_5$ | S | O | $CH_3$ |
| 5-$C_2H_5$ | S | O | $CH_3$ |
| 5-*n*-$C_7H_9$ | S | O | $CH_3$ |
| 5-$CH(CH_3)_2$ | S | O | $CH_3$ |
| 4-$CF_3$ | S | O | $CH_3$ |
| 5-$OCH_3$ | S | O | $CH_3$ |

TABLE XI-b

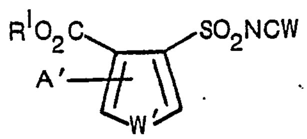

| A' | W' | W | R' |
|---|---|---|---|
| H | S | O | $C_2H_5$ |
| H | S | O | $CH(CH_3)_2$ |
| H | S | O | $CH_2CH_2Cl$ |
| H | S | O | $(CH_2)_6H$ |
| H | S | O | $CH—CH_2CH_3$<br>\|<br>$CH_3$ |
| H | S | O | $CH_2—CH{=}CH_2$ |
| H | S | O | $CH_2—CH{=}CH—CH_3$ |
| H | S | O | $CH_2CH{=}CH—CH_2CH_3$ |
| H | S | O | $CH_2$<br>‖<br>$CH_2—C—CH_3$ |
| H | S | O | $CH_2CH(CH_3)_2$ |
| H | S | O | $CH_2CH(CH_2CH_3)_2$ |
| H | S | O | $CH_2CH_2Cl$ |
| H | S | O | $CH_2CH(CH_2Cl)_2$ |

TABLE XI-b (cont'd)

| A′ | W′ | W | R′ |
|---|---|---|---|
| H | S | O | $CH_2OCl_3$ |
| H | S | O | $(CH_2)_6Cl$ |
| H | S | O | $(CH_2)_6Br$ |
| H | S | O | $CH_2CH_2Br$ |
| H | S | O | $(CH_2)_6F$ |
| H | S | O | $(CH_2)_4F$ |
| H | S | O | $CH_2CN$ |
| H | S | O | $CH_2CH_2CN$ |
| H | S | O | $CH_2CH_2OCH_3$ |
| H | S | O | $(CH_2)_6OCH_3$ |
| H | S | O | $(CH_2)_3OCH_3$ |
| H | S | O | $CH—CH_2OCH_3$<br>\|<br>$CH_3$ |
| H | S | O | $CHCH_2OCH_3$<br>\|<br>$CH_3$ |
| H | S | O | $CH—CH_2OCH(CH_3)_2$<br>\|<br>$CH_3$ |
| H | S | O | $CH—CH_2OCH_2OCl_3$ ,<br>\|<br>$CH_3$ |
| H | S | O | $CH_2CN$ |
| H | S | O | 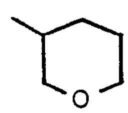 |
| H | S | O | 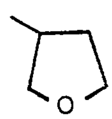 |
| H | S | O | 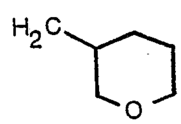 |
| H | S | O | 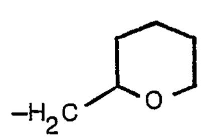 |

TABLE XI-b (cont'd)

| A' | W' | W | R' |
|---|---|---|---|
| H | S | O | 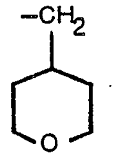 |
| H | S | O | 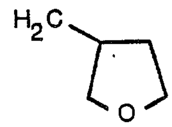 |
| H | S | O | 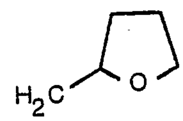 |
| H | S | O | 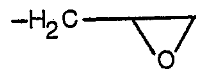 |
| H | S | O | $CH_2OCH_3$ |
| H | S | O | $CH_2OC_2H_5$ |
| H | S | O | $CH_2O(CH_2)_4H$ |
| H | S | O | $CH_2CH{=}CHCl$ |
| H | S | O | $CH_2CH{=}CH{-}CH_2Cl$ |
| H | S | O | $CH_2CH{=}CH(CH_2)_3Cl$ |
| H | S | O | $CH_2C{\equiv}C{-}CH_3$ |
| H | S | O | $CH_2C{\equiv}C{-}CH_2Cl$ |
| H | S | O | $CH_2C{\equiv}C{-}CH_2CH_2{-}Cl$ |
| H | S | O | 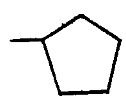 |
| H | S | O | 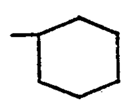 |
| H | S | O | 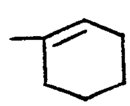 |
| H | S | O | 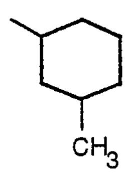 |

TABLE XI-b (cont'd)

| A' | W' | W | R' |
|---|---|---|---|
| H | S | O | 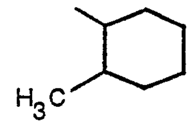 |
| H | S | O | 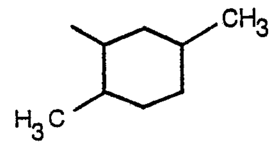 |
| H | S | O | 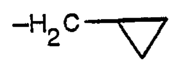 |
| H | S | O | 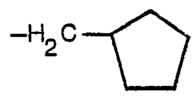 |
| H | S | O | 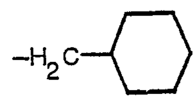 |
| H | S | O | 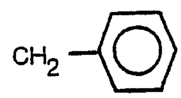 |
| H | S | O | 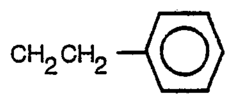 |
| H | S | O | 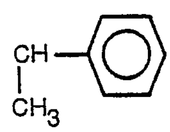 |
| H | S | O | 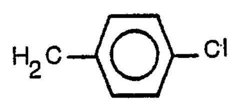 |
| H | S | O | 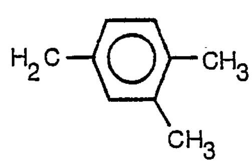 |
| H | S | O | 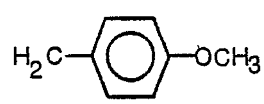 |
| H | S | O | $CH_2CH_2OCH_2CH_3$ |
| H | S | O | $CH_2CH_2OCH(CH_3)_2$ |
| H | S | O | 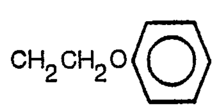 |

TABLE XI-b (cont'd)

| A′ | W′ | W | R′ |
|---|---|---|---|
| H | S | O | $CH_2CH_2OCH_2CH_2Cl$ |
| H | S | O | $CH_2CH_2OCH_2OCl_3$ |
| H | S | O | $CH(CH_3)CH_2OC_2H_5$ |
| H | S | O | $CH(CH_3)—CH_2OCH(CH_3)_2$ |
| H | S | O | $(CH_2)_3OCH(CH_3)_2$ |
| H | S | O | $(CH_2)_3OCH_2CH_3$ |
| H | S | O | $CH_2CH_2OCH_2CH_2OCH_3$ |
| H | S | O | $CH_2CH_2(OCH_2CH_2)_2OC_2H_5$ |
| 5-$CH_3$ | S | O | $CH_3$ |
| H | O | O | $CH_3$ |
| H | S | S | $CH_3$ |
| 4-$CH_3$ | S | O | $CH_3$ |
| 4-Cl | S | O | $CH_3$ |
| 4-Br | S | O | $CH_3$ |
| 5-Br | S | O | $CH_3$ |
| 4-$NO_2$ | S | O | $CH_3$ |
| 4-$C_2H_5$ | S | O | $CH_3$ |
| 5-$C_2H_5$ | S | O | $CH_3$ |
| 5-*n*-$C_7H_9$ | S | O | $CH_3$ |
| 5-$CH(CH_3)_2$ | S | O | $CH_3$ |
| 4-$CF_3$ | S | O | $CH_3$ |
| 5-$OCH_3$ | S | O | $CH_3$ |

TABLE XI-c

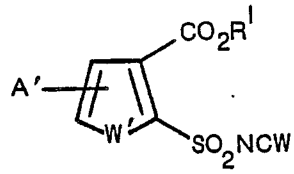

| A′ | W′ | W | R′ |
|---|---|---|---|
| H | S | O | $C_2H_5$ |
| H | S | O | $CH(CH_3)_2$ |
| H | S | O | $CH_2CH_2Cl$ |
| H | S | O | $(CH_2)_6H$ |
| H | S | O | $CH-CH_2CH_3$<br>\|<br>$CH_3$ |
| H | S | O | $CH_2-CH=CH_2$ |
| H | S | O | $CH_2-CH=CH-CH_3$ |
| H | S | O | $CH_2CH=CH-CH_2CH_3$ |
| H | S | O | $CH_2$<br>‖<br>$CH_2-C-CH_3$ |
| H | S | O | $CH_2CH(CH_3)_2$ |
| H | S | O | $CH_2CH(CH_2CH_3)_2$ |
| H | S | O | $CH_2CH_2Cl$ |
| H | S | O | $CH_2CH(CH_2Cl)_2$ |
| H | S | O | $CH_2OCl_3$ |
| H | S | O | $(CH_2)_6Cl$ |
| H | S | O | $(CH_2)_6Br$ |
| H | S | O | $CH_2CH_2Br$ |
| H | S | O | $(CH_2)_6F$ |
| H | S | O | $(CH_2)_4F$ |
| H | S | O | $CH_2CN$ |
| H | S | O | $CH_2CH_2CN$ |
| H | S | O | $CH_2CH_2OCH_3$ |
| H | S | O | $(CH_2)_6OCH_3$ |
| H | S | O | $(CH_2)_3OCH_3$ |
| H | S | O | $CH-CH_2OCH_3$<br>\|<br>$CH_3$ |

TABLE XI-c (cont'd)

| A' | W' | W | R' |
|---|---|---|---|
| H | S | O | $CHCH_2OCH_3$<br>$CH_3$ |
| H | S | O | $CH—CH_2OCH(CH_3)_2$<br>$CH_3$ |
| H | S | O | $CH—CH_2OCH_2OCl_3$<br>$CH_3$ |
| H | S | O | $CH_2CN$ |
| H | S | O | 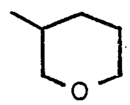 |
| H | S | O | 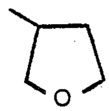 |
| H | S | O | 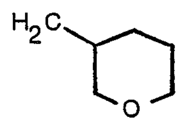 |
| H | S | O | 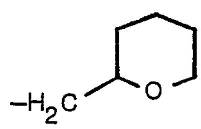 |
| H | S | O | 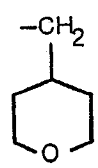 |
| H | S | O | 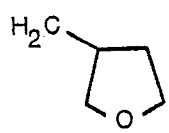 |
| H | S | O | 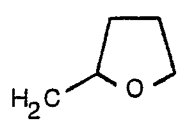 |
| H | S | O | 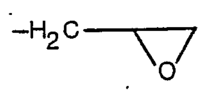 |
| H | S | O | $CH_2OCH_3$ |
| H | S | O | $CH_2OC_2H_5$ |
| H | S | O | $CH_2O(CH_2)_4H$ |

TABLE XI-c (cont'd)

| A' | W' | W | R' |
|---|---|---|---|
| H | S | O | $CH_2CH{=}CHCl$ |
| H | S | O | $CH_2CH{=}CH{-}CH_2Cl$ |
| H | S | O | $CH_2CH{=}CH(CH_2)_3Cl$ |
| H | S | O | $CH_2C{\equiv}C{-}CH_3$ |
| H | S | O | $CH_2C{\equiv}C{-}CH_2Cl$ |
| H | S | O | $CH_2C{\equiv}C{-}CH_2CH_2{-}Cl$ |
| H | S | O | 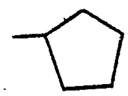 |
| H | S | O | 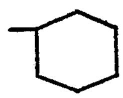 |
| H | S | O | 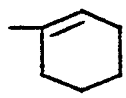 |
| H | S | O | 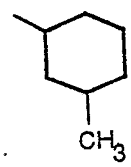 |
| H | S | O | 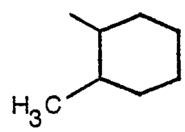 |
| H | S | O | 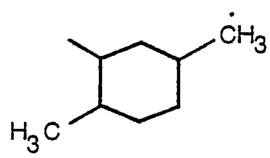 |
| H | S | O | 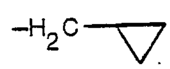 |
| H | S | O | 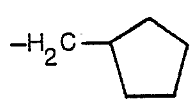 |
| H | S | O | 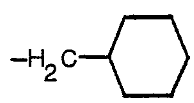 |
| H | S | O | 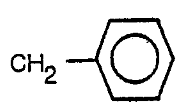 |
| H | S | O | 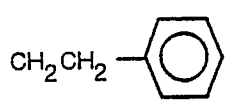 |

TABLE XI-c (cont'd)

| A′ | W′ | W | R′ |
|---|---|---|---|
| H | S | O | 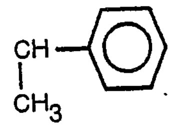 |
| H | S | O | 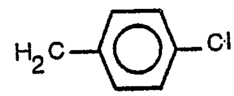 |
| H | S | O | 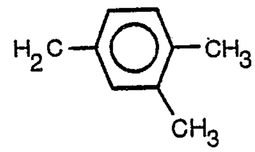 |
| H | S | O | 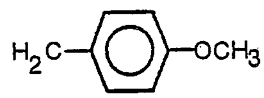 |
| H | S | O | $CH_2CH_2OCH_2CH_3$ |
| H | S | O | $CH_2CH_2OCH(CH_3)_2$ |
| H | S | O | 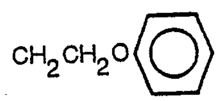 |
| H | S | O | $CH_2CH_2OCH_2CH_2Cl$ |
| H | S | O | $CH_2CH_2OCH_2CCl_3$ |
| H | S | O | $CH(CH_3)CH_2OC_2H_5$ |
| H | S | O | $CH(CH_3)—CH_2OCH(CH_3)_2$ |
| H | S | O | $(CH_2)_3OCH(CH_3)_2$ |
| H | S | O | $(CH_2)_3OCH_2CH_3$ |
| H | S | O | $CH_2CH_2OCH_2CH_2OCH_3$ |
| H | S | O | $CH_2CH_2(OCH_2CH_2)_2OC_2H_5$ |
| 5-$CH_3$ | S | O | $CH_3$ |
| H | O | O | $CH_3$ |
| H | S | S | $CH_3$ |
| 4-$CH_3$ | S | O | $CH_3$ |
| 4-Cl | S | O | $CH_3$ |
| 4-Br | S | O | $CH_3$ |

TABLE XI-c (cont'd)

| A' | W' | W | $R^1$ |
|---|---|---|---|
| 5-Br | S | O | $CH_3$ |
| 4-$NO_2$ | S | O | $CH_3$ |
| 4-$C_2H_5$ | S | O | $CH_3$ |
| 5-$C_2H_5$ | S | O | $CH_3$ |
| 5-*n*-$C_7H_9$ | S | O | $CH_3$ |
| 5-$CH(CH_3)_2$ | S | O | $CH_3$ |
| 4-$CF_3$ | S | O | $CH_3$ |
| 5-$OCH_3$ | S | O | $CH_3$ |

Formulations

Useful formulations of the compounds of Formulas I, II or III can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions set forth in Table XVIII.

TABLE XVIII

| | Weight Percent* | | |
|---|---|---|---|
| | Active Ingredient | Diluent(s) | Surfactant(s) |
| Wettable Powders | 20—90 | 0—74 | 1—10 |
| Oil Suspensions, Emulsions, Solutions (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

*Active ingredients plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers," 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates;

solution concentrates are preferably stable against phase separation at 0°C. "McCutcheon's Detergents and Emulsifiers Annual," MC Publishing Corp., Ridgewood, New Jersey, as well as Sisely and Wood, "Encyclopedia of Surface Active Agents," Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon performed granular carriers or by agglomeration techniques. See J. E. Browning, "Agglomeration," *Chemical Engineering,* December 4, 1967, pp. 147ff. and "Perry's Chemical Engineer's Handbook," 4th Ed., McGraw-Hill, New York, 1963, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41.

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167, 169—182.

H. Gysin and E. Knusli, U.S. Patent 2,891,855, June 23, 1959, Col. 3 line 66 through Col. 5, line 17 and Examples 1—4.

G. C. Klingman, "Weed Control as a Science", John Wiley & Sons, Inc., New York, 1961, pp. 81—96.

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 9

<u>Wettable Powder</u>

| | |
|---|---|
| methyl 3-[[(4,6-dimethylpyrimidin-2-yl)amino-carbonyl]aminosulfonyl]-2-thiophenecarboxylate | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 10

<u>Wettable Powder</u>

| | |
|---|---|
| methyl 3-[[(4,6-dimethoxypyrimidin-2-yl)amino-carbonyl]aminosulfonyl]-2-thiophenecarboxylate | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 11

<u>Granule</u>

| | |
|---|---|
| wettable powder of Example 10 | 5% |
| attapulgite granules | 95% |
| (U.S.S. 20—40 mesh; 0.84—0.42 mm) | |

A slurry of wettable powder containing ≈25% solids is sprayed on the surface of attapulgite granules in a double-cone blender. The granules are dried and packaged.

Example 12

Extruded Pellet

| | |
|---|---|
| methyl 3-[[(4-methoxy-6-methoxypyrimidin-2-yl)amino-carbonyl]aminosulfonyl]-2-thiophenecarboxylate | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 13

Oil Suspension

| | |
|---|---|
| methyl 3-[[(4,6-dimethylpyrimidin-2-yl)amino-carbonyl]aminosulfonyl]-2-thiophenecarboxylate | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting suspension may be applied directly, but preferably after being extended with oils or emulsified in water.

Example 14

Wettable Powder

| | |
|---|---|
| methyl 3-[[(4,6-dimethoxypyrimidin-2-yl)amino-carbonyl]aminosulfonyl]-2-thiophenecarboxylate | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer mill to produce particles essentially all below 100 microns, the material is reblended and sifted through a U.S.S. No. 50 sieve (0.3 mm opening) and packaged.

Example 15

Oil Suspension

| | |
|---|---|
| methyl 3-[[(4-methoxy-6-methylpyrimidin-2-yl)amino-carbonyl]aminosulfonyl]-2-thiophenecarboxylate | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 16

High Strength Concentrates

| | |
|---|---|
| methyl 3-[[(4,6-dimethylpyrimidin-2-yl)amino-carbonyl]aminosulfonyl]-2-thiophenecarboxylate | 99% |
| silica aerogel | 0.5% |
| synthetic amorphous silica | 0.5% |

The ingredients are blended and ground in a hammer mill to produce a material essentially all passing a U.S.S. No. 50 screen (0.3 mm opening). The concentrate may be formulated further if necessary.

Example 17

Low Strength Granule

| | |
|---|---|
| methyl 3-[[(4,6-dimethoxypyrimidin-2-yl)amino-carbonyl]aminosulfonyl]-2-thiophenecarboxylate | 1% |
| N,N-dimethylformamide | 9% |
| attapulgite granules | 90% |
| (U.S.S. 20—40 mesh). | |

The active ingredient is dissolved in the solvent and the solution is sprayed upon dedusted granules in a rotating blender. After spraying of the solution has been completed, the blender is allowed to run for a short period and then the granules are packaged.

Example 18

Aqueous Suspension

| | |
|---|---|
| methyl 3-[[(4-methoxy-6-methylpyrimidin-2-yl)amino-carbonyl]aminosulfonyl]-2-thiophenecarboxylate | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 19

Solution

| | |
|---|---|
| methyl 3-[[(4,6-dimethylpyrimidin-2-yl)amino-carbonyl]aminosulfonyl]-2-thiophenecarboxylate | 5% |
| water | 95% |

The salt is added directly to the water with stirring to produce the solution, which may then be packaged for use.

Example 20

Granule

| | |
|---|---|
| methyl 3-[[(4,6-dimethoxypyrimidin-2-yl)amino-carbonyl]aminosulfonyl]-2-thiophenecarboxylate | 80% |
| wetting agent | 1% |
| crude ligninsulfonate salt (containing 5—20% of the natural sugars) | 10% |
| attapulgite clay | 9% |

The ingredients are blended and milled to pass ghrough a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 21

Low Strength Granule

| | |
|---|---|
| methyl 3-[[(4-methoxy-6-methylpyrimidin-2-yl)amino-carbonyl]aminosulfonyl]-2-thiophenecarboxylate | 0.1% |
| attapulgite granules (U.S.S. 20—40 mesh) | 99.9% |

The active ingredient is dissolved in a solvent and the solution is sprayed upon dedusted granules in a double cone blender. After spraying of the solution has been completed, the material is warmed to evaporate the solvent. The material is allowed to cool and then packaged.

UTILITY

The compounds of the present invention are powerful herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for the selective pre- or post-emergence weed control in crops, such as wheat and soybeans.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.06 to 10 kg/ha, the lower rates being suggested for use on ighter soils and/or those having a low organic matter content, for selective weed control or for situations where only short-term persistence is required.

The compounds of the invention may be used in combination with any other commercial herbicide examples of which are those of the triazine, triazole, uracil, urea, amide, diphenylether, carbamate and bipyridylium types.

For use as a plant growth regulant, the compound will be used in such manner and amount as to be effective but substantially non-phytotoxic to the desirable plant species whose growth is to be regulated.

The herbicidal properties of the subject compounds were discovered in a number of greenhouse tests. The test procedures and results follow.

TEST A

Seeds of crabgrass (*Digitaria* spp.), barnyardgrass (*Echinochloa crusgalli*), wild oats (*Avena fatua*), *Cassia tora*, morningglory (*Ipomoea* spp.), cocklebur (*Xanthium* spp.), sorghum, corn, soybean, rice, wheat as well as nutsedge tubers were planted in a growth medium and treated preemergence with the

chemicals dissolved in a non-phytotoxic solvent. At the same time, cotton having five leaves (including cotyledonary ones), bush beans with the third trifoliate leaf expanding, crabgrass, barnyardgrass and wild oats with two leaves, cassia with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three-five leaves were sprayed. Treated plants and controls were maintained in a greenhouse for sixteen days, whereupon all species were compared to controls and visually rated for response to treatment. The ratings are based on a numerical scale extending from 0 = no injury, to 10 = complete kill. The accompanying descriptive symbols have the following meanings: B = burn; G = growth retardation; C = chlorosis/necrosis; D = defoliation; 6Y = abscised buds or flowers; 6F = delayed flowring; U = unusual pigmentation; S = albinism; E = emergence inhibition; and H = formative effects. The ratings for the compounds tested by this procedure are presented in Table A. It will be seen that certain of the compounds tested have utility for selective pre- and post-emergence weed control in soybeans and wheat.

TABLE A

| | 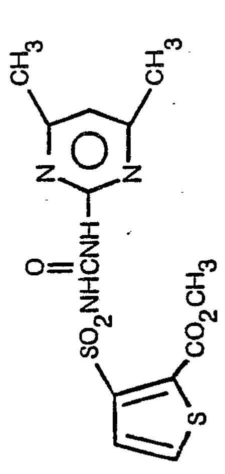 | 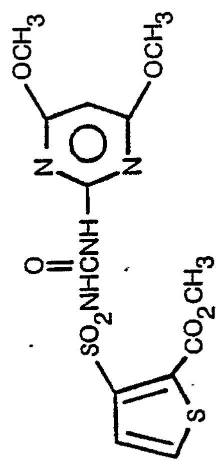 | 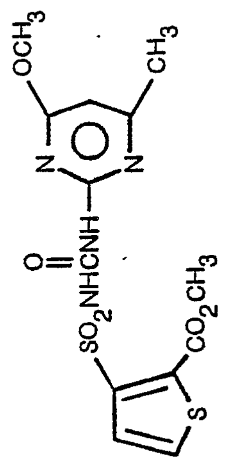 |
|---|---|---|---|
| Rate, kg/ha | 0.1 | 0.1 | 0.1 |
| POST-EMERGENCE | | | |
| Bushbean | 6C, 9G, 6Y | 5C, 8G, 6Y | 8D, 9G, 6Y |
| Cotton | 3U, 9G | 7C, 9G | 7C, 9G |
| Morningglory | 9C | 10C | 10C |
| Cocklebur | 5C, 9G | 9C | 3C, 9G |
| Cassia | 0 | 2C | 1C, 3G |
| Nutsedge | 5C, 9G | 1C, 9G | 4C, 9G |
| Crabgrass | 5C, 9G | 3C, 8G | 6C, 9G |
| Barnyardgrass | 6C, 9G | 9C | 9C |
| Wild Oats | 2G | 1C, 7G | 2C, 8G |
| Wheat | 1C, 9G | 2C, 8G | 3C, 7G |
| Corn | 1C, 9H | 2U, 9G | 1C, 9G |
| Soybean | 3G | 5C, 9G | 2C, 8G |
| Rice | 2C, 9G | 5C, 9G | 3C, 9G |
| Sorghum | 1C, 9G | 5U, 9G | 3C, 9G |
| PRE-EMERGENCE | | | |
| Morningglory | 9G | 9G | 9G |
| Cocklebur | 9G | 9H | 9H |
| Cassia | 6G | 8G | 8G |
| Nutsedge | 10E | 10E | 10E |
| Crabgrass | 1C, 7G | 1C, 7G | 1C, 8G |
| Barnyardgrass | 1C, 9H | 1C, 9H | 1C, 9H |
| Wild Oats | 1C, 6G | 1C, 8G | 1C, 8G |
| Wheat | 1C, 9G | 1C, 9G | 1C, 9H |
| Corn | 1C, 9H | 9G | 9G |
| Soybean | 0 | 6H | 6H |
| Rice | 10E | 10E | 10E |
| Sorghum | 10H | 10E | 1C, 9H |

TABLE A (Continued)

| | 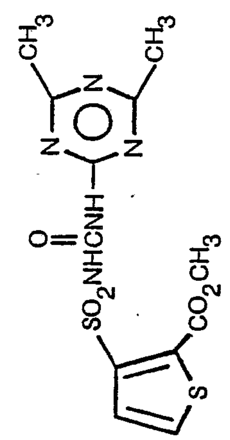 | 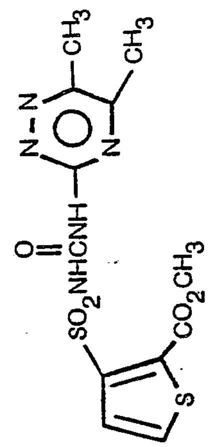 | 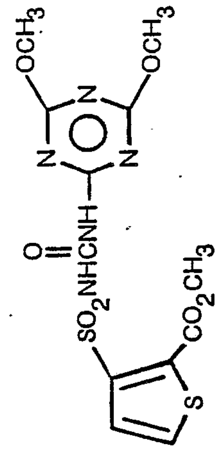 |
|---|---|---|---|
| Rate, kg/ha | 0.1 | 0.4 | 0.1 |
| POST-EMERGENCE | | | |
| Bushbean | 9C | 6C, 9G | 2C, 8G, 6Y |
| Cotton | 3C, 7G | 3C, 3H | 9C |
| Sorghum | 1C, 9G | 2C, 9H | 2C, 7H |
| Corn | 2C, 8H | 2C, 7H | 9H |
| Soybean | 1C, 2H | 2C, 3G | 5C, 9G |
| Wheat | 0 | 0 | 1H |
| Wild Oats | 0 | 0 | 1H |
| Rice | – | 1C, 5G | 2C, 8G |
| Barnyardgrass | 2C, 8H | 2C, 5H | 2C, 9H |
| Crabgrass | 1C, 5G | 4G | 1C |
| Morningglory | 5C, 8G | 1C, 3H | 10C |
| Cocklebur | 10C | 1C, 5G | 10C |
| Cassia | 1C | 2C, 2H | 2C, 7H |
| Nutsedge | 0 | 1C | 1C |
| PRE-EMERGENCE | | | |
| Sorghum | 1C, 9H | 3C, 9H | 1C, 7H |
| Corn | 1C, 4G | 1C, 5H | 5G |
| Soybean | 0 | 1C, 1H | 2C, 5H |
| Wheat | 0 | 0 | 0 |
| Wild Oats | 0 | 0 | 0 |
| Rice | 10E | 10E | 10E |
| Barnyardgrass | 1C, 5G | 3C | 2C, 7G |
| Crabgrass | 0 | 3C | 5G |
| Morningglory | 9G | 5G | 9H |
| Cocklebur | 8H | 9G | 9H |
| Cassia | 2C | 2G | 6G |
| Nutsedge | 0 | 10E | 7G |

TABLE A (Continued)

| | 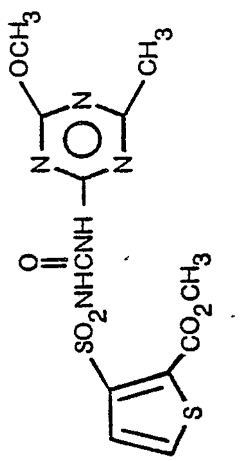 | 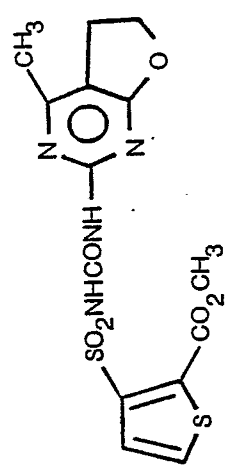 | 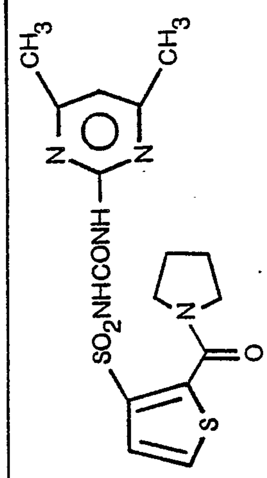 |
|---|---|---|---|
| Rate, kg/ha | 0.1 | 0.4 | 0.05 |
| POST-EMERGENCE | | | |
| Bushbean | 9C | 3C, 4G, 6F | 3C |
| Cotton | 9C | 1C, 7G | 4C, 8G |
| Sorghum | 9G | 3C, 4G | 1C, 5H |
| Corn | 7C | 3U, 5G | 1C, 2G |
| Soybean | 2C | 4G | 1H |
| Wheat | 0 | 3C, 5G | 2C |
| Wild Oats | 0 | 2C, 3G | 1C |
| Rice | 5C | 3C, 4G | 1C, 2G |
| Barnyardgrass | 10C | 10C | 1C |
| Crabgrass | 3C | 10C | 0 |
| Morningglory | 10C | 10C | 4C, 9H |
| Cocklebur | 6C | 10C | 2C, 9G |
| Cassia | 1C | 0 | 2C |
| Nutsedge | 1C | 5C, 5G | 0 |
| PRE-EMERGENCE | | | |
| Sorghum | 1C, 9H | 7C, 8G | 0 |
| Corn | 2C, 8G | | 0 |
| Soybean | 2C, 4G | 1C, 3G | 0 |
| Wheat | 3G | 10E | 0 |
| Wild Oats | 0 | 3C, 4G | 0 |
| Rice | 9H | 10E | 0 |
| Barnyardgrass | 2C, 8G | 5C, 8G | 0 |
| Crabgrass | 2C | 4C, 7G | 0 |
| Morningglory | 9G | 5G, 3C | 0 |
| Cocklebur | 8H | 6C, 5G | 0 |
| Cassia | 8G | 4G | 0 |
| Nutsedge | 3G | 10E | 0 |

TABLE A (Continued)

| | 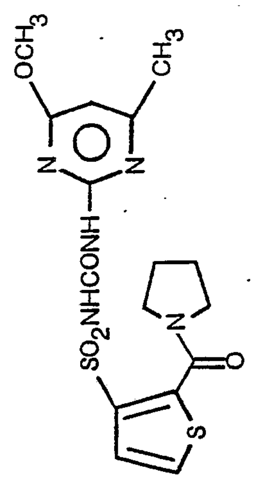 | 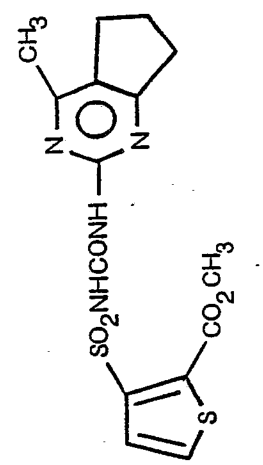 | 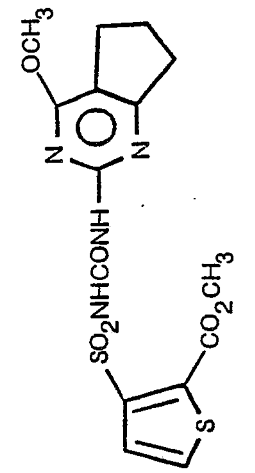 |
|---|---|---|---|
| Rate, kg/ha | 0.05 | 0.05 | 0.05 |
| POST-EMERGENCE | | | |
| Bushbean | 6Y, 7C, 9G | 2B | 1B |
| Cotton | 6C, 9G | 0 | 3G |
| Sorghum | 2C, 9H | 2C, 8H | 0 |
| Corn | 2C, 8H | 0 | 0 |
| Soybean | 2C, 8G | 1H | 0 |
| Wheat | 2C, 9G | 0 | 0 |
| Wild Oats | 2C | 0 | 0 |
| Rice | 2C, 9G | 5G | 0 |
| Barnyardgrass | 3C, 7H | 0 | 0 |
| Crabgrass | 2C | 0 | 0 |
| Morningglory | 10C | 1C, 3G | 0 |
| Cocklebur | 6C, 9G | 1C | 0 |
| Cassia | 1C, 4G | 1C | 0 |
| Nutsedge | 2C, 8G | 0 | 0 |
| PRE-EMERGENCE | | | |
| Sorghum | 2C, 8H | 6H | 0 |
| Corn | 1C, 9H | 1C, 5G | 0 |
| Soybean | 1C, 3H | 0 | 0 |
| Wheat | 9G | 0 | 0 |
| Wild Oats | 2C, 7G | | 0 |
| Rice | 4C, 9H | 2C | 0 |
| Barnyardgrass | 9H, 3C | 1C | 0 |
| Crabgrass | 1C | 0 | 0 |
| Morningglory | 9H | 0 | 0 |
| Cocklebur | 9H | 9H | 9H |
| Cassia | 5G | 2C | 0 |
| Nutsedge | 10E | 0 | 0 |

TABLE A (Continued)

| | 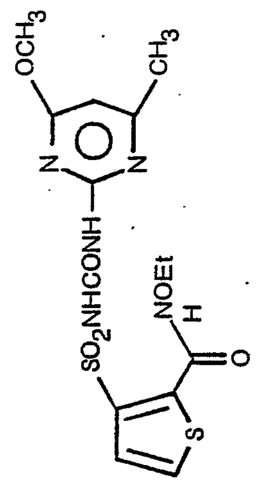 | 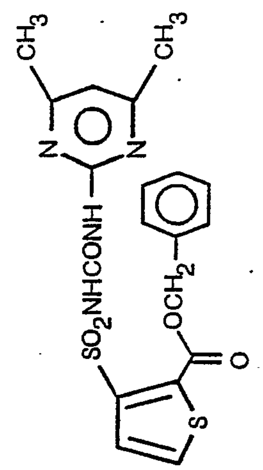 | 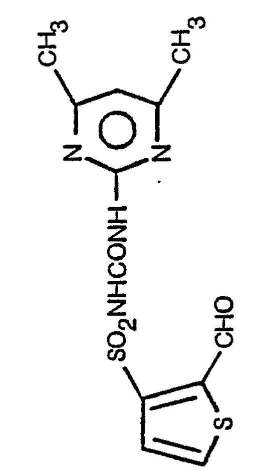 |
|---|---|---|---|
| Rate, kg/ha | 0.05 | 0.05 | 0.05 |
| POST-EMERGENCE | | | |
| Bushbean | 6Y, 1B, 2H | 0 | 3D, 6Y |
| Cotton | 4C, 9G | 0 | 0 |
| Sorghum | 1C, 9G | 0 | 2C, 9G |
| Corn | 2C, 8H | 0 | 2C, 7H |
| Soybean | 2C, 3G | 0 | 1H |
| Wheat | 0 | 0 | 0 |
| Wild Oats | 0 | 0 | 0 |
| Rice | 1C, 3G | 0 | 8G |
| Barnyardgrass | 2C, 6H | 0 | 2G |
| Crabgrass | 1C | 0 | 0 |
| Morningglory | 3C, 9G | 0 | 0 |
| Cocklebur | 3C, 9H | 0 | 0 |
| Cassia | 2C | 0 | 0 |
| Nutsedge | 2C, 5G | 0 | 2G |
| PRE-EMERGENCE | | | |
| Sorghum | 2C | 0 | 6H |
| Corn | 1C, 5C | 0 | 2G |
| Soybean | 2C, 4H | 0 | 3H |
| Wheat | 0 | 0 | 0 |
| Wild Oats | 0 | 0 | 0 |
| Rice | 1C, 6G | 0 | 5G |
| Barnyardgrass | 5H | 0 | 1H |
| Crabgrass | 0 | 0 | 0 |
| Morningglory | 9G | 0 | 0 |
| Cocklebur | 5G | 0 | 3C, 8H |
| Cassia | 0 | 0 | 1H |
| Nutsedge | 0 | 0 | 0 |

TABLE A (Continued)

| | 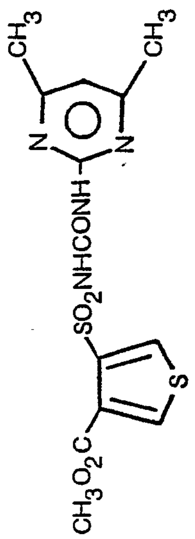 | 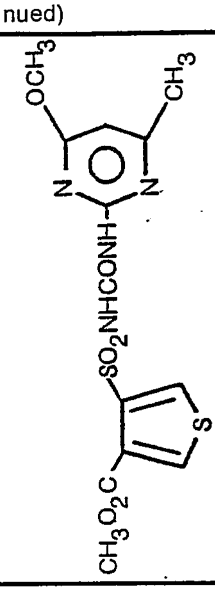 | 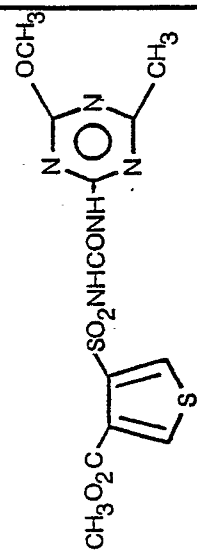 |
|---|---|---|---|
| Rate, kg/ha | 0.4 | 0.4 | 0.4 |
| POST-EMERGENCE | | | |
| Bushbean | 9C | 9C | 9C |
| Cotton | 10C | 9C | 9C |
| Sorghum | 9C | 10C | 6C, 9G |
| Corn | 7U, 9C | 10C | 5U, 10C |
| Soybean | 9C | 9C | 6C, 9G |
| Wheat | 9C | 9C | 4C, 8G |
| Wild Oats | 9C | 9C | 8C |
| Rice | 8C | 8C | 5C, 9G |
| Barnyardgrass | 9C | 9C | 9C |
| Crabgrass | 9C | 9C | 9C |
| Morningglory | 10C | 10C | 10C |
| Cocklebur | 10C | 10C | 9C |
| Cassia | 9C | 9C | 9C |
| Nutsedge | 7C, 9G | 9C | 6C, 9G |
| PRE-EMERGENCE | | | |
| Sorghum | 10E | 10E | 6C, 9H |
| Corn | 10E | 10E | 10E |
| Soybean | 9H | 9H | 9H |
| Wheat | 10E | 10E | 3C, 9G |
| Wild Oats | 5C, 9H | 5C, 9H | 3C, 9G |
| Rice | 10E | 10E | 10E |
| Barnyardgrass | 9H | 5C, 9H | 5C, 9H |
| Crabgrass | 10E | 10E | 6C, 9G |
| Morningglory | 9H | 9H | 9G |
| Cocklebur | 9H | 9H | 9H |
| Cassia | 10C | 9H | 9G |
| Nutsedge | 10E | 10E | 9G |

TABLE A (Continued)

| | 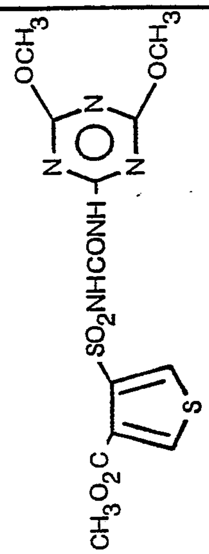 | 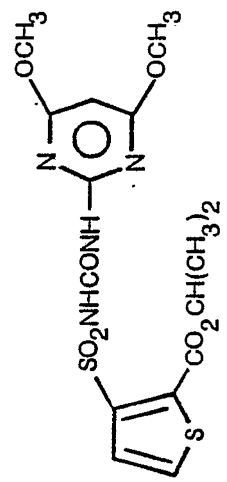 | 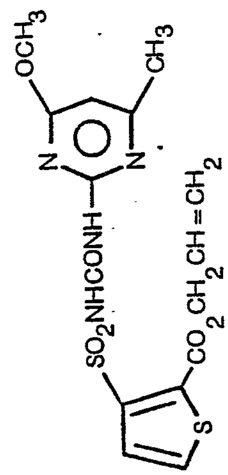 |
|---|---|---|---|
| Rate, kg/ha | 0.4 | 0.1 | 0.05 |
| POST-EMERGENCE | | | |
| Bushbean | 9C | 9C | 7C, 9G, 6Y |
| Cotton | 9C | 9C | 6C, 9G |
| Sorghum | 2C, 9G | 2C | 1C, 9G |
| Corn | 8U, 9C | 3C | 5C, 9G |
| Soybean | 5C, 9G | 9C | 6H |
| Wheat | 1C, 5G | 0 | 1C, 6G |
| Wild Oats | 2C, 8G | 0 | 2G |
| Rice | 9C | 5C | 8G |
| Barnyardgrass | 9C | 3C | 4C, 9H |
| Crabgrass | 8C | 1C | 5G |
| Morningglory | 10C | 10C | 10C |
| Cocklebur | 9C | 10C | 5C, 9G |
| Cassia | 9C | 2C | 1C |
| Nutsedge | 9C | 1C | 9G |
| PRE-EMERGENCE | | | |
| Sorghum | 6C, 9H | 4C, 9G | 3C, 9G |
| Corn | 3C, 9H | 3C, 9G | 1C, 9G |
| Soybean | 9H | 9H | 1C, 3G |
| Wheat | 1C, 8H | 1C, 3G | 1C, 9G |
| Wild Oats | 3C, 9G | 2C, 7G | 1C, 8G |
| Rice | 10E | 9H | 10E |
| Barnyardgrass | 5C, 9H | 3C, 9H | 2C,9H |
| Crabgrass | 3C, 9H | 2G | 1C |
| Morningglory | 9H | 9G | 9G |
| Cocklebur | 9H | 9H | 9H |
| Cassia | 3C, 9G | 6G | 8G |
| Nutsedge | 10E | 10E | 10E |

TABLE A (Continued)

| | 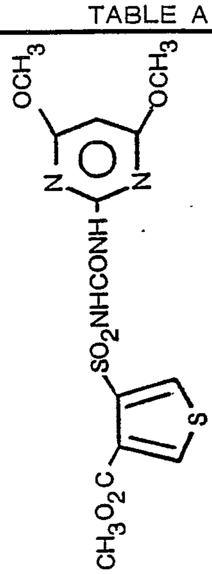 | 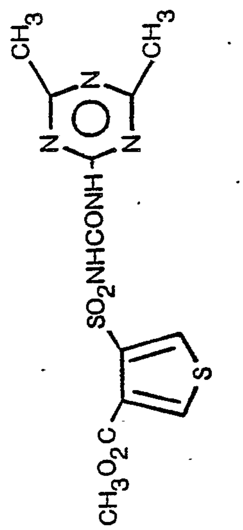 | 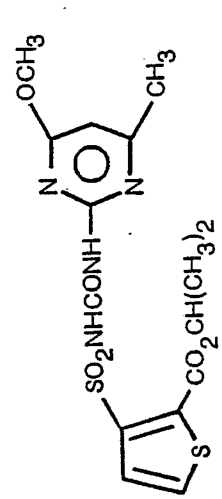 |
|---|---|---|---|
| Rate, kg/ha | 0.4 | 0.4 | 0.1 |
| POST-EMERGENCE | | | |
| Bushbean | 9C | 9C | 9C |
| Cotton | 9C | 9C | 9C |
| Sorghum | 9C | 9C | 5C |
| Corn | 6U, 9C | 5U, 9C | 5C |
| Soybean | 9C | 9C | 9C |
| Wheat | 9C | 8C | 1C |
| Wild Oats | 9C | 9C | 2C |
| Rice | 6C, 9G | 6C, 9G | 9C |
| Barnyardgrass | 9C | 9C | 9C |
| Crabgrass | 9C | 9C | 1C |
| Morningglory | 10C | 10C | 9C |
| Cocklebur | 9C | 9C | 3C |
| Cassia | 9C | 6C, 9G | 1C |
| Nutsedge | 9C | 9C | 2C |
| PRE-EMERGENCE | | | |
| Sorghum | 2C, 9G | 10E | 5C, 9H |
| Corn | 10E | 10E | 5C, 9G |
| Soybean | 9H | 9H | 9H |
| Wheat | 10E | 1E, 9G | 9G |
| Wild Oats | 2C, 9H | 5C, 9H | 3C, 9G |
| Rice | 10E | 10E | 10E |
| Barnyardgrass | 3C, 9H | 5C, 9H | 5C, 9H |
| Crabgrass | 10E | 5C, 9G | 2C, 7G |
| Morningglory | 9G | 9G | 9H |
| Cocklebur | 9H | 9H | 9H |
| Cassia | 6C, 9G | 9G | 9G |
| Nutsedge | 10E | 9G | 9G |

TEST B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinum*), mustard (*Brassica arvensis*), cocklebur (*Xanthium pennsylvanicum*), pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A 12.5 cm diameter plastic pot was also filled with prepared oil and planted with rice and wheat. Another 12.5 pot was planted with sugarbeets. The above four containers were treated preemergence with certain test compounds within the scope of the invention.

Twenty-eight days after treatment, the plants were evaluated and visually rated for response to the chemical treatments utilizing the rating system described previously for Test A. The data are summarized in Table B. Note that certain compounds are useful as pre-emergence treatments for weed control in crops such as soybeans and wheat.

TABLE B

| PRE-EMERGENCE ON FALLSINGTON SILT LOAM | | |
|---|---|---|
| | 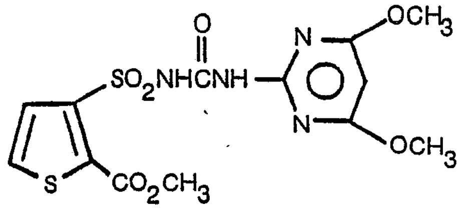 | |
| Rate, kg/ha | 0.03 | 0.12 |
| | | |
| Crabgrass | 8G, 7C | 10E |
| Barnyardgrass | 7G, 3H | 8G, 9C |
| Sorghum | 10E | 10E |
| Wild Oats | 2G | 5G |
| Johnsongrass | 7G, 3H | 8G, 5H |
| Dallisgrass | 3G | 4G |
| Giant Foxtail | 8G, 8C | 9G, 9C |
| Ky. bluegrass | – | – |
| Cheatgrass | 8G | 7G |
| Sugarbeets | 8G, 6C | 8G, 7C |
| Corn | 5G, 5H | 7G, 5H |
| Mustard | 8G, 8C | 9G, 9C |
| Cocklebur | 6G, 3H | 6G, 3H |
| Pigweed | 10C | 10C |
| Nutsedge | 7G | 8G |
| Cotton | 8G | 8G |
| Morningglory | 7G | 8G |
| Cassia | 3G | 3G |
| Teaweed | 5G | 6G |
| Velvetleaf | 6G, 3H | 7G, 5C |
| Jimsonweed | 6G, 6C | 6G, 6C |
| Soybean | 0 | 4G |
| Rice | 10E | 10E |
| Wheat | 3G, 3C | 4G, 3C |
| | | |
| | | |
| | | |

TABLE B (Continued)

| PRE-EMERGENCE ON FALLSINGTON SILT LOAM | | |
|---|---|---|
| | 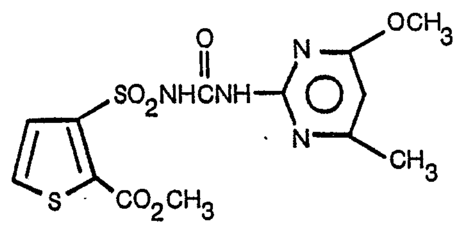 | |
| Rate, kg/ha | 0.03 | 0.12 |
| | | |
| Crabgrass | 7G, 5C | 10C |
| Barnyardgrass | 7G, 6C | 8G, 9C |
| Sorghum | 10E | 10E |
| Wild Oats | 5G | 7G |
| Johnsongrass | 6G, 5H | 9G, 8C |
| Dallisgrass | 7G | 8G |
| Giant Foxtail | 6G, 3C | 9G, 3C |
| Ky. bluegrass | – | – |
| Cheatgrass | 8G | 10E |
| Sugarbeets | 8G, 7C | 8G, 7C |
| Corn | 5G, 5H | 4G, 3H |
| Mustard | 9G, 9C | 9G, 9C |
| Cocklebur | 7G, 4C | 6G, 5H |
| Pigweed | 9G, 9C | 10C |
| Nutsedge | 7G | 9G |
| Cotton | 8G | 8G |
| Morningglory | 7G | 8G |
| Cassia | 3G | 4G |
| Teaweed | 4G | 5G, 2C |
| Velvetleaf | 7G, 7C | 10C |
| Jimsonweed | 5G | 5G, 3C |
| Soybean | 0 | 6G, 4C |
| Rice | 10E | 10E |
| Wheat | 7G, 4C | 7G, 4C |
| | | |
| | | |
| | | |

TABLE B (Continued)

| PRE-EMERGENCE ON FALLSINGTON SILT LOAM | | |
|---|---|---|
| | 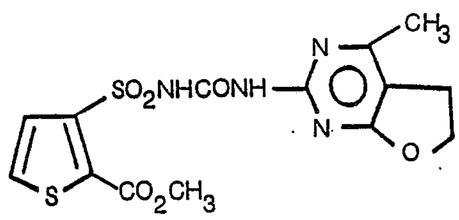 | |
| Rate, kg/ha | 0.03 | 0.12 |
| | | |
| Crabgrass | 3G | 4G |
| Barnyardgrass | 3G | 5G |
| Sorghum | 3G | 5G, 3H |
| Wild Oats | 0 | 0 |
| Johnsongrass | 0 | 4G, 3H |
| Dallisgrass | 3G | 4G |
| Giant Foxtail | 0 | 3G |
| Ky. bluegrass | 0 | 5G |
| Cheatgrass | 3G | 5G |
| Sugarbeets | 0 | 3G |
| Corn | 2C | 2C, 2U |
| Mustard | 5G | 7G |
| Cocklebur | 0 | 0 |
| Pigweed | 3G | 4G |
| Nutsedge | 0 | 0 |
| Cotton | 0 | 3G |
| Morningglory | 3G | 5G |
| Cassia | 0 | 0 |
| Teaweed | 0 | 0 |
| Velvetleaf | 0 | 0 |
| Jimsonweed | 0 | 0 |
| Soybean | 0 | 0 |
| Rice | 5G, 3C | 7G, 8C |
| Wheat | 0 | 3G |
| | | |
| | | |
| | | |

TABLE B (Continued)

| PRE-EMERGENCE ON FALLSINGTON SILT LOAM | | | |
|---|---|---|---|
| | 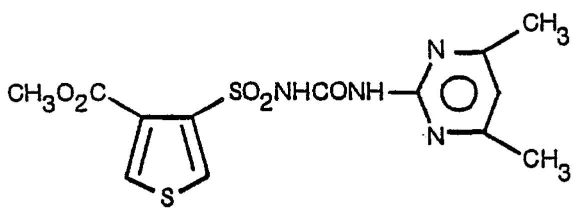 | | |
| Rate, kg/ha | 0.03 | 0.12 | 0.007 |
| | | | |
| Crabgrass | 8G | 9G, 8C | 6G |
| Barnyardgrass | 9G, 7C | 9G, 7C | 9G, 9C |
| Sorghum | 10E | 10E | 8G, 9C |
| Wild Oats | 6G, 3H | 8G, 8C | 6G, 3H |
| Johnsongrass | 8G, 3C | 9G, 9C | 8G, 5C |
| Dallisgrass | – | – | 5G |
| Giant Foxtail | 6G, 3H | 9G, 8C | 3G |
| Ky. bluegrass | 7G, 7C | 8G, 9C | 7G |
| Cheatgrass | 10E | 10E | 10E |
| Sugarbeets | 9G, 9C | 9G, 9C | 7G, 6C |
| Corn | 7G, 5H | 9G, 9C | 5G, 2C |
| Mustard | 9G, 9C | 9G, 9C | 8G, 8C |
| Cocklebur | 6G, 3H | 7G, 5H | 6G |
| Pigweed | 9G, 8C | 10E | 10C |
| Nutsedge | 7G | 6G | 6G |
| Cotton | 7G | 8G, 3C | 2G |
| Morningglory | 8G, 3C | 9G, 8C | 7G |
| Cassia | 7G | 7G | 5G |
| Teaweed | 6G | 7G | 2G |
| Velvetleaf | 8G, 3C | 10C | 9G, 5H |
| Jimsonweed | 7G | 8G, 4C | 4G |
| Soybean | 4G | 7G, 5H | 2G |
| Rice | 10E | 10E | 9G, 9C |
| Wheat | 6G, 3H | 8G, 8C | 6G |
| | | | |
| | | | |
| | | | |

TABLE B (Continued)

| PRE-EMERGENCE ON FALLSINGTON SILT LOAM | | | |
|---|---|---|---|
| | 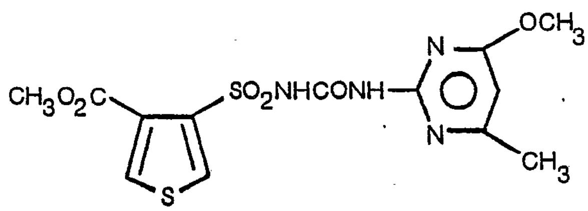 | | |
| Rate, kg/ha | 0.03 | 0.12 | 0.007 |
| | | | |
| Crabgrass | 8G | 9G, 8C | 7G |
| Barnyardgrass | 9G, 8C | 9G, 9C | 9C, 8G |
| Sorghum | 10E | 10E | 9G, 9C |
| Wild Oats | 7G, 3H | 8G, 6C | 4G |
| Johnsongrass | 8G, 3C | 10C | 8G, 3C |
| Dallisgrass | – | – | 5G |
| Giant Foxtail | 9G, 5H | 10C | 5G, 2C |
| Ky. bluegrass | 9G, 9C | 10C | 8G |
| Cheatgrass | 10E | 10E | 10E |
| Sugarbeets | 9G, 9C | 9G, 9C | 7G, 8C |
| Corn | 7G, 5H | 9G, 9C | 7G, 3H |
| Mustard | 9G, 9C | 10C | 7G, 8C |
| Cocklebur | 6G | 8G, 5H | 4G |
| Pigweed | 10E | 10E | 9G, 9C |
| Nutsedge | 5G | 7G | 5G |
| Cotton | 7G, 3H | 9G, 8C | 4G, 2H |
| Morningglory | 8G, 3C | 9G, 6C | 7G, 3C |
| Cassia | 7G | 7G | 4G |
| Teaweed | 7G | 7G, 5C | 4G |
| Velvetleaf | 8G, 6C | 10C | 8G, 5H |
| Jimsonweed | 7G, 5C | 9G, 9C | 4G |
| Soybean | 7G, 5H | 8G, 5H | 3G |
| Rice | 10E | 10E | 9G, 9C |
| Wheat | 6G | 8G, 8C | 5G |
| | | | |
| | | | |
| | | | |

TABLE B (Continued)

| PRE-EMERGENCE ON FALLSINGTON SILT LOAM | | | |
|---|---|---|---|
| | 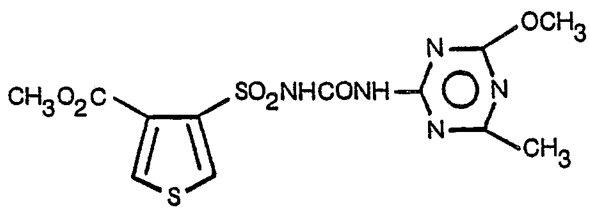 | | |
| Rate, kg/ha | 0.03 | 0.12 | 0.007 |
| | | | |
| Crabgrass | 7G, 3C | 9G, 9C | 2G |
| Barnyardgrass | 6G, 3C | 7G, 3C | 4G |
| Sorghum | 8G, 3C | 10C | 8G, 3H |
| Wild Oats | 4G | 6G | 2G |
| Johnsongrass | 8G, 4C | 8G, 4C | 4G |
| Dallisgrass | 0 | 5G | 0 |
| Giant Foxtail | 0 | 7G, 3H | 0 |
| Ky. bluegrass | 8G, 8C | 8G, 8C | 0 |
| Cheatgrass | 5G | 7G, 3H | 3G |
| Sugarbeets | 9G, 9C | 10C | 10C |
| Corn | 9G, 7C | 9G, 9C | 7G, 3H |
| Mustard | 10C | 10C | 10C |
| Cocklebur | 8G, 5H | 9G, 5H | 6G |
| Pigweed | 9G, 9C | 10C | 9G, 9C |
| Nutsedge | 6G | 6G | 3G |
| Cotton | 8G, 5H | 8G, 5H | 6G, 3H |
| Morningglory | 8G, 3H | 9G, 5H | 9G, 8C |
| Cassia | 9G, 8C | – | 7G |
| Teaweed | 7G, 3C | 7G, 3C | 4G |
| Velvetleaf | 9G, 9C | 10C | 8G |
| Jimsonweed | 6G, 5C | 7G, 7C | 5G |
| Soybean | 9G, 5H | 9G, 5H | 6G, 3H |
| Rice | 10E | 10E | 6G |
| Wheat | 4G | 4G | 0 |
| | | | |
| | | | |
| | | | |

TABLE B (Continued)

| PRE-EMERGENCE ON FALLSINGTON SILT LOAM | | | |
|---|---|---|---|
| | 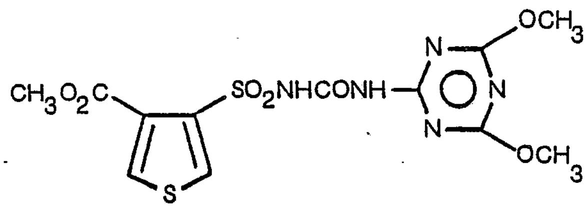 | | |
| Rate, kg/ha | 0.03 | 0.12 | 0.007 |
| | | | |
| Crabgrass | 3G | 6G, 3C | 0 |
| Barnyardgrass | 5G | 7G, 4C | 4G |
| Sorghum | 8G, 3H | 9G, 9C | 7G, 3H |
| Wild Oats | 2G | 4G | 0 |
| Johnsongrass | 5G, 3H | 7G, 3H | 5G |
| Dallisgrass | 0 | 6G | 2G |
| Giant Foxtail | 0 | 3G | 0 |
| Ky. bluegrass | 6G | 8G, 8C | 3G |
| Cheatgrass | 0 | 7G | 0 |
| Sugarbeets | 10C | 9G, 9C | 9G, 9C |
| Corn | 6G, 3H | 6G, 5H | 2G, 1U |
| Mustard | 10C | 10C | 10E |
| Cocklebur | 6G | 7G, 5H | 6G |
| Pigweed | 8G, 8C | 10C | 7G, 5E |
| Nutsedge | 2G | 7G | 6G |
| Cotton | 6G, 5H | 9G, 5H | 6G, 3H |
| Morningglory | 8G, 3H | 8G, 3H | 9G, 8C |
| Cassia | 6G | – | 4G |
| Teaweed | 6G, 2C | 7G, 3C | 3G |
| Velvetleaf | 8G, 5H | 8G, 8C | 6G |
| Jimsonweed | 5G | 6G, 3C | 5G |
| Soybean | 5G | 9G, 5H | 4G, 3H |
| Rice | 7G | 10E | 5G |
| Wheat | 2G | 2G | 0 |
| | | | |
| | | | |
| | | | |

TABLE B (Continued)

| PRE-EMERGENCE ON FALLSINGTON SILT LOAM | | |
|---|---|---|
| | 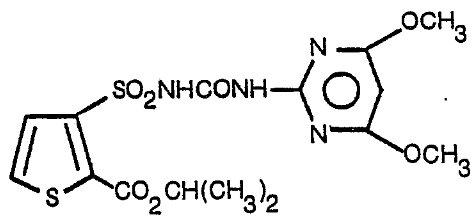 | |
| Rate, kg/ha | 0.03 | 0.12 |
| | | |
| Crabgrass | 0 | 0 |
| Barnyardgrass | 3G | 3G |
| Sorghum | 0 | 6G, 3H |
| Wild Oats | 0 | 0 |
| Johnsongrass | 0 | 5G, 3H |
| Dallisgrass | 0 | 3G |
| Giant Foxtail | 0 | 2H |
| Ky. bluegrass | 4G | 6G |
| Cheatgrass | 0 | 10E |
| Sugarbeets | 3G | 3G |
| Corn | 0 | 0 |
| Mustard | 8G, 5C | 10C |
| Cocklebur | 0 | 0 |
| Pigweed | 4G | 8G, 8C |
| Nutsedge | 0 | 7G |
| Cotton | 0 | 3G |
| Morningglory | 3G | 4G |
| Cassia | 0 | 0 |
| Teaweed | 0 | 3G |
| Velvetleaf | 5G, 3H | 6G, 3H |
| Jimsonweed | 3G | 5G |
| Soybean | 0 | 2G |
| Rice | 4G | 5G, 3H |
| Wheat | 0 | 0 |
| | | |
| | | |
| | | |

TABLE B (Continued)

| PRE-EMERGENCE ON FALLSINGTON SILT LOAM | | |
|---|---|---|
| | 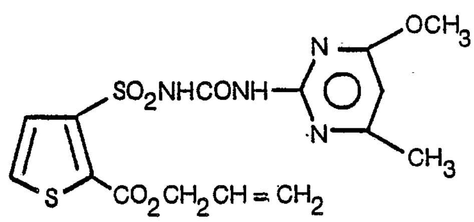 | |
| Rate, kg/ha | 0.03 | 0.12 |
| | | |
| Crabgrass | 0 | 0 |
| Barnyardgrass | 6G | 6G, 5H |
| Sorghum | 7G, 3H | 10C |
| Wild Oats | 3G | 3G |
| Johnsongrass | 6G, 3H | 8G, 5E |
| Dallisgrass | 0 | 4G |
| Giant Foxtail | 0 | 3G |
| Ky. bluegrass | 7G, 4C | 7G, 4C |
| Cheatgrass | 7G, 5C | 9G, 9C |
| Sugarbeets | 6G, 3H | 10C |
| Corn | 4G, 2U | 6G, 5H |
| Mustard | 10C | 10C |
| Cocklebur | 2G | 3G |
| Pigweed | 8G, 8C | 10C |
| Nutsedge | 7G | 7G |
| Cotton | 5G | 5G, 2H |
| Morningglory | 5G | 4G |
| Cassia | 5G | 5G |
| Teaweed | 2G | 4G |
| Velvetleaf | 8G, 5H | 9G,5H |
| Jimsonweed | 8G | 8G |
| Soybean | 4G | 4G |
| Rice | 7G | 8G |
| Wheat | 4G | 6G |
| | | |
| | | |
| | | |

TABLE B (Continued)

| PRE-EMERGENCE ON FALLSINGTON SILT LOAM | | |
|---|---|---|
| | 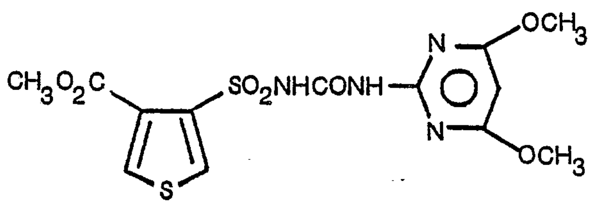 | |
| Rate, kg /ha | 0.03 | 0.12 |
| | | |
| Crabgrass | 8G, 5C | 9G, 7C |
| Barnyardgrass | 9G, 7C | 9G, 7C |
| Sorghum | 10E | 10E |
| Wild Oats | 7G, 3C | 8G, 5E |
| Johnsongrass | 9G, 7C | 9G, 9C |
| Dallisgrass | 7G | 8G |
| Giant Foxtail | 7G, 3H | 9G, 5H |
| Ky. bluegrass | 10C | 9G, 9C |
| Cheatgrass | 10C | 10E |
| Sugarbeets | 9G, 9C | 9G, 9C |
| Corn | 7G, 5H | 8G, 3C |
| Mustard | 10C | 10E |
| Cocklebur | 6G | 7G |
| Pigweed | 10E | 10E |
| Nutsedge | 8G | 10E |
| Cotton | 3G | 7G, 5H |
| Morningglory | 6G | 8G, 3H |
| Cassia | 5G | 7G |
| Teaweed | 6G | 8G, 4C |
| Velvetleaf | 8G, 7C | 10C |
| Jimsonweed | 6G | 7G, 7C |
| Soybean | 3G | 6G, 3H |
| Rice | 10E | 10E |
| Wheat | 7G, 3C | 7G, 5C |
| | | |
| | | |
| | | |

TABLE B (Continued)

| PRE-EMERGENCE ON FALLSINGTON SILT LOAM | | | |
|---|---|---|---|
| | 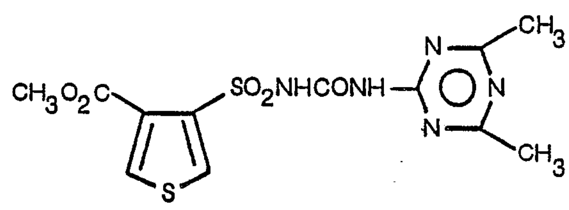 | | |
| Rate, kg/ha | 0.03 | 0.12 | 0.007 |
| | | | |
| Crabgrass | 7G, 3H | 8G, 5C | 0 |
| Barnyardgrass | 6G, 3H | 8G, 4C | 0 |
| Sorghum | 9G, 9C | 10E | 5G, 3H |
| Wild Oats | 2G | 7G, 3H | 0 |
| Johnsongrass | 8G, 5H | 8G, 5C | 0 |
| Dallisgrass | 6G | 0 | 0 |
| Giant Foxtail | 5G, 2H | 7G, 5H | 0 |
| Ky. bluegrass | 7G, 7C | 7G, 5C | 0 |
| Cheatgrass | 7G, 3C | 10C | 0 |
| Sugarbeets | 9G, 9C | 9G, 9C | 5G, 3H |
| Corn | 10C | 10E | 3G |
| Mustard | 10C | 10C | 7G, 3C |
| Cocklebur | 7G, 5H | 8G, 5H | 2G |
| Pigweed | 10C | 10C | 5G |
| Nutsedge | 6G | 7G | 0 |
| Cotton | 7G | 8G, 5H | 0 |
| Morningglory | 8G, 3H | 8G, 3H | 6G, 5H |
| Cassia | 6G | – | 0 |
| Teaweed | 6G | 7G, 4C | 0 |
| Velvetleaf | 8G, 8C | 8G, 8C | 3G |
| Jimsonweed | 6G, 4C | 6G, 5C | 0 |
| Soybean | 2H | 7G, 5H | 0 |
| Rice | 10E | 10E | 7G, 3H |
| Wheat | 5G | 6G | 0 |
| | | | |
| | | | |
| | | | |

TABLE B (Continued)

| PRE-EMERGENCE ON FALLSINGTON SILT LOAM | | |
|---|---|---|
| | 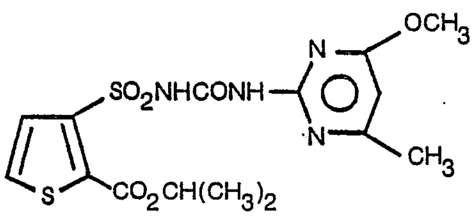 | |
| Rate, kg/ha | 0.03 | 0.12 |
| | | |
| Crabgrass | 0 | 5G |
| Barnyardgrass | 5G, 3H | 4G, 2H |
| Sorghum | 8G, 5H | 10C |
| Wild Oats | 0 | 3C |
| Johnsongrass | 7G, 3H | 8G, 5H |
| Dallisgrass | 0 | 4G |
| Giant Foxtail | 0 | 4H |
| Ky. bluegrass | 7G | 8G, 8C |
| Cheatgrass | 10E | 10E |
| Sugarbeets | 6G, 3H | 10C |
| Corn | 5G, 3H | 6G, 3H |
| Mustard | 8G, 5C | 10C |
| Cocklebur | 0 | 5G |
| Pigweed | 5G | 10E |
| Nutsedge | 0 | 5G |
| Cotton | 3G | 6G, 3H |
| Morningglory | 4G | 5G |
| Cassia | 3G | 3G |
| Teaweed | 3G | 4G, 2C |
| Velvetleaf | 3H | 6G, 3H |
| Jimsonweed | 3G | 7G, 3C |
| Soybean | 0 | 4G, 3H |
| Rice | 6G | 7G, 5H |
| Wheat | 3G | 3G |
| | | |
| | | |
| | | |

TABLE B (Continued)

| PRE-EMERGENCE ON FALLSINGTON SILT LOAM | | |
|---|---|---|
| | 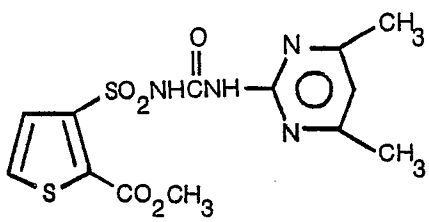 | |
| Rate, kg /ha | 0.12 | 0.5 |
| | | |
| Crabgrass | 0 | 0 |
| Barnyardgrass | 0 | 0 |
| Sorghum | 0 | 7G, 5H |
| Wild Oats | 0 | 4G |
| Johnsongrass | 0 | 5G |
| Dallisgrass | 0 | 3G |
| Giant Foxtail | 0 | 2G |
| Ky. bluegrass | – | – |
| Cheatgrass | 0 | 5G |
| Sugarbeets | 2G | 6G |
| Corn | 0 | 4G |
| Mustard | 8G, 5C | 10C |
| Cocklebur | 6G, 3C | 6G, 2C |
| Pigweed | 4G, 4C | 7G, 7C |
| Nutsedge | 0 | 5G |
| Cotton | 3G | 7G |
| Morningglory | 0 | 6G |
| Cassia | 0 | 3G |
| Teaweed | 0 | 0 |
| Velvetleaf | 4G, 3C | 5G |
| Jimsonweed | 0 | 0 |
| Soybean | 0 | 4G, 2H |
| Rice | 0 | 2G |
| Wheat | 0 | 3G |
| | | |
| | | |
| | | |

TABLE B (Continued)

| PRE-EMERGENCE ON FALLSINGTON SILT LOAM | | | |
|---|---|---|---|
| | 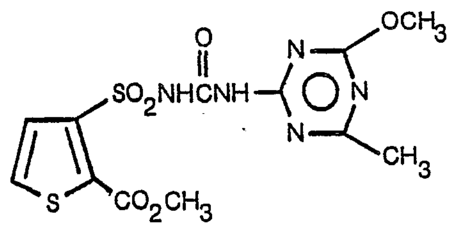 | | |
| Rate, kg/ha | 0.06 | 0.12 | 0.5 |
| | | | |
| Crabgrass | 6G, 7C | 4G | 7G |
| Barnyardgrass | 4G | 6G | 8G, 4C |
| Sorghum | 9G, 5C | 9G, 8C | 10C |
| Wild Oats | 0 | 0 | 0 |
| Johnsongrass | 4G, 3H | 4G | 7G, 3H |
| Dallisgrass | 0 | 0 | 3G |
| Giant Foxtail | 3G | 3G | 6G, 3C |
| Ky. bluegrass | 3G | 3G | 5G |
| Cheatgrass | 3G | – | – |
| Sugarbeets | 9G, 9C | 10C | 10C |
| Corn | 5G | 6G, 3H | 9G, 5H |
| Mustard | 10C | 8G | 10C |
| Cocklebur | 6G, 3H | 7G, 3H | 8G, 5H |
| Pigweed | 8G, 8C | 10C | 10E |
| Nutsedge | 3G | 7G | 8G |
| Cotton | 8G, 3H | 9G, 5C | 9G, 5C |
| Morningglory | 8G, 3H | 8G, 3H | 9G, 9C |
| Cassia | 6G | 5G | 7G |
| Teaweed | 5G, 3H | 4G | 8G, 5C |
| Velvetleaf | 8G, 7C | 10C | 10C |
| Jimsonweed | 9G, 9C | 7G | 10C |
| Soybean | 0 | 3G | 5G, 3H |
| Rice | 10E | 8G, 6C | 10E |
| Wheat | 0 | 0 | 0 |
| | | | |
| | | | |
| | | | |

TEST PROCEDURE C

*Wheat and Barley Herbicide Screen*

Two ten-inch in diameter plastic pans lined with polyethylene liners were filled with prepared Fallsington silt loam soil. One pan was planted with seeds of wheat *(Triticum aestivum)*, barley *(Hordeum vulgare)*, wild oats *(Avena fatua)*, down brome *(bromus tectorum)*, cheatgrass *(Bromum secalinus)*, blackgrass (*Alopecurus myosuroides*), annual bluegrass (*Poa annua*), green foxtail (*Setaria viridis*), quackgrass (*Agropyron repens*), Italian ryegrass (*Lolium multiflorum*) and ripgut brome (*Bromus rigidus)*. The other pan was planted with seeds of Russian thistle *(Salsola kali)*, tansy mustard *(Descuraina pinnata)*, smartweed *(Polygonum pennsylvanicum)*, tumble mustard *(Sisymbrium altissium)*, kochia (*Kochia scoparia*), shephard's purse (*Capsella bursa-pastoris*), *Matricaria indora*, black nightshade (*Solanum nigrum*), yellow rocket (*Barbarea vulgaris*), wild mustard (*Brassica kaber*) and wild buckwheat (*Polygonum convolvulus*). The above two pans were treated pre-emergence. At the same time two pans in which the above plant species were growing were treated post-emergence. Plant height at the time of treatment ranged from 1—15 cm depending on plant species.

The compounds applied were diluted with a non-pytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table C. It will be seen that the compounds have utility for selective weed control in cereal crops.

TABLE C

| | 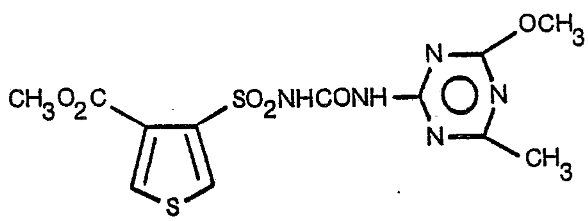 | |
|---|---|---|
| | Post-emergence | Pre-emergence |
| Rate, kg/ha | 0.008 | 0.008 |
| | | |
| Wheat | 1C, 1G | 0 |
| Barley | 1G | 1G |
| Wild Oats | 1C, 1G | 0 |
| Downy Brome | 2G | 2G |
| Cheatgrass | 1C, 4G | 7G |
| Blackgrass | 3C, 3G | 5G |
| Annual Bluegrass | 8C, 8G | 3C, 6G |
| Green Foxtail | 1C, 3G | 1C, 2G |
| Quackgrass | 2C, 3G | 3G |
| Italian Ryegrass | 6C, 7G | 1C, 5G |
| Ripgut Brome | 1C, 2G | 0 |
| Russian Thistle | 10C | 3G |
| Tansy Mustard | 10C | 10C |
| Smartweed | – | – |
| Jimhill Mustard | 10C | 10C |
| Kochia | 10C | 10C |
| Shepherd's Purse | 10C | 10C |
| False Chamomile | 10C | 6C, 7G |
| Black Nightshade | 10C | 7G |
| Yellow Rocket | 10C | 10C |
| Wild Mustard | 10C | 10C |
| Wild Buckwheat | 10C | 7C, 6G |
| | | |
| | | |
| | | |
| | | |
| | | |

TABLE C (Continued)

| | 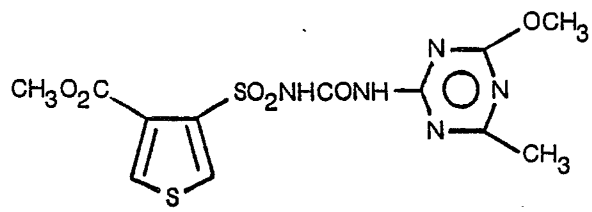 | |
|---|---|---|
| | Post-emergence | Pre-emergence |
| Rate, kg/ha | 0.03 | 0.03 |
| | | |
| Wheat | 6C, 5G | 0 |
| Barley | 2C, 3G | 2G |
| Wild Oats | 7C, 5G | 0 |
| Downy Brome | 7C, 7G | 5C, 7G |
| Cheatgrass | 10C | 10C |
| Blackgrass | 9C, 8G | 7C, 7G |
| Annual Bluegrass | 10C | 7C, 8G |
| Green Foxtail | 10C | 5C, 6G |
| Quackgrass | 10C | 2C, 5G |
| Italian Ryegrass | 10C | 5C, 7G |
| Ripgut Brome | 3C, 4G | 1C, 3G |
| Russian Thistle | 10C | 7C, 6G |
| Tansy Mustard | 10C | 10C |
| Smartweed | – | – |
| Jimhill Mustard | 10C | 10C |
| Kochia | 10C | 10C |
| Shepherd's Purse | 10C | 10C |
| False Chamomile | 10C | 10C |
| Black Nightshade | 10C | 7C, 8G |
| Yellow Rocket | 10C | 10C |
| Wild Mustard | 10C | 10C |
| Wild Buckwheat | 10C | 7C, 7G |
| | | |
| | | |
| | | |
| | | |
| | | |

TABLE C (Continued)

| | 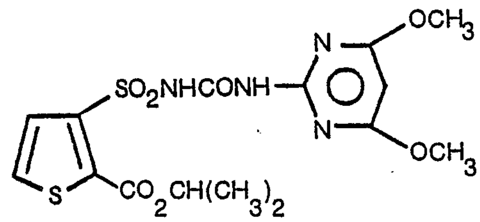 | |
|---|---|---|
| | Post-emergence | Pre-emergence |
| Rate, kg/ha | 0.015 | 0.015 |
| | | |
| Wheat | 0 | 0 |
| Barley | 0 | 0 |
| Wild Oats | 0 | 2C, 2G |
| Downy Brome | 0 | 1C, 3G |
| Cheatgrass | 0 | 1C, 4G |
| Blackgrass | 0 | 3G |
| Annual Bluegrass | 0 | 2G |
| Green Foxtail | 0 | 0 |
| Quackgrass | 0 | 0 |
| Italian Ryegrass | 0 | 0 |
| Ripgut Brome | 0 | 0 |
| Russian Thistle | 10C | 0 |
| Tansy Mustard | 10C | 10C |
| Smartweed | – | – |
| Jimhill Mustard | 10C | 10C |
| Kochia | 10C | 2C, 5G |
| Shepherd's Purse | 10C | 10C |
| False Chamomile | 10C | 4C, 6G |
| Black Nightshade | 0 | 2C, 6G |
| Yellow Rocket | 10C | 3C, 8G |
| Wild Mustard | 10C | 9C, 9G |
| Wild Buckwheat | 3C, 2G | 4G |
| | | |
| | | |
| | | |
| | | |
| | | |

TABLE C (Continued)

| | | |
|---|---|---|
| | 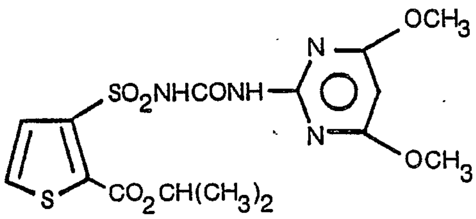 | |
| | Post-emergence | Pre-emergence |
| Rate, kg/ha | 0.06 | 0.06 |
| | | |
| Wheat | 2G | 0 |
| Barley | 0 | 0 |
| Wild Oats | 0 | 3C, 4G |
| Downy Brome | 0 | 4C, 6G |
| Cheatgrass | 7C, 5G | 10C |
| Blackgrass | 7C, 7G | 10C |
| Annual Bluegrass | 2G | 3C, 5G |
| Green Foxtail | 1C, 3G | 2C, 4G |
| Quackgrass | 0 | 2C, 3G |
| Italian Ryegrass | 0 | 0 |
| Ripgut Brome | 0 | 0 |
| Russian Thistle | 10C | 7C, 8G |
| Tansy Mustard | 10C | 10C |
| Smartweed | – | – |
| Jimhill Mustard | 10C | 10C |
| Kochia | 10C | 7C, 7G |
| Shepherd's Purse | 10C | 10C |
| False Chamomile | 10C | 7C, 9G |
| Black Nightshade | 0 | 2C, 7G |
| Yellow Rocket | 10C | 9C, 9G |
| Wild Mustard | 10C | 10C |
| Wild Buckwheat | 7C, 6G | 2C, 7G |
| | | |
| | | |
| | | |
| | | |
| | | |

TABLE C (Continued)

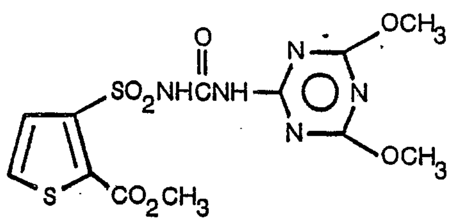

| | Post-emergence | Pre-emergence |
|---|---|---|
| Rate, kg/ha | 0.015 | 0.015 |
| | | |
| Wheat | 0 | 0 |
| Barley | 0 | 0 |
| Wild Oats | 0 | 0 |
| Downy Brome | 0 | 2G |
| Cheatgrass | 0 | 1G |
| Blackgrass | 0 | 3G |
| Annual Bluegrass | 0 | 2G |
| Green Foxtail | 0 | 0 |
| Quackgrass | 0 | 0 |
| Italian Ryegrass | 0 | 0 |
| Ripgut Brome | 0 | 0 |
| Russian Thistle | 10C | 2C, 2G |
| Tansy Mustard | 10C | 10C |
| Smartweed | – | – |
| Jimhill Mustard | 10C | 10C |
| Kochia | 2C, 4G | 3C, 5G |
| Shepherd's Purse | 10C | 10C |
| False Chamomile | 10C | 7C, 8G |
| Black Nightshade | 1C, 3G | 1C, 8G |
| Yellow Rocket | 2C, 3G | 5C, 6G |
| Wild Mustard | 10C | 9C, 9G |
| Wild Buckwheat | 9C, 8G | 4C, 7G |

TABLE C (Continued)

| | | |
|---|---|---|
| | 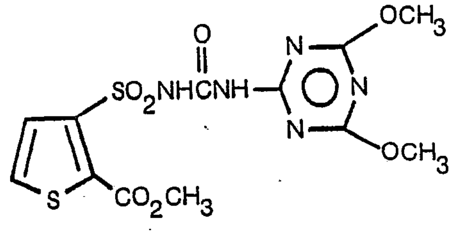 | |
| | Post-emergence | Pre-emergence |
| Rate, kg/ha | 0.06 | 0.06 |
| | | |
| Wheat | 0 | 0 |
| Barley | 0 | 0 |
| Wild Oats | 0 | 0 |
| Downy Brome | 2C, 3G | 2C,3G |
| Cheatgrass | 2C, 5G | 1C,5G |
| Blackgrass | 1C, 3G | 2C, 7G |
| Annual Bluegrass | 1C, 4G | 5G |
| Green Foxtail | 2G | 2C, 3G |
| Quackgrass | 0 | 1C, 2G |
| Italian Ryegrass | 3G | 3G |
| Ripgut Brome | 0 | 0 |
| Russian Thistle | 10C | 7C, 8G |
| Tansy Mustard | 10C | 10C |
| Smartweed | – | – |
| Jimhill Mustard | 10C | 10C |
| Kochia | 7C, 7G | 7C, 7G |
| Shepherd's Purse | 10C | 10C |
| False Chamomile | 10C | 9C, 9G |
| Black Nightshade | 3C, 6G | 2C, 8G |
| Yellow Rocket | 10C | 6C, 8G |
| Wild Mustard | 10C | 9C, 9G |
| Wild Buckwheat | 10C | 7C, 7G |
| | | |
| | | |
| | | |
| | | |
| | | |

TABLE C (Continued)

| | 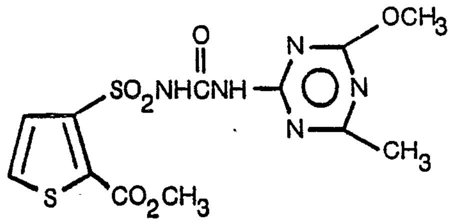 | |
|---|---|---|
| | Post-emergence | Pre-emergence |
| Rate, kg/ha | 0.008 | 0.008 |
| | | |
| Wheat | 0 | 0 |
| Barley | 0 | 0 |
| Wild Oats | 0 | 0 |
| Downy Brome | 0 | 1C, 2G |
| Cheatgrass | 0 | 3G |
| Blackgrass | 1G | 1C, 3G |
| Annual Bluegrass | 1G | 5C, 6G |
| Green Foxtail | 2G | 0 |
| Quackgrass | 0 | 2G |
| Italian Ryegrass | 0 | 4G |
| Ripgut Brome | 0 | 0 |
| Russian Thistle | 10C | 4C, 5G |
| Tansy Mustard | 10C | 10C |
| Smartweed | – | – |
| Jimhill Mustard | 10C | 10C |
| Kochia | 8C, 8G | 7C, 8G |
| Shepherd's Purse | 9C, 8G | 10C |
| False Chamomile | 10C | 7C, 8G |
| Black Nightshade | 1G | 2C,5G |
| Yellow Rocket | 10C | 10C |
| Wild Mustard | 10C | 10C |
| Wild Buckwheat | 8C, 7G | 4C, 7G |

TABLE C (Continued)

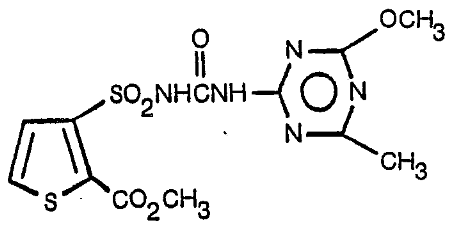

| | Post-emergence | Pre-emergence |
|---|---|---|
| Rate, kg/ha | 0.03 | 0.03 |
| | | |
| Wheat | 0 | 0 |
| Barley | 0 | 0 |
| Wild Oats | 0 | 0 |
| Downy Brome | 1G | 2C, 5G |
| Cheatgrass | 2G | 2C, 5G |
| Blackgrass | 1C, 3G | 3C, 7G |
| Annual Bluegrass | 3G | 5C, 7G |
| Green Foxtail | 2C, 3G | 3G |
| Quackgrass | 1G | 2C, 7G |
| Italian Ryegrass | 2G | 1G |
| Ripgut Brome | 0 | |
| Russian Thistle | 10C | 10C |
| Tansy Mustard | 10C | 10C |
| Smartweed | – | – |
| Jimhill Mustard | 10C | 10C |
| Kochia | 10C | 8C, 9G |
| Shepherd's Purse | 10C | 10C |
| False Chamomile | 10C | 9C, 9G |
| Black Nightshade | 2C, 3G | 3C, 8G |
| Yellow Rocket | 10C | 10C |
| Wild Mustard | 10C | 10C |
| Wild Buckwheat | 10C | 8C, 8G |
| | | |
| | | |
| | | |
| | | |
| | | |

**Claims for the Contracting States: BE CH DE FR IT LI LU NL SE**

1. A compound selected from:

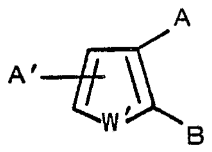
I

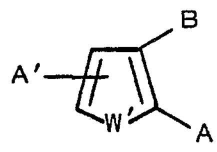
II

or

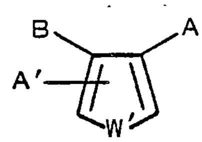
III

wherein

W' is O or S;

A' is H, Cl, Br, $C_1$—$C_4$ alkyl, $OCH_3$, $NO_2$ or $CF_3$;

A is 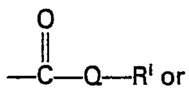 or 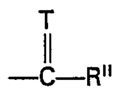 where

Q is O, S or 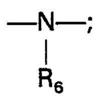;

T is O or 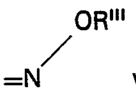 where

$R^{III}$ is H, $C_1$—$C_4$ alkyl or $C_3$—$C_4$ alkenyl;

when Q is O or S then

$R^I$ is $C_1$—$C_6$ alkyl; $C_3$—$C_6$ alkenyl; $C_3$—$C_6$ alkynyl; $C_2$—$C_6$ alkyl substituted with 1—3 Cl, F or Br, or one of CN or $OCH_3$; $C_3$—$C_6$ alkenyl substituted with 1—3 Cl; $C_3$—$C_6$ alkynyl substituted with Cl; $C_5$—$C_6$ cycloalkyl; cyclohexenyl; cyclohexyl substituted with 1—3 $CH_3$; $C_4$—$C_7$ cycloalkyl-alkyl or

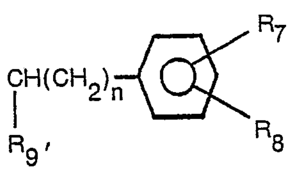

where $R_7$ and $R_8$ are independently H, Cl, $CH_3$ or $OCH_3$;

n is 0 or 1; and

$R_9$ is H or $CH_3$;

when Q is O then $R^I$ can be $CH_2CH_2OR_{15}$; $CH_2CH_2CH_2OR_{15}$; 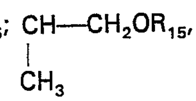

where $R_{15}$ is $C_2H_5$, $CH(CH_3)_2$, phenyl, $CH_2CH_2Cl$, $CH_2CCl_3$; $\leftarrow CH_2CH_2O \rightarrow_{n'} R_{16}$, 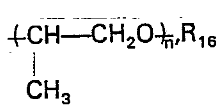

where $R_{16}$ is $CH_3$, $C_2H_5$, $CH(CH_3)_2$, phenyl, $CH_2CH_2Cl$, $CH_2CCl_3$, and n' is 2 or 3;

$CH_2CN$; 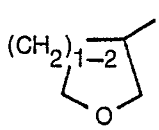 ; 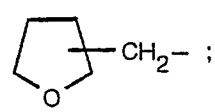 ; 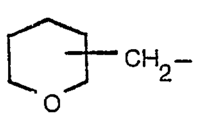 ; 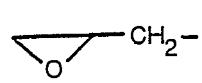 ; $CH_2OR_6'$ ;

where $R_6'$ is $C_1$—$C_4$ alkyl;

provided $R^I$ has a total of ≤13 carbon atoms;

when Q is 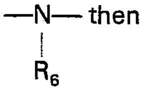 then

$R^I$ is H; $C_1$—$C_6$ alkyl; —$CH_2CH_2OR_{10}$; —$CH_2CH_2CH_2OR_{10}$; where $R_{10}$ is $CH_3$, $CH_3CH_2$, $CH(CH_3)_2$, or phenyl; $C_3$—$C_6$ alkenyl; $C_3$—$C_6$ cycloalkyl; $C_5$—$C_6$ cycloalkenyl; $C_6$ cycloalkyl substituted with any one of 1—2 $OCH_3$, 1—3 $CH_3$, —$CH_2CH_3$ or $CF_3$; $C_4$—$C_7$ cycloalkylalkyl; —$CH_2CN$; —$CH_2CH_2CN$;

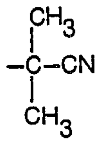 ; $OCH_3$ ; $OCH_2CH_3$ ; 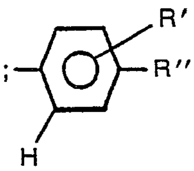 or 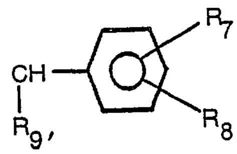 ;

where

$R'$ is H, $C_1$—$C_4$ alkyl, $OCH_3$, F, Cl, Br, CN, $NO_2$ or $CF_3$;

$R''$ is H, $CH_3$, Cl, F or Br;

$R_7$, $R_8$ and $R_9'$ are as previously defined;

$R_6$ is H, $C_1$—$C_3$ alkyl; $CH_2CN$; $CH_2CH_2$—CN or —$CH_2$—CH=$CH_2$ and $R_6$ and $R^I$ may be taken together to form —$(CH_2)_4$—, —$(CH_2)_5$— or —$CH_2CH_2O$—$CH_2CH_2$—;

with the proviso that when $R_6$ is $CH_2CH_2CN$ or $CH_2CN$, then $R^1$ is $CH_2CH_2CN$ or $CH_2CN$; and $R^1$ and $R_6$ have a total carbon atom count of $\leq 13$; and when $R^I$ is $OCH_3$ or $OCH_2CH_3$ then $R_6$ is $CH_3$ or H;

when A is 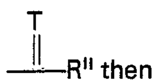 then

$R^{II}$ is H, $C_1$—$C_6$ alkyl; $C_3$—$C_6$ alkenyl; phenyl; benzyl; benzyl or phenyl substituted with 1—2 Cl, 1—2 $OCH_3$, 1—2 $CH_3$; $C_5$—$C_6$ cycloalkyl; $C_4$—$C_7$ cycloalkylalkyl with the proviso that when T is =N—$OR^{III}$, then $R^{II}$ must be $C_1$—$C_6$ alkyl or $C_3$—$C_6$ alkenyl;

B is 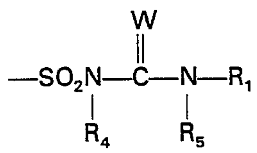

where $R_4$ is H or $CH_3$; W is O or S; $R_5$ is H, $CH_3$ or $CH_3O$; with the further proviso that either $R_4$ or $R_5$ must be H;

$R_1$ is 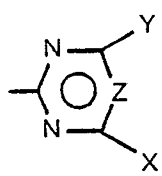 , 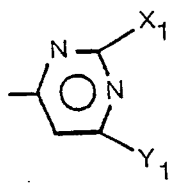 , 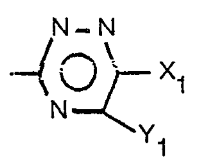 ,

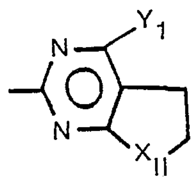 or 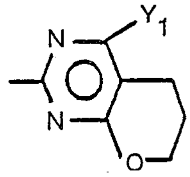 ;

where

Z is N, CH or C—F;

X=H, Cl, —$CH_3$, —$OCH_3$ or —$OCH_2CH_3$;

Y=H; Cl; $C_1$—$C_4$ alkyl; $C_1$—$C_4$ alkyl substituted with —$OCH_3$, —$OC_2H_5$, —CN, —$CO_2CH_3$, —$CO_2C_2H_5$,

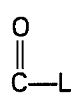 ,

or 1—3 atoms of F, Cl, Br; $C_3$—$C_4$ alkenyl; —O—$(CH_2)_{n'}$O—($C_1$—$C_3$ alkyl) where n′ is 2 or 3;

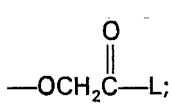 ; 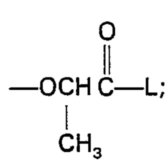 ; 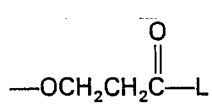 where

L is OH, —$NH_2$, —$NCH_3$,
|
$OCH_3$

—NH($C_1$—$C_4$ alkyl), —N($C_1$—$C_4$ alkyl)$_2$ or $C_1$—$C_6$ alkoxy; SCN; —$N_3$; $NR_{11}R_{12}$ where $R_{11}$ is H or $CH_3$ and $R_{12}$ is H, —$OCH_3$, $C_1$—$C_4$ alkyl, $C_3$—$C_6$ cycloalkyl, $C_3$—$C_4$ alkenyl, $C_2$—$C_3$ alkyl substituted with $OCH_3$ or $OC_2H_5$, $C_1$—$C_2$ alkyl substituted with —CN, $CO_2H$, $CO_2CH_3$ or $CO_2C_2H_5$, and $R_{11}$ and $R_{12}$ can be taken together to form —$CH_2CH_2CH_2CH_2$— or $CH_2CH_2OCH_2CH_2$—; —O—$R_9$ where $R_9$ is $C_1$—$C_4$ alkyl, $C_2$—$C_4$ alkyl substituted with 1—3 atoms of F, Cl or Br, $C_1$—$C_2$ alkyl substituted with cyano, $C_3$—$C_4$ alkenyl,

—$CH_2C{\equiv}CR_{13}$, 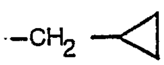 , 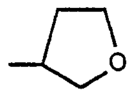 or 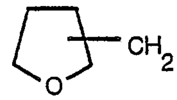 :

$R_{13}$ is H, $CH_3$ or $CH_2Cl$; $SR_{14}$;
where
$R_{14}$ is $C_1$—$C_4$ alkyl, allyl, propargyl or $C_1$—$C_2$ alkyl substituted with CN; with the proviso that when X and Y are both H, then $R^I$ and $R^{II}$ are less than 5 carbons;
$X_1$ = H, Cl, $OCH_3$, $OCH_2CH_3$ or $CH_3$;
$Y_1$ = H, $OCH_3$ or $CH_3$; and
$X_{II}$ = O or $CH_2$
and further provided that when A contains greater than 5 carbon atoms, then Y must contain ≤4 carbon atoms,
and their agriculturally suitable salts.

O
‖
2. A compound of Claim 1 in which B is $SO_2$—NH—C—$NHR_1$.

3. A compound of Claim 1 in which T is oxygen.
4. A compound of Claim 2 or 3 in which

$R_1$ is 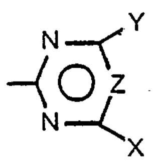 , 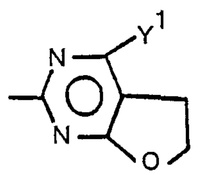 or 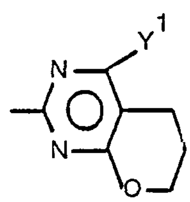

5. A compound of Claim 4 where Q is O or S and $R^I$ is $C_1$—$C_4$ alkyl; $C_3$—$C_4$ alkenyl; $C_3$—$C_4$ alkynyl; $C_2$—$C_3$ alkyl substituted with CN, $OCH_3$ or 1—3F, Cl or Br; $C_3$—$C_4$ alkenyl substituted with 1—3 Cl or $C_3$—$C_4$ alkynyl substituted with Cl.
6. A compound of Claim 4 in which Q is oxygen and $R^I$ is $CH_2CH_2OR_{15}$; $CH_2CH_2CH_2OR_{15}$;

$CHCH_2OR_{15}$
|
$CH_3$

where $R_{15}$ is $CH_2CH_3$; $CH_2CN$; $CH_2OR'_6$ where $R'_6$ is $CH_3$ or $CH_3CH_2$; and 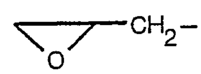 ;

7. A compound of Claim 4 in which Q is —N— and
|
$R_6$

$R^I$ is H, $C_1$—$C_4$ alkyl, $CH_2CH_2OR_{10}$, $CH_2CH_2CH_2OR_{10}$ where $R_{10}$ is $CH_3$ or $CH_3CH_2$; $C_3$—$C_4$ alkenyl; $CH_2CN$; $CH_2CH_2CN$; $OCH_3$ or $OCH_2CH_3$;
$R^6$ is H, $C_1$—$C_2$ alkyl, $CH_2CN$ or $CH_2CH_2CN$ and $R_6$ and $R^I$ can be taken together to form $-(CH_2)_4-$.
8. A compound of Claim 4 in which $R^{II}$ is H or $C_1$—$C_3$ alkyl.
9. A compound of any of claims 5—8 in which
Z is CH or N;
X is $CH_3$ or $CH_3O$; and

Y is $C_1$—$C_2$ alkyl; $C_1$—$C_2$ alkyl substituted with $OCH_3$; $OCH_2CH_3$, CN or 1—3 atoms of F, Cl or Br;

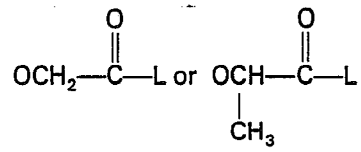
or

where L is $NH_2$, OH, 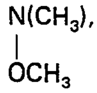

$N(CH_3)$, $NHCH_3$, $C_1$—$C_2$ alkoxy; SCN; $N_3$; $NR_{11}R_{12}$ where $R_{11}$ is H or $CH_3$; $R_{12}$ is H, $CH_3$, $CH_3CH_2$, $OCH_3$; $OR_9$ where $R_9$ is $CH_3$, $CH_3CH_2$; $CH_2CH{=}CH_2$; $CH_2C{\equiv}CH$; or $C_2$ alkyl substituted with 1—3 F, Cl or Br; $CH_3S$.

10. A compound of Claim 4 in which A′ is H, Cl or Br.

11. A compound of Claim 10 in which Q is O or S and $R^I$ is $C_1$—$C_4$ alkyl, $CH_2CH{=}CH_2$ or $CH_2CH_2Cl$.

12. A compound of Claim 10 in which Q is O and $R^I$ is $CH_2CH_2OCH_3$, 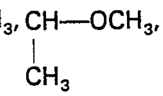

$CH_2OCH_3$ or $CH_2OCH_2CH_3$.

13. A compound of Claim 10 in which Q is 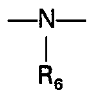

and $R^I$ is H; $C_1$—$C_3$ alkyl, $OCH_3$ or $OCH_2CH_3$ and $R_6$ is H or $C_1$—$C_2$ alkyl.

14. A compound of Claim 10 in which $R^{II}$ is H or $CH_3$.

15. A compound of any of Claims 11—14 in which A′ is H; Y is $CH_3$, $OCH_3$, $OCH_2CH_3$, $OCH_2CF_3$, $OCH_2CH{=}CH_2$ or $OCH_2{\equiv}CH$.

16. A compound of Claim 9 in which 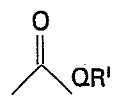

and Q is oxygen or sulfur and $R^1$ is $CH_3$ or $CH_2CH_3$; Q is 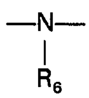

and $R^I$ is H, $CH_3$ or $OCH_3$ and $R_6$ is $CH_3$;

$R_1$ is 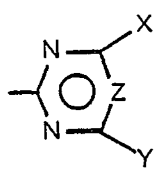

and Y is $CH_3$ or $OCH_3$.

17. A compound of Claim 16 in which Formula I is utilized.

18. A compound of Claim 16 in which Formula II is utilized.

19. A compound of Claim 16 in which Formula III is utilized.

20. A compound of any of Claims 1 to 19 in which W′ is sulfur.

21. A compound of any of Claims 1 to 19 in which W′ is oxygen.

22. A compound of Claim 1 or an agriculturally suitable salt thereof selected from

methyl 3-[[(4-methoxy-6-methylpyrimidin-2-yl)aminocarbonyl]aminosulfonyl]-2-thiophenecarboxylate

methyl 3-[[(4,6-dimethoxy-1,3,5-triazin-2-yl)aminocarbonyl]aminosulfonyl]-2-thiophenecarboxylate

methyl 3-[[(4-methoxy-6-methyl-1,3,5-triazin-2-yl)-aminocarbonyl]aminosulfonyl]-2-thiophenecarboxylate

methyl 3-[[(4,6-dimethoxypyrimidin-2-yl)aminocarbonyl]aminosulfonyl]-2-thiophenecarboxylate or

methyl 3-[[(4,6-dimethylpyrimidin--2-yl)aminocarbonyl]aminosulfonyl]-2-thiophenecarboxylate.

23. A compound of Claim 1 or an agriculturally suitable salt thereof selected from

methyl 2-[[(4,6-dimethoxypyrimidin-2-yl)aminocarbonyl]aminosulfonyl]-3-thiophenecarboxylate

methyl 2-[[(4,6-dimethylpyrimidin-2-yl)aminocarbonyl]aminosulfonyl]-3-thiophenecarboxylate

methyl 2-[[(4-methoxy-6-methylpyrimidin-2-yl)aminocarbonyl]aminosulfonyl]-3-thiophenecarboxylate

methyl 2-[[(4,6-dimethoxy-1,3,5-triazin-2-yl)aminocarbonyl]aminosulfonyl]-3-thiophenecarboxylate or

methyl 2-[[(4-methoxy-6-methyl-1,3,5-triazin-2-yl)aminocarbonyl]aminosulfonyl]-3-thiophenecarboxylate.

24. A compound of Claim 1 or an agriculturally suitable salt thereof selected from

methyl 4-[[(4,6-dimethoxypyrimidin-2-yl)aminocarbonyl]-aminosulfonyl]-3-thiophenecarboxylate

methyl 4-[[(4,6-dimethylpyrimidin-2-yl)aminocarbonyl]aminosulfonyl]-3-thiophenecarboxylate

methyl 4-[[(4-methoxy-6-methylpyrimidin-2-yl)aminocarbonyl]-aminosulfonyl]-3-thiophenecarboxylate

methyl 4-[[(4,6-dimethoxy-1,3,5-triazin-2-yl)aminocarbonyl]-aminosulfonyl]-3-thiophenecarboxylate or

methyl 4-[[(4-methoxy-6-methyl-1,3,5-triazin-2-yl)aminocarbonyl]-aminosulfonyl-3-thiophene-carboxylate.

25. A compound of Claim 1 or an agriculturally suitable salt thereof which is

N-[(4-methoxy-6-methylpyrimidin-2-yl)aminocarbonyl]-2-(1-pyrolidinylcarbonyl)-3-thiophene-sulphonamide.

26. A compound of Claim 1 or an agriculturally suitable salt thereof which is

1-methylethyl 3-[[(4,6-dimethoxypyrimidin-2-yl)aminocarbonyl]aminoxulfonyl]-2-thiophenecarboxylate.

27. A compound of Claim 1 wherein at least one of the following applies:

(a) A′ is not $OCH_3$, $NO_2$ or $CF_3$

(b) $R^I$ is not $OCH_2CH_3$ or $C_2$—$C_6$ alkyl substituted with F or Br

(c) $R^I$ is not

$CH_2CN$; 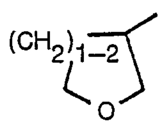 ; 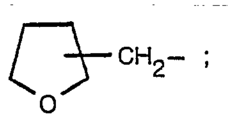 ; 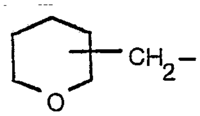 ; 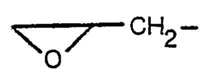 ; $CH_2OR'_6$ ;

(d) R′ is not CN, $NO_2$ or $CF_3$

(e) when $R^I$ is $OCH_3$, $R_6$ is not H

(f) $R^{II}$ is not H

(g) $R_1$ is not 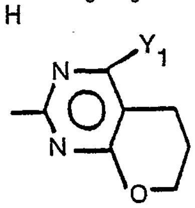

(h) $R_9$ is not 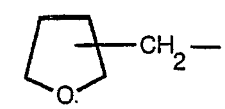

(i) Z is not C—F.

28. A composition suitable for controlling the growth of undesired vegetation comprising an effective amount of a herbicidal compound and at least one of the following: surfactant, solid or liquid diluent, characterised in that said herbicidal compound comprises a compound of any of Claims 1 to 27.

29. A method for controlling the growth of undesired vegetation by applying to the locus to be protected an effective amount of a herbicidal compound, characterised in that said herbicidal compound comprises a compound of any of Claims 1 to 27.

30. A method for regulating the growth of plants by applying to the locus of said plants an effective but substantially non-phytotoxic amount of a plant growth regulant, characterised in that said plant growth regulant comprises a compound of any of Claims 1 to 27.

31. A process for preparing a compound of Claim 1 which comprises reacting an appropriate isocyanate or isothiocyanate of formula

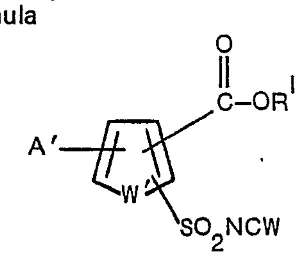

with an amine of formula $HNR_1R_5$, wherein A′, W, W′, $R^I$, $R_1$ and $R_5$ are as defined in Claim 1.

32. A compound selected from

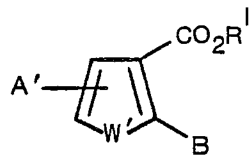 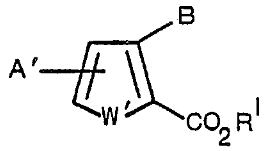 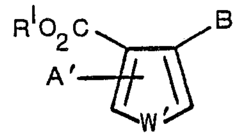

I′ II′ III′

wherein

$R^I$ is H or M;

M is a cation of an alkali metal or of a tertiary amine of up to 12 carbon atoms;

W′ is O or S;

A′ is H, Cl, Br, $C_1$—$c_4$ alkyl, $OCH_3$, $NO_2$ or $CF_3$;

B is 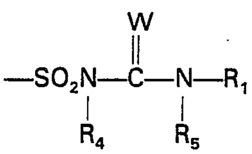

where

$R_4$ is H or $CH_3$; W is O or S; $R_5$ is H, $CH_3$ or $CH_3O$; with the proviso that either $R_4$ or $R_5$ must be H;

$R_1$ is 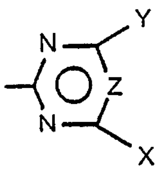, 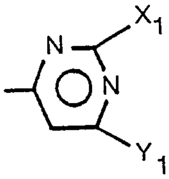, 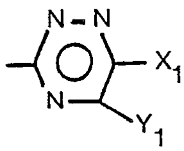, 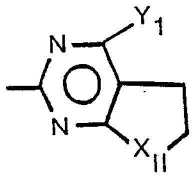 or 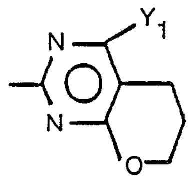;

where

Z is N, CH or C—F;

X=H, Cl, —$CH_3$, —$OCH_3$ or —$OCH_2CH_3$;

Y=H; Cl; $C_1$—$C_4$ alkyl; $C_1$—$C_4$ alkyl substituted with —$OCH_3$, —$OC_2H_5$, —CN, —$CO_2CH_3$, —$CO_2C_2H_5$,

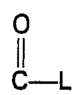

or 1—3 atoms of F, Cl, Br; $C_3$—$C_4$ alkenyl; —O—$(CH_2)_{n'}$O—($C_1$—$C_3$ alkyl) where n′ is 2 or 3;

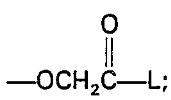; 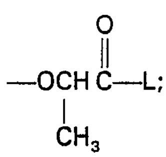; 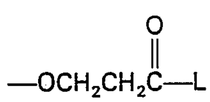 where

L is OH, —$NH_2$, 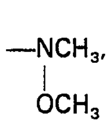

—NH($C_1$—$C_4$ alkyl), —N($C_1$—$C_4$ alkyl)$_2$ or $C_1$—$C_6$ alkoxy; SCN; —$N_3$; $NR_{11}R_{12}$ where $R_{11}$ is H or $CH_3$ and $R_{12}$ is H, —$OCH_3$, $C_1$—$C_4$ alkyl, $C_3$—$C_6$ cycloalkyl, $C_3$—$C_4$ alkenyl, $C_2$—$C_3$ alkyl substituted with $OCH_3$ or $OC_2H_5$, $C_1$—$C_2$ alkyl substituted with —CN, $CO_2H$, $CO_2CH_3$ or $CO_2C_2H_5$, and $R_{11}$ and $R_{12}$ can be taken together to form —$CH_2CH_2CH_2CH_2$— or $CH_2CH_2OCH_2CH_2$—; —O—$R_9$ where $R_9$ is $C_1$—$C_4$ alkyl, $C_2$—$C_4$ alkyl substituted with 1—3 atoms of F, Cl or Br, $C_1$—$C_2$ alkyl substituted with cyano, $C_3$—$C_4$ alkenyl, —$CH_2C{\equiv}CR_{13}$,

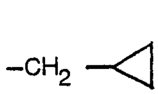, 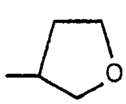 or 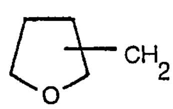 :

$R_{13}$ is H, $CH_3$ or $CH_2Cl$; $SR_{14}$; where

$R_{14}$ is $C_1$—$C_4$ alkyl, allyl, propargyl or $C_1$—$C_2$ alkyl substituted with CN;
$X_1$ = H, Cl, $OCH_3$, $OCH_2CH_3$ or $CH_3$;
$Y_1$ = H, $OCH_3$ or $CH_3$; and
$X_{II}$ = O or $CH_2$.

33. A process for the preparation of a compound of Claim 32 which comprises hydrolyzing a corresponding starting compound wherein $R^I$ is other than H, under basic conditions, and if desired acidifying to obtain a product wherein $R^I$ is H.

**Claims for the Contracting State: AT**

1. A process for the preparation of a compound selected from:

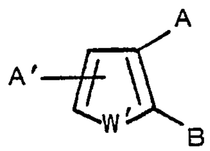
I

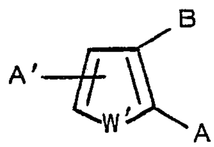
II

or

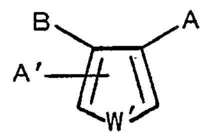
III

wherein
W' is O or S;
A' is H, Cl, Br, $C_1$—$C_4$ alkyl, $OCH_3$, $NO_2$ or $CF_3$;

A is 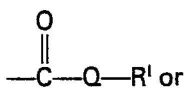 or 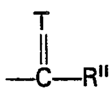 where

Q is O, S or 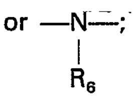;

T is O or 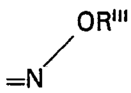 where

$R^{III}$ is H, $C_1$—$C_4$ alkyl or $C_3$—$C_4$ alkenyl;
when Q is O or S then
$R^I$ is $C_1$—$C_6$ alkyl; $C_3$—$C_6$ alkenyl; $C_3$—$C_6$ alkynyl; $C_2$—$C_6$ alkyl substituted with 1—3 Cl, F or Br, or one of CN or $OCH_3$; $C_3$—$C_6$ alkenyl substituted with 1—3 Cl; $C_3$—$C_6$ alkynyl substituted with Cl; $C_5$—$C_6$ cycloalkyl; cyclohexenyl; cyclohexyl substituted with 1—3 $CH_3$; $C_4$—$C_7$ cycloalkyl-alkyl or

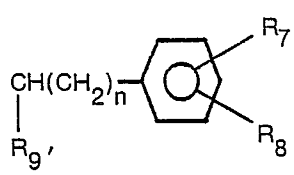

where $R_7$ and $R_8$ are independently H, Cl, $CH_3$ or $OCH_3$;
n is 0 or 1; and
$R_9$ is H or $CH_3$;
when Q is O then $R^I$ can be $CH_2CH_2OR_{15}$; $CH_2CH_2CH_2OR_{15}$; 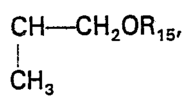

where $R_{15}$ is $C_2H_5$, $CH(CH_3)_2$, phenyl, $CH_2CH_2Cl$, $CH_2CCl_3$, $-(CH_2CH_2O)_{n'}R_{16}$, 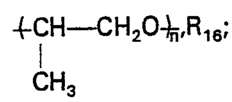;

where $R_{16}$ is $CH_3$, $C_2H_5$, $CH(CH_3)_2$, phenyl, $CH_2CH_2Cl$, $CH_2CCl_3$, and n' is 2 or 3;

$CH_2CN$; 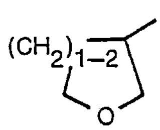 ; 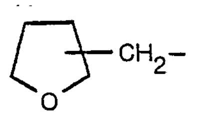 ; 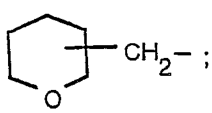 ; 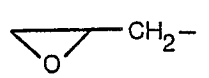 ; $CH_2OR'_6$ ;

where $R_6'$ is $C_1$—$C_4$ alkyl;
provided $R^I$ has a total of $\leq 13$ carbon atoms;
when Q is 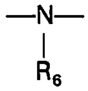 then

$R^I$ is H; $C_1$—$C_6$ alkyl; $—CH_2CH_2OR_{10}$; $—CH_2CH_2CH_2OR_{10}$; where $R_{10}$ is $CH_3$, $CH_3CH_2$, $CH(CH_3)_2$, or phenyl; $C_3$—$C_6$ alkenyl; $C_3$—$C_6$ cycloalkyl; $C_5$—$C_6$ cycloalkenyl; $C_6$ cycloalkyl substituted with any one of 1—2 $OCH_3$, 1—3 $CH_3$, $—CH_2CH_3$ or $CF_3$; $C_4$—$C_7$ cycloalkylalkyl; $—CH_2CN$; $—CH_2CH_2CN$;

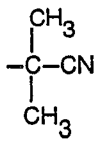 ; $OCH_3$ ; $OCH_2CH_3$ ; 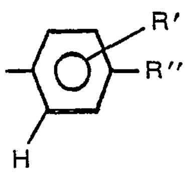 or 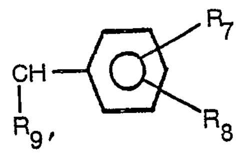 ;

where

$R'$ is H, $C_1$—$C_4$ alkyl, $OCH_3$, F, Cl, Br, CN, $NO_2$ or $CF_3$;

$R''$ is H, $CH_3$, Cl, F or Br;

$R_7$, $R_8$ and $R_9'$ are as previously defined;

$R_6$ is H, $C_1$—$C_3$ alkyl; $CH_2CN$; $CH_2CH_2$—CN or $—CH_2—CH{=}CH_2$ and $R_6$ and $R^I$ may be taken together to form $—(CH_2)_4—$, $—(CH_2)_5—$ or $—CH_2CH_2O—CH_2CH_2—$;

with the proviso that when $R_6$ is $CH_2CH_2CN$ or $CH_2CN$, then $R^1$ is $CH_2CH_2CN$ or $CH_2CN$; and $R^1$ and $R_6$ have a total carbon atom count of $\leq 13$; and when $R^I$ is $OCH_3$ or $OCH_2CH_3$ then $R_6$ is $CH_3$ or H;

when A is 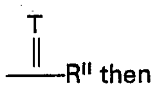 then

$R^{II}$ is H, $C_1$—$C_6$ alkyl; $C_3$—$C_6$ alkenyl; phenyl; benzyl; benzyl or phenyl substituted with 1—2 Cl, 1—2 $OCH_3$, 1—2 $CH_3$; $C_5$—$C_6$ cycloalkyl; $C_4$—$C_7$ cycloalkylalkyl with the proviso that when T is $=N—OR^{III}$, then $R^{II}$ must be $C_1$—$C_6$ alkyl or $C_3$—$C_6$ alkenyl;

B is 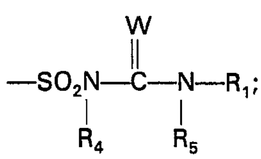;

where $R_4$ is H or $CH_3$; W is O or S; $R_5$ is H, $CH_3$ or $CH_3O$; with the further proviso that either $R_4$ or $R_5$ must be H;

$R_1$ is 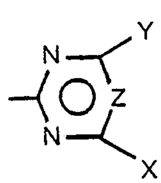 , 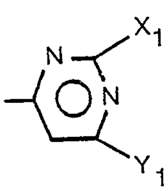 , 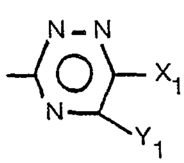 , 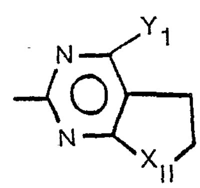 or 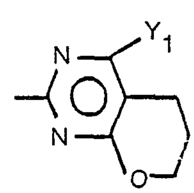 ;

where

Z is N, CH or C—F;

X=H, Cl, $—CH_3$, $—OCH_3$ or $—OCH_2CH_3$;

Y=H; Cl; $C_1$—$C_4$ alkyl; $C_1$—$C_4$ alkyl substituted with $—OCH_3$, $—OC_2H_5$, —CN, $—CO_2CH_3$, $—CO_2C_2H_5$,

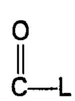

or 1—3 atoms of F, Cl, Br; $C_3$—$C_4$ alkenyl; $—O—(CH_2)_{n'}O—(C_1—C_3$ alkyl) where n' is 2 or 3;

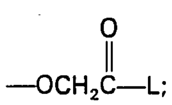; 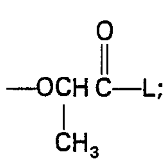; 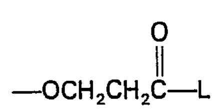 where

L is OH, $—NH_2$, 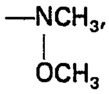

$—NH(C_1—C_4$ alkyl), $—N(C_1—C_4$ alkyl$)_2$ or $C_1—C_6$ alkoxy; SCN; $—N_3$; $NR_{11}R_{12}$ where $R_{11}$ is H or $CH_3$ and $R_{12}$ is H, $—OCH_3$, $C_1—C_4$ alkyl, $C_3—C_6$ cycloalkyl, $C_3—C_4$ alkenyl, $C_2—C_3$ alkyl substituted with $OCH_3$ or $OC_2H_5$, $C_1—C_2$ alkyl substituted with —CN, $CO_2H$, $CO_2CH_3$ or $CO_2C_2H_5$, and $R_{11}$ and $R_{12}$ can be taken together to form $—CH_2CH_2CH_2CH_2—$ or $CH_2CH_2OCH_2CH_2—$; $—O—R_9$ where $R_9$ is $C_1—C_4$ alkyl, $C_2—C_4$ alkyl substituted with 1—3 atoms of F, Cl or Br, $C_1—C_2$ alkyl substituted with cyano, $C_3—C_4$ alkenyl, $—CH_2C{\equiv}CR_{13}$,

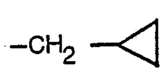 , 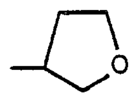 or 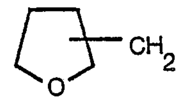 :

$R_{13}$ is H, $CH_3$ or $CH_2Cl$; $SR_{14}$

where

$R_{14}$ is $C_1—C_4$ alkyl, allyl, propargyl or $C_1—C_2$ alkyl substituted with CN; with the proviso that when X and Y are both H, then $R^I$ and $R^{II}$ are less than 5 carbons;

$X_1 = H$, Cl, $OCH_3$, $OCH_2CH_3$ or $CH_3$;

$Y_1 = H$, $OCH_3$ or $CH_3$; and

$X_{II} = O$ or $CH_2$

and further provided that when A contains greater than 5 carbon atoms, then Y must contain $\leq 4$ carbon atoms,

or an agriculturally suitable salt thereof, which comprises (a) reacting an appropriate isocyanate or isothiocyanate of formula

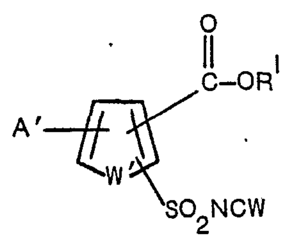

with an amine of formula $HNR_1R_5$,

wherein W, W', $R_1$ and $R_5$ are as defined above,

A' is H, Cl, Br, $C_1—C_4$ alkyl, $OCH_3$, $NO_2$ or $CF_3$;

$R^I$ is $C_1—C_6$ alkyl; $C_3—C_6$ alkenyl; $C_3—C_6$ alkynyl; $C_2—C_6$ alkyl substituted with Cl, CN or $OCH_3$; $C_3—C_6$ alkenyl substituted with 1—3 Cl; $C_3—C_6$ alkynyl substituted with Cl; $C_5—C_6$ cycloalkyl; cyclohexenyl; cyclohexyl substituted with 1—3 $CH_3$; $C_4—C_7$ cycloalkyl-alkyl or

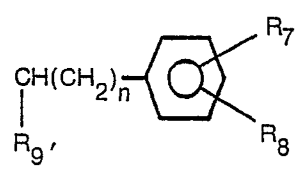

where $R_7$ and $R_8$ are independently H, Cl, $CH_3$ or $OCH_3$;

$C_2H_5$, $CH(CH_3)_2$, phenyl, $CH_2CH_2Cl$, $CH_2CCl_3$; $\text{+}CH_2CH_2O\text{+}_{n'}$ $R_{16}$, $\text{+}\underset{CH_3}{CH}—CH_2O\text{+}_{n'}R_{16}$

where $R_{16}$ is $CH_3$, $C_2H_5$, $CH(CH_3)_2$, phenyl, $CH_2CH_2Cl$, $CH_2CCl_3$, and n' is 2 or 3;

$CH_2CN$; 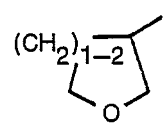 ; 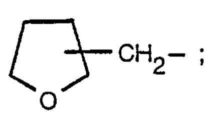 ; 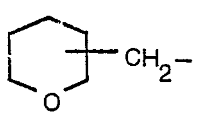 ; 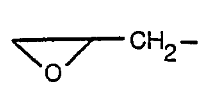 ; $CH_2OR'_6$ ;

where $R'_6$ is $C_1—C_4$ alkyl;

provided $R^I$ has a total of $\leq 13$ carbon atoms;

(b) reacting an appropriate sulfonamide of formula

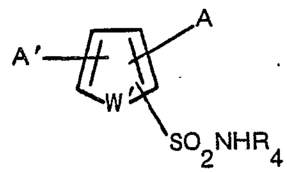

with an isocyanate or isothiocyanate of formula

$$R_1NCW$$

wherein A, A', W, W', $R_1$ and $R_4$ are as defined above to prepare a compound of formula (I), (II) or (III) wherein $R_5$ is H;

(c) methylating a compound of formula (I), (II) or (III) wherein $R_4$ is H and W is O to obtain a compound of formula (I), (II) or (III) wherein $R_4$ is methyl;

(d) reacting a carbamyl chloride of formula

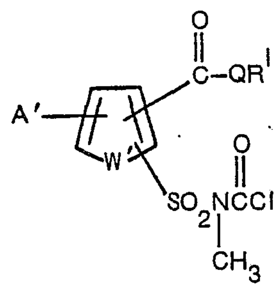

wherein A', W, W', Q and $R^I$ are as defined above, with an amine of formula

$$HNR_1R_5$$

wherein $R_1$ and $R_5$ are as defined above;

(e) esterifying a corresponding starting compound wherein A is COOH to obtain a compound of formula (I), (II) or (III) wherein A is $COOR^I$;

(f) reacting a compound of formula (I), (II), or (III) wherein A is $CO_2R^I$, where $R^I$ has 1—4 carbon atoms, with a compound of formula

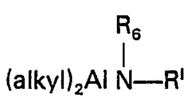

to obtain a compound of formula (I), (II) or (III) wherein

A is 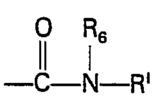

wherein $R^I$ and $R_6$ are as defined above;

(g) reacting a compound of formula (I), (II) or (III) wherein $QR^I$ is $O(C_1—C_4$ alkyl) with a compound of formula

$$(R^IS)_2Al(alkyl)$$

to obtain a corresponding compound of formula (I), (II) or (III) wherein $QR^I$ is $SR^I$, $R^I$ being as defined above;

(h) reacting an ester of formula (I), (II) or (III) wherein $R^I$ is a lower primary alkyl group with a compound of formula

$$(alkyl)_2AlOR^I$$

where $R^I$ is a secondary alkyl group within the definition of $R^I$ above, to replace said lower primary alkyl group by $R^I$;

(i) reacting a compound of formula (I), (II) or (III) wherein A is $CO_2H$ with $R^{II}Li$ to obtain a compound of formula (I), (II) or (III) wherein A is $COR^{II}$, $R^{II}$ being as defined above; whereafter if desired said ketone is reacted with a hydroxylamine of formula

$$H_2NOR^{III}$$

to obtain a compound of formula I, II or III wherein A is $-\overset{\overset{\displaystyle NOR'''}{\|}}{C}-R''$;

(j) hydrolyzing a compound of formula I, II or III wherein Y is $-Q'-\overset{\overset{\displaystyle O}{\|}}{C}-L$

where Q' is $C_1$—$C_4$ alkylene, $-OCH_2-$, $-OCH_2CH_2-$, $-\underset{\underset{\displaystyle CH_3}{|}}{OCH}-$, $-\underset{\underset{\displaystyle R_{11}}{|}}{N}-$ or $-\underset{\underset{\displaystyle R_{11}}{|}}{N}CH_2-$

where $R_{11}$ is as defined above and L is other than OH, to obtain a compound of formula I, II or III wherein L is OH, or an agriculturally suitable salt thereof; or

(k) reacting a compound of formula I, II or III wherein A is $CO_2Me$ and A' is other than $NO_2$ with sodium bis-(2-methoxyethoxy)aluminium hydride or with diisobutylaluminum hydride to obtain a compound of formula I, II or III wherein A is —CHO.

2. A process of Claim 1 wherein at least one of the following applies:

(a) A' is not $OCH_3$, $NO_2$ or $CF_3$

(b) $R^I$ is not $OCH_2CH_3$ or $C_2$—$C_6$ alkyl substituted with F or Br

(c) $R^I$ is not

$CH_2CN$; 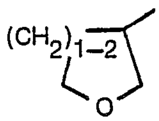 ; 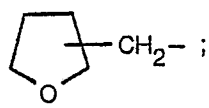 ; 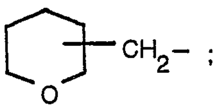 ; 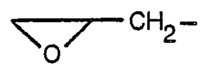 ; $CH_2OR'_6$ ;

(d) R' is not CN, $NO_2$ or $CF_3$

(e) when $R^I$ is $OCH_3$, $R_6$ is not H

(f) $R^{II}$ is not H

(g) $R_1$ is not 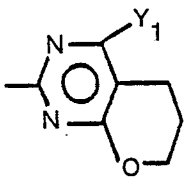

(h) $R_9$ is not 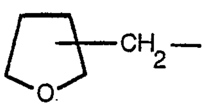

(i) Z is not C—F.

3. A process of Claim 1 or 2 in which

B is $-SO_2-NH-\overset{\overset{\displaystyle O}{\|}}{C}-NHR_1$

and T is oxygen; and in which

$R_1$ is 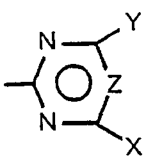 , 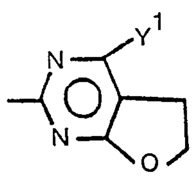 or 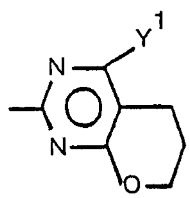

Q is O or S and $R^I$ is $C_1$—$C_4$ alkyl; $C_3$—$C_4$ alkenyl; $C_3$—$C_4$ alkynyl; $C_2$—$C_3$ alkyl substituted with CN, $OCH_3$ or 1—3F, Cl or Br; $C_3$—$C_4$ alkenyl substituted with 1—3 Cl or $C_3$—$C_4$ alkynyl substituted with Cl; or

in which Q is oxygen and $R^I$ is $CH_2CH_2OR_{15}$; $CH_2CH_2CH_2OR_{15}$; $\underset{\underset{\displaystyle CH_3}{|}}{CH}CH_2$ $OR_{15}$ where $R_{15}$ is $CH_2CH_3$;

$CH_2CN$; $CH_2OR'_6$ where $R'_6$ is $CH_3$ or $CH_3CH_2$; or 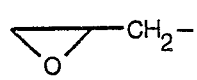 ;

4. A process of Claim 1 or 2 in which Q is 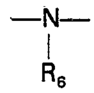 and

$R^I$ is H, $C_1$—$C_4$ alkyl, $CH_2CH_2OR_{10}$, $CH_2CH_2CH_2OR_{10}$ where $R_{10}$ is $CH_3$ or $CH_3CH_2$; $C_3$—$C_4$ alkenyl; $CH_2CN$; $CH_2CH_2CN$; $OCH_3$ or $OCH_2CH_3$;

$R^6$ is H, $C_1$—$C_2$ alkyl, $CH_2CN$ or $CH_2CH_2CN$ and $R_6$ and $R^I$ can be taken together to form $-(CH_2)_4-$.

5. A process of Claim 3 in which $R^{II}$ is H or $C_1$—$C_3$ alkyl.

6. A process of any of Claims 3—5 in which

Z is CH or N;

X is $CH_3$ or $CH_3O$; and

Y is $C_1$—$C_2$ alkyl; $C_1$—$C_2$ alkyl substituted with $OCH_3$; $OCH_2CH_3$, CN or 1—3 atoms of F, Cl or Br;

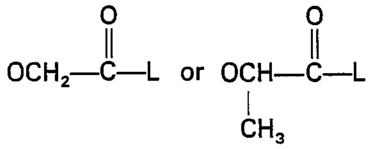 where

L is $NH_2$, $OH_5$ 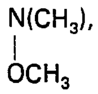

$N(CH_3)_2$, $NHCH_3$, $C_1$—$C_2$ alkoxy; SCN; $N_3$; $NR_{11}R_{12}$ where $R_{11}$ is H or $CH_3$; $R_{12}$ is H, $CH_3$, $CH_3CH_2$, $OCH_3$; $OR_9$ where $R_9$ is $CH_3$, $CH_3CH_2$; $CH_2CH{=}CH_2$; $CH_2C{\equiv}CH$; or $C_2$ alkyl substituted with 1—3 F, Cl or Br; $CH_3S$.

7. A process of Claim 2 or 3 in which A′ is H, Cl or Br;

Q is O or S and $R^I$ is $C_1$—$C_4$ alkyl, $CH_2CH{=}CH_2$ or $CH_2CH_2Cl$; or in which Q is O and $R^I$ is $CH_2CH_2OCH_3$, 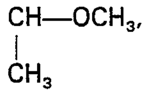 $CH_2OCH_3$ or $CH_2OCH_2CH_3$;

or in which Q is 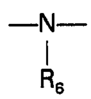

and $R^I$ is H; $C_1$—$C_3$ alkyl, $OCH_3$ or $OCH_2CH_3$ and $R_6$ is H or $C_1$—$C_2$ alkyl.

8. A process of Claim 7 in which A′ is H; Y is $CH_3$, $OCH_3$, $OCH_2CH_3$, $OCH_2CF_3$, $OCH_2CH{=}CH_2$ or $OCH_2{\equiv}CH$.

9. A process of Claim 6 in which A is 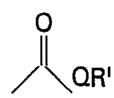

and Q is oxygen or sulfur and $R^I$ is $CH_3$ or $CH_2CH_3$; Q is 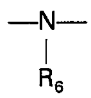

and $R^I$ is H, $CH_3$ or $OCH_3$ and $R_6$ is $CH_3$;

$R_1$ is 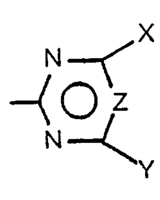

Y is $CH_3$ or $OCH_3$ and W′ is sulfur.

10. A process of Claim 1 wherein the product is a compound or an agriculturally suitable salt thereof selected from

methyl 3-[[(4-methoxy-6-methylpyrimidin-2-yl)aminocarbonyl]aminosulfonyl]-2-thiophenecarboxylate

methyl 3-[[(4,6-dimethoxy-1,3,5-triazin-2-yl)aminocarbonyl]aminosulfonyl]-2-thiophenecarboxylate

methyl 3-[[(4,6-dimethyl-1,3,5-triazin-2-yl)aminocarbonyl]aminosulfonyl]-2-thiophenecarboxylate

methyl 3-[[(4-methoxy-6-methyl-1,3,5-triazin-2-yl)aminocarbonyl]aminosulfonyl]-2-thiophenecarboxylate.

11. A process of Claim 1 wherein the product is a compound or an agriculturally suitable salt thereof selected from
methyl 2-[[(4,6-dimethoxypyrimidin-2-yl)aminocarbonyl]aminosulfonyl]-3-thiophenecarboxylate
methyl 2-[[(4,6-dimethylpyrimidin-2-yl)aminocarbonyl]aminosulfonyl]-3-thiophenecarboxylate
methyl 2-[[(4-methoxy-6-methylpyrimidin-2-yl)aminocarbonyl]aminosulfonyl]-3-thiophenecarboxylate
methyl 2-[[(4,6-dimethoxy-1,3,5-triazin-2-yl)aminocarbonyl]aminosulfonyl]-3-thiophenecarboxylate or
methyl 2-[[(4-methoxy-6-methyl-1,3,5-triazin-2-yl)aminocarbonyl]aminoxulfonyl]-3-thiophenecarboxylate.

12. A process of Claim 1 wherein the product is a compound or an agriculturally suitable salt thereof selected from
methyl 4-[[(4,6-dimethoxypyrimidin-2-yl)aminocarbonyl]aminosulfonyl]-3-thiophenecarboxylate
methyl 4-[[(4,6-dimethylpyrimidin-2-yl)aminocarbonyl]aminosulfonyl]-3-thiophenecarboxylate
methyl 4-[[(4-methoxy-6-methylpyrimidin-2-yl)aminocarbonyl]aminosulfonyl]-3-thiophenecarboxylate
methyl 4-[[(4,6-dimethoxy-1,3,5-triazin-2-yl)aminocarbonyl]aminosulfonyl]-3-thiophenecarboxylate or
methyl 4-[[(4-methoxy-6-methyl-1,3,5-triazin-2-yl)aminocarbonyl]aminosulfonyl]-3-thiophenecarboxylate.

13. A process of Claim 1 wherein the product is N-[(4-methoxy-6-methylpyrimidin-2-yl)aminocarbonyl]-2-(1-pyrrolidinylcarbonyl)-3-thiophenesulfonamide, or an agriculturally suitable salt thereof.

14. A process of Claim 1 wherein the product is 1-methylethyl 3 - [[(4,6 - dimethoxypyrimidin-2-yl)aminocarbonyl]aminosulfonyl]-2-thiophenecarboxylate or an agriculturally suitable salt thereof.

15. A method for controlling the growth of undesired vegetation by applying to the locus to be protected an effective amount of a herbicidal compound, characterised in that said herbicidal compound comprises a compound of formula (I), (II) or (III) as defined in any of Claims 1 to 14.

16. A method for regulating the growth of plants by applying to the locus of said plants an effective but substantially non-phytotoxic amount of a plant growth regulant, characterised in that said plant growth regulant comprises a compound of formula (I), (II) or (III) as defined in any of Claims 1 to 14.

17. A process for the preparation of a compound selected from

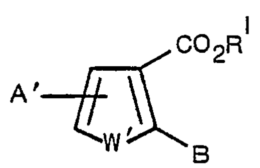
I′

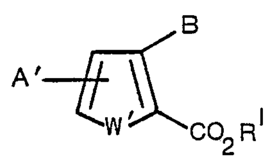
II′

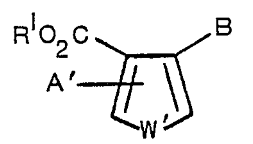
III′

wherein
$R^I$ is H or M;
M is a cation of an alkali metal or of a tertiary amine of up to 12 carbon atoms;
W′ is O or S;
A′ is H, Cl, Br, $C_1$—$C_4$ alkyl, $OCH_3$, $NO_2$ or $CF_3$;

B is 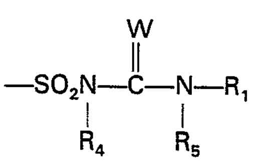

where

$R_4$ is H or $CH_3$; W is O or S; $R_5$ is H, $CH_3$ or $CH_3O$; with the proviso that either $R_4$ or $R_5$ must be H;

$R_1$ is 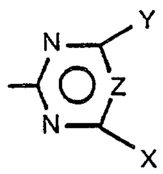 , 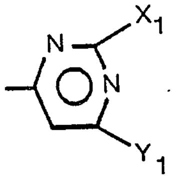 , 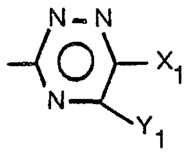 , 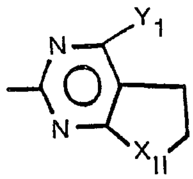 or 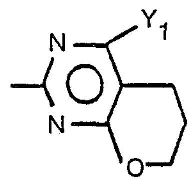 ;

where

Z is N, CH or C—F;

X=H, Cl, $—CH_3$, $—OCH_3$ or $—OCH_2CH_3$;

Y=H; Cl; $C_1$—$C_4$ alkyl; $C_1$—$C_4$ alkyl substituted with $—OCH_3$, $—OC_2H_5$, —CN, $—CO_2CH_3$, $—CO_2C_2H_5$,

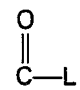

or 1—3 atoms of F, Cl, Br; $C_3$—$C_4$ alkenyl; $—O—(CH_2)_{n'}O—(C_1—C_3$ alkyl) where n' is 2 or 3;

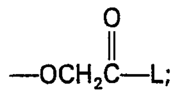 ; 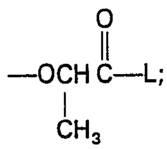 ; 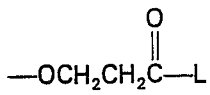 where

L is OH, $—NH_2$, 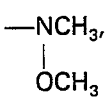

$—NH(C_1—C_4$ alkyl), $—N(C_1—C_4$ alkyl$)_2$ or $C_1$—$C_6$ alkoxy; SCN; $—N_3$; $NR_{11}R_{12}$ where $R_{11}$ is H or $CH_3$ and $R_{12}$ is H, $—OCH_3$, $C_1$—$C_4$ alkyl, $C_3$—$C_6$ cycloalkyl, $C_3$—$C_4$ alkenyl, $C_2$—$C_3$ alkyl substituted with $OCH_3$ or $OC_2H_5$, $C_1$—$C_2$ alkyl substituted with —CN, $CO_2H$, $CO_2CH_3$ or $CO_2C_2H_5$, and $R_{11}$ and $R_{12}$ can be taken together to form $—CH_2CH_2CH_2CH_2—$ or $CH_2CH_2OCH_2CH_2—$; O—$R_9$ where $R_9$ is $C_1$—$C_4$ alkyl, $C_2$—$C_4$ alkyl substituted with 1—3 atoms of F, Cl or Br, $C_1$—$C_2$ alkyl substituted with cyano, $C_3$—$C_4$ alkenyl, $—CH_2C{\equiv}CR_{13}$,

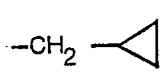 , 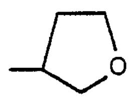 or 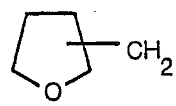 :

$R_{13}$ is H, $CH_3$ or $CH_2Cl$; $SR_{14}$;

where

$R_{14}$ is $C_1$—$C_4$ alkyl, allyl, propargyl or $C_1$—$C_2$ alkyl substituted with CN;

$X_1$ = H, Cl, $OCH_3$, $OCH_2CH_3$ or $CH_3$;

$Y_1$ = H, $OCH_3$ or $CH_3$; and

$X_{II}$ = O or $CH_2$;

which comprises hydrolyzing a corresponding starting compound wherein $R^I$ is other than H, under basic conditions, and if desired acidifying to obtain a product wherein $R^I$ is H.

**Patentansprüche für die Vertragsstaaten: BE CH DE FR IT LI LU NL SE**

1. Verbindung ausgewählt aus

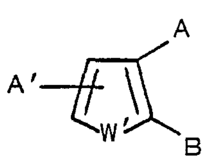
I

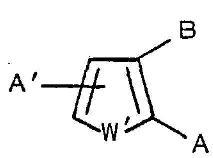
II

oder

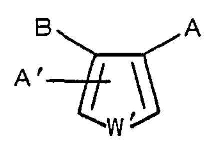
III

worin

W' O oder S ist;

A' H, Cl, Br, $C_1$—$C_4$-Alkyl, $OCH_3$, $NO_2$ oder $CF_3$ ist;

A $-\overset{\overset{O}{\|}}{C}-Q-R^I$ oder $-\overset{\overset{T}{\|}}{C}-R^{II}$ ist, worin

Q O, S oder $-\underset{\underset{R_6}{|}}{N}-$ ist;

T O oder 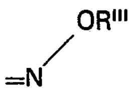

ist, worin

$R^{III}$ H, $C_1$—$C_4$-Alkyl oder $C_3$—$C_4$-Alkenyl ist;

wenn

Q O oder S ist, dann

ist $R^I$ $C_1$—$C_6$-Alkyl; $C_3$—$C_6$-Alkenyl; $C_3$—$C_6$-Alkinyl; $C_2$—$C_6$-Alkyl substituiert mit 1—3 Cl, F oder Br, oder einem von CN oder $OCH_3$; $C_3$—$C_6$-Alkenyl substituiert mit 1—3 Cl; $C_3$—$C_6$-Alkinyl substituiert mit Cl; $C_5$—$C_6$-Cycloalkyl; Cyclohexenyl; Cyclohexyl substituiert mit 1—3 $CH_3$; $C_4$—$C_7$-Cycloalkylalkyl oder

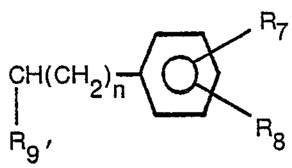

worin $R_7$ und $R_8$ unabhängig H, Cl, $CH_3$ oder $OCH_3$ sind;

n 0 oder 1 ist; und

$R_9$ H oder $CH_3$ ist;

wenn Q

O ist dann kann $R^I$ sein: $CH_2CH_2OR_{15}$; $CH_2CH_2CH_2OR_{15}$; $\underset{\underset{CH_3}{|}}{CH}-CH_2OR_{15}$;

worin $R_{15}$ $C_2H_5$, $CH(CH_3)_2$, Phenyl, $CH_2CH_2Cl$, $CH_2CCl_3$; $+CH_2CH_2O+_{n'}R_{16}$, $+\underset{\underset{CH_3}{|}}{CH}-CH_2O+_{n'}R_{16}$ ist;

worin $R_{16}$ $CH_3$, $C_2H_5$, $CH(CH_3)_2$, Phenyl, $CH_2CH_2Cl$, $CH_2CCl_3$ ist und n' 2 oder 3 ist;

$CH_2CN$; 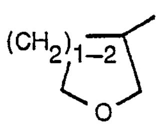 ; 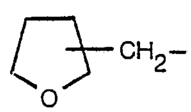 ; 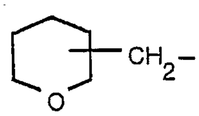 ; 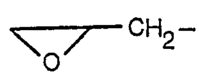 ; $CH_2OR'_6$ ;

worin $R'_6$ $C_1$—$C_4$-Alkyl ist;

vorausgesetzt daß $R^I$ insgesamt ≤13 Kohlenstoffatome aufweist;

wenn Q $-\underset{\underset{R_6}{|}}{N}-$ ist dann

ist $R^I$ H; $C_1$—$C_6$-Alkyl; $-CH_2CH_2OR_{10}$; $-CH_2CH_2CH_2OR_{10}$; worin $R_{10}$ $CH_3$, $CH_3CH_2$, $CH(CH_3)_2$ oder Phenyl ist; $C_3$—$C_6$-Alkenyl; $C_3$—$C_6$-Cycloalkyl; $C_5$—$C_6$-Cycloalkenyl; $C_6$-Cycloalkyl substituiert mit beliebigen von 1—2 $OCH_3$, 1—3 $CH_3$, $-CH_2CH_3$ oder $CF_3$; $C_4$—$C_7$-Cycloalkylalkyl; $-CH_2CN$; $-CH_2CH_2CN$;

$-\overset{\overset{CH_3}{|}}{\underset{\underset{CH_3}{|}}{C}}-CN$ ; $OCH_3$ ; $OCH_2CH_3$ ; 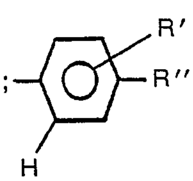 oder 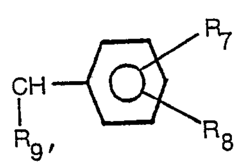 ;

worin

$R'$ H, $C_1$—$C_4$-Alkyl, $OCH_3$, F, Cl, Br, CN, $NO_2$ oder $CF_3$ ist;

$R''$ H, $CH_3$, Cl, F oder Br ist;

$R_7$, $R_8$ und $R_9'$ wie vorstehend definiert sind;

$R_6$ H, $C_1$—$C_3$-Alkyl; $CH_2CN$; $CH_2CH_2$—CN oder —$CH_2$—CH=$CH_2$ ist und $R_6$ und $R^I$ zusammengenommen werden können, unter Bildung von —$(CH_2)_4$—, —$(CH_2)_5$— oder —$CH_2CH_2O$—$CH_2CH_2$—; mit der Maßgabe, daß wenn $R_6$ $CH_2CH_2CN$ oder $CH_2CN$ ist, dann $R^I$ $CH_2CH_2CN$ oder $CH_2CN$ ist; und $R^I$ und $R_6$ eine Gesamtkohlenstoffanzahl von $\leq 13$ aufweisen; und wenn $R^I$ und $OCH_3$ oder $OCH_2CH_3$ ist, dann $R_6$ $CH_3$ oder H ist;

wenn A 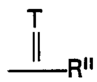

ist, dann

ist $R^{II}$ H, $C_1$—$C_6$-Alkyl; $C_3$—$C_6$-Alkenyl; Phenyl; Benzyl; Benzyl oder Phenyl substituiert mit 1—2 Cl, 1—2 $OCH_3$, 1—2 $CH_3$; $C_5$—$C_6$-Cycloalkyl; $C_4$—$C_7$-Cycloalkylalkyl;
mit der Maßgabe, daß wenn T die Bedeutung von =N—$OR^{III}$ hat, dann $R^{II}$ $C_1$—$C_6$-Alkyl oder $C_3$—$C_6$-Alkenyl sein muß;

B 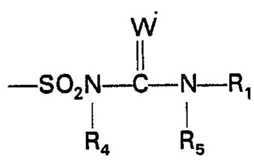 ist;

worin

$R_4$ H oder $CH_3$ ist;

W O oder S ist;

$R_5$ H, $CH_3$ oder $CH_3O$ ist; mit der weiteren Maßgabe, daß entweder $R_4$ oder $R_5$ H sein muß;

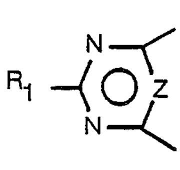 , 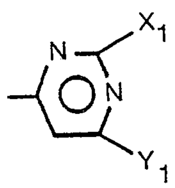 , 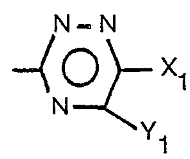 , 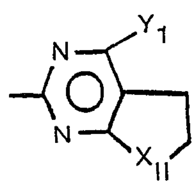 oder 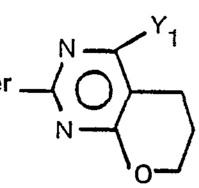 ist;

worin

Z N, CH oder C—F ist;

X=H, Cl, —$CH_3$, —$OCH_3$ oder —$OCH_2CH_3$;

Y=H; Cl; $C_1$—$C_4$-Alkyl; $C_1$—$C_4$-Alkyl substituiert mit —$OCH_3$, —$OC_2H_5$, —CN, —$CO_2CH_3$, —$CO_2C_2H_5$,

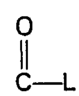

oder 1—3 Atomen von F, Cl, Br; $C_3$—$C_4$-Alkenyl; —O—$(CH_2)_{n'}$ O—($C_1$—$C_3$-Alkyl), worin

n' 2 oder 3 ist; 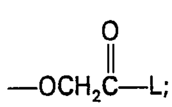 ; 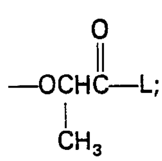 ; 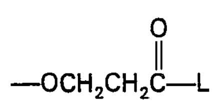

worin L OH, —$NH_2$, 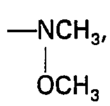

—NH($C_1$—$C_4$-Alkyl), —N—($C_1$—$C_4$-Alkyl)$_2$ oder $C_1$—$C_6$-Alkoxy ist; SCN; —$N_3$; $NR_{11}R_{12}$, worin $R_{11}$ H oder $CH_3$ ist und $R_{12}$ H, —$OCH_3$, $C_1$—$C_4$-Alkyl, $C_3$—$C_6$-Cycloalkyl, $C_3$—$C_4$-Alkenyl, $C_2$—$C_3$-Alkyl substituiert mit $OCH_3$ oder $OC_2H_5$, $C_1$—$C_2$-Alkyl substituiert mit —CN, $CO_2H$, $CO_2CH_3$ oder $CO_2C_2H_5$ ist, und $R_{11}$ und $R_{12}$ zusammengenommen werden können, unter Bildung von —$CH_2CH_2CH_2CH_2$— oder $CH_2CH_2OCH_2CH_2$—; —O—$R_9$, worin $R_9$ $C_1$—$C_4$-Alkyl, $C_2$—$C_4$-Alkyl substituiert mit 1—3 Atomen von

F, Cl oder Br, $C_1$—$C_2$-Alkyl substituiert mit Cyano, $C_3$—$C_4$-Alkenyl, —$CH_2C{\equiv}CR_{13}$,

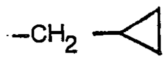 , 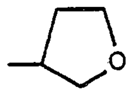 oder 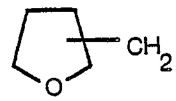 ist:

$R_{13}$ H, $CH_3$ oder $CH_2Cl$ ist; $SR_{14}$;
worin

$R_{14}$ $C_1$—$C_4$-Alkyl, Allyl, Propargyl oder $C_1$—$C_2$-Alkyl substituiert mit CN ist; mit der Maßgabe, daß wenn X und Y beide H sind, dann $R^I$ und $R^{II}$ weniger als 5 Kohlenstoffatome sind;

$X_1$=H, Cl, $OCH_3$, $OCH_2CH_3$ oder $CH_3$;

$Y_1$=H, $OCH_3$ oder $CH_3$; und

$X_{II}$=O oder $CH_2$

und weiter vorausgesetzt, daß wenn A mehr als 5 Kohlenstoffatome enthält, dann Y ≤4 Kohlenstoffatome enthalten muß,
und ihre landwirtschaftlich brauchbaren Salze.

2. Verbindung nach Anspruch 1, worin

$$B\ SO_2—NH—\overset{\overset{\displaystyle O}{\|}}{C}—NHR_1$$

ist.

3. Verbindung nach Anspruch 2, worin T Sauerstoff ist.

4. Verbindung nach Anspruch 2 oder 3, worin $R_1$ die Bedeutung hat von

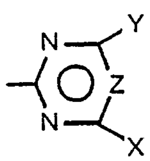 , 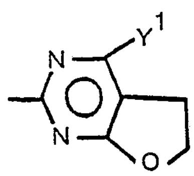 oder 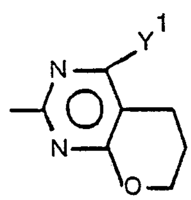

5. Verbindung nach Anspruch 4, worin Q O oder S ist, und $R^I$ $C_1$—$C_4$-Alkyl; $C_3$—$C_4$-Alkenyl; $C_3$—$C_4$-Alkinyl; $C_2$—$C_3$-Alkyl, substituiert mit CN, $OCH_3$ oder 1—3 F, Cl oder Br; $C_3$—$C_4$-Alkenyl substituiert mit 1—3 Cl oder $C_3$—$C_4$-Alkinyl, substituiert mit Cl ist.

6. Verbindung nach Anspruch 4, worin Q Sauerstoff ist und $R^I$ $CH_2CH_2OR_{15}$; $CH_2CH_2CH_2OR_{15}$;

$$\underset{\underset{\displaystyle CH_3}{|}}{CH}CH_2OR_{15},$$

worin $R_{15}$ $CH_2CH_3$ ist; $CH_2CN$; $CH_2OR_6'$, worin $R_6'$ $CH_3$ oder $CH_3CH_2$ ist; und 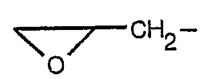 ;

7. Verbindung nach Anspruch 4, worin Q $—\underset{\underset{\displaystyle R_6}{|}}{N}—$ ist und

$R^I$ H, $C_1$—$C_4$-Alkyl, $CH_2CH_2OR_{10}$, $CH_2CH_2CH_2OR_{10}$ ist, worin $R_{10}$ $CH_3$ oder $CH_3CH_2$ ist; $C_3$—$C_4$-Alkenyl; $CH_2CN$; $CH_2CH_2CN$; $OCH_3$ oder $OCH_2CH_3$ ist;

$R^6$ H, $C_1$—$C_2$-Alkyl, $CH_2CN$ oder $CH_2CH_2CN$ und $R_6$ und $R^I$ zusammengenommen $-(CH_2)_n-$ bilden können, ist.

8. Verbindung nach Anspruch 4, worin $R^{II}$ H oder $C_1$—$C_3$-Alkyl ist.

9. Verbindung nach einem der Ansprüche 5—8, worin

Z CH oder N ist;

X $CH_3$ oder $CH_3O$ ist; und

Y $C_1$—$C_2$-Alkyl; $C_1$—$C_2$-Alkyl substituiert mit $OCH_3$; $OCH_2CH_3$, CN oder 1—3 Atomen von F, Cl oder Br;

$$OCH_2—\overset{\overset{\displaystyle O}{\|}}{C}—L \text{ oder } \underset{\underset{\displaystyle CH_3}{|}}{OCH}—\overset{\overset{\displaystyle O}{\|}}{C}—L \text{ worin}$$

L $NH_2$, OH, $N(CH_3)$,
$OCH_3$

$N(CH_3)_2$, $NHCH_3$, $C_1$—$C_2$-Alkoxy ist; SCN; $N_3$; $NR_{11}R_{12}$, worin $R_{11}$ H oder $CH_3$ ist; $R_{12}$ H, $CH_3$, $CH_3CH_2$, $OCH_3$ ist; $OR_9$, worin $R_9$ $CH_3$, $CH_3CH_2$ ist; $CH_2CH{=}CH_2$, $CH_2C{\equiv}CH$; oder $C_2$ Alkyl substituiert mit 1—3 F, Cl oder Br; $CH_3S$ ist.

10. Verbindung nach Anspruch 4, worin A' H, Cl oder Br ist.

11. Verbindung nach Anspruch 10, worin Q O oder S ist und R' $C_1$—$C_4$-Alkyl, $CH_2CH{=}CH_2$ oder $CH_2CH_2Cl$ ist.

12. Verbindung nach Anspruch 10, worin Q O ist, und R' $CH_2CH_2OCH_3$, $CH(CH_3)$—$OCH_3$ $CH_2OCH_3$ oder $CH_2OCH_2CH_3$ ist.

13. Verbindung nach Anspruch 10, worin Q —N($R_6$)— ist und R' H ist; $C_1$—$C_3$-Alkyl, $OCH_3$ oder $OCH_2CH_3$ ist, und $R_6$ H oder $C_1$—$C_2$-Alkyl ist.

14. Verbindung nach Anspruch 10, worin R'' H oder $CH_3$ ist.

15. Verbindung nach einem der Ansprüche 11—14, worin A' H ist; Y $CH_3$, $OCH_3$, $OCH_2$ $CH_3$, $OCH_2$ $CF_3$, $OCH_2CH{=}CH_2$ oder $OCH_2C{\equiv}CH$ ist.

16. Verbindung nach Anspruch 9, worin A 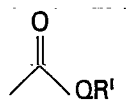 ist und Q Sauerstoff oder Schwefel ist und R' $CH_3$ oder $CH_2CH_3$ ist; Q —N($R_6$)— ist und R' H, $CH_3$ oder $OCH_3$ ist und $R_6$ $CH_3$ ist;

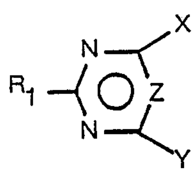 ist

und Y $CH_3$ oder $OCH_3$ ist.

17. Verbindung nach Anspruch 16, in der die Formel I verwendet wird.

18. Verbindung nach Anspruch 16, in der die Formel II verwendet wird.

19. Verbindung nach Anspruch 16, in der die Formel III verwendet wird.

20. Verbindung nach einem der Ansprüche 1 bis 19, worin W' Schwefel ist.

21. Verbindung nach einem der Ansprüche 1 bis 19, worin W' Sauerstoff ist.

22. Verbindung nach Anspruch 1 oder ein landwirtschaftlich brauchbares Salz davon, ausgewählt aus

Methyl 3-[[(4-methoxy-6-methylpyrimidin-2-yl)aminocarbonyl]-aminosulfonyl]-2-thiophen-carboxylat

Methyl 3-[[(4,6-dimethoxy-1,3,5-triazin-2-yl)aminocarbonyl]-aminosulfonyl]-2-thiophen-carboxylat

Methyl 3-[[(4-methoxy-6-methyl-1,3,5-triazin-2-yl)aminocarbonyl]-aminosulfonyl]-2-thiophen-carboxylat

Methyl 3-[[(4,6-dimethoxypyrimidin-2-yl)aminocarbonyl]-aminosulfonyl]-2-thiophen-carboxylat oder

Methyl 3-[[(4,6-dimethylpyrimidin-2-yl)aminocarbonyl]-aminosulfonyl]-2-thiophen-carboxylat.

23. Verbindung nach Anspruch 1 oder ein landwirtschaftlich brauchbares Salz davon ausgewählt aus

Methyl 2-[[(4,6-dimethoxypyrimidin-2-yl)aminocarbonyl]-aminosulfonyl]-3-thiophen-carboxylat

Methyl 2-[[(4,6-dimethylpyrimidin-2-yl)aminocarbonyl]-aminosulfonyl]-3-thiophen-carboxylat

Methyl 2-[[(4-methoxy-6-methylpyrimidin-2-yl)aminocarbonyl]-aminosulfonyl]-3-thiophen-carboxylat

Methyl 2-[[(4,6-dimethoxy-1,3,5-triazin-2-yl)aminocarbonyl]-aminosulfonyl]-3-thiophen-carboxylat oder

Methyl 2-[[(4-methoxy-6-methyl-1,3,5-triazin-2-yl)aminocarbonyl]-aminosulfonyl]-3-thiophen-carboxylat.

24. Verbindung nach Anspruch 1 oder ein landwirtschaftlich brauchbares Salz davon ausgewählt aus

Methyl 4-[[(4,6-dimethoxypyrimidin-2-yl)aminocarbonyl]-aminosulfonyl]-3-thiophen-carboxylat

Methyl 4-[[(4,6-dimethylpyrimidin-2-yl)aminocarbonyl]-aminosulfonyl]-3-thiophen-carboxylat

Methyl 4-[[(4-methoxy-6-methylpyrimidin-2-yl)aminocarbonyl]-aminosulfonyl]-3-thiophen-carboxylat

Methyl 4-[[(4,6-dimethoxy-1,3,5-triazin-2-yl)aminocarbonyl]-aminosulfonyl]-3-thiophen-carboxylat oder

Methyl 4-[[(4-methoxy-6-methyl-1,3,5-triazin-2-yl)aminocarbonyl]-aminosulfonyl]-3-thiophen-carboxylat.

25. Verbindung nach Anspruch 1 oder ein landwirtschaftlich brauchbares Salz davon, nämlich N - [(4 - Methoxy - 6 - methylpyrimidin - 2 - yl) - aminocarbonyl] - 2 - (1 - pyrrolidinylcarbonyl) - 3 - thiophensulfonamid.

26. Verbindung nach Anspruch 1 oder ein landwirtschaftlich brauchbares Salz davon, nämlich 1 - Methylethyl - 3 - [[(4,6 - dimethoxypyrimidin - 2 - yl) - aminocarbonyl] - aminosulfonyl] - 2 - thiophencarboxylat.

27. Verbindung nach Anspruch 1, worin mindestens eines der folgenden Gültigkeit hat:

(a) A′ ist nicht $OCH_3$, $NO_2$ oder $CF_3$

(b) $R^I$ ist nicht $OCH_2CH_3$ oder $C_2$—$C_6$ Alkyl substituiert mit F oder Br

(c) $R^I$ ist nicht

$CH_2CN$; 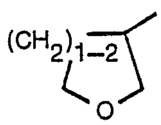 ; 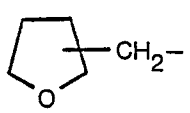 ; 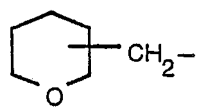 ; 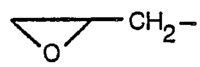 ; oder $CH_2OR'_6$ ;

(d) R′ ist nicht CN, $NO_2$ oder $CF_3$

(e) wenn $R^I$ $OCH_3$ ist, ist $R_6$ nicht H

(f) $R^{II}$ ist nicht H

(g) $R_1$ ist nicht 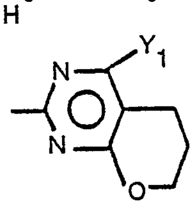

(h) $R_9$ ist nicht 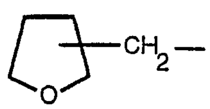

(i) Z ist nicht C—F.

28. Zusammensetzung geeignet zur Bekämpfung des Wachstums unerwünschter Vegetation enthaltend eine wirksame Menge einer herbiziden Verbindung und mindestens eines der folgenden: oberflächenaktives Mittel, festes oder flüssige Verdünnungsmittel, dadurch gekennzeichnet, daß die herbizide Verbindung eine Verbindung gemäß einem der Ansprüche 1 bis 27 umfaßt.

29. Verfahren zur Bekämpfung des Wachstums unerwünschter Vegetation durch Auftrag auf den zu schützenden Ort von einer wirksamen Menge einer herbiziden Verbindung, dadurch gekennzeichnet, daß die herbizide Verbindung eine Verbindung gemäß einem der Ansprüche 1 bis 27 umfaßt.

30. Verfahren zur Regulierung des Wachstums von Pflanzen durch Auftrag auf den Ort dieser Pflanzen von einer wirksamen, jedoch im wesentlichen nicht phytotoxischen Menge eines das Pflanzenwachstum regulierenden Mittels, dadurch gekennzeichnet, daß das Pflanzenwachstum regulierende Mittel eine Verbindung gemäß einem der Ansprüche 1 bis 27 umfaßt.

31. Verfahren zur Herstellung einer Verbindung nach Anspruch 1 durch Reaktion eines geeigneten Isocyanats oder Isothiocyanats der Formel

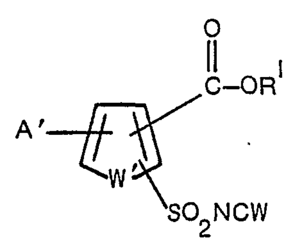

mit einem Amin der Formel $HNR_1H_5$, worin A', W, W', $R^I$, $R_1$ und $R_5$ jeweils wie in Anspruch 1 definiert sind.

32. Verbindung ausgewählt aus

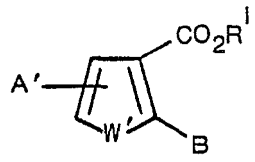
I'

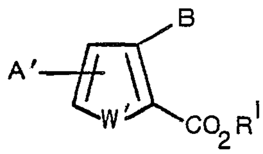
II'

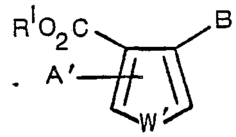
III'

worin $R^I$ H oder M ist;

M ein Kation eines Alkalimetalls oder eines tertiären Amins mit bis zu 12 Kohlenstoffatomen ist;

W' O oder S ist;

A', H, Cl, Br, $C_1$—$C_4$-Alkyl, $OCH_3$, $NO_2$ oder $CF_3$ ist;

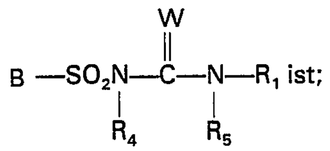

worin $R_4$ H oder $CH_3$ ist; W O oder S ist;

$R_5$ H, $CH_3$ oder $CH_3O$ ist; mit der Maßgabe, daß entweder $R_4$ oder $R_5$ H sein muß;

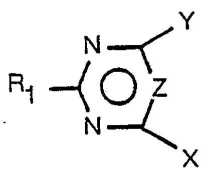 , 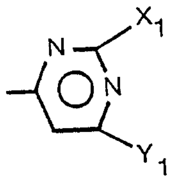 , 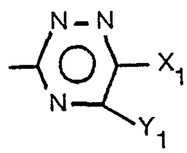 , 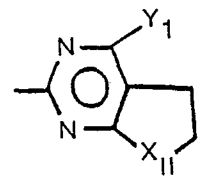 oder 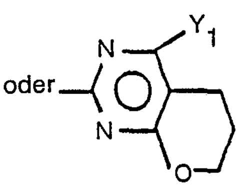 ist;

worin

Z N, CH oder C—F ist;

X=H, Cl, —$CH_3$, —$OCH_3$ oder —$OCH_2CH_3$ ist,

Y=H; Cl; $C_1$—$C_4$-Alkyl; $C_1$—$C_4$-Alkyl substituiert mit —$OCH_3$, —$OC_2H_5$, —CN, —$CO_2CH_3$, —$CO_2C_2H_5$,

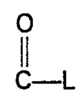

oder 1—3 Atomen von F, Cl, Br; $C_3$—$C_4$-Alkenyl; —O—$(CH_2)_{n'}$O—($C_1$—$C_3$-Alkyl), worin

n' 2 oder 3 ist; 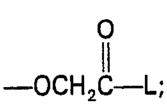; 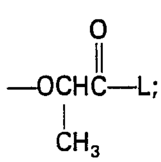; 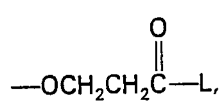,

worin L OH, —$NH_2$, 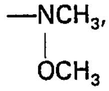,

—NH($C_1$—$C_4$-Alkyl), —N($C_1$—$C_4$-Alkyl)$_2$ oder $C_1$—$C_6$-Alkoxy ist; SCN; —$N_3$; $NR_{11}R_{12}$, worin $R_{11}$ H oder $CH_3$ ist und $R_{12}$ H, —$OCH_3$, $C_1$—$C_4$-Alkyl, $C_3$—$C_6$-Cycloalkyl, $C_3$—$C_4$-Alkenyl, $C_2$—$C_3$-Alkyl substituiert mit $OCH_3$ oder $OC_2H_5$, $C_1$—$C_2$-Alkyl substituiert mit —CN, $CO_2H$, $CO_2CH_3$ oder $CO_2C_2H_5$ ist, und $R_{11}$ und $R_{12}$ zusammengenommen bilden können: —$CH_2CH_2CH_2CH_2$— oder $CH_2CH_2OCH_2CH_2$—; —O—$R_9$, worin $R_9$ $C_1$—$C_4$-Alkyl, $C_2$—$C_4$-Alkyl substituiert mit 1—3 Atomen von F, Cl oder Br, $C_1$—$C_2$-Alkyl substituiert mit Cyano, $C_3$—$C_4$-Alkenyl, —$CH_2C{\equiv}CR_{13}$,

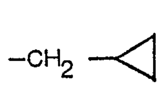 , 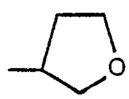 oder 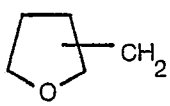 ist;

$R_{13}$ H, $CH_3$ oder $CH_2Cl$ ist; $SR_{14}$;

worin

$R_{14}$ $C_1$—$C_4$-Alkyl, Allyl, Propargyl oder $C_1$—$C_2$-Alkyl substituiert mit CN ist;

$X_1$=H, Cl, $OCH_3$, $OCH_2CH_3$ oder $CH_3$ ist,

$Y_1$=H, $OCH_3$ oder $CH_3$ ist; und

$X_{II}$=O oder $CH_2$ ist.

33. Verfahren zur Herstellung einer Verbindung nach Anspruch 32 durch Hydrolyse einer entsprechenden Ausgangsverbindung, worin $R^I$ von H unterschiedlich ist, unter basischen Bedingungen, und — falls gewünscht — Ansäuern unter Erzielung eines Produkts, worin $R^I$ H ist.

**Patentansprüche für den Vertragsstaat: AT**

1. Verfahren zur Herstellung einer Verbindung ausgewählt aus

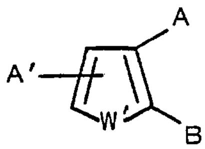

I

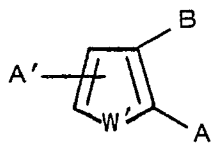

II

oder

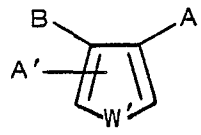

III

worin

W' O oder S ist;

A' H, Cl, Br, $C_1$—$C_4$-Alkyl, $OCH_3$, $NO_2$ oder $CF_3$ ist;

A 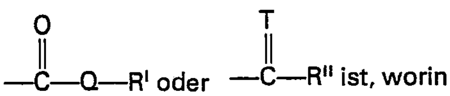 ist, worin

Q O, S oder 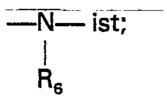 ist;

T O oder 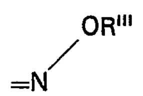

ist, worin

$R^{III}$ H, $C_1$—$C_4$-Alkyl oder $C_3$—$C_4$-Alkenyl ist;

wenn

Q O oder S ist, dann

ist $R^I$ $C_1$—$C_6$-Alkyl; $C_3$—$C_6$-Alkenyl; $C_3$—$C_6$-Alkinyl; $C_2$—$C_6$-Alkyl substituiert mit 1—3 Cl, F oder Br, oder einem von CN oder $OCH_3$; $C_3$—$C_6$-Alkenyl substituiert mit 1—3 Cl; $C_3$—$C_6$-Alkinyl substituiert mit Cl; $C_5$—$C_6$-Cycloalkyl; Cyclohexenyl; Cyclohexyl substituiert mit 1—3 $CH_3$; $C_4$—$C_7$-Cycloalkylalkyl oder

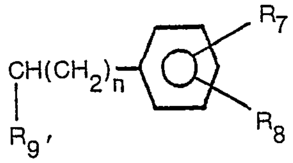

worin $R_7$ und $R_8$ unabhängig H, Cl, $CH_3$ oder $OCH_3$ sind;

n 0 oder 1 ist; und

$R_9$ H oder $CH_3$ ist;

wenn Q O ist dann kann $R^I$ sein: $CH_2CH_2OR_{15}$; $CH_2CH_2CH_2OR_{15}$; 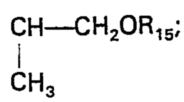;

worin $R_{15}$ $C_2H_5$, $CH(CH_3)_2$, Phenyl, $CH_2CH_2Cl$, $CH_2CCl_3$; $-(CH_2CH_2O)_{n'}R_{16}$, 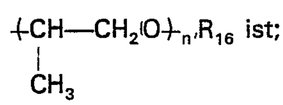 ist;

worin $R_{16}$ $CH_3$, $C_2H_5$, $CH(CH_3)_2$, Phenyl, $CH_2CH_2Cl$, $CH_2CCl_3$ ist und n′ 2 oder 3 ist;

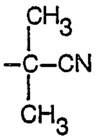 ; $OCH_3$ ; $OCH_2CH_3$ ; 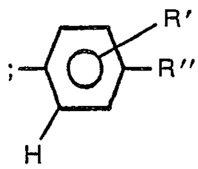 oder 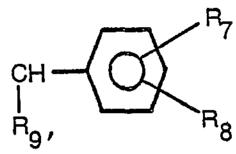 ;

worin $R_9'$ $C_1$—$C_4$-Alkyl ist;
vorausgesetzt daß $R^I$ insgesamt ≤13 Kohlenstoffatome aufweist;
wenn Q 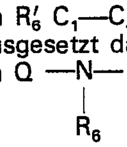 ist dann

ist $R^I$ H; $C_1$—$C_6$-Alkyl; —$CH_2CH_2OR_{10}$; —$CH_2CH_2CH_2OR_{10}$; worin $R_{10}$ $CH_3$, $CH_3CH_2$, $CH(CH_3)_2$ oder Phenyl ist; $C_3$—$C_6$-Alkenyl; $C_3$—$C_6$-Cycloalkyl; $C_5$—$C_6$-Cycloalkenyl; $C_6$-Cycloalkyl substituiert mit beliebigen von 1—2 $OCH_3$, 1—3 $CH_3$, —$CH_2CH_3$ oder $CF_3$; $C_4$—$C_7$-Cycloalkylalkyl; —$CH_2CN$; —$CH_2CH_2CN$;

$CH_2CN$; 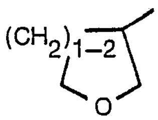 ; 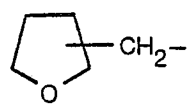 ; 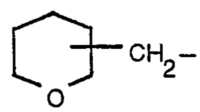 ; 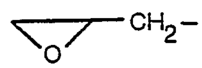 ; $CH_2OR_6'$ ;

worin

$R'$ H, $C_1$—$C_4$-Alkyl, $OCH_3$, F, Cl, Br, CN, $NO_2$ oder $CF_3$ ist;

$R''$ H, $CH_3$, Cl, F oder Br ist;

$R_7$, $R_8$ und $R_9'$ wie vorstehend definiert sind;

$R_6$ H, $C_1$—$C_3$-Alkyl; $CH_2CN$; $CH_2CH_2$—CN oder —$CH_2$—CH=$CH_2$ ist und $R_6$ und $R^I$ zusammengenommen werden können, unter Bildung von —$(CH_2)_4$—, —$(CH_2)_5$— oder —$CH_2CH_2O$—$CH_2CH_2$—; mit der Maßgabe, daß wenn $R_6$ $CH_2CH_2CN$ oder $CH_2CN$ ist, dann $R^I$ $CH_2CH_2CN$ oder $CH_2CN$ ist; und $R^I$ und $R_6$ eine Gesamtkohlenstoffanzahl von ≤13 aufweisen; und wenn $R^I$ und $OCH_3$ oder $OCH_2CH_3$ ist, dann $R_6$ $CH_3$ oder H ist;

wenn A 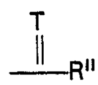

ist, dann

ist $R^{II}$ H, $C_1$—$C_6$-Alkyl; $C_3$—$C_6$-Alkenyl; Phenyl; Benzyl; Benzyl oder Phenyl substituiert mit 1—2 Cl, 1—2 $OCH_3$, 1—2 $CH_3$; $C_5$—$C_6$-Cycloalkyl; $C_4$—$C_7$-Cycloalkylalkyl;

mit der Maßgabe, daß wenn T die Bedeutung von =N—$OR^{III}$ hat, dann $R^{II}$ $C_1$—$C_6$-Alkyl oder $C_3$—$C_6$-Alkenyl sein muß;

B 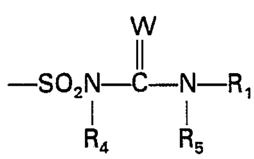 ist;

worin

$R_4$ H oder $CH_3$ ist;

W O oder S ist;

$R_5$ H, $CH_3$ oder $CH_3O$ ist; mit der weiteren Maßgabe, daß entweder $R_4$ oder $R_5$ H sein muß;

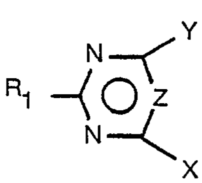 , 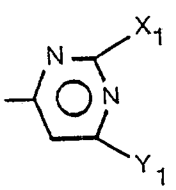 , 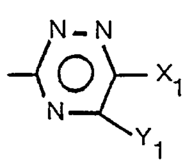 , 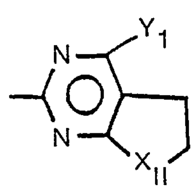 oder 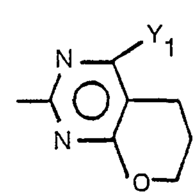 ist;

worin

Z N, CH oder C—F ist;

X=H, Cl, —$CH_3$, —$OCH_3$ oder —$OCH_2CH_3$;

Y=H; Cl; $C_1$—$C_4$-Alkyl; $C_1$—$C_4$-Alkyl substituiert mit —$OCH_3$, —$OC_2H_5$, —CN, —$CO_2CH_3$, —$CO_2C_2H_5$,

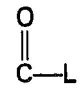

oder 1—3 Atomen von F, Cl, Br; $C_3$—$C_4$-Alkenyl; —O—$(CH_2)_{n'}$O—($C_1$—$C_3$-Alkyl), worin

n' 2 oder 3 ist; 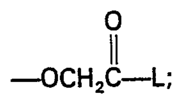; 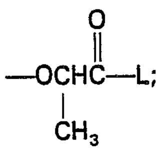; 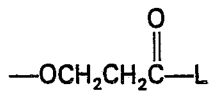

worin L OH, —$NH_2$, 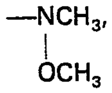

—NH($C_1$—$C_4$-Alkyl), —N—($C_1$—$C_4$-Alkyl)$_2$ oder $C_1$—$C_6$-Alkoxy ist; SCN; —$N_3$; $NR_{11}R_{12}$, worin $R_{11}$ H oder $CH_3$ ist und $R_{12}$ H, —$OCH_3$, $C_1$—$C_4$-Alkyl, $C_3$—$C_6$-Cycloalkyl, $C_3$—$C_4$-Alkenyl, $C_2$—$C_3$-Alkyl substituiert mit $OCH_3$ oder $OC_2H_5$, $C_1$—$C_2$-Alkyl substituiert mit —CN, $CO_2H$, $CO_2CH_3$ oder $CO_2C_2H_5$ ist, und $R_{11}$ und $R_{12}$ zusammengenommen werden können, unter Bildung von —$CH_2CH_2CH_2CH_2$— oder $CH_2CH_2OCH_2CH_2$—; —O—$R_9$, worin $R_9$ $C_1$—$C_4$-Alkyl, $C_2$—$C_4$-Alkyl substituiert mit 1—3 Atomen von F, Cl oder Br, $C_1$—$C_2$-Alkyl substituiert mit Cyano, $C_3$—$C_4$-Alkenyl, —$CH_2C{\equiv}CR_{13}$,

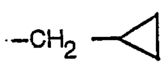 , 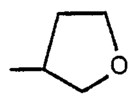 oder 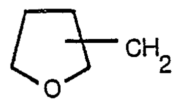 ist:

$R_{13}$ H, $CH_3$ oder $CH_2Cl$ ist; $SR_{14}$;

worin

$R_{14}$ $C_1$—$C_4$-Alkyl, Allyl, Propargyl oder $C_1$—$C_2$-Alkyl substituiert mit CN ist; mit der Maßgabe, daß wenn X und Y beide H sind, dann $R^I$ und $R^{II}$ weniger als 5 Kohlenstoffatome sind;

$X_1$=H, Cl, $OCH_3$, $OCH_2CH_3$ oder $CH_3$;

$Y_1$=H, $OCH_3$ oder $CH_3$; und

$X_{II}$=O oder $CH_2$

und weiter vorausgesetzt, daß wenn A mehr als 5 Kohlenstoffatome enthält, dann Y ≤4 Kohlenstoffatome enthalten muß,

oder eines landwirtschaftlich brauchbaren Salzes davon, durch

(a) Reaktion eines entsprechenden Isocyanats oder Isothiocyanats der Formel

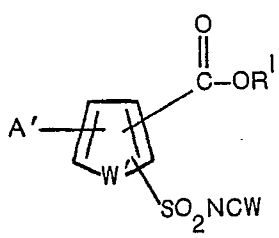

mit einem Amin der Formel $HNR_1R_5$, worin W, W', $R_1$ und $R_5$ wie vorstehend definiert sind,

A' H, Cl, Br, $C_1$—$C_4$-Alkyl, $OCH_3$, $NO_2$ oder $CF_3$ ist;

$R^I$ $C_1$—$C_6$-Alkyl; $C_3$—$C_6$-Alkenyl, $C_3$—$C_6$-Alkinyl; $C_2$—$C_6$-Alkyl substituiert mit Cl, CN oder $OCH_3$; $C_3$—$C_6$-Alkenyl substituiert mit 1—3 Cl; $C_3$—$C_6$-Alkinyl substituiert mit Cl; $C_5$—$C_6$-Cycloalkyl; Cyclohexenyl; Cyclohexyl substituiert mit 1—3 $CH_3$; $C_4$—$C_7$ Cycloalkylalkyl oder

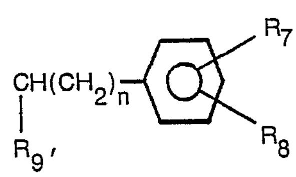

worin $R_7$ und $R_8$ unabhängig H, Cl, $CH_3$ oder $OCH_3$ sind;

$C_2H_5$, $CH(CH_3)_2$, Phenyl, $CH_2CH_2Cl$, $CH_2CCl_3$; $-(CH_2CH_2O)_{n'}-R_{16}$, $-(CH(CH_3)-CH_2O)_{n'}-R_{16}$ worin $R_{16}$ $CH_3$, $C_2H_5$, $CH(CH_3)_2$, Phenyl, $CH_2CH_2Cl$, $CH_2CCl_3$ ist, und n' 2 oder 3 ist;

$CH_2CN$; 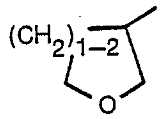 ; 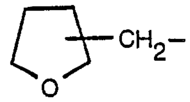 ; 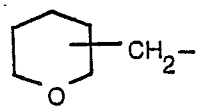 ; 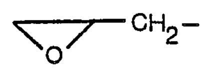 ; $CH_2OR'_6$ ;

worin $R'_6$ $C_1$—$C_4$-Alkyl ist; ist;
vorausgesetzt daß $R^I$ insgesamt $\leq 13$ Kohlenstoffatome aufweist;

(b) Reaktion eines geeigneten Sulfonamids der Formel

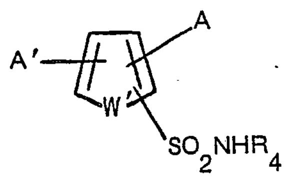

mit einem Isocyanat oder Isothiocyanat der Formel

$$R_1NCW$$

worin A, A', W, W', $R_1$ und $R_4$ wie vorstehend definiert sind, unter Herstellung einer Verbindung der Formel (I), (II) oder (III), worin $R_5$ H ist;

(c) Methylieren einer Verbindung der Formel (I), (II) oder (III), worin $R_4$ H ist und W O ist, unter Bildung einer Verbindung der Formel (I), (II) oder (III), worin $R_4$ Methyl ist;

(d) Reaktion eines Carbamylchlorids der Formel

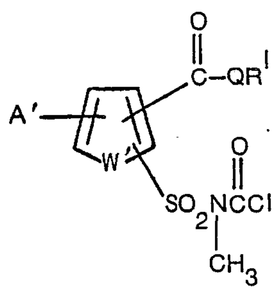

worin A', W, W', Q und $R^I$ wie vorstehend definiert sind, mit einem Amin der Formel

$$HNR_1R_5$$

worin $R_1$ und $R_5$ wie vorstehend definiert sind;

(e) Verestern einer entsprechenden Ausgangsverbindung, worin A COOH ist, unter Bildung einer Verbindung der Formel (I), (II) oder (III), worin A $COOR^I$ ist;

(f) Reaktion einer Verbindung der Formel (I), (II) oder (III), worin A $CO_2R^I$ ist, worin $R^I$ 1—4 Kohlenstoffatome aufweist mit einer Verbindung der Formel

$$(Alkyl)_2Al\,N(R_6)-R^I,$$

unter Erzielung einer Verbindung der Formel (I), (II) oder (III)

worin A $-C(=O)-N(R_6)-R^I$
ist,
worin $R^I$ und $R_6$ wie vorstehend definiert sind;

(g) Reaktion einer Verbindung der Formel (I), (II) oder (III), worin $QR^I$ die Bedeutung von $O(C_1—C_4$-Alkyl) hat mit einer Verbindung der Formel

$$(R^IS)_2Al(Alkyl),$$

unter Erzielung einer entsprechenden Verbindung der Formel (I), (II) oder (III), worin $QR^I$ die Bedeutung von $SR^I$ hat, wobei $R^I$ wie vorstehend definiert ist;

(h) Reaktion eines Esters der Formel (I), (II) oder (III), worin $R^I$ eine niedrige primäre Alkylgruppe ist mit einer Verbindung der Formel

$$(Alkyl)_2AlOR^I,$$

worin $R^I$ eine sekundäre Alkylgruppe mit der vorstehenden Definition von $R^I$ ist, unter Ersatz der niedrigen primären Alkylgruppe durch $R^I$;

(i) Reaktion einer Verbindung der Formel (I), (II) oder (III), worin A $CO_2H$ ist mit $R^{II}Li$, unter Erzielung einer Verbindung der Formel (I), (II) oder (III), worin A $COR^{II}$ ist, wobei $R^{II}$ wie vorstehend definiert ist; worauf gegebenenfalls das Keton umgesetzt wird mit einem Hydroxylamin der Formel

$$H_2NOR^{III},$$

unter Erzielung einer Verbindung der Formel (I), (II) oder (III),

worin A 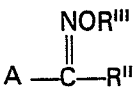

ist;

(j) Hydrolyse einer Verbindung der Formel (I), (II) oder (III),

worin Y 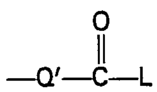

ist, worin Q' $C_1—C_4$-Alkylen, $—OCH_2—$, $—OCH_2CH_2—$, 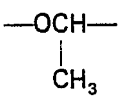, 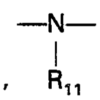 oder 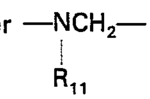 ist,

worin $R_{11}$ wie vorstehend definiert ist, und L von OH unterschiedlich ist, unter Bildung einer Verbindung der Formel (I), (II) oder (III), worin L OH ist oder eines landwirtschaftlich brauchbaren Salzes davon; oder

(k) Reaktion einer Verbindung der Formel (I), (II) oder (III), worin A $CO_2Me$ ist und A' von $NO_2$ verschieden ist mit Natrium-bis-(2-methoxyethoxy)-aluminium-hydrid oder mit Diisobutylaluminiumhydrid, unter Erzielung einer Verbindung der Formel (I), (II) oder (III), worin A —CHO ist.

2. Verfahren nach Anspruch 1, worin mindestens eines der folgenden Gültigkeit hat:

(a) A' ist nicht $OCH_3$, $NO_2$ oder $CF_3$

(b) $R^I$ ist nicht $OCH_2CH_3$ oder $C_2—C_6$-Alkyl substituiert mit F oder Br

(c) $R^I$ ist nicht

$CH_2CN$; 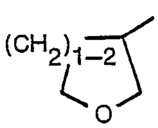 ; 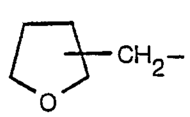 ; 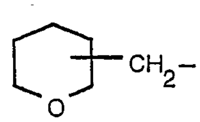 ; 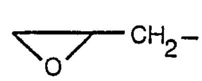 ; oder $CH_2OR_6'$ ;

(d) R' ist nicht CN, $NO_2$ oder $CF_3$

(e) wenn $R^I$ $OCH_3$ ist, ist $R_6$ nicht H

(f) $R^{II}$ ist nicht H

(g) $R_1$ ist nicht 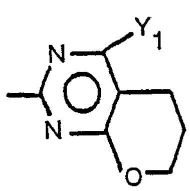

(h) $R_9$ ist nicht 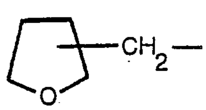

(i) Z ist nicht C—F.

3. Verfahren nach Anspruch 1 oder 2, worin

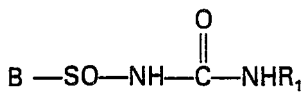

ist und T Sauerstoff ist; und worin

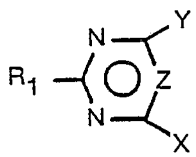 , 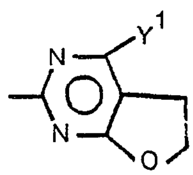 oder 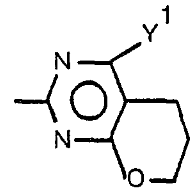 ist;

Q O oder S ist und R' $C_1$—$C_4$-Alkyl; $C_3$—$C_4$-Alkenyl; $C_3$—$C_4$-Alkinyl; $C_2$—$C_3$-Alkyl substituiert mit CN, $OCH_3$ oder 1—3 F, Cl oder Br ist; $C_3$—$C_4$-Alkenyl substituiert mit 1—3 Cl oder $C_3$—$C_4$-Alkinyl substituiert mit Cl; ist; oder

worin Q Sauerstoff ist und R' $CH_2CH_2OR_{15}$; $CH_2CH_2CH_2OR_{15}$; 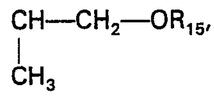

worin $R_{15}$ $CH_2CH_3$ ist; $CH_2CN$; $CH_2OR_6'$, worin $R_6'$ $CH_3$ oder $CH_3CH_2$ ist; oder 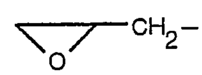 ist.

4. Verfahren zur Herstellung einer Verbindung nach Anspruch 1 oder 2, worin Q 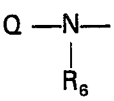 ist und

R' H, $C_1$—$C_4$-Alkyl, $CH_2CH_2OR_{10}$, $CH_2CH_2CH_2OR_{10}$, worin $R_{10}$ $CH_3$ oder $CH_3CH_2$ ist; $C_3$—$C_4$-Alkenyl; $CH_2CN$; $CH_2CH_2CN$; $OCH_3$ oder $OCH_2CH_3$ ist;

$R^6$ H, $C_1$—$C_2$-Alkyl, $CH_2CN$ oder $CH_2CH_2CN$ ist und $R_6$ und R' zusammengenommen $-(CH_2)_4$ bilden können.

5. Verfahren nach Anspruch 3, worin R'' H oder $C_1$—$C_3$-Alkyl ist.

6. Verfahren nach einem der Ansprüche 3 bis 5, worin

Z CH oder N ist;

X $CH_3$ oder $CH_3O$ ist; und

Y $C_1$—$C_2$-Alkyl; $C_1$—$C_2$-Alkyl substituiert mit $OCH_3$; $OCH_2CH_3$, CN oder 1—3 Atome von F, Cl oder Br;

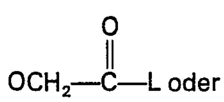 oder 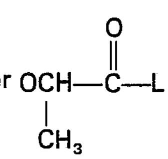 worin

L $NH_2$, OH, 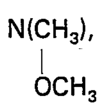

$N(CH_3)_2$, $NHCH_3$, $C_1$—$C_2$-Alkoxy ist; SCN; $N_3$; $NR_{11}R_{12}$, worin $R_{11}$ H oder $CH_3$ ist; $R_{12}$ H, $CH_3$, $CH_3CH_2$, $OCH_3$ ist; $OR_9$, worin $R_9$ die Bedeutung hat von $CH_3$, $CH_3CH_2$; $CH_2CH{=}CH_2$; $CH_2C{\equiv}CH$; oder $C_2$-Alkyl substituiert mit 1—3 F, Cl oder Br; $CH_3S$ ist.

7. Verfahren nach Anspruch 2 oder 3, worin A' H, Cl oder Br ist;

Q O oder S ist und R' $C_1$—$C_4$-Alkyl, $CH_2CH{=}CH_2$ oder $CH_2CH_2Cl$ ist.

oder worin Q O ist und R' $CH_2CH_2OCH_3$, 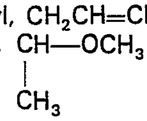

$CH_2OCH_3$ oder $CH_2OCH_2CH_3$ ist;

oder worin Q 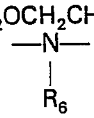 ist

und R' H; $C_1$—$C_3$-Alkyl, $OCH_3$ oder $OCH_2CH_3$ ist und $R_6$ H oder $C_1$—$C_2$-Alkyl ist.

8. Verfahren nach Anspruch 7, worin A′ H ist; Y $CH_3$, $OCH_3$, $OCH_2CH_3$, $OCH_2CF_3$, $OCH_2CH{=}CH_2$ oder $OCH_2C{\equiv}CH$ ist.

9. Verfahren nach Anspruch 6, worin A 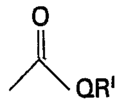

ist und Q Sauerstoff oder Schwefel ist und R' $CH_3$ oder $CH_2CH_3$ ist; Q 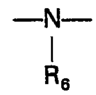 ist

und R' H, $CH_3$ oder $OCH_3$ ist und $R_6$ $CH_3$ ist;

$R_1$ is 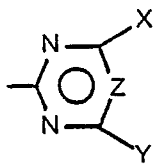 ist

Y $CH_3$ oder $OCH_3$ ist und W′ Schwefel ist.

10. Verfahren nach Anspruch 1, bei dem das Produkt eine Verbindung oder ein landwirtschaftlich brauchbares Salz davon ist, ausgewählt aus

Methyl 3-[[(4-methoxy-6-methylpyrimidin-2-yl)aminocarbonyl]-aminosulfonyl]-2-thiophencarboxylat

Methyl 3-[[(4,6-dimethoxy-1,3,5-triazin-2-yl)aminocarbonyl]-aminosulfonyl]-2-thiophencarboxylat

Methyl 3-[[(4,6-dimethyl-1,3,5-triazin-2-yl)aminocarbonyl]-aminosulfonyl]-2-thiophencarboxylat

Methyl-3-[[(4-methoxy-6-methyl-1,3,5-triazin-2-yl)aminocarbonyl]-aminosulfonyl]-2-thiophencarboxylat

11. Verfahren nach Anspruch 1, worin das Produkt eine Verbindung oder ein landwirtschaftlich brauchbares Salz davon ist, ausgewalt aus

Methyl 2-[[(4,6-dimethoxypyrimidin-2-yl)aminocarbonyl]-aminosulfonyl] -3-thiophencarboxylat

Methyl 2-[[(4,6-dimethylpyrimidin-2-yl)aminocarbonyl]-aminosulfonyl]·-3-thiophencarboxylat

Methyl 2-[[(4-methoxy-6-methylpyrimidin-2-yl)aminocarbonyl]-aminosulfonyl]-3-thiophencarboxylat

Methyl 2-[[(4,6-dimethoxy-1,3,5-triazin-2-yl)aminocarbonyl]-aminosulfonyl]-3-thiophencarboxylat oder

Methyl 2-[[(4-methoxy-6-methyl-1,3,5-triazin-2-yl)aminocarbonyl]-aminosulfonyl]-3-thiophencarboxylat.

12. Verfahren nach Anspruch 1, bei dem das Produkt eine Verbindung oder ein landwirtschaftlich brauchbares Salz davon ist, ausgewählt aus

Methyl 4-[[(4,6-dimethoxypyrimidin-2-yl)aminocarbonyl]-aminosulfonyl] -3-thiophencarboxylat

Methyl 4-[[(4,6-dimethylpyrimidin-2-yl)aminocarbonyl]-aminosulfonyl] -3-thiophencarboxylat

Methyl 4-[[(4-methoxy-6-methylpyrimidin-2-yl)aminocarbonyl]-aminosulfonyl]-3-thiophencarboxylat

Methyl 4-[[(4,6-dimethoxy-1,3,5-triazin-2-yl)aminocarbonyl]-aminosulfonyl]-3-thiophencarboxylat oder

Methyl 4-[[(4-methoxy-6-methyl-1,3,5-triazin-2-yl)aminocarbonyl]-aminosulfonyl]-3-thiophencarboxylat.

13. Verfahren nach Anspruch 1, bei dem das Produkt N - [(4 - Methoxy - 6 - methylpyrimidin - 2 - yl) - aminocarbonyl] - 2 - (1 - pyrrolidinylcarbonyl) - 3 - thiophensulfonamid oder ein landwirtschaftlich brauchbares Salz davon ist.

14. Verfahren nach Anspruch 1, bei dem das Produkt 1 - Methylethyl - 3 - [[(4,6 - dimethoxypyrimidin - 2 - yl) - aminocarbonyl] - aminosulfonyl] - 2 - thiophencarboxylat oder ein landwirtschaftlich brauchbares Salz davon ist.

15. Verfahren zur Bekämpfung des Wachstums unerwünschter Vegetation durch Auftrag auf den zu schützenden Ort von einer wirksamen Menge einer herbiziden Verbindung, dadurch gekennzeichnet, daß die herbizide Verbindung eine Verbindung der Formel (I), (II) oder (III), wie in einem der Ansprüche 1 bis 14 definiert, umfasst.

16. Verfahren zur Regulierung des Wachstums von Pflanzen durch Auftrag auf den Ort dieser Pflanzen von einer wirksamen jedoch im wesentlichen nicht-phytotoxischen Menge eines das Pflanzenwachstum regulierenden Mittels, dadurch gekennzeichnet, daß das das Pflanzenwachstum regulierende Mittel eine Verbindung der Formel (I), (II) oder (III), wie in einem der Ansprüche 1 bis 14 definiert, umfasst.

17. Verfahren zur Herstellung einer Verbindung ausgewählt aus

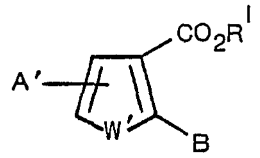

I′

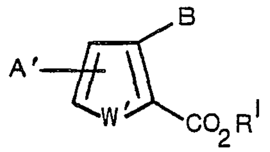

II′

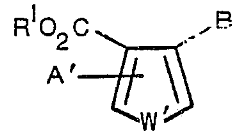

III′

worin $R^I$ H oder M ist;
M ein Kation eines Alkalimetalls oder eines tertiären Amins mit bis zu 12 Kohlenstoffatomen ist;
W′ O oder S ist;
A′, H, Cl, Br, $C_1$—$C_4$-Alkyl, $OCH_3$, $NO_2$ oder $CF_3$ ist;

$$-SO_2\underset{R_4}{N}-\overset{W}{\overset{\|}{C}}-\underset{R_5}{N}-R_1 \text{ ist;}$$

worin $R_4$ H oder $CH_3$ ist; W O oder S ist;
$R_5$ H, $CH_3$ oder $CH_3O$ ist; mit der Maßgabe, daß entweder $R_4$ oder $R_5$ H sein muß;

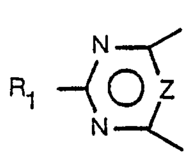 , 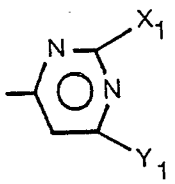 , 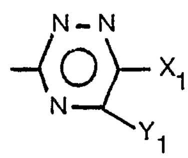 , 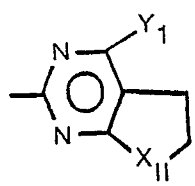 oder 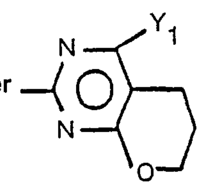 ist;

worin
Z N, CH oder C—F ist;
X=H, Cl, —$CH_3$, —$OCH_3$ oder —$OCH_2CH_3$;
Y=H; Cl; $C_1$—$C_4$-Alkyl; $C_1$—$C_4$-Alkyl substituiert mit —$OCH_3$, —$OC_2H_5$, —CN, —$CO_2CH_3$, —$CO_2C_2H_5$,

$$\overset{O}{\overset{\|}{C}}-L$$

oder 1—3 Atomen von F, Cl, Br; $C_3$—$C_4$-Alkenyl; —O—$(CH_2)_{n'}$—O—($C_1$—$C_3$-Alkyl), worin

n′ 2 oder 3 ist; $-OCH_2\overset{O}{\overset{\|}{C}}-L$; $-O\underset{CH_3}{CH}\overset{O}{\overset{\|}{C}}-L$; $-OCH_2CH_2\overset{O}{\overset{\|}{C}}-L$

worin L OH, —$NH_2$, $-\underset{OCH_3}{N}CH_3$,

—NH($C_1$—$C_4$-Alkyl), —N($C_1$—$C_4$-Alkyl)$_2$ oder $C_1$—$C_6$-Alkoxy ist; SCN; —$N_3$; $NR_{11}R_{12}$, worin $R_{11}$ H oder $CH_3$ ist und $R_{12}$ H, —$OCH_3$, $C_1$—$C_4$-Alkyl, $C_3$—$C_6$-Cycloalkyl, $C_3$—$C_4$-Alkenyl, $C_2$—$C_3$-Alkyl substituiert mit $OCH_3$ oder $OC_2H_5$, $C_1$—$C_2$-Alkyl substituiert mit —CN, $CO_2H$, $CO_2CH_3$ oder $CO_2C_2H_5$ ist, und $R_{11}$ und $R_{12}$ zusammengenommen —$CH_2CH_2CH_2CH_2$— oder $CH_2CH_2OCH_2CH_2$ bilden können; O—$R_9$, worin $R_9$ $C_1$—$C_4$-Alkyl, $C_2$—$C_4$-Alkyl substituiert mit 1—3 Atomen von F, Cl oder Br, $C_1$—$C_2$-Alkyl substituiert mit Cyano, $C_3$—$C_4$-Alkenyl, —$CH_2C{\equiv}CR_{13}$,

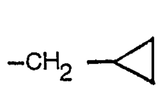 , 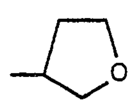 oder 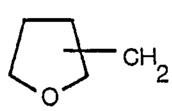 ist:

$R_{13}$ H, $CH_3$ oder $CH_2Cl$ ist; $SR_{14}$;
worin

$R_{14}$ $C_1$—$C_4$-Alkyl, Allyl, Propargyl oder $C_1$—$C_2$-Alkyl substituiert mit CN ist;
$X_1$=H, Cl, $OCH_3$, $OCH_2CH_3$ oder $CH_3$;
$Y_1$=H, $OCH_3$ oder $CH_3$; und
$X_{II}$=O oder $CH_2$;
durch Hydrolyse einer entsprechenden Ausgangsverbindung, worin $R^I$ von H unterschiedlich ist, unter basischen Bedingungen, und gegebenenfalls Ansäuern zur Erzielung eines Produkts worin $R^I$ H ist.

**Revendications pour les Etats contractants: BE CH DE FR IT LI LU NL SE**

1. Composé choisi parmi:

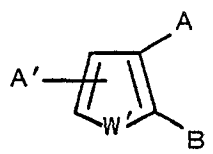
I

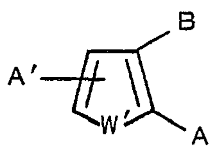
II

ou

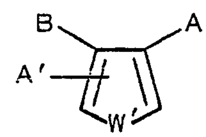
III

W' est O ou S;
A' est H, Cl, Br, un radical alcoyle en $C_1$—$C_4$, $OCH_3$, $NO_2$ ou $CF_3$.

A est 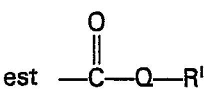 ou 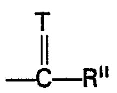 où

Q est O, S ou 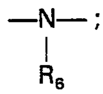 ;

T est 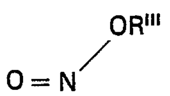 où

$R^{III}$ est H, un radical alcoyle en $C_1$—$C_4$ ou alcényle en $C_3$—$C_4$;
quand Q est O ou S, alors

$R^I$ est un radical alcoyle en $C_1$—$C_6$; alcényle en $C_3$—$C_6$; alcynyle en $C_3$—$C_6$; alcoyle en $C_2$—$C_6$ substitué par 1 à 3 atomes de Cl, F ou Br ou un radical CN ou $OCH_3$; alcényle en $C_3$—$C_6$ substitué par 1 à 3 atomes de Cl, alcynyle en $C_3$—$C_6$ substitué par Cl; cycloalcoyle en $C_5$—$C_6$; cyclohexényle; cyclohexyle substitué par 1 à 3 radicaux $CH_3$; cycloalcoylalcoyle en $C_4$—$C_7$ ou

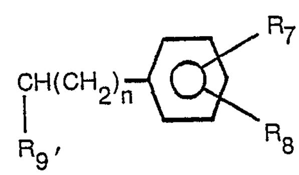

où $R_7$ et $R_8$ sont indépendamment, H, Cl, $CH_3$ ou $OCH_3$;
n est 0 ou 1; et
$R_9$' est H ou $OCH_3$;
quand Q est O, alors $R^I$ peut être $CH_2CH_2OR_{15}$; $CH_2CH_2CH_2OR_{15}$; 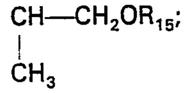

où $R_{15}$ est $C_2H_5$, $CH(CH_3)_2$, un radical phényle, $CH_2CH_2Cl$, $CCH_2CCl_3$; $-(CH_2CH_2O)-_{n'}R_{16}$, 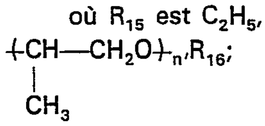

où $R_{16}$ est $CH_3$, $C_2H_5$, $CH(CH_3)_2$, un radical phényle, $CH_2CH_2Cl$, $CH_2CCl_3$, et n′ est 2 ou 3;

$CH_2CN$; 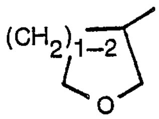 ; 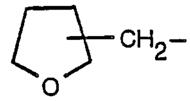 ; 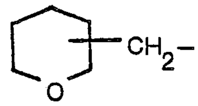 ; 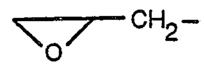 ; $CH_2OR'_6$ ;

où $R'_6$ est un radical alcoyle en $C_1$—$C_4$

avec la condition que $R^I$ a un total de ⩽13 atomes de carbone;

quand Q est 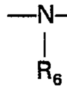, alors

$R^I$ est H; un radical alcoyle en $C_1$—$C_6$; —$CH_2CH_2OR_{10}$; —$CH_2CH_2CH_2OR_{10}$; où $R_{10}$ est un radical $CH_3$, $CH_3CH_2$, $CH(CH_3)_2$ ou phényle; alcényle en $C_3$—$C_6$; cycloalcoyle en $C_3$—$C_6$; cycloalcényle en $C_5$—$C_6$; cycloalcoyle en $C_6$ substitué par 1—2 radicaux $OCH_3$, 1—3 radicaux $CH_3$, —$CH_2CH_3$ ou $CF_3$; cycloalcoylalcoyle en $C_4$—$C_7$; —$CH_2CN$; —$CH_2CH_2CN$;

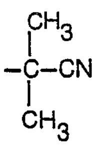 ; $OCH_3$ ; $OCH_2CH_3$ ; 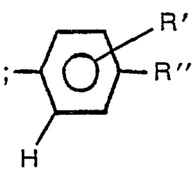 ou 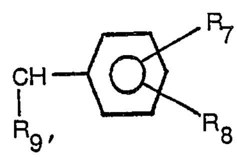 ;

où

R′ est H, un radical alcoyle en $C_1$—$C_4$, $OCH_3$, F, Cl, Br, CN, $NO_2$ ou $CF_3$;

R″ est H, $CH_3$, Cl, F ou Br;

$R_7$, $R_8$ et $R_9'$ sont tels que définis précédemment;

$R_6$ est H, un radical alcoyle en $C_1$—$C_3$; $CH_2CN$; $CH_2CH_2$—CN ou —$CH_2$—CH=$CH_2$ et $R_6$ et $R^I$ peuvent être pris ensemble pour former —$(CH_2)_4$, —$(CH_2)_5$— ou —$CH_2CH_2O$—$CH_2CH_2$—;

avec la condition que, quand $R_6$ est $CH_2CH_2CN$ ou $CH_2CN$, alors $R^I$ est $CH_2CH_2CN$ ou $CH_2CN$; et $R^I$ et $R_6$ ont un nombre total d'atomes de carbone ⩽13; et quand $R^I$ est $OCH_3$ ou $OCH_2CH_3$, alors $R_6$ est $CH_3$ ou H;

Quand A est 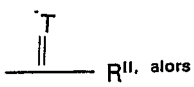 alors

$R^{II}$ est H, un radical alcoyle en $C_1$—$C_6$, alcényle en $C_3$—$C_6$; phényle; benzyle; benzyle ou phényle substitué par 1—2 Cl, 1—2 $OCH_3$, 1—2 $CH_3$; cycloalcoyle en $C_5$—$C_6$; cycloalcoylalcoyle en $C_4$—$C_7$ avec la condition que, quand T est =N—$OR^{III}$, alors $R^{II}$ soit être un radical alcoyle en $C_1$—$C_6$ ou alcényle en $C_3$—$C_6$;

B est 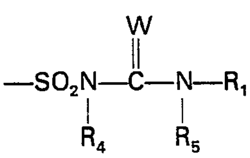

où $R_4$ est H ou $CH_3$; W est O ou S; $R_5$ est H, $CH_3$ ou $CH_3O$; avec la condition supplémentaire que $R_4$ ou $R_5$ doit être H;

$R_1$ est 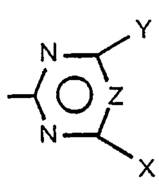 , 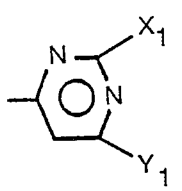 , 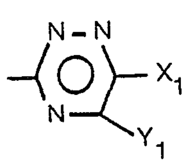 , 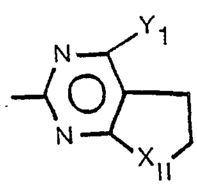 ou 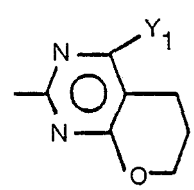 ;

où Z est N, CH ou C—F;

X = H, Cl, —$CH_3$, —$OCH_3$ ou —$OCH_2CH_3$;

Y = H; Cl; un radical alcoyle en $C_1$—$C_4$; alcoyle en $C_1$—$C_4$ substitué par —$OCH_3$, —$OC_2H_5$, —CN, —$CO_2CH_3$, —$CO_2C_2H_5$,

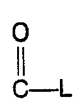

ou par 1—3 atomes de F, Cl, Br; alcényle en $C_3$—$C_4$; —O—$(CH_2)_{n'}$ O-(alcoyle en $C_1$—$C_3$) où n′ est 2 ou 3;

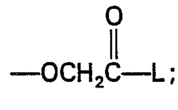 ; 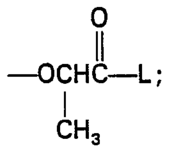 ; 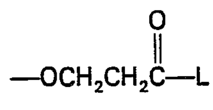

où L est OH, —$NH_2$, 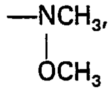

—NH(alcoyle en $C_1$—$C_4$), —N(alcoyle en $C_1$—$C_4$)$_2$ ou alcoxy en $C_1$—$C_6$; SCN; —$N_3$; $NR_{11}R_{12}$ où $R_{11}$ est H ou $CH_3$ et $R_{12}$ est H, —$OCH_3$, alcoyle en $C_1$—$C_4$, cycloalcoyle en $C_3$—$C_6$, alcényle en $C_3$—$C_4$, alcoyle en $C_2$—$C_3$ substitué par $OCH_3$ ou $OC_2H_5$, alcoyle en $C_1$—$C_2$ substitué par —CN, $CO_2H$, $CO_2CH_3$ ou $CO_2C_2H_5$, et $R_{11}$ et $R_{12}$ peuvent être pris ensemble pour former —$CH_2CH_2CH_2CH_2$— ou $CH_2CH_2OCH_2CH_2$—; —O—$R_9$ où $R_9$ est un radical alcoyle en $C_1$—$C_4$, alcoyle en $C_2$—$C_4$ substitué par 1—3 atomes de F, Cl, ou Br, alcoyle en $C_1$—$C_2$ substitué par un radical cyano, alcényle en $C_3$—$C_4$, —$CH_2C{\equiv}CR_{13}$,

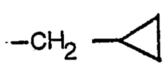 , 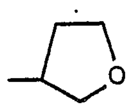 ou 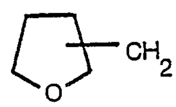 ;

$R_{13}$ est H, $CH_3$ ou $CH_2Cl$;
$SR_{14}$;
où
$R_{14}$ est un radical alcoyle en $C_1$—$C_4$, allyle, propargyle ou alcoyle en $C_1$—$C_2$ substitué par CN;
avec la condition que, quand X et Y sont tous deux H, alors $R^I$ et $R^{II}$ ont moins de 5 atomes de carbone;
$X_1$ = H, Cl, $OCH_3$, $OCH_2CH_3$ ou $CH_3$;
$Y_1$ = H, $OCH_3$ ou $CH_3$; et
$X_{II}$ = O ou $CH_2$
et avec la condition en outre que, quand A contient plus de 5 atomes de carbone, alors Y doit contenir ⩽ 4 atomes de carbone et leurs sels acceptables en agriculture.

2. Un composé selon la revendication 1, dans lequel B est 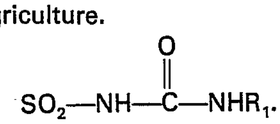.

3. Un composé selon la revendication 2, dans lequel T est de l'oxygène.

4. Un composé selon la revendication 2 ou 3, dans lequel

$R_1$ est 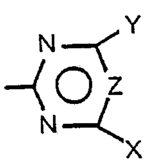 , 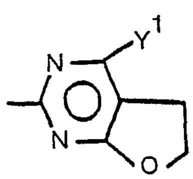 ou 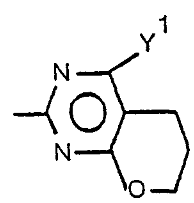

5. Un composé selon la revendication 4, dans lequel Q est O ou S et $R^I$ est un radical alcoyle en $C_1$—$C_4$; alcényle en $C_3$—$C_4$; alcynyle en $C_3$—$C_4$; alcoyle en $C_2$—$C_3$ substitué par CN, $OCH_3$ ou 1—3 F, Cl ou Br; alcényle en $C_3$—$C_4$ substitué par 1—3 Cl ou alcynyle en $C_3$—$C_4$ substitué par Cl.

6. Un composé selon la revendication 4, dans lequel Q est de l'oxygène et $R^I$ est $CH_2CH_2OR_{15}$; 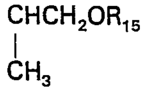

où $R_{15}$ est $CH_2CH_3$; $CH_2CN$; $CH_2OR_6'$ où $R_6'$ est $CH_3$ ou $CH_3CH_2$; et 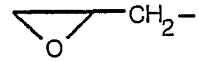 .

7. Un composé selon la revendication 4, dans lequel Q est 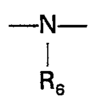

et $R^I$ est H, un radical alcoyle en $C_1$—$C_4$, $CH_2CH_2OR_{10}$, $CH_2CH_2CH_2OR_{10}$ où $R_{10}$ est $CH_3$ ou $CH_3CH_2$; alcényle en $C_3$—$C_4$; $CH_2CN$; $CH_2CH_2CN$; $OCH_3$; ou $OCH_2CH_3$;

$R^6$ est H, un radical alcoyle en $C_1$—$C_2$, $CH_2CN$ ou $CH_2CH_2CN$ et $R^6$ et $R^I$ peuvent être pris ensemble pour former $-(CH_2)_4$.

8. Un composé selon la revendication 4, dans lequel $R^{II}$ est H ou un radical alcoyle en $C_1$—$C_3$.

9. Un composé selon l'une quelconque des revendications 5 à 8, dans lequel

Z est CH ou N;

X est $CH_3$ ou $CH_3O$; et

Y est un radical alcoyle en $C_1$—$C_2$; alcoyle en $C_1$—$C_2$ substitué par $OCH_3$, $OCH_2CH_3$, CN ou par 1—3 atomes de F, Cl ou Br;

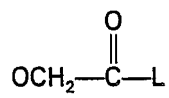 ou 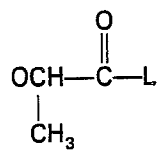

ou L est $NH_2$, OH, 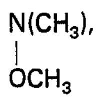

$N(CH_3)_2$, $NHCH_3$, un radical alcoxy en $C_1$—$C_2$; SCN, $N_3$; $NR_{11}R_{12}$ où $R_{11}$ est H ou $CH_3$ et $R_{12}$ est H, $CH_3$, $CH_3CH_2$, $OCH_3$; $OR_9$ où $R_9$ est $CH_3$, $CH_3CH_2$; $CH_2CH{=}CH_2$; $CH_2C{\equiv}CH$; ou un radical alcoyle substitué par 1—3 F, Cl ou Br; $CH_3S$.

10. Un composé selon la revendication 4, dans lequel A' est H, Cl ou Br.

11. Un composé selon la revendication 10, dans lequel Q est O ou S et $R^I$ est un radical alcoyle en $C_1$—$C_4$, $CH_2CH{=}CH_2$ ou $CH_2CH_2Cl$.

12. Un composé selon la revendication 10, dans lequel Q est O et $R^I$ est $CH_2CH_2OCH_3$, 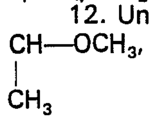 $CH_2OCH_3$ ou $CH_2OCH_2CH_3$.

13. Un composé selon la revendication 10, dans lequel Q est 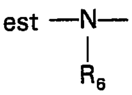

et $R^I$ est H; un radical alcoyle en $C_1$—$C_3$, $OCH_3$ ou $OCH_2CH_3$ et $R_6$ est H ou un radical alcoyle en $C_1$—$C_2$.

14. Un composé selon la revendication 10, dans lequel $R^{II}$ est H ou $CH_3$.

15. Un composé selon l'une quelconque des revendications 11 à 14, dans lequel A' est H; Y est $CH_3$, $OCH_3$, $OCH_2CH_3$, $OCH_2CF_3$, $OCH_2CH{=}CH_2$ ou $OCH_2C{\equiv}CH$.

16. Un composé selon la revendication 9, dans lequel A est 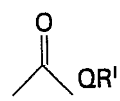

et Q est de l'oxygène ou du soufre et $R^I$ est $CH_3$ ou $CH_2CH_3$; Q est 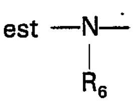

et $R^I$ est H, $CH_3$ ou $OCH_3$ et $R_6$ est $CH_3$.

$R_1$ est 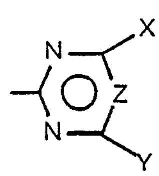

et Y est $CH_3$ ou $OCH_3$.

17. Un composé selon la revendication 16, dans lequel la formule I est utilisée.

18. Un composé selon la revendication 16, dans lequel la formule II est utilisée.

19. Un composé selon la revendication 16, dans lequel la formule III est utilisée.

20. Un composé selon l'une quelconque des revendications 1 à 19, dans lequel W' est du soufre.

21. Un composé selon l'une quelconque des revendications 1 à 19, dans lequel W' est de l'oxygène.

22. Un composé selon la revendication 1 ou un sel acceptable en agriculture d'un tel composé, choisi parmi les suivants:

3-[[(4-méthoxy-6-méthylpyrimidin-2-yl)aminocarbonyl]-aminosulfonyl]-2-thiophènecarboxylate de méthyle

3-[[(4,6-diméthoxy-1,3,5-triazin-2-yl)aminocarbonyl]-aminosulfonyl]-2-thiophènecarboxylate de méthyle

3-[[(4-méthoxy-6-méthyl-1,3,5-triazin-2-yl)aminocarbonyl]-aminosulfonyl]-2-thiophènecarboxylate de méthyle

3-[[(4,6-diméthoxypyrimidin-2-yl)aminocarbonyl]-aminosulfonyl]-2-thiophènecarboxylate de méthyle

3-[[(4,6-diméthylpyrimidin-2-yl)aminocarbonyl]-aminosulfonyl]-2-thiophènecarboxylate de méthyle.

23. Un composé selon la revendication 1 ou un sel acceptable en agriculture d'un tel composé, choisi parmi les suivants:

2-[[(4,6-diméthoxypyrimidin-2-yl)aminocarbonyl]-aminosulfonyl]-3-thiophènecarboxylate de méthyle

2-[[(4,6-diméthylpyrimidin-2-yl)aminocarbonyl]-aminosulfonyl]-3-thiophènecarboxylate de méthyle

2-[[(4-méthoxy-6-méthylpyrimidin-2-yl)aminocarbonyl]-aminosulfonyl]-3-thiophènecarboxylate de méthyle

2-[[(4,6-diméthoxy-1,3,5-triazin-2-yl)aminocarbonyl]-aminosulfonyl]-3-thiophènecarboxylate de méthyle

2-[[(4-méthoxy-6-méthyl-1,3,5-triazin-2-yl)aminocarbonyl]-aminosulfonyl]-3-thiophènecarboxylate de méthyle.

24. Un composé selon la revendication 1 ou un sel acceptable en agriculture d'un tel composé, choisi parmi les suivants:

4-[[(4,6-diméthoxypyrimidin-2-yl)aminocarbonyl]-aminosulfonyl]-3-thiophènecarboxylate de méthyle

4-[[(4,6-diméthylpyrimidin-2-yl)aminocarbonyl]-aminosulfonyl]-3-thiophènecarboxylate de méthyle

4-[[(4-méthoxy-6-méthylpyrimidin-2-yl)aminocarbonyl]-aminosulfonyl]-3-thiophènecarboxylate de méthyle

4-[[(4,6-diméthoxy-1,3,5-triazin-2-yl)aminocarbonyl]-aminosulfonyl]-3-thiophènecarboxylate de méthyle

4-[[(4-méthoxy-6-méthyl-1,3,5-triazin-2-yl)aminocarbonyl]-aminosulfonyl]-3-thiophènecarboxylate de méthyle.

25. Un composé selon la revendication 1, qui est le:

N-[(4-méthoxy-6-méthylpyrimidin-2-yl)aminocarbonyl]-2-(1-pyrrolidinylcarbonyl)-3-thiophènesulfonamide.

ou un sel acceptable en agriculture de ce composé.

26. Un composé selon la revendication 1, qui est le:

3-[[(4,6-diméthoxypyrimidin-2-yl)aminocarbonyl]-aminosulfonyl]-2-thiophènecarboxylate de 1-méthyléthyle.

ou un sel acceptable en agriculture de ce composé.

27. Un composé selon la revendication 1, dans lequel au moins une condition suivante est remplie:

(a) A' n'est pas $OCH_3$, $NO_2$ ou $CF_3$

(b) $R^I$ n'est pas $OCH_2CH_3$ ou un radical alcoyle en $C_2$—$C_6$ substitué par F ou Br

(c) $R^I$ n'est pas

$CH_2CN$ ; 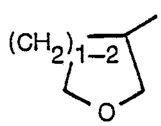 ; 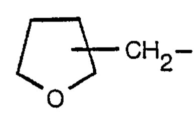 ; 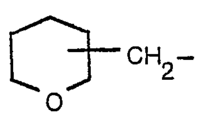 ; 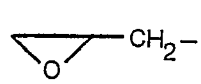 ou $CH_2OR'_6$ ;

(d) R' n'est pas CN, $NO_2$ ou $CF_3$

(e) quand $R^I$ est $OCH_3$, $R_6$ n'est pas H

(f) $R^{II}$ n'est pas H

(g) $R_1$ n'est pas 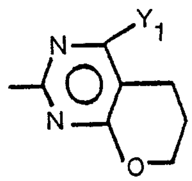

(h) $R_9$ n'est pas 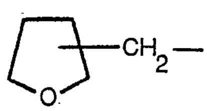

(i) Z n'est pas C—F.

28. Une composition utile pour lutter contre la croissance de végétation indésirable comprenant une quantité efficace d'un composé herbicide et au moins un agent tensio-actif et/ou un diluant solide ou liquide, caractérisée en ce que le composé herbicide comprend un composé selon l'une quelconque des revendications 1 à 27.

29. Un procédé pour lutter contre la croissance de végétation indésirable par application sur le lieu de cette végétation d'une quantité efficace d'un composé herbicide, caractérisé en ce que ce composé herbicide comprend un composé selon l'une quelconque des revendications 1 à 27.

30. Un procédé pour régler la croissance de plantes par application au site de ces plantes d'uné quantité efficace, mais substantiellement non-phytotoxique, d'un agent de réglage de la croissance de plantes, caractérisé en ce que l'agent de réglage de la croissance de plantes comprend un composé selon l'une quelconque des revendications 1 à 27.

31. Un procédé de préparation d'un composé de la revendication 1, qui comprend la réaction d'un isocyanate ou isothiocyanate approprié de formule

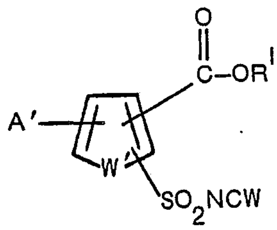

avec une amine de formule $HNR_1R_5$, où A', W, W', $R^I$, $R_1$ et $R_5$ sont tels que définis dans la revendication 1.

32. Un composé choisi parmi

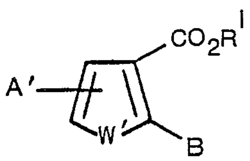
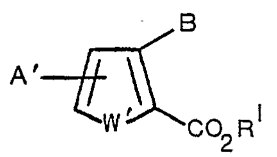
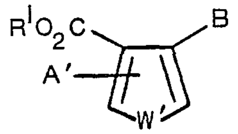

I II III

où

$R^I$ est H ou M;

M est un cation d'un métal alcalin ou d'une amine tertiaire ayant jusqu'à 12 atomes de carbone;

W' est O ou S;

A' est H, Cl, Br, un radical alcoyle en $C_1$—$C_4$, $OCH_3$, $NO_2$ ou $CF_3$;

B est 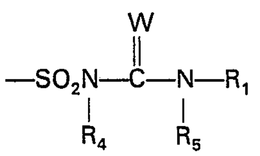

où $R_4$ est H ou $CH_3$; W est O ou S; $R_5$ est H, $CH_3$ ou $CH_3O$; avec la condition que $R_4$ ou $R_5$ doit être H;

$R_1$ est 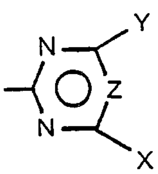 , 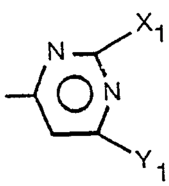 , 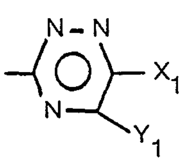 , 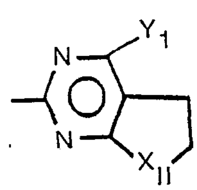 ou 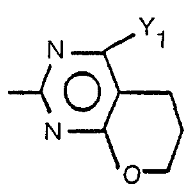 ;

où

Z est N, CH ou C—F;

X = H, Cl, —$CH_3$, —$OCH_3$ ou —$OCH_2CH_3$;

Y = H; Cl; un radical alcoyle en $C_1$—$C_4$; alcoyle en $C_1$—$C_4$ substitué par —$OCH_3$, —$OC_2H_5$, —CN, —$CO_2CH_3$, —$CO_2C_2H_5$,

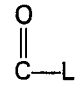

ou par 1—3 atomes de F, Cl, Br; alcényle en $C_3$—$C_4$; —O—$(CH_2)_{n'}$ O-(alcoyle en $C_1$—$C_3$) où n' est 2 ou 3;

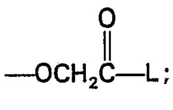 ; 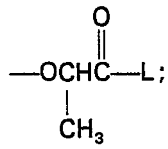 ; 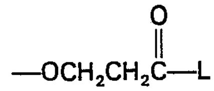

où L est OH, —$NH_2$, 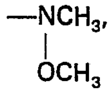

—NH(alcoyle en $C_1$—$C_4$), —N(alcoyle en $C_1$—$C_4$)$_2$ ou alcoxy en $C_1$—$C_6$; SCN; —$N_3$; $NR_{11}R_{12}$ où $R_{11}$ est H ou $CH_3$ et $R_{12}$ est H, —$OCH_3$, un radical alcoyle en $C_1$—$C_4$, cycloalcoyle en $C_3$—$C_6$, alcényle en $C_3$—$C_4$, alcoyle en $C_2$—$C_3$ substitué par $OCH_3$ ou $OC_2H_5$, alcoyle en $C_1$—$C_2$ substitué par —CN, $CO_2H$, $CO_2CH_3$ ou $CO_2C_2H_5$, et $R_{11}$ et $R_{12}$ peuvent être pris ensemble pour former —$CH_2CH_2CH_2CH_2$— ou $CH_2CH_2OCH_2CH_2$—; —O—$R_9$ où $R_9$ est un radical alcoyle en $C_1$—$C_4$, alcoyle en $C_2$—$C_4$ substitué par 1—3 atomes de F, Cl, ou Br, alcoyle en $C_1$—$C_2$ substitué par un radical cyano, alcényle en $C_3$—$C_4$, —$CH_2C{\equiv}CR_{13}$,

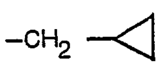 , 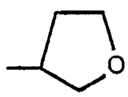 ou 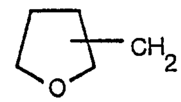 :

$R_{13}$ est H, $CH_3$ ou $CH_2Cl$;

$SR_{14}$, où $R_{14}$ est un radical alcoyle en $C_1$—$C_4$, allyle, propargyle ou alcoyle en $C_1$—$C_2$ substitué par CN;

$X_1$ = H, Cl, $OCH_3$, $OCH_2CH_3$ ou $CH_3$;

$Y_1$ = H, $OCH_3$ ou $CH_3$; et

$X_{II}$ = O ou $CH_2$.

33. Un procédé de préparation d'un composé de la revendication 32, qui comprend l'hydrolyse d'un composé de départ correspondant dans lequel $R^I$ est différent de H, dans des conditions basiques et, si on le désire, une acidification pour obtenir un produit dans lequel $R^I$ est H.

**Revendications pour le Etat contractant: AT**

1. Un procédé de préparation d'un composé choisi parmi:

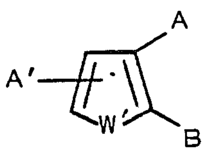
I

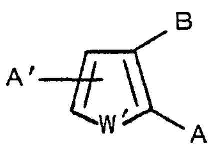
II

ou

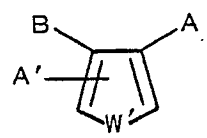
III

où

W' est O ou S;

A' est H, Cl, Br, un radical alcoyle en $C_1$—$C_4$, $OCH_3$, $NO_2$ ou $CF_3$.

A est 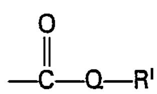 ou 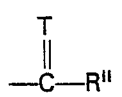 où

Q est O, S ou 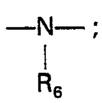 ;

T est O = 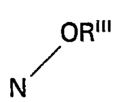

$R^{III}$ est H, un radical alcoyle en $C_1$—$C_4$ ou alcényle en $C_3$—$C_4$;

quand Q est O ou S, alors

$R^I$ est un radical alcoyle en $C_1$—$C_6$; alcényle en $C_3$—$C_6$; alcynyle en $C_3$—$C_6$; alcoyle en $C_2$—$C_6$

substitué par 1 à 3 atomes de Cl, F ou Br ou un radical CN ou $OCH_3$; alcényle en $C_3$—$C_6$ substitué par 1 à 3 atomes de Cl, alcynyle en $C_3$—$C_6$ substitué par Cl; cycloalcoyle en $C_5$—$C_6$; cyclohexényle; cyclohexyle substitué par 1 à 3 radicaux $CH_3$; cycloalcoylalcoyle en $C_4$—$C_7$ ou

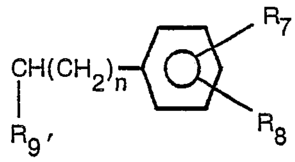

où $R_7$ et $R_8$ sont, indépendamment, H, Cl, $CH_3$ ou $OCH_3$;
n est 0 ou 1; et
$R_{9'}$ est H ou $OCH_3$;
quand Q est O, alors $R^I$ peut être $—CH_2CH_2OR_{15}$; $CH_2CH_2CH_2OR_{15}$; $CH—CH_2OR_{15}$;
$|$
$CH_3$

où $R_{15}$ est $C_2H_5$, $CH(CH_3)_2$, un radical phényle, $CH_2CH_2Cl$, $CH_2CCl_3$; $\leftarrow\!CH_2CH_2O\!\rightarrow_{n'}R_{16}$,
$\leftarrow\!CH—CH_2O\!\rightarrow_{n'}R_{16}$;
$|$
$CH_3$

où $R_{16}$ est $CH_3$, $C_2H_5$, $CH(CH_3)_2$, un radical phényle, $CH_2CH_2Cl$, $CH_2CCl_3$, et n' est 2 ou 3;

$CH_2CN$; 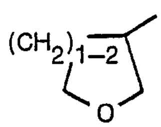 ; 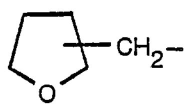 ; 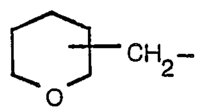 ; 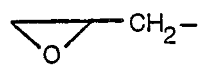 ; $CH_2OR'_6$ ;

où $R'_6$ est un radical alcoyle en $C_1$—$C_4$
avec la condition que $R^I$ a un total de $\leqslant 13$ atomes de carbone;
quand Q est —N—, alors
$|$
$R_6$

$R^I$ est H; un radical alcoyle en $C_1$—$C_6$; $—CH_2CH_2OR_{10}$; $—CH_2CH_2CH_2OR_{10}$; où $R_{10}$ est un radical $CH_3$, $CH_3CH_2$, $CH(CH_3)_2$ ou phényle; alcényle en $C_3$—$C_6$; cycloalcoyle en $C_3$—$C_6$; cycloalcényle en $C_5$—$C_6$; cycloalcoyle en $C_6$ substitué par 1—2 radicaux $OCH_3$, 1—3 radicaux $CH_3$, $—CH_2CH_3$ ou $CF_3$; cycloalcoylalcoyle en $C_4$—$C_7$; $—CH_2CN$; $—CH_2CH_2CN$;

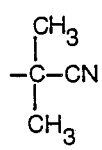 ; $OCH_3$ ; $OCH_2CH_3$ ; 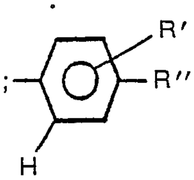 ou 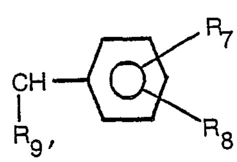 ;

où
R' est H, un radical alcoyle en $C_1$—$C_4$, $OCH_3$, F, Cl, Br, CN, $NO_2$ ou $CF_3$;
R'' est H, $CH_3$, Cl, F ou Br;
$R_7$, $R_8$ et $R_{9'}$ sont tels que définis précédemment;
$R_6$ est H, un radical alcoyle en $C_1$—$C_3$; $CH_2CN$; —CN ou $—CH_2—CH{=}CH_2$ et $R_6$ et $R^I$ peuvent être pris ensemble pour former $—(CH_2)_4$, $—(CH_2)_5—$ ou $—CH_2CH_2O—CH_2CH_2—$;

avec la condition que, quand $R_6$ est $CH_2CH_2CN$ ou $CH_2CN$, alors $R^I$ est $CH_2CH_2CN$ ou $CH_2CN$; et $R^I$ et $R_6$ ont un nombre total d'atomes de carbone $\leqslant 13$; et quand $R^I$ est $OCH_3$ ou $OCH_2CH_3$, alors $R_6$ est $CH_3$ ou H;

Quand A est 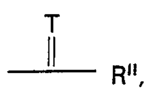 alors

$R^{II}$ est H, un radical alcoyle en $C_1$—$C_6$, alcényle en $C_3$—$C_6$; phényle; benzyle; benzyle ou phényle substitué par 1—2 Cl, 1—2 $OCH_3$, 1—2 $CH_3$; cycloalcoyle en $C_5$—$C_6$; cycloalcoylalcoyle en $C_4$—$C_7$

avec la condition que quand T est =N—OR''', alors R'' doit être un radical alcoyle en $C_1$—$C_6$ ou alcényle en $C_3$—$C_6$;

B est 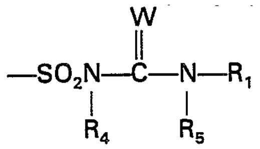

où $R_4$ est H ou $CH_3$; W est O ou S; $R_5$ est H, $CH_3$ ou $CH_3O$; avec la condition supplémentaire que $R_4$ ou $R_5$ doit être H;

$R_1$ est 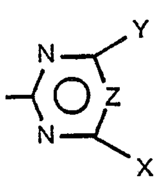 , 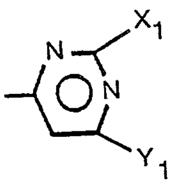 , 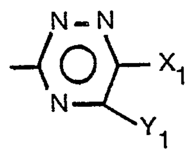 , 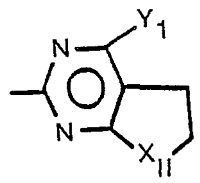 ou 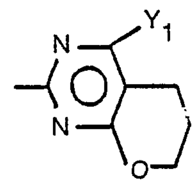 ;

où Z est N, CH ou C—F;
X = H, Cl, —$CH_3$, —$OCH_3$ ou —$OCH_2CH_3$;
Y = H; Cl; un radical alcoyle en $C_1$—$C_4$; alcoyle en $C_1$—$C_4$ substitué par —$OCH_3$, —$OC_2H_5$, —CN, —$CO_2CH_3$, —$CO_2C_2H_5$,

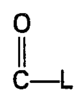

ou par 1—3 atomes de F, Cl, Br; alcényle en $C_3$—$C_4$; —O—$(CH_2)_{n'}$ O-(alcoyle en $C_1$—$C_3$) où n' est 2 ou 3;

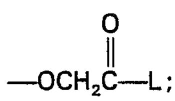 ; 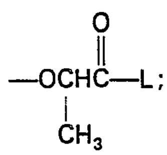 ; 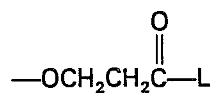

où L est OH, —$NH_2$, 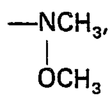

—NH(alcoyle en $C_1$—$C_4$), —N(alcoyle en $C_1$—$C_4$)$_2$ ou alcoxy en $C_1$—$C_6$; SCN; —$N_3$; $NR_{11}R_{12}$ où $R_{11}$ est H ou $CH_3$ et $R_{12}$ est H, —$OCH_3$, alcoyle en $C_1$—$C_4$, cycloalcoyle en $C_3$—$C_6$, alcényle en $C_3$—$C_4$, alcoyle en $C_2$—$C_3$ substitué par $OCH_3$ ou $OC_2H_5$, alcoyle en $C_1$—$C_2$ substitué par —CN, $CO_2H$, $CO_2CH_3$ ou $CO_2C_2H_5$, et $R_{11}$ et $R_{12}$ peuvent être pris ensemble pour former —$CH_2CH_2CH_2CH_2$— ou $CH_2CH_2OCH_2CH_2$—; —O—$R_9$ où $R_9$ est un radical alcoyle en $C_1$—$C_4$, alcoyle en $C_2$—$C_4$ substitué par 1—3 atomes de F, Cl, ou Br, alcoyle en $C_1$—$C_2$ substitué par un radical cyano, alcényle en $C_3$—$C_4$, —$CH_2C{\equiv}CR_{13}$,

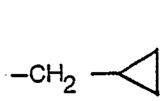 , 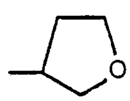 ou 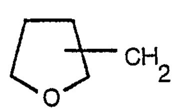 ;

$R_{13}$ est H, $CH_3$ ou $CH_2Cl$;
$SR_{14}$;
où
$R_{14}$ est un radical alcoyle en $C_1$—$C_4$, allyle, propargyle ou alcoyle en $C_1$—$C_2$ substitué par CN;
avec la condition que, quand X et Y sont deux H, alors R' et R'' ont moins de 5 atomes de carbone;
$X_1$ = H, Cl, $OCH_3$, $OCH_2CH_3$ ou $CH_3$;
$Y_1$ = H, $OCH_3$ ou $CH_3$; et
$X_{II}$ = O ou $CH_2$
et avec la condition en outre que, quand A contient plus de 5 atomes de carbone, alors Y doit contenir ≤ 4 atomes de carbone ou leurs sels acceptables en agriculture, procédé qui comprend:

(a) la réaction d'un isocyanate ou isothiocyanate approprié de formule

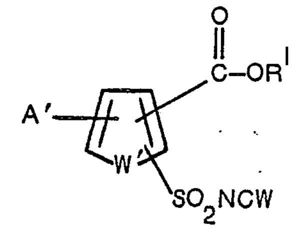

avec une amine de formule $NHR_1R_5$,
où W, W', $R_1$ et $R_5$ sont tels que définis ci-dessus,

A' est H, Cl, Br, un radical alcoyle en $C_1$—$C_4$, $OCH_3$, $NO_2$ ou $CF_3$;

$R^I$ est un radical alcoyle en $C_1$—$C_6$; alcényle en $C_3$—$C_6$; alcynyle en $C_3$—$C_6$; alcoyle en $C_2$—$C_6$ substitué par Cl, CN ou $OCH_3$; alcényle en $C_3$—$C_6$ substitué 1—3 Cl; alcynyle en $C_3$—$C_6$ substitué par Cl; cycloalcoyle en $C_5$—$C_6$; cyclohexényle; cyclohexyle substitué par 1—3 $CH_3$ cycloalcoylalcoyle en $C_4$—$C_7$ ou

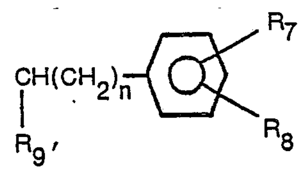

où $R_7$ et $R_8$ sont, indépentamment, H, Cl, $CH_3$ ou $OCH_3$; $C_2H_5$, $CH(CH_3)_2$, un radical phényle $CH_2CH_2Cl$, $CH_2CCl_3$; $(CH_2CH_2O)_{n'}R_{16}$, $(CH(CH_3)—CH_2O)_{n'}R_{16}$

où $R_{16}$ est $CH_3$, $C_2H_5$, $CH(CH_3)_2$, un radical phényle, $CH_2CH_2Cl$, $CH_2CCl_3$, et n' est 2 ou 3;

$CH_2CN$ ; 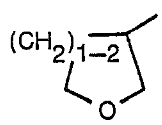 ; 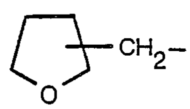 ; 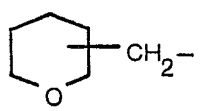 ; 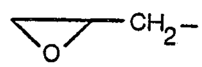 ; $CH_2OR'_6$ ;

où $R'_6$ est un radical alcoyle en $C_1$—$C_4$;
avec la condition que $R^I$ ait au total ⩽ 13 atomes de carbone;

(b) la réaction d'un sulfonamide approprié de formule

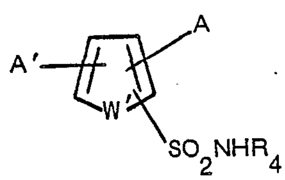

avec un isocyanate ou isothiocyanate de formule

$R_1NCW$

où A, A', W, W', $R_1$ et $R_4$ sont tels que définis ci-dessus pour préparer un composé de formule (I), (II) ou (III) dans lequel $R_5$ est H;

(c) la méthylation d'un composé de formule (I), (II) ou (III) dans lequel $R_4$ est H et W est O pour obtenir un composé de formule (I), (II) ou (III) dans lequel $R_4$ est un radical méthyle;

(d) la réaction d'un chlorure de carbamyle de formule

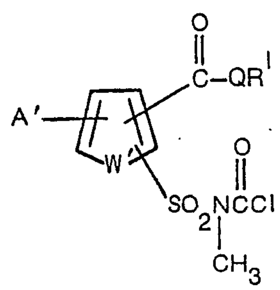

où A', W, W', Q et $R^I$ sont tels que définis ci-dessus, avec une amine de formule: $HNR_1R_5$ où $R_1$ et $R_5$ sont tels que définis ci-dessus;

(e) l'estérification d'un composé de départ correspondant dans lequel A est COOH pour obtenir un composé de formule (I), (II) ou (III) dans lequel A est $COOR^I$;

(f) la réaction d'un composé de formule (I), (II) ou (III) dans lequel A est $CO_2R^I$, où $R^I$ a 1—4 atomes de carbone, avec un composé de formule:

$$\begin{array}{c} R_6 \\ | \\ (alcoyl)_2Al\,N—R^I \end{array}$$

pour obtenir un composé de formule (I), (II) ou (III) dans lequel A est $\begin{array}{c} O \quad R_6 \\ \| \quad | \\ —C—N—R^I \end{array}$

où $R^I$ et $R_6$ sont tels que définis ci-dessus;

(g) la réaction d'un composé de formule (I), (II) ou (III) dans lequel $QR^I$ est O(alcoyle en $C_1$—$C_4$) avec un composé de formule

$$(R^IS)_2Al(alcoyle)$$

pour obtenir un composé correspondant de formule (I), (II) ou (III) dans lequel $QR^I$ est $SR^I$, $R^I$ étant tel que défini ci-dessus;

(h) la réaction d'un ester de formule (I), (II) ou (III) dans lequel $R^I$ est un groupe alcoyle primaire inférieur avec un composé de formule

$$(alcoyle)_2AlOR^I$$

où $R^I$ est un groupe alcoyle secondaire compris dans la définition de $R^I$ ci-dessus, pour remplacer le groupe alcoyle primaire inférieur par $R^I$;

(i) la réaction d'un composé de formule (I), (II) ou (III) dans lequel A est $CO_2H$ avec $R^{II}Li$ pour obtenir un composé de formule (I), (II) ou (III) dans lequel A est $COR^{II}$, $R^{II}$ étant tel que défini ci-dessus; aprés quoi, si on le désire, on fait réagir cette cétone avec une hydroxylamine de formule

$$H_2NOR^{III}$$

pour obtenir un composé de formule I, II ou III dans lequel A est $\begin{array}{c} NOR^{III} \\ \| \\ —C—R^{II} \end{array}$ ;

(j) l'hydrolyse d'un composé de formule I, II ou III dans lequel Y est $\begin{array}{c} O \\ \| \\ —Q'—C—L \end{array}$

où Q' est un radical alcoylène en $C_1$—$C_4$, —$OCH_2$—, —$OCH_2CH_2$—, $\begin{array}{c} —OCH— \\ | \\ CH_3 \end{array}$,

$\begin{array}{c} —N— \\ | \\ R_{11} \end{array}$ ou $\begin{array}{c} —NCH_2— \\ | \\ R_{11} \end{array}$

où $R_{11}$ est tel que défini ci-dessus et L est différent de OH, pour obtenir un composé de formule I, II ou III dans lequel L est OH, ou un sel de ce composé acceptable en agriculture; ou

(k) la réaction d'un composé de formule I, II ou III dans lequel A est $CO_2Me$ et A' est différent de $NO_2$ avec de l'hydrure de sodium et de bis-(2-méthoxyéthoxy) aluminium ou avec de l'hydrure de diisobutylaluminium pour obtenir un composé de formule I, II ou III dans lequel A est —CHO.

2. Un procédé selon la revendication 1, dans lequel au moins une des conditions suivantes est remplie:

(a) A' n'est pas $OCH_3$, $NO_2$ ou $CF_3$

(b) $R^I$ n'est pas $OCH_2CH_3$ ou un radical alcoyle en $C_2$—$C_6$ substitué par F ou Br

(c) $R^I$ n'est pas

$CH_2CN$ ; 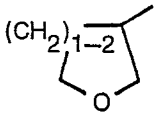 ; 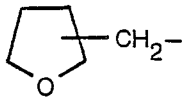 ; 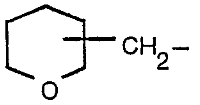 ; 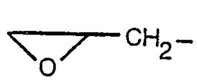 ou $CH_2OR'_6$ ;

(d) R′ n'est pas CN, $NO_2$ ou $CF_3$
(e) quand $R^I$ est $OCH_3$, $R_6$ n'est pas H
(f) $R^{II}$ n'est pas H

(g) $R_1$ n'est pas 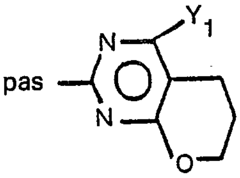

(h) $R_9$ n'est pas 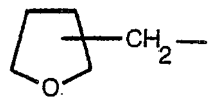

(i) Z n'est pas C—F.

3. Un procédé selon la revendication 1 ou 2, dans lequel

B est $-SO_2-NH-\overset{O}{\overset{\|}{C}}-NHR_1$

et T est l'oxygène; et dans lequel

$R_1$ est 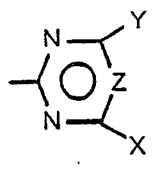, 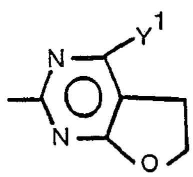 ou 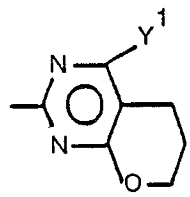

Q est O ou S et $R^I$ est un radical alcoyle en $C_1$—$C_4$, alcényle en $C_3$—$C_4$; alcynyle en $C_3$—$C_4$; alcoyle en $C_2$—$C_3$ substitué par CN, $OCH_3$ ou 1—3 F, Cl ou Br; alcényle en $C_3$—$C_4$ substitué par 1—3 Cl ou alcynyle en $C_3$—$C_4$ substitué par Cl; ou dans lequel Q est de l'oxygène et $R^I$ est $CH_2CH_2OR_{15}$; $CH_2CH_2CH_2OR_{15}$; $\underset{\underset{CH_2}{|}}{CH}CH_2OR_{15}$ où

$R_{15}$ est $CH_2CH_3$; $CH_2CN$; $CH_2OR'_6$ ou $R'_6$ est $CH_3$ ou $CH_3CH_2$; ou 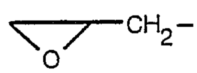 ;

4. Un procédé selon la revendication 1 ou 2, dans lequel Q est $-\underset{\underset{R_6}{|}}{N}-$;

$R^I$ est H, un radical alcoyle en $C_1$—$C_4$, $CH_2CH_2OR_{10}$, $CH_2CH_2CH_2OR_{10}$ où $R_{10}$ est $CH_3$ ou $CH_3CH_2$; un radical alcényle en $C_3$—$C_4$; $CH_2CN$; $CH_2CH_2CN$; $OCH_3$ ou $OCH_2CH_3$; $R^6$ est H, un radical alcoyle en $C_1$—$C_2$, $CH_2CN$, $CH_2CH_2CN$ et $R_6$ et $R^I$ peuvent être pris ensemble pour former $-(CH_2)_4-$.

5. Un procédé selon la revendication 3, dans lequel $R^{II}$ est H ou un radical alcoyle en $C_1$—$C_3$.

6. Un procédé selon l'une quelconque des revendications 3—5, dans lequel
Z est CH ou N;
X est $CH_3$ ou $CH_3O$; et
Y est un radical alcoyle en $C_1$—$C_2$; alcoyle en $C_1$—$C_2$ substitué par $OCH_3$; $OCH_2CH_3$, CN ou 1—3 atomes de F, Cl ou Br;

$OCH_2-\overset{O}{\overset{\|}{C}}-L$ ou $\underset{\underset{CH_3}{|}}{OCH}-\overset{O}{\overset{\|}{C}}-L$

où L est $NH_2$, OH, $\underset{\underset{OCH_3}{|}}{N(CH_3)}$,

$N(CH_3)_2$, $NHCH_3$, un radical alcoxy en $C_1$—$C_2$; SCN, $N_3$; $NR_{11}R_{12}$ où $R_{11}$ est H ou $CH_3$ et $R_{12}$ est H, $CH_3$,

$CH_3CH_2$, $OCH_3$; $OR_9$ où $R_9$ est $CH_3$, $CH_3CH_2$; $CH_2CH{=}CH_2$; $CH_2C{\equiv}CH$; ou un radical alcoyle en $C_2$substitué par 1—3 F, Cl ou Br; $CH_3S$.

7. Un procédé selon la revendication 2 ou 3, dans lequel A′ est H, Cl ou Br;

Q est O ou S et R' est un radical alcoyle en $C_1$—$C_4$, $CH_2CH{=}CH_2$ ou $CH_2CH_2Cl$; ou dans lequel Q est O et R' est $CH_2CH_2OCH_3$, 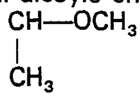

$CH_2OCH_3$ ou $CH_2OCH_2CH_3$; ou dans lequel Q est 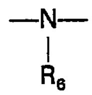

et R' est H; un radical alcoyle en $C_1$—$C_3$, $OCH_3$ ou $OCH_2CH_3$ et $R_6$ est H ou un radical alcoyle en $C_1$—$C_2$.

8. Un procédé selon la revendication 7, dans lequel A′ est H; Y est $CH_3$, $OCH_3$, $OCH_2CH_3$, $OCH_2CF_3$, $OCH_2CH{=}CH_2$ ou $OCH_2C{\equiv}CH$.

9. Un procédé selon la revendication 6, dans lequel A est 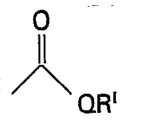

et Q est de l'oxygène ou du soufre et R' est $CH_3$ ou $CH_2CH_3$; Q est 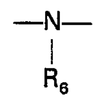

et R' est H, $CH_3$ ou $OCH_3$ et $R_6$ est $CH_3$;

$R_1$ est 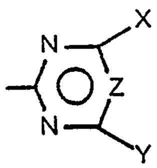

Y est $CH_3$ ou $OCH_3$ et W′ est du soufre.

10. Un procédé selon la revendication 1, dans lequel le produit est un composé, ou un sel acceptable en agriculture de ce composé, choisi parmi les suivants:

3-[[(4-méthoxy-6-méthylpyrimidin-2-yl)aminocarbonyl]-aminosulfonyl]-2-thiophènecarboxylate de méthyle

3-[[(4,6-diméthoxy-1,3,5-triazin-2-yl)aminocarbonyl]-aminosulfonyl]-2-thiophènecarboxylate de méthyle

3-[[(4,6-diméthyl-1,3,5-triazin-2-yl)aminocarbonyl]-aminosulfonyl]-2-thiophènecarboxylate de méthyle

3-[[(4-méthoxy-6-méthyl-1,3,5-triazin-2-yl)aminocarbonyl]-aminosulfonyl] -2-thiophènecarboxylate de méthyle.

11. Un procédé selon la revendication 1, dans lequel le produit est un composé, ou un sel acceptable en agriculture de ce composé, choisi parmi les suivants:

2-[[(4,6-diméthoxypyrimidin-2-yl)aminocarbonyl]-aminosulfonyl]-3-thiophènecarboxylate de méthyle

2-[[(4,6-diméthylpyrimidin-2-yl)aminocarbonyl]-aminosulfonyl]-3-thiophènecarboxylate de méthyle

2-[[(4-méthoxy-6-méthylpyrimidin-2-yl)aminocarbonyl]-aminosulfonyl]-3-thiophènecarboxylate de méthyle

2-[[(4,6-diméthoxy-1,3,5-triazin-2-yl)aminocarbonyl]-aminosulfonyl]-3-thiophènecarboxylate de méthyle

2-[[(4-méthoxy-6-méthyl-1,3,5-triazin-2-yl)aminocarbonyl]-aminosulfonyl] -3-thiophène-carboxylate de méthyle.

12. Un procédé selon la revendication 1, dans lequel le produit est un composé, ou un sel acceptable en agriculture de ce composé, choisi parmi les suivants:

4-[[(4,6-diméthoxypyrimidin-2-yl)aminocarbonyl]-aminosulfonyl]-3-thiophènecarboxylate de méthyle

4-[[(4,6-diméthylpyrimidin-2-yl)aminocarbonyl]-aminosulfonyl]-3-thiophènecarboxylate de méthyle

4-[[(4-méthoxy-6-méthylpyrimidin-2-yl)aminocarbonyl]-aminosulfonyl]-3-thiophènecarboxylate de méthyle

4-[[(4,6-diméthoxy-1,3,5-triazin-2-yl)aminocarbonyl]-aminosulfonyl]-3-thiophènecarboxylate de méthyle

4-[[(4-méthoxy-6-méthyl-1,3,5-triazin-2-yl)aminocarbonyl]-aminosulfonyl] -3-thiophènecarboxylate de méthyle.

13. Un procédé selon la revendication 1, dans lequel le produit est le N-[[(4 - méthoxy - 6 - méthylpyrimidin - 2 - yl)aminocarbonyl] - 2 - (1 - pyrrolidinylcarbonyl) - 3 - thiophènesulfonamide ou un sel acceptable en agriculture de ce composé.

14. Un procédé selon la revendication 1, dans lequel le produit est le 3 - [[(4,6 - diméthoxypyrimidin - 2 - yl)aminocarbonyl] - aminosulfonyl] - 2 - thiophènecarboxylate de 1-méthyléthyle ou un sel acceptable en agriculture de ce composé.

15. Un procédé pour lutter contre la croissance de végétation indésirable par application sur le lieu à protéger d'une quantité efficace d'un composé herbicide, caractérisé en ce que ce composé herbicide comprend un composé de formule (I), (II) ou (III) tel que défini dans l'une quelconque des revendications 1 à 14.

16. Un procédé pour régler la croissance de plantes par application au site de ces plantes d'une quantité efficace, mais substantiellement non-phytotoxique, d'un agent de réglage de la croissance de plantes, caractérisé en ce que l'agent de réglage de la croissance de plantes comprend un composé de formule (I), (II) ou (III) tel que défini dans l'une quelconque des revendications 1 à 14.

17. Un procédé de préparation d'un composé choisi parmi

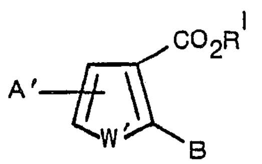
I

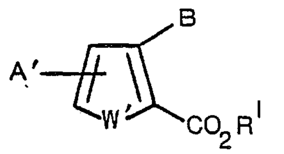
II

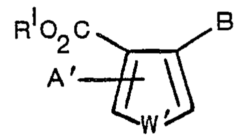
III

où $R^I$ est H ou M;

M est un cation d'un métal alcalin ou d'une amine tertiaire ayant jusqu'à 12 atomes de carbone;

W' est O ou S;

A' est H, Cl, Br, un radical alcoyle en $C_1$—$C_4$, $OCH_3$, $NO_2$ ou $CF_3$;

B est 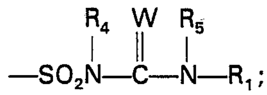

où $R_4$ est H ou $CH_3$; W est O ou S; $R_5$ est H, $CH_3$ ou $CH_3O$; avec la condition que $R_4$ ou $R_5$ doit être H;

$R_1$ est 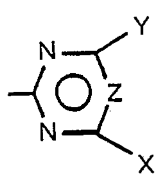 , 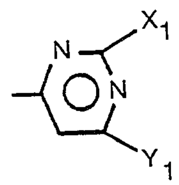 , 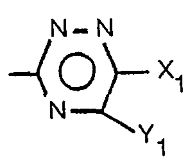 , 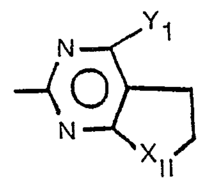 ou 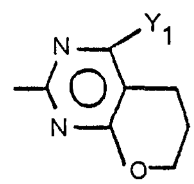 ;

où Z est N, CH ou C—F;

X = H, Cl, —$CH_3$, —$OCH_3$ ou —$OCH_2CH_3$;

Y = H; Cl; un radical alcoyle en $C_1$—$C_4$; un radical alcoyle en $C_1$—$C_4$ substitué par —$OCH_3$, —$OC_2H_5$, —CN, —$CO_2CH_3$, —$CO_2C_2H_5$,

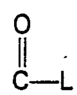

ou par 1—3 atomes de F, Cl, Br; alcényle en $C_3$—$C_4$; —O—$(CH_2)_{n'}$ O-(alcoyle en $C_1$—$C_3$) où n' est 2 ou 3;

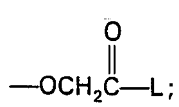 ; 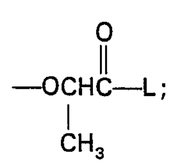 ; 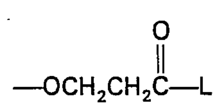

où L est OH, —$NH_2$, 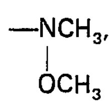

—NH(alcoyle en $C_1$—$C_4$), —N(alcoyle en $C_1$—$C_4$)$_2$ ou alcoxy en $C_1$—$C_6$; SCN; —$N_3$; $NR_{11}R_{12}$ où $R_{11}$ est H ou $CH_3$ et $R_{12}$ est H, —$OCH_3$, alcoyle en $C_1$—$C_4$, cycloalcoyle en $C_3$—$C_6$, alcényle en $C_3$—$C_4$, alcoyle en $C_2$—$C_3$ substitué par $OCH_3$ ou $OC_2H_5$, alcoyle en $C_1$—$C_2$ substitué par —CN, $CO_2H$, $CO_2CH_3$ ou $CO_2C_2H_5$, et $R_{11}$ et $R_{12}$ peuvent être pris ensemble pour former —$CH_2CH_2CH_2CH_2$— ou $CH_2CH_2OCH_2CH_2$—; —O—$R_9$ où $R_9$ est un radical alcoyle en $C_1$—$C_4$, alcoyle en $C_2$—$C_4$ substitué par 1—3 atomes de F, Cl, ou Br, alcoyle en $C_1$—$C_2$ substitué par un radical cyano, alcényle en $C_3$—$C_4$, —$CH_2C{\equiv}CR_{13}$,

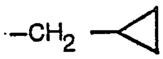 , 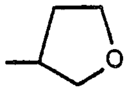 ou 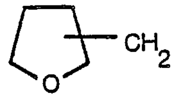 ;

$R_{13}$ est H, $CH_3$ ou $CH_2Cl$;

$SR_{14}$, où

$R_{14}$ est un radical alcoyle en $C_1$—$C_4$, allyle, propargyle ou alcoyle en $C_1$—$C_2$ substitué par CN;

$X_1$ = H, Cl, $OCH_3$, $OCH_2CH_3$ ou $CH_3$;

$Y_1$ = H, $OCH_3$ ou $CH_3$; et

$X_{II}$ = O ou $CH_2$; qui comprend l'hydrolyse d'un composé de départ correspondant dans lequel $R^I$ est différent de H, dans des conditions basiques, et, si on le désire, une acidification pour obtenir un produit dans lequel $R^I$ est H.